(19) Europäisches Patentamt
European Patent Office
Office européen des brevets

(11) EP 1 541 696 A1

(12) **EUROPEAN PATENT APPLICATION**

(43) Date of publication:
**15.06.2005 Bulletin 2005/24**

(51) Int Cl.[7]: **C12Q 1/68**

(21) Application number: **03293079.4**

(22) Date of filing: **09.12.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **INSTITUT PASTEUR**
**75015 Paris (FR)**

(72) Inventors:
- **El Sohl, Névine**
**94300 Vincennes (FR)**
- **Trad, Salim**
**75007 Paris (FR)**
- **Frangeul, Lionel**
**75014 Paris (FR)**
- **Allignet, Jeanine**
**92000 Nanterre (FR)**
- **Davi, Marilyne**
**92320 Chatillon (FR)**
- **Glaser, Philippe**
**75013 Paris (FR)**
- **Buchrieser, Carmen**
**75013 Paris q (FR)**

(74) Representative: **Ahner, Francis et al**
**Cabinet Régimbeau**
**20, rue de Chazelles**
**75847 Paris cedex 17 (FR)**

(54) **A DNA macro-array for staphylococcus aureus identification and typing**

(57) The present invention relates to a method for identification at the species level of a *Staphylococcus aureus* bacterial strain or to a method for typing it by using an extract of its labelled genomic DNA, comprising the use of a solid support coated with a set of amplicons, each amplicon being representative of a gene relevant for the characterization of a *Staphylococcus aureus* bacterial strain. Also comprised herein are solid supports and DNA macro-arrays coated with the set of amplicons, and kits comprising such solid supports and DNA macro-arrays.



Printed by Jouve, 75001 PARIS (FR)

## Description

The best known staphylococcal species is *Staphylococcus aureus,* by virtue of its frequent and highly versatile pathogenicity in human and animals. The isolates belonging to this species are responsible for suppurative infections and syndromes provoked by toxins.

If toxins are excluded such as enterotoxins and exfoliative or toxic shock syndrome toxins (McCormick and al. 2001. Annual Review of Microbiology 55:77-104), the pathology of a staphylococcal infection is attributable not to a single factor but to the coordinate actions of several different factors whose expression is controled by several regulatory systems (Becker and al. 2003. J Clin Microbiol 41:1434-9; Novick, R. P. 2003. Molecular Microbiology 48: 1429-1449; Peacock and al. 2002. Infect Immun 70:4987-96; Projan, S.J. and R.P. Novick. 1997. The Molecular Basis of Pathogenicity, p. 55-81. *In* K. B. Crossley and G. L. Archer (ed.), Churchill Livingstone).

*S. aureus* is one of the most common causes of nosocomial infections. The emergence of such infections is of particular concern since most isolates are resistant to multiple antibiotics (Berger-Bächi, B. 1997, p. 158-174. *In* K. B. Crossley and G. L. Archer (ed.), Churchill Livingstone ; Paulsen and al. 1997, p. 175-212. *In* K. B. Crossley and G. L. Archer (ed.), Churchill Livingstone) such as methicillin-resistant *S. aureus* (MRSA), and because the spread of these strains in hospitals often increases the overall incidence of nosocomial *S. aureus* infections in the institution. MRSA clinical isolates with decreased susceptibility to glycopeptides (2002. First U.S. case of Vancomycin-Resistant *Staphylococcus aureus* infection reported; patient has chronic renal failure. Dialysis and transplantation:602-603; Hiramatsu, K. 2001. Lancet Infect Dis 1:147-55) threaten to compromise the ability to treat hospital-acquired *S. aureus* infections.

*S. aureus* typing is a useful adjunct in several different clinical settings in addition to dramatic acute outbreaks. Despite the use of several phenotypic and genotypic methods (antibiotyping, phage typing, multilocus enzyme electrophoresis, restriction analysis of cellular DNA, analysis of PCR products or multilocus sequencing typing) (Enright and al. 2000. J Clin Microbiol 38:977-86; Grundmann and al. 2002. J Clin Microbiol 40:4544-6; Morvan and al. 1997. Journal of Clinical Microbiology 35:1415-1423; Musser, J. M., and R. K. Selander. 1990. Genetic analysis of natural populations of *Staphylococcus aureus.,* p. 59-67. *In* R. P. Novick (ed.), Molecular Biology of the Staphylococci. VCH Publishers, New York; Sabat and al. 2003. J Clin Microbiol 41:1801-4; Tenover and al. 1995. Journal of Clinical Microbiology 33:2233-2239; van Leeuwen and al. 2003. J Clin Microbiol 41:3323-6; van Leeuwen and al. 1999. J Clin Microbiol 37:664-74), indistinguishable or closely related isolates were detected, not only among those responsible for outbreaks, but also among those isolated in different countries, at time intervals of several years and without any obvious epidemiological links. Indeed, Oliveira *and al.* ( 2001, Microb Drug Resist 7:349-61) identified five major pandemic MRSA clones that accounted for almost 70% of the 3000 isolates analysed.

The whole genome sequencing of seven *S. aureus* strains (N315 ( Kuroda *and al.,* 2001, Lancet 357:1225-40), Mu50 (Kuroda *and al.,* 2001), COL (The Institute for Genomic Research. TIGR databases), MW2, NCTC8325 ( Oklahoma University. Staphylococcus aureus NCTC8325 Genome Sequencing), MSSA-strain 476 (Sanger Centre. Staphylococcus aureus) and EMRSA 16-strain 252 ( Sanger Centre. Staphylococcus aureus)) revealed the presence of large amounts of well conserved DNA regions in the chromosomes. Fitzgerald *and al.* ( Proc Natl Acad Sci U S A 98:8821-6) demonstrated that 2,198 (78%) of the 2,817 COL chromosomal orfs represented on the DNA micro-array were shared by the 36 analysed *S. aureus* isolates from various sources, which belonged to 14 multilocus enzyme electrophoretic types. Ten of the eighteen large regions of difference have genes that encode putative virulence factors and proteins that mediate antibiotic resistance.

Despite the use of several methods of typing based on genome analysis, *S. aureus* bacterial strains isolated in various countries at and with time intervals from time which can exceed 10 years, are sometimes indistinguishable. Moreover, the typing of the strains is an essential complement for the epidemiologic investigations, in order to check wether spread of the strains results from a recent epidemy or from the persistence of an endemy.

So, there is a need to design a DNA macro-array to identify at species level *S. aureus* isolates and to type unknown bacteria as *S. aureus* bacteria.

The present invention resolves this technical problem, which overcomes all the drawbacks of the prior art previously mentioned.

One first aspect of the present invention is a method for identification at species level of *Staphylococcus aureus* bacteria from an extract of bacterial genomic DNA, characterized in that it comprises:

- labeling the genomic DNA after its extraction from the bacterial strain, and
- putting said labeled genomic DNA into contact with a solid support coated with a set of amplicons in suitable conditions for hybridization, each amplicon being able to specifically hybridize with a relevant gene or with a representative fragment thereof, susceptible to be present in said genomic DNA to be tested, wherein said relevant genes whose presence or absence is to be determined using the set of amplicons comprise the following genes:

a) at least one gene considered as a positive control whose presence in a genome is characteristic for the *Staphylococcus aureus* species, and

b) at least one gene considered as a negative control, said gene being absent in the genome of the *Staphylococcus aureus* species, and

c) genes selected from the group A, said group A consisting in the following categories of genes :

(i) genes encoding staphylococcal and enterococcal proteins mediating drug resistances,

(ii) genes encoding factors known to be involved in *Staphylococcus aureus* pathogenicity and genes encoding structurally related proteins,

(iii)genes encoding proteins produced by mobile elements,

(iv)genes encoding factors involved in the regulation of pathological proteins,

(v) genes encoding proteins involved in the expression of antibiotic resistance or in the transfer of antibiotics.

so as to obtain an hybridization profile containing detectable signals characteristic for the presence or the absence of the relevant genes in said genomic DNA to be tested, and

- identifying the *Staphylococcus aureus* bacterial strain relative to the characterization of the presence or absence of the relevant genes in its genomic DNA.

Another aspect of the present invention is a method for typing a bacterial strain from an extract of its genomic DNA, characterized in that it comprises:

- identifying a least one *Staphylococcus aureus* bacterial strain according to claim 1, so as to obtain at least one hybridization profile corresponding to said *Staphylococcus aureus* bacterial strain,
- labeling the genomic DNA of the bacterial strain to be tested after its extraction and obtaining its hybridization profile using the solid support of claim 1,
- comparing the obtained hybridization profile of the bacterial strain to be tested with the hybridization profile of the *Staphylococcus aureus* bacterial strain previously identified, and
- typing the bacterial strain to be tested as a *Staphylococcus aureus* bacterial strain when the hybridization profile of the bacterial strain to be tested is close up to the hybridization profile of said *Staphylococcus aureus* bacterial strain previously identified.

The present invention has two applications, which are complementary, but distinct: firstly, the identification of the *Staphylococcus aureus* bacterial strains (isolates), and secondly the typing of the unknown and/or new bacterial strains. The typing application is useful in epidemiology.

The inventors of the present invention have selected, from all the genes of *S. aureus,* relevant genes, and consequently have selected a set of amplicons which are necessary to hybridize with said relevant genes. These experiments also permitted to calibrate the solid support on which the amplicons are coated, so as to exploit the intensity values obtained after hybridization of said amplicons with the genomic DNA extracted from the unknown and/or new bacterial strains to be tested.

The selection of the genes of interest has been performed by analyzing the hybridization intensity signals obtained with the genomic DNA extracted from strains whose typing was pre-established by other typing techniques, such as using the macrorestriction profile of the cellular DNA by digestion with enzymes, such as the restriction macroenzyme SmaI.

The typing, that is to say the closing up of an *a priori* unknown strain with other known strains, can then be performed by comparison of the genetic profile of said unknown bacterial strain with the genetic profiles of known bacterial strains. The hybridization profile of the bacterial strain to be tested is compared with the hybridization profile of the known *S. aureus* bacterial strain, as described herein. Then the bacterial strain to be tested is typed as a known *S. aureus* bacterial strain when its hybridization profile is close up to the hybridization profile of the known *Staphylococcus aureus* bacterial strain.

Preferably, the method for typing of the invention is characterized in that a plurality of *Staphylococcus aureus* bacterial strains is identified, so as to obtain a plurality of hybridization profiles corresponding to said *S. aureus* bacterial strains, such as to type the bacterial strain to be tested as one of the plurality of *S. aureus* bacterial strains when its hybridization profile is close up to the one of the plurality of hybridization profiles, which corresponds to the one of the *S. aureus* bacterial strains.

This method allows also the comparison of several known strains of *S. aureus* between them.

The term plurality is used herein to refer to a number of identified and typed *S. aureus* bacterial strains and corresponding hybridization porfiles sufficient to type the bacterial strain to be tested as one of the identified *S. aureus* bacterial strains. It may be for example at least 1, 2, 5, 10, 20, 30, 40, 50, 60 or 70 identified *S. aureus* bacterial strains. Preferably, there is at least 80 identified *S. aureus* bacterial strains, such as those characterized in the Table V. The more *S. aureus* bacterial strains are identified according to the invention, the more the treshlod used to cluster the strains is correctly selected.

Preferably, a phylogenetic tree is designed on the basis of a plurality of the hybridization profiles obtained for each of the known *Staphylococcus aureus* bacterial strains, by clustering these hybridization profiles on the basis of the presence/absence of the relevant genes having been tested. Then this *Staphylococcus aureus* phylogenetic tree can be used for typing a bacterial strain according to the present invention, by comparing the hybridization profile of the bacterial strain to be tested obtained using the solid support with the hybridization profiles of the strains used for the design of the phylogenetic tree.

Preferably, for designing the phylogenetic tree, the gene content from different categories of *Staphylococcus aureus* bacterial strains is comparatively analysed using the following test:

When categories of *n* and *m* isolates are compared, the probability that a given gene is present by chance in $n_1$ isolates of the first category of isolates and $n_2$ of the second, is given by the binomial formula :

$$p=\binom{n}{n1}\binom{m}{n2}q^{n1+n2}(1-q)^{n+m-n1-n2}$$

- p is estimated by maximum likelihood as $q = (n_1 + n_2) / (n + m)$.
- $p_g$=p g is the normalized probability, "g" representing the total number of genes investigated.
  The gene ditribution is considered as significant if the normalized probability $p_g < 0.10$.

This test can also be employed for typing a bacterial strain according to the present invention.

The term amplicon is used in the present invention to design any nucleotide sequence which is able to specifically hybridize with a relevant gene or with a representative fragment thereof, susceptible to be present in the genomic DNA to be tested, said genomic DNA being deposited on each of the amplicons coated on said support in suitable conditions for hybridization, so as to obtain, for each amplicon, a signal characteristic of the presence or the absence of said gene in said genomic DNA to be tested.

Such amplicons may be amplification products obtained from a genomic DNA of a bacterial strain containing said genes with a couple of primers. A nucleotide sequence amplification technique currently used by the man skilled in the art is the polymerase chain reaction (PCR).

The molecular techniques employed for carrying out the present invention are well known by the person skilled in the art, and are described in the literature (see for example Sambrook, Russell and al, *"Molecular cloning: A laboratory manual ",* Vol.3. January 15, 2001, Cold Spring Harbor Laboratory ; « *Nucleic acid hybridization* », BD Haines, SJ Higgins (Eds), 1985, IRL Press).

The expression "labeling the genomic DNA" presently refers to a step for the obtention of a collection of labeled DNA fragments representative of a whole given genomic DNA after its extraction. It may consist for example in any method of DNA fragmentation complemented with a labeling step, which are well known by the skilled person.

The expression "genomic DNA" refers in the present invention to the whole genetic content of a bacterial strain, in particular the nuclear DNA and plasmid DNA.

The labeling step allows the emission of a signal which is directly or indirectly detectable, and which is characteristic of the presence or the absence of a relevant gene, or a representative fragment thereof, said signal being obtained when the labeled genomic DNA is put into contact with the solid support coated with the set of amplicons in suitable conditions for hybridization, said gene being susceptible to be present in said genomic DNA.

It may consist, for example, in radio-labeling (such as $^{14}C$, $^{36}Cl$, $^{57}Co$, $^{58}Co$, $^{51}Cr$, $^{152}Eu$, $^{59}Fe$, $^{3}H$, $^{125}I$, $^{131}I$, $^{32}P$, $^{35}S$, $^{75}SE$ and $^{99m}Tc$ which may be detected using for example gamma-ray counter or scintillation counter, autoradiography...), enzymatic-labeling (biotin, digoxigenin,...), chimioluminescent-labeling (luminol, dioxetane, luciferase, luciferin) or fluorescent-labeling.

Such methods of nucleotide sequence labeling are well known by the person skilled in the art (see for example "Pratique de l'utilisation des sondes nucleiques et de l'hybridation moleculaire", Gerard Lacotte, May 1992, Ed. Lavoisier, Paris), are well described in the literature, and kits and markers for nucleotide labeling may be commercially available (for example, Random Priming DNA Labeling Kit (Roche, Diagnostics GmbH, Penzberg, Germany), and 50 μCi 5'-(α-$^{33}$P) triphosphate dCTP (Amersham, Piscataway, NJ, US ).

Examples of fluorescent-labeling can be, without any limitation, fluorescein and its derivates, such as fluorescein isothiocyanate (FITC), allophycocyanin (APC), phycoerythrin-cyanin 5 (PC5) and phycoerythrine (PE), calcein (AM), red fluorescent tetramethyl-rhodamin or rhodamin and its derivates, GFP (Green Fluorescent Protein), dansyl, umbelliferone etc..

The solid support used for carrying out the present invention may be in general any support which can be coated with nucleotide sequences, in particular with amplicons of the present invention, and which can support hybridization of those nucleotide sequences with complementary sequences. It may be for example, but without any limitation, membranes such as high density nylon membranes (for example Performa, Genetix, New Milton, UK), or glass supports. Such solid supports are well known by the man of the art, who will know which suitable solid supports to employ relative to the test which will be made.

One example of an array preparation using such solid supports is described in Example 1: "Array construction".

By the expression "representative fragment" of a relevant gene in the present invention it is meant any fragment of a relevant gene which is sufficiently large to specifically hybridize, under high stringency conditions, with one amplicon of the set of amplicons coated on the solid support and which has a percentage of G (guanin) and C (cystein) nucleotides (% GC) identical at least at 80 % with the GC percentage of the relevant gene, so as to obtain a significant signal of the presence or the absence of said relevant gene according to the invention.

The expression "suitable conditions for hybridization" in the invention refers to high stringency conditions which allow hybridization when complementary sequences having at least 80, 82, 85, 87, 89, 90, 92, 95, 97 or 99 % of identity are able to specifically hybridize. Conditions for hybridization are sufficiently described in the literature. An example, which doesn't limit the scope of the present invention, of such suitable conditions is described in Example 1: "Hybridization".

By percentage of identity (the terms identity and similarity are used in the same way in the present invention) between two nucleic acid sequences in the present invention, it is meant a percentage of identical nucleotides between the two compared sequences, obtained after the best alignment ; the differences between the two sequences can, eventually, being distributed randomly throughout their length, but not necessarily. The best alignment or optimal alignment is the alignment corresponding to the highest percentage of identity between the two sequences compared, which is calculated such as herein after. The sequence comparisons between two nucleic acid sequences are usually performed by comparing these sequences after their optimal alignment, said comparison being performed for one segment or for one "comparison window", to identify and compare local regions of sequence similarity. The optimal alignment of sequences for the comparison can be performed manually or by means of the algorithm of local homology of Smith and Waterman (1981) (Ad. App. Math. 2:482), by means of the algorithm of local homology of Neddleman and Wunsch (1970) (J. Mol. Biol. 48:443), by means of the similarity research method of Pearson and Lipman (1988) (Proc. Natl. Acad. Sci. USA 85:2444), by means of computer softwares using these algorithms (GAP, BESTFIT, FASTA and TFASTA in the Wisconsin Genetics Software Package, Genetics Computer Group, 575 Science Dr., Madison, WI).

The percentage of identity between two nucleic acid sequences is determined by comparing these two aligned sequences in an optimal manner with a "comparison window" in which the region of the nucleic acid sequence to compare may comprise additions or deletions with regard to the sequence of reference for an optimal alignment between these two sequences. The percentage of identity is calculated by determining the number of positions for which the nucleotide is identical between the two sequences, by dividing this number of identical positions by the total number of positions of the shorter sequence in the "comparison window" and by multiplying the result obtained by 100, to obtain the percentage of identity between these two sequences.

Preferably, the method for identification or typing of the present invention is characterized in that the specificity of the set of amplicons coated on the solid support is checked by randomly sequencing some of the amplicons of the set. Sequencing methods are well described in the previously mentioned literature and also in Example 1: "Verification of the specificity of the DNA macro-array".

When a gene corresponding to an amplicon is present in the genomic DNA to be tested, it will specifically hybridize with the corresponding amplicon, and a characteristic signal will be emitted.

Preferably, after the specific hybridization step and after reading of the significant signal obtained for one amplicon, the value 0 is attributed when this signal is significant of the absence of the gene, or the value 1 is attributed when this signal is significant of the presence of the gene, said values being attributed in the following manner, after determination of the following ratio R:

$$R = \frac{\text{Normalized Hybridization Intensity Value (NHIV)}}{\text{Normalized Hybridization Intensity Value of Reference (NHIVR)}},$$

- 0, when the ratio R is strictly inferior to 0.3,

- 1, when the ratio R is equal or superior to 0.3;

where NHIV is the ratio of the hybridization intensity value of the signal obtained for one amplicon to the average of all hybridization intensity values of the significant signals obtained on the solid support, and
where NHIVR is obtained by combining the average of NHIV values obtained for each of the amplicons after hybridization in suitable conditions of the amplified genomic DNA of the previously identified *S. aureus* bacterial strains with the set of amplicons coated on the solid support.

Preferably, the method for identification or typing according to the present invention is
characterized in that the solid support coated with a set of amplicons comprises two deposits of each amplicon corresponding to a relevant gene.

In order to compare the various signals obtained, the inventors have developed a calibration of the solid support, by normalizing the signals obtained after hybridization of the amplicons coated on the solid support with the labeled genomic DNA. The normalization step allows becoming free from the differences caused by the background noise, the duration of hybridization exposure, the more or less intensive labeling of the genomic DNA to be tested, the variable quantities of labeled genomic DNA which is put into contact with the solid support.

Preferably, this calibration allows concluding about the absence or the presence of the relevant genes in the tested genomic DNA.

The normalization step comprises two steps:

The first step allows the determination of the threshold value of the signal intensity which discriminates between the significant signals and the background noise signals. This threshold value is objectively established for each test on the basis of the curve representing the intensity values of the signals obtained for each of the amplicons (see Figure 3). This threshold value corresponds to the signal hybridization value observed when there is a change in the slope of the curve (see Figure 3).

In the second step, the NHIV is obtained for each significant signal value which is located over the signal hybridization value of the change in the slope of the curve.

When the genes were known to be present in the tested strains used as substrates such as the five strains whose genomes have been sequenced : N315, Mu50, COL, MW2 and NCTC8325, the ratios between the normalized signal intensity of the gene hybridized with the tested strain and that of the reference array was always higher than 0.3. Thus, the threshold for the presence of a gene or variants related by at least 80 % similarity was defined as > 0.3. The data were then converted into binary score : at ≥ 0.3, a gene was scored as present (score=1) and at < 0.3 a gene was scored as absent (score=0). The binary data were used to cluster the isolates hierarchically, using the program J-Express. The threshold adopted to distribute the isolates into clusters was that which enabled each of the outbreak-related isolates belonging to SmaI genotypes 100 or 101 (Table 1) to be grouped and distinguished from any of the other isolates.

In other words, this normalization step comprises:

1. classifying the hybridization signals obtained in an ascending order,
2. plotting a curve of the hybridization intensities relative to the number of amplicons.
3. measuring the change in the slope of the curve,
4. determining the signal hybridization value observed when this change in the slope of the curve occurs,
5. eliminating all the signals whose hybridization intensity is inferior to this signal hybridization value,
6. calculating the average of the intensity values retained, herein considered as hybridization intensity values of the significant signals,
7. obtaining the NHIV by dividing the intensity value obtained for each amplicon corresponding spot to the average previously calculated.

The NHIVR value is obtained beforehand, by applying the above strategy for calculating the NHIV value, but from the signals obtained after hybridization of the amplicons coated on the solid support with the genomic DNA extracted from identified *S. aureus* bacterial strains. Preferably, these identified *S. aureus* bacterial strains are the strains Mu50, N315, NCTC8325, COL and MW2.

The 0.3 value has been established on the basis the NHIVR values calculated for each of the amplicons coated on the solid support: the value 1 is attributed when the ratio R is equal or superior to 0.3, and the value 0 is attributed when the ratio R is strictly inferior to 0.3.

Alternately, the present invention also comprises a method for analysis of the levels of gene expression in the isolates used as probe, in particular with amplicons allowing the detection of mRNA isolated from each bacterial strain. This approach allows the establishment of the transcriptoma of each bacterial strain.

Preferably, the method for identification or typing according to the present invention is characterized in that at least one of the two genes *nuc SA* and *sodM* as defined in Table I, preferably the two genes, is selected as positive control for the *Staphylococcus aureus* species.

More preferably, the method for identification or typing according to the present invention is characterized in that the gene *nuc SI* as defined in Table I is selected as a negative control for the *Staphylococcus aureus* species.

The amplicons which may be coated on the solid support of the present invention other than the control genes are the amplicons necessary for specific hybridization with genes of the group A whose presence has been characterized in at least 30 to 60 % of the *Staphylococcus aureus* strains presently studied.

These *Staphylococcus aureus* strains presently studied comprise the strains which are characterized in Table 5 and the following strains : N315, Mu50, COL, MW2, NCTC8325 and the 80 strains of this study.

Accordingly, the amplicons which may be coated on the solid support of the present invention are the amplicons necessary for specific hybridization with genes whose presence has been characterized in at least 30 % of the *Staphylococcus aureus* strains (isolates) presently studied. The genes corresponding to these amplicons are listed in Table T30.

So, in a prefered embodiment, the method for identification or typing according to the present invention is characterized in that the genes selected from the group A are all the genes of the Table T30.

Preferably, the amplicons which may be coated on the solid support of the present invention are the amplicons necessary for specific hybridization with genes whose presence has been characterized in at least 60 % of the *Staphylococcus aureus* strains (isolates) presently studied. The genes corresponding to these amplicons are listed in Table T60.

Accordingly, the method for identification or typing according to the present invention is characterized in that the genes selected from the group A are all the genes of the Table T60.

In one another embodiment, the method for identification or typing according to the present invention is characterized in that the genes selected from the group A comprise all the genes of the Table II.

The genes of the table II correspond to the genes which are not present in the genome of all of the seven strains N315, Mu50, COL, MW2, NCTC8325, MSSA-strain and EMRSA 16-strain (p=0/7).

Preferably, the method for identification or typing according to the present invention is characterized in that the genes selected from the group A comprise all the genes of the Table III.

The genes of the table III correspond to the genes which are present in the genome of all of the seven strains N315, Mu50, COL, MW2, NCTC8325, MSSA-strain and EMRSA 16-strain (p=7/7).

Furthermore, the method for identification or typing according to the present invention is characterized in that the genes selected from the group A comprise putative genes selected from at least n *Staphylococcus aureus* strains whose genomes have been sequenced or may be sequenced, in the following manner, with n = 3, 4, 5, 6 or = 7 :

a) the sequences of the putative genes of the *Staphylococcus aureus* strains genomes are identified, and the sequences identities of the putative genes compared with each other are determined, and

b) the putative genes which have the following characteristics are selected:

- putative genes which are considered as distinct from other putative genes previously selected, and
- putative genes which are not present in all of the n *Staphylococcus aureus* strains whose genomes have been sequenced or may be sequenced, and
- putative genes which encode putative proteins having at least 150 amino-acids.

In the present invention, the putative gene sequences are considered as homologuous when they comprise a sub-sequence of at least 30 contiguous nucleotides having at least 80 % of identity with each other. On the contrary, the putative genes will be considered as distinct when their sequences don't encompass these conditions.

One example of a method used for annotation and comparative analysis of the available genome sequences is the program CAAT-Box (Frangeul *and al,* 2003. CAAT-Box, Contigs-Assembly and Annotation tool-box for genome sequencing projects. Bioinformatics *in press*). But it can be any other method which allows to select putative genes in a genome.

By the expression "putative genes which are not present in all of the n *Staphylococcus aureus* strains whose genomes have been sequenced or may be sequenced" it is meant that the putative genes are present in 1, .... to (n-1) of the n *Staphylococcus aureus* strains whose genomes have been sequenced or may be sequenced.

Preferably, the method for identification or typing according to the present invention is characterized in that n=7, the *Staphylococcus aureus* strains used for the selection of the putative genes being the strains N315, Mu50, COL, MW2, NCTC8325, MSSA-strain and EMRSA 16-strain 252.

More preferably, the method for identification or typing according to the present invention is characterized in that the putative genes are all the genes of the Table IV.

The genes of the table IV correspond to the genes which are present in the genome of one to six of the seven strains N315, Mu50, COL, MW2, NCTC8325, MSSA-strain and EMRSA 16-strain (p=1 to 6/7).

In a most prefered embodiment, the method for identification or typing according to the present invention is characterized in that the relevant genes whose presence or absence is to be determined using the set of amplicons comprise the following genes:

- all the control genes of the Table I ;
- all the genes of the Table II ;
- all the genes of the Table III ;
- all the putative genes of the Table IV.

One further prefered embodiment is the method for identification or typing according to the present invention, characterized in that each amplicon of the set of amplicons is an amplification product of a relevant gene, said amplification product being obtained from the genomic DNA of a bacterial strain containing said gene with a couple of primers specific for the gene.

Preferably, the method for identification or typing according to the present invention is characterized in that the couple of primers used for obtention of each amplicon of the set of amplicons is selected in a manner such that the amplicons have a size of 400 to 500 base pairs.

More preferably, the method for identification or typing according to the present invention is characterized in that when at least one of the genes *nuc SA* and *sodM* as defined in the Table I, preferably the two genes, is selected as positive control, the couple of primers specific for the gene *nuc SA* is SEQ ID No. 1 and SEQ ID No. 2, and the couple of primers specific for the gene *sodM* is SEQ ID No. 3 and SEQ ID No. 4.

Even more preferably, the method for identification or typing according to the present invention is characterized in that when the gene *nuc SI* as defined in the Table I is selected as negative control, the couple of primers specific for this gene is SEQ ID No. 5 and SEQ ID No. 6.

One further prefered embodiment is the method for identification or typing according to the present invention, characterized in that the couples of primers specific for the genes of the Tables II, III and IV are the couples of primers corresponding to these genes as defined in column "couple of primers" of these Tables.

One another embodiment is the method for identification or typing according to the present invention, characterized in that the bacterial strains whose genomic DNA containing said gene is used for obtaining amplification products are the following:

- the strain N315 for the gene *nuc SA,* and/or
- the strain NCTC8325 for the gene *sodM,* and/or
- the strain LRA076 of *Staphylococcus intermedius* for the gene *nuc SI,* and/or
- the corresponding strains to the genes of Table II as defined in column "Strains" of Table II, and/or
- the corresponding strains to the genes of Table III as defined in column "Strains" of Table III, and/or
- the corresponding strains to the genes of Table IVas defined in column "Strains" of Table IV.

Preferably, the method for identification or typing according to the present invention is characterized in that the absence or the presence of a relevant gene in the genomic DNA to be tested is determined in the following manner:

after the specific hybridization step and after reading of the signal obtained for one amplicon, the value 0 is attributed when this signal is significant of the absence of the gene, or the value 1 is attributed when this signal is significant of the presence of the gene,
said values being attributed in the following manner, after determination of the following ratio R:

$$R = \frac{\text{Normalized Hybridization Intensity Value (NHIV)}}{\text{Normalized Hybridization Intensity Value of Reference (NHIVR)}},$$

- 0, when the ratio R is strictly inferior to 0.3,

- 1, when the ratio R is equal or superior to 0.3;

where NHIV is the ratio of the hybridization intensity value of the signal obtained for one amplicon to the average of all hybridization intensity values of the significant signals obtained on the solid support, and
where NHIVR is obtained by combining the average of NHIV values obtained for each of the amplicons after hybridization in suitable conditions of the amplified genomic DNA of the previously identified *S. aureus* bacterial strains with the set of amplicons coated on the solid support.

Furthermore, the method for identification or typing at species level of a *Staphylococcus aureus* bacterial strain according to the present invention is characterized in that a phylogenetic tree is designed on the basis of a plurality of the hybridization profiles obtained for each of the identified *Staphylococcus aureus* bacterial strains, said hybridization profiles being clustered relative to the presence or the absence of the relevant genes.

In one prefered embodiment, the method for typing a bacterial strain according to the present invention is characterized in that a phylogenetic tree is designed on the basis of a plurality of the hybridization profiles obtained for each of the identified *Staphylococcus aureus* bacterial strains, said hybridization profiles being clustered relative to the presence or the absence of the relevant genes, and the bacterial strain to be tested is typed as a known *Staphylococcus aureus* bacterial strain when its hybridization profile is close up to the clustered hybridization profiles of the identified *Staphylococcus aureus* bacterial strains.

One another aspect of the present invention is a solid support coated with a set of amplicons for identification or typing of *Staphylococcus aureus* bacterial strains from an extract of its genomic DNA, each amplicon being able to specifically hybridize with a relevant gene or with a representative fragment thereof susceptible to be present in said genomic DNA to be tested,
characterized in that said relevant genes whose presence or absence is to be determined using the set of amplicons comprise the following genes :

a) at least one gene considered as a positive control whose presence in a genome is characteristic for the *Staphylococcus aureus* species, and

b) at least one gene considered as a negative control, said gene being absent in the genome of said *Staphylococcus aureus* species, and

c) genes selected from the group A, said group A consisting in the following categories of genes:

(i) genes encoding staphylococcal and enterococcal proteins mediating drug resistances,

(ii) genes encoding factors known to be involved in *Staphylococcus aureus* pathogenicity and genes encoding structurally related proteins,

(iii)genes encoding proteins produced by mobile elements,

(iv)genes encoding factors involved in the regulation of pathological proteins,

(v)genes encoding proteins involved in the expression of antibiotic resistance or in the transfer of antibiotics.

Preferably, the solid support of the present invention is characterized in that the set of amplicons comprises the amplicons necessary for specifically hybridizing with at least 50 % of the relevant genes of the group A.

Indeed, the analysis of the results demonstrates that:

- the average of the positive hybridizations for each strain (for example NCTC8325: 241 positive spots for 472 tested genes: Blast and hybridizations) ist about 50 %;

- the diversity is of 385 amplicons non present in the five reference strains entirely sequenced (N315, Mu50, COL, MW2 and NCTC8325).

A prefered embodiment is the solid support of the present invention, characterized in that at least one of the two genes *nuc SA* and *sodM* as defined in Table I, preferably the two genes, is selected as positive control for the *Staphylococcus aureus* species.
Another prefered embodiment is the solid support of the present invention, characterized in that the gene *nuc SI* as defined in Table I is selected as a negative control for the *Staphylococcus aureus* species.
Another prefered embodiment is the solid support of the present invention, characterized in that the genes selected from the group A are all the genes of the Table T30.
Another prefered embodiment is the solid support of the present invention, characterized in that the genes selected from the group A are all the genes of the Table T60.
One antoher prefered embodiment is the solid support of the present invention, characterized in that the genes selected from the group A comprise all the genes of the Table II.
Preferably, the solid support of the present invention is characterized in that the genes selected from the group A comprise all the genes of the Table III.
More preferably, the solid support of the present invention is characterized in that the genes selected from the group A comprise all the putative genes of the Table IV.
In a most prefered embodiment, the solid support of the present invention is characterized in that the relevant genes whose presence or absence is to be determined using the set of amplicons comprise the following genes:

- all the control genes of the Table I ;
- all the genes of the Table II ;
- all the genes of the Table III ;
- all the putative genes of the Table IV.

More preferably, the solid support of the present invention is characterized in that each amplicon of the set of amplicons is an amplification product of a relevant gene, said amplification product being obtained from the genomic DNA of a bacterial strain containing said gene with a couple of primers specific for the gene.
More preferably, the solid support of the present invention is characterized in that the couple of primers used for the obtention of each amplicon of the set of amplicons is selected in a manner such that the amplicons have a size of 400 to 500 base pairs.
Even more preferably, the solid support of the present invention is characterized in that when at least one of the genes *nuc SA* and *sodM* as defined in the Table I, preferably the two genes, is selected as positive control, the couple of primers specific for the gene *nuc SA* is SEQ ID No. 1 and SEQ ID No. 2, and the couple of primers specific for the gene *sodM* is SEQ ID No. 3 and SEQ ID No. 4.
In a most prefered embodiment, the solid support of the present invention is characterized in that when the gene *nuc SI* as defined in the Table I is selected as negative control, the couple of primers specific for this gene is SEQ ID No. 5 and SEQ ID No. 6.
In another most prefered embodiment, the solid support of the present invention is characterized in that the couples of primers specific for the genes of the Tables II, III and IV are the couples of primers corresponding to these genes as defined in column "couple of primers" of these Tables.
Preferably, the solid support of the present invention is characterized in that the bacterial strains whose genomic DNA containing said gene is used for obtaining amplification products are the following:

- the strain N315 for the gene *nuc SA,* and/or
- the strain NCTC8325 for the gene *sodM,* and/or
- the strain LRA076 of *Staphylococcus intermedius* for the gene *nuc SI,* and/or
- the corresponding strains to the genes of Table II as defined in column "Strains" of Table II, and/or
- the corresponding strains to the genes of Table III as defined in column "Strains" of Table III, and/or

- the corresponding strains to the genes of Table IV as defined in column "Strains" of Table IV.

One further aspect of the present invention is a DNA macro-array for identification and typing of a *Staphylococcus aureus* bacterial strain comprising the solid support of the present invention.
One another aspect of the present invention is a kit for typing a bacterial strain comprising :

- the solid support of anyone of the present invention or the DNA macro-array of the present invention.

Preferably, the kit for typing a bacterial strain according to the present invention is characterized in that it also comprises :

- a plurality of hybridization profiles obtained from previously identified *Staphylococcus aureus* bacterial strains and/or possibly a phylogenetic tree designed on the basis of this plurality of hybridization profiles.

Kits comprising a solid support or a DNA macro-array according to the present invention are well described in the literature; they may further comprise in particular reagents and/or a notice where the conditions suitable for the specific hybridization are described.
One further aspect of the present invention is a set of couples of primers capable of specifically amplifying, in suitable conditions for hybridization, genes or representative fragments which are relevant for the identification at the species level of a *Staphylococcus aureus* strain or for typing it, said relevant genes comprising the following genes:

a) at least one gene considered as a positive control whose presence in a genome is characteristic for the *Staphylococcus aureus* species, and

b) at least one gene considered as a negative control, said gene being absent in the genome of the *Staphylococcus aureus* species, and

c) genes selected from the group A, said group A consisting in the following categories of genes :

(i) genes encoding staphylococcal and enterococcal proteins mediating drug resistances,

(ii) genes encoding factors known to be involved in *Staphylococcus aureus* pathogenicity and genes encoding structurally related proteins,

(iii) genes encoding proteins produced by mobile elements,

(iv)genes encoding factors involved in the regulation of pathological proteins,

(v) genes encoding proteins involved in the expression of antibiotic resistance or in the transfer of antibiotics.

Preferably, the set of couples of primers according to the present invention is characterized in that at least one of the two genes *nuc SA* and *sodM* as defined in Table I, preferably the two genes, is selected as positive control for the *Staphylococcus aureus* species, the couple of primers specific for the gene *nuc SA* having the sequences SEQ ID No. 1 and SEQ ID No. 2, and the couple of primers specific for the gene *sodM* having the sequences SEQ ID No. 3 and SEQ ID No. 4.
More preferably, the set of couples of primers according to the present invention is characterized in that the gene *nuc SI* as defined in Table I is selected as a negative control for the *Staphylococcus aureus* species, the couple of primers specific for this gene having the sequences SEQ ID No. 5 and SEQ ID No. 6.
More preferably, the set of couples of primers according to the present invention is characterized in that the genes selected from the group A comprise all the genes of the Table II, the couples of primers corresponding to these genes being defined in column "couple of primers" of this Table II.
Even more preferably, the set of couples of primers according to the present invention is characterized in that the genes selected from the group A comprise all the genes of the Table III, the couples of primers corresponding to these genes being defined in column "couple of primers" of this Table III.
More preferably, the set of couples of primers according to the present invention is characterized in that the genes selected from the group A comprise all the putative genes of the Table IV, the couples of primers corresponding to these genes being defined in column "couple of primers" of this Table IV.
More preferably, the solid support according to the present invention is characterized in that the genes relevant for the *Staphylococcus aureus* species comprise the following genes:

- all the control genes of the Table I ;
- all the genes of the Table II ;
- all the genes of the Table III ;
- all the putative genes of the Table IV;

the couples of primers corresponding to these genes being defined in column "couple of primers" of these Tables.

The purpose of the legends of the figures and of the examples below is to illustrate the invention. It doesn't limit the scope of the claimed invention.

## LEGENDS OF THE FIGURES

**Fig. 1 : Hierarchical clustering of the 80 *S. aureus* isolates investigated according to their gene content, using the J-Express program (Dysvick, 2001).**

The threshold cleavage site was chosen to distinguish each of the outbreak-related isolates belonging to the *Sma* I genotype 100 or 101(clusters 9 and 28, respectively) from any of the other isolates.
The isolates in grey background represent outbreak-related isolates marked with the superscript letter O.
The isolates with an asterisque represent the *mecA*+ isolates.

**Fig 2 : Images resulting from scanning of the DNA macro-arrays hybridized with the total cellular DNA from two isolates. Fig. 2A : BM9290 (cluster 46, Fig. 1) and Fig. 2B : BM12188 (cluster 47, Fig. 1).**

Even belonging to the same SmaI subtypes ($45a^{A}$), the two isolates were found in two close but separate clusters (Fig.1) due to the lack, in BM12188, of the following five drug resistance genes : *blaZ, qacA, qac*C, CZ040 and CZ041 encoding β lactamase, resistance to antiseptics, organomercurial lyase and mercuric reductase, respectively.

**Fig 3 : Normalization result curve (calibration curve)**

## EXEMPLE 1 : MATERIAL AND METHODS

### Bacterial isolates :

The relevant characteristics of the 80 *S. aureus* clinical isolates used to evaluate the usefulness of the DNA macro-array designed in this study are given in Table 5. The 44 staphylococcal, enterococcal and *E. coli* stains used as substrates for PCR amplification of the genes chosen for the construction of the macro-array are reported in the Tables I, II, III and IV.

### DNA extraction:

Total cellular DNA was extracted and purified using QIAamp DNA Mini kit (Qiagen, Hilden, Germany ), but the method was modified by the inclusion of lysostaphin (Applied Microbiology, NY, US ) at a final concentration of 100 mg/l at the lysis step. RNA was removed after 30 min incubation at 37°C with 5 mg/l of Rnase (DNAse-free) (Roche, Meylan, France).

### Choice of the primers.

The data-processing program "primer 2" was used for the choice of the primers. The programmed size of each amplicon lay between 400 and 500 Pb. For certain genes of pathogeny and resistance to antibiotics, the research of the primers was preceded by a comparative analysis with other genes by the same class. The primers were synthesized by the Eurogentec company.

**Intragenic Amplification by polymerization in chain (PCR)**

**• Genomic Extraction of DNA**

The bacteria were initially put in culture 18h under agitation. A volume of one ml was collected and centrifuged. The pellet was suspended in 180ml of Tp buffer (TRIS 50Mm HCl pH 8,5/ EDTA 5mM/1% X-100 Triton). The bacteria were lysed by addition of 20ml of lysostaphine (1mg/ml) followed by incubation during 30 minutes at 37°C. The Kit Qlamp DNA Mini Kit of Qiagen was used for the extraction and the purification of DNA. The RNA was inactivated by addition of 2mg of Rnase (Rock) followed by incubation during 30minutes at 37°C. The total DNA preparations were controlled by electrophoresis of 2ml of the solution the DNA in 07% agarose gel.

**• Amplification of the fragments intragenic by PCR.**

The solutions of total DNA of the matrix strains were diluted beforehand at the 1/10 to reduce the quantities of substrates associated with the amplified products. The amplification experiments were carried out, by series, on plates of 96 wells adapted to Robotcycler (Gene Amp PCR system 9700 Applied Biosystem).
The reactive medium had the following composition:

- Genomic DNA diluted at 1/10ème : 1µl
- Primers F+R (10 µM): 6µl
- Tp buffer 10X: 10µl
- dNTP 25mM: 0,8µl
- Taq 0,8: µl
- H2O (qsp 100µl): 81,4µl

The program recommended by the provider was used, that is : 5 minutes at 94°C and 35 cycles of 1 minute at 94°C, 1 minute at 50°C, 1 minute at 72°C, then 7 minute at 72°C.
The solution is preserved at 4°C. The amplified products, presenting two bands or more at 50°C, were again amplified at 55°C to improve specificity of the reaction. To obtain a final volume of 300 µl for each amplicon, three series of PCR were carried out. The three fractions of 100 µl were joined together in the wells of plates of 96 wells. A control on agarose gel of product PCR was carried out to confirm the presence of the amplicon, to evaluate its intensity, its size and the absence of other non-specific bands.

**Sequencing of the amplified products**

On a systematic basis 15% of randomly selected amplicons as well as the negative and positive controls were sequenced in order to control the specificity of the amplified products. Moreover, all the products amplified with poor yield or obtained at a temperature of 55°C were also sequenced. Sequenced amplicons are marked by an asterisk in table 2.
The sequencing was carried out in three stages on plates of 96 wells:

a) Treatment of let us amplicons with exonuclease I:

- PCR Product: 4µl
- Exonuclease I (10u /µl)(Amersham): 0,3µl
- SAP * (1u/µl)(Amersham): 1 µl
- Plug 5X : 2,5µl
- H2O (qsp 10µl): 2,2µl

SAP *: Shrimp Alkaline Phosphatase. This mixture was incubated 15 minutes at 37°C, 15 minutes at 80°C then preserved at 4°C.

**Comparative genome analysis, primer design, PCR amplification:**

For annotation and comparative analysis of the available genome sequences from the seven S.aureus isolates cited above, the program CAAT-Box ( Frangeul *and al,* 2003). was used. The genes whose nt sequences exhibited less than 80% similarity were considered as distinct.CAAT-Box uses the blast programme which presents the least similarity with the rest of genome. The Primer3 programme (http://www.broad.mit.edu/cgi-bin/primer/primer3_www.cg) identifies primer pairs in this specific area which are unlikely to produce non-specific amplifications with regard to the seven sequenced S.aureus genomes. The criteria used by CAAT-Box and Primer3 were : match threshold : 21, max length of non-spe PCR : 3000 bases, min PCR length: 250 bases, optimum PCR length : 400-500 bases, primer size : 18-20-25 bases (minimum-optimum-maximum), primer-Tm : 51-55-60°C, GC%:25-50-80%, max diff Tm in a pair: 5 °C. Each of the 478 selected genes encoded at least 150 amino acids. Primers were designed to amplify a fragment of 400 to 500 bp specific for each gene. PCR amplification reactions were performed in a 100 µl reaction volume containing 10-20 ng DNA and 1µM of each primer (Eurogentec, Liege, Belgium): initial cycle of 5 min at 94 °C, followed by 35 cycles of 1 min at 94° C, 1 min at 50°C, and 1 min at 72°C, with a final extension step of 7 min at 72° C. The concentration and size of each PCR product was verified by electrophoresis on agarose gels.

**Array construction:**

For array preparation, high density nylon membranes Performa (Genetix, New Milton, UK) were soaked in TE solution (10 mM Tris-pH 7.6, 1mM EDTA). Double spot blots of each PCR product were printed using a Qpix robot (Genetix). Following spot deposition, membranes were fixed for 15 min in 0.5 M NaOH, 1.5 M NaCl, washed briefly in distilled water and stored wet at -20 °C until use.

**Hybridization:**

The cellular DNA of the *S. aureus* strains (50ng) was labeled using a Random Priming DNA Labeling Kit (Roche, Diagnostics GmbH, Penzberg, Germany), and 50 µCi 5'-(α-$^{33}$P) triphosphate dCTP (Amersham, Piscataway, NJ, US ). Labeled probes were purified using QIAquick Nucleotide Removal Kit (Quiagen). The membranes were moistened in 2x SSC((0 .3M NaCl, 0.03M Na citrate) and pre-hybridized for 1 h in 10 ml of 5x SSPE (0.9M NaCl, 6mM NaH2PO4 and 7.5mM EDTA, pH8), SDS 4%, 1x Denhardt (0,02% Ficoll, 0,02% polyvinylpyrrolidone and 0,02% BSA) and 1 mg of denatured salmon sperm DNA. Hybridization was performed overnight at 65 °C. Membranes were washed twice at room temperature and twice at 65 °C in 0.5X SSPE, and 0.2% SDS. Arrays were then sealed in polypropylene bags and exposed to a PhosphorImager screen for 24 h.

**Verification of the specificity of the DNA macro-array:**

Of the 478 DNA fragment amplified, 106 were randomly chosen and sequenced. Sequencing of the PCR products was carried out using ABI3700 capillary sequencer. To test the correct spotting, the membranes loaded with the amplicons were hybridized with the cellular DNA of the *S. aureus* strains used as substrates in PCR experiments. For 465 amplicons, the results were as expected, i.e. specific. Thirteen of the 478 genes selected were eliminated, either because two aspecific DNA bands were amplified (one gene), or because hybridization experiments revealed false positive or negative results (10 and 2 genes, respectively). The characteristics of the amplicons and the strains used as substrates, as well as the sequences of the primers and their position in the genome, are available in the Tables I, II, III and IV.

**Data analysis:**

For scanning, the Typhoon 9400 PhosphorImager (Molecular Dynamics, Amersham) was used. The Array Vision software (Imaging Research) was used for quantification of the hybridization intensities and for normalisation. For each spot, the hybridization intensity value was normalised by dividing it by the average of all significant intensity values on each filter. For ratio calculation, a reference array was built by combining the average normalised data when two replicate hybridization experiments were carried out (this was the case when probes consisted of the cellular DNA of strains N315, MW2, MU50, NCTC8325, and COL used to amplify DNA fragments from within 385 genes). When the genes were present in multiple copies, the intensity values were often higher than those present in a single copy, and the lowest significant values were chosen.

When the genes were known to be present in the tested strains used as substrates such as the five strains whose genomes have been sequenced : N315, Mu50, COL, MW2 and NCTC8325, the ratios between the normalized signal intensity of the gene hybridized with the tested strain and that of the reference array was always higher than 0.3. Thus the threshold for the presence of a gene or variants related by at least 80% similarity was defined as > 0.3. The data were then converted into binary score : at ≥ 0.3, a gene was scored as present (score=1) and at < 0.3 a gene was scored as absent (score=0). The binary data were used to cluster the isolates hierarchically, using the J-Express program (Dysvik, B., and I. Jonassen. 2001. Bioinformatics 17:369-70) .

**EXEMPLE 2 : RESULTS**

**Choice of the genes for the construction of the DNA macro-array:**

Comparative analysis of the seven *S. aureus* genomes sequenced (N315 ( Kuroda *and al.,* 2001, Lancet 357: 1225-40), Mu50 (Kuroda *and al.,* 2001), COL (The Institute for Genomic Research. TIGR databases), MW2, NCTC8325 ( Oklahoma University. Staphylococcus aureus NCTC8325 Genome Sequencing), MSSA-strain 476 (Sanger Centre. Staphylococcus aureus) and EMRSA 16-strain 252 ( Sanger Centre. Staphylococcus aureus) enabled us to choose, first, 397 amplicons. Among these latters, 305 amplicons were not shared by all of them and thus, were putatively useful for typing. Although shared by the seven sequenced genomes, 92 genes were selected. They included two *S. aureus* specific genes (*nuc* ( Chesneau *and al,* 1994, p. 83-85. *In* R. Möllby, J.-I. Flock, C. E. Nord, and B. Christensen (ed.), Staphylococci and Staphylococcal Infections., Gustav Fischer Verlag, Stuttgart, Jena, New York, 1994 ed. Gustav Fischer Verlag, Stuttgart, New York, Jena), *sodM* ( Valderas *and al,* Journal of Bacteriology 184:2465-2472)) for identification, at species level, and genes encoding either putative virulence proteins and factors involved in their regulation or proteins involved in the expression of antibiotic resistance.

Furthter, 67 amplicons not detected in the seven genomes compared were selected for typing because the corresponding genes encode (i) staphylococcal and enterococcal proteins mediating drug resistances, 30 antibiotic resistance genes were found in Gram positive species other than *S. aureus*: *S. hyicus* (*tetL*), *S.cohnii* (*vatC*, *vgbB*), *S. epidermidis* (*fos*, *InuA*), *E. faecium* (*vatD*, *vatE*, *msrA*, *InuB*, *vanA*), *E. faecalis* (*vanB*, *Isa*) and *E. gallinarum* (*vanC*). These latter genes were chosen because of their possible transfer to *S. aureus,* (ii) factors known to be involved in *S. aureus* pathogenicity and structurally related proteins (toxins, adhesins, enzymes involved in the biosynthesis of capsule or slime, etc.), or (iii) proteins produced by mobile elements (transposons, insertion sequences, plasmids). The negative control consisted of the *S. intermedius* specific *nucI* gene ( Chesneau *and al,* 1994).

Thus, a total number of 465 amplicons was spotted on the membranes. The five *S. aureus* strains, N315, Mu50, COL, MW2, and NCTC8325, were used for amplifying 385 intragenic fragments. The 80 other genes were previously amplified from 39 other strains used as substrates.

**Distribution of the 465 genes among the 80 *S. aureus* clinical isolates analysed:**

A DNA macro-array was designed with several intragenic amplicons to identify, at the species level, and to type S. aureus isolates. To evaluate the DNA macro-arrays' usefulness for typing and for investigation of a putative pathogenicity index correlated with bone infection, they were probed with the cellular DNA of 80 clinical isolates previously typed by determining their antibiograms and SmaI restriction patterns. They included outbreaks related isolates as well as unrelated isolates responsible for bone infections and from nasal samples of uninfected carriers to check whether these two categories of isolates can be distinguished.

The gene content of each of the 80 isolates is shown on the Tables T30 and T60. Of the 92 genes shared by the seven sequenced genomes and used in the macro-array, 76 including *S. aureus nuc* and *sodM* were detected in all the analysed isolates. Therefore a total number of 388 genes were useful for typing.

**Antibiotic resistance genes and their phenotypic expressions:**

Analysis of the data reported in Table V enabled us to check whether the genes detected by hybridization were correlated with their phenotypic expression in the isolates. Resistance to βlactams (PEN, OXA) of 35 isolates is associated with the presence of *mecA* (four isolates) or *mecA* and *blaZ* (31 isolates), whereas resistance to penicillin G (PEN) only (39 isolates) was associated with *blaZ.* A single *mecA*+ isolate was susceptible to β lactams. The other associations consisted of (i) resistance to gentamicin and *aacA-aphD* (30 isolates), (ii) resistance to neomycin and *aadD* (24 isolates) or *aphA-3* (eight isolates), (iii) resistance to spectinomycin and *spc* (31 isolates), (iv) resistance to tetracycline and minocycline and *tetM* (34 isolates) or *tetM* and *tetK* (three isolates), (v) resistance to tetracycline and *teK* (two isolates), (vi) inducible or constitutive resistance to macrolides, lincosamides and streptograminB (MLS) and *ermA* (31 isolates), *ermC* (11 isolates) or *ermA* and *ermC* (one isolate), (vii) resistance to erythromycin and *msrA* (three isolates), and (viii) resistance to lincosamides and *lnuA* (one isolate).

The streptogramin resistance genes previously found by PCR in the isolates resistant to streptogramin A ( Haroche and al, Journal of Clinical Microbiology 41:586-591) (Table V), were detectable by hybridization with the DNA macro-arrays designed in this study. The intragenic amplicons from vgaA and vgaAv appear to be specific to each variant despite the 83.2% similarity relating them. This is due to the fact that the divergences are distributed along the entire sequences of the gene variants, without more than 29 consecutive matching nucleotides between the amplicon and the variant gene. The resistances to fosfomycin, streptomycin, thrimetoprime and fusidic acid, which can result from mutations in pre-existing genes are rarely associated with acquired genes (Table VI). In contrast, resistances to β-lactams, aminocyclitols except streptomycin, tetacycline, minocycline, macrolides, lincosamides and streptogramin B were correlated with the presence of at least one acquired gene (Table VI). Two of the 12 isolates resistant to streptogramin A (BM10761 and BM10759, Table V) did not carry any of the investigated genes encoding resistance to this antibiotic.
The S. aureus fosB gene, included in the arrays because of its similarity with fos, was found in 69 of the isolates, independantly of their phenotypes of resistance to fosfomycin.

The combination of the genes carried by each of the transposons,Tn *554* (*spe, ermA, tnpA,* and *tnpB),* Tn *5406* (*vgaAv, tnpA,* and *tnpB*), and Tn *4001* (*aacA-aphD*,and IS*256 tnp*) was found in the isolates exhibiting the antibiotic resistance phenotypes mediated by these transposons. The genes *blaZ* and *tnp480* co-carried by Tn *552*, were associated in only 28 of the 70 isolates containing *blaZ.* As stated before ( Derbise *and al.* 1997. Antimicrobial Agents & Chemotherapy 41:1024-1032), the three genes, *aadE, sat4,* and *aphA*-3 initially found in Tn *5405,* were always combined and found in seven isolates of this study. This latter combination was occasionally associated with other Tn *5405* genes, i.e. *orfX* (two isolates), *orfX* and IS*1182 tnp* (four isolates) or *orfX,* IS*1182 tnp,* and IS*l181 tnp* (one isolate).

**Distribution of the genes in *mecA*+ and *mecA*- isolates:**

As reported in Table V, 36 of the 80 tested isolates were *mecA*+ and 44 were *mecA*-. Several genes including those coding for antibiotic resistance and putative virulence factors have a distribution which is significantly different ($p_g < 0.1$) in the two categories of isolates compared. The distribution of those genes encoding putative toxins or adhesins is reported in Table VI. Note that the enterotoxin encoding genes *seg, sei, sem, sen,* and *seo,* co-detected in the same pathogenicity island of N315 and Mu50 *S.aureus* strains (Kuroda *and al,* 2001), were always associated in the isolates of our study and significantly predominant in the *mecA*- isolates (30 of the 44 tested isolates) compare to the *mecA*+ isolates (one of the 36 isolates).

**Distribution of the genes in 16 Bone Infection isolates (BI) and 12 isolates from Nasal Carriers (NC):**

Unrelated isolates were selected for the comparative analysis of these two categories of isolates (Table V). No significant differences in the gene contents were observed between the BI and NC isolates, when the 388 genes were taken into account for the calculation of the probability that a given gene is present by chance. However, taking into account only 11 genes not shared by all isolates and encoding adhesins (*sdrD*, *sdrC*, *fnbA*, *fnbB*, *efb*, *map*, *cna*, *bbp*, *vwb*, *bap* and *ebpS*), the two categories of isolates became significantly distinguishable ($p_g = 0.059$) by their content in the *sdrD* gene coding for a putative SD (Serine-Aspartate) adhesin (Josefsson *and al,* 1998, J Biol Chem 273: 31145-52) and detected in 15 of the 16 BI isolates vs. seven of the 12 NC isolates.

**Clustering of the 80 *S. aureus* clinical isolates on the basis of their genes contents investigated with the DNA macro-array designed in this study:**

The hierarchical clustering of the isolates by neighbor joining is represented in the dendrogram shown in Figure 1. First we checked whether the outbreak-related and endemic isolates were more closely linked to each other.

Within each *Sma*I genotype 100 or 101 (Table V), the isolates were more linked to each other than to any of the other isolates (Fig.1). The former isolates were included in this study because they were responsible for documented acute outbreaks in the hospitals of Villeneuve St Georges and Calais, respectively. Such isolates were not detected in the hospitals before the outbreaks. Analysis of their gene contents revealed the absence of two and seven widespread genes, detected in at least 84 % of the other isolates : *fnbB* and MW2409 in the four *Sma*I 100 isolates and *set14*, *lukM*, *splcC*, *splD*, *vwb*, *emp* and SA0276 in the five *Sma*I 101 isolates. The lack of widespread genes confirmed the hypothesis of a close relatedness of the isolates belonging to each of the two *Sma*I genotypes. As found previously by PCR, the four isolates from Villeneuve St Georges responsible for scalded skin syndrome in neonates (Table V), carried the *eta* gene encoding the exfoliative toxin A. Moreover, the single *Sma*I 101 isolate distinguishable from the other four *Sma*I 101 isolates by its susceptibility to erythromycin lacked the *ermC* gene found in the latter isolates (Table V).

The 24 *mecA*+ isolates belonging to the *Sma*I genotypes $39^A$ and $45^A$ were more linked to each other than any of the 56 other isolates (Fig.1). Isolates belonging to *Sma*I genotype $39^A$ were phenotypically recognizable because of their decreased susceptibility to glycopeptides. Those belonging to *Sma*I genotype $45^A$ and phage type 77 were initially discovered in 1987, during the emergence of resistance to fluoroquinolones in French hospitals. Even being isolated in several European countries and at time intervals of several years, these endemic isolates were considered as putatively related according to their *Sma*I genotypes. Some of these latter isolates are clearly divergent in the dendrogram and the mode of their linkage is not correlated to the *Sma*I genotypes. Note that the isolates belonging to *Sma*I genotypes $39a^A$ (IPF 555, IPF 557, IPF 562) ( Guerin *and al,* 2000. J Clin Microbiol 38:2985-8) or $45a^A$ (BM 9586, BM 12184, BM 12188) (Morvan *and al,* 1997) and considered as outbreak-related in hospital C (Paris) in 1999 and 1987, respectively, pre-existed in French hospitals before the outbreaks, but at very low frequencies.

**Clustering of the 80 clinical isolates after the choice of the threshold cleavage site on the hierarchical clustering dendrogram (Fig. 1):**

The four categories of outbreak-related isolates cited above are framed on Fig.1. To distribute the isolates into clusters, it was necessary to choose a threshold cleavage site on the dendrogram. For this purpose, the adopted threshold was that which enabled to distinguish each of the outbreak-related isolates belonging to *Sma*I genotypes 100 or 101 from any of the other isolates. These isolates were taken into consideration because they were not detected before the outbreaks, by contrast with the *Sma*I type 39a$^A$ or 45a$^A$ outbreak- related isolates. The choice of this cleavage site enabled the discrimination of 52 clusters among the 80 isolates belonging to 45 *Sma*I genotypes (Fig. 1). The genes common to the isolates linked in the same cluster can be found in the Tables T30 and T60.

With the chosen cleavage site, a total of five clusters were found among the ten *Sma*I type 39$^A$ isolates and eigth clusters among the 14 *Sma*I type 45$^A$ isolates (Fig. 1). Among these isolates endemically spread in European cities, those collected in the same hospital or city are not necessary the most closely linked. The three *Sma*I type 39a$^A$ isolates collected in the C hospital (Paris) in 1999 (IPF 555, IPF 557, and IPF 562) and considered as outbreak-related are linked in cluster 41 including another *Sma*I type 39a$^A$ isolate (BM 12612), collected in 1998 in Villiers St Denis. Moreover, four of the five isolates belonging to *Sma*I subtypes 45a$^A$ - 45d$^A$ and collected in three French hospitals in 1987 are within cluster 46 (BM 9290, BM 9343, BM 9586, and BM 12184). The fifth isolate, BM 12188, located in the separate but close cluster 47, was distinguishable by the lack of five drug resistance genes : *blaZ, qacA, qac*C, CZ040 and CZ041 encoding β lactamase, resistance to antiseptics, organomercurial lyase and mercuric reductase, respectively. Fig.2A and 2B represents the images resulting from scanning of the two DNA macro-arrays hybridized with the total cellular DNA from BM9290 and BM12188 isolates, respectively (Table V).

Each of the isolates linked in clusters 6, 13, 37 and 40 belonged to the same *Sma*I genotypes. By contrast the isolates linked in clusters 11, 14, and 33 belonged to unrelated *Sma*I genotypes and cluster 31 included two distinct but related *Sma*I genotypes. In addition, isolates with the same *Sma*I genotype, 117 or 144, were separated. Note that the two isolates belonging to the *Sma*I genotype 144 had no epidemiological link since they were from distinct sources (Tunisia and Sweden) and collected at a ten years time interval. In this latter case, analysis of gene contents is more appropriate than *Sma*I patterns for the discrimination between the two isolates.

**DISCUSSION**

DNA macro-arrays offer a rapid, robust, and easily standardizible method for the simultaneous detection of several genes of interest and may be used for analysis of their transcriptional expression in the isolates grown under different *in vitro* and *in vivo* conditions. The 465 genes spotted in each DNA macro-array of this study were chosen for their potential usefulness for the identification, at species level, and typing of *S.aureus* isolates. They included, in particular, those encoding antibiotic resistance and putative virulence factors.

The detection of antibiotic resistance genes is particularly interesting when they mediate low antibiotic resistance levels not detectable reproducibly by antibiograms and contribute to the selection of isolates carrying genes not yet described. For 77 of the 80 clinical isolates tested, the resistance phenotype conferred by the resistance genes found were expressed whereas in one isolate, the *mecA* gene was not expressed and in two of the streptogramin A resistant isolates, none of the known staphylococcal or enterococcal genes conferring resistance to this antibiotic was detected. However, the mutations in pre-existing genes associated with antibiotic resistances cannot be visualized and would necessitate hybridization with oligonucleotides.

The assessment of the isolates for the presence of all known *S.aureus* genes encoding putative virulence factors may contribute to the determination of the pathogenic potentiel correlated with a particular type of infection and identification of emerging pathotypes. In this study, we checked if some genes were more prevalent in isolates responsible for bone infections (BI) compare to isolates from uninfected nasal carriers (NC). For this purpose, only unrelated isolates of our collection were included. This constraint explains why the number of isolates analysed was only 16 BI and 12 NC.

Despite the fact that bone infections were contracted by children outside the hospital, several patients were infected by *S.aureus* isolates considered as monoclonal on the basis of their *Sma*I patterns. Even if a few genes including *sdrD* encoding a putative SD adhesin appeared predominant in one of the two categories of isolates, the differences were not significant when the 388 genes used for typing were taken into account in the calculation of the probability that a given gene is present by chance. Thus, a larger number of unrelated isolates from various sources have to be further analysed. Meanwhile, when only the 11 genes encoding putative adhesions were taken into account, the higher prevalence of *sdrD* in BI isolates, compare to NC isolates, became significant. Some SD proteins were shown to bind fibrinogen (ClfA (McDevitt *and al,* 1994. Mol Microbiol 11:237-48), ClfB (Ni Eidhin *and al,* 1998. Mol Microbiol 30:245-57) and SdrG (Hartford *and al* 2001. Microbiology 147:2545-52)) or bone sialoprotein (Bbp) (Tung *and al,* 2000. Biochemical Journal 345 Pt 3:611-9), but the ability to bind matrix protein(s) of SdrD has not been investigated. The impact of *sdrD* inactivation would merit to be evaluated in a bone infection animal model.

The significantly distinct distribution of some genes encoding enterotoxins or adhesins among the *mecA*+ and *mecA*- isolates of this study may not be found among isolates from various sources (Table VI). Indeed, most of the 80 tested isolates were collected in France and in Tunisia and the *mecA*+ isolates belonged to a limited number of *Sina*I genotypes. Nevertheless, the low frequency of *cna* detection in *mecA*+ isolates has been already reported by Booth *et al.* (2001) ( Infection & Immunity 69:345-52).

Due to the use of a large number of genes fo typing (388), all 80 isolates tested were typeable. The method based on the analysis of a large number of genes was expected to yield a better discrimination between the isolates than the typing methods based on sequencing a limited number of genes or on analysis of *Sma*I patterns which depends on the number and location of *Sma*I sites in the genome. This was confirmed in this study, among the *mecA*+ isolates endemically spread in several European cities and collected at large time intervals (*Sma*I genotype $39^A$ and $45^A$). Among the latter isolates, those considered as outbreak-related in the same hospital appeared to be in the same or in close cluster(s) : 41 or 46-47. In such a context, the typing method proposed in this study provides a better discrimination of the isolates responsible for acute outbreaks than the determination of *Sma*I patterns. For the other isolates, if we exclude the three pairs of isolates which were linked in the same cluster despite belonging to unrelated *Sma*I types, our results revealed a good correlation between the mode of isolate clustering based on the two typing methods, i.e. analyis of gene contents and *Sma*I patterns. Indeed, the isolates belonging to the same or related *SmaI* types appear to be more linked to each other than to those belonging to unrelated *Sma*I types.

In conclusion, the typing method proposed here performed better that based on the analysis of *Sma*I patterns, in particular, to distinguish outbreak related isolates from those spread endemically. It also has the advantage to be faster and to provide additional information concerning the content in genes of interest. This macro-array should be updated when additional genes are described, and has to be validated for transcriptome analysis in order to evaluate the levels of gene expression which may be correlated with particular types of infections.

Table T30: Gene content of each of the 80 S. aureus isolates tested. mecA+ isolates are marked by an asterisk. The gene was scored as present (1) or absent (0). The cluster numbers are those reported in Figure 1.

° Outbreak related isolates

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 | 32 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| Gene | BM12987 (O24) | IPF143 | BM12685 | BM12947 | IPF493 | BM12286 | BM18287 | IPF140 | IPF150 | IPF735° | IPF736° | IPF741° | IPF743° | IPF738° | IPF511 | BM12633 | BM12623 | BM12863 | BM12718 | BM12771 | BM12889 | IPF497 | BM12766 | BM12816* | IPF139 | IPF166 | BM12764 | IPF524 | BM12881 | IPF161* | IPF498 | IPF488 | IPF494 | BM12666 | IPF153 | IPF308° | IPF310° | IPF323° | IPF311° | IPF145 | IPF520 | IPF490 | IPF159 | BM12681* |
| *SA1208* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 0 | 1 | 1 | 1 | 0 | 0 | 0 | 0 | 0 | 0 | 0 | 0 | 0 | 0 | 0 | 0 | 0 |
| *MW0378* | 0 | 0 | 0 | 0 | 0 | 0 | 0 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 1 | 0 | 1 | 1 | 1 | 0 | 0 | 0 | 0 | 0 | 1 | 1 | 1 | 1 | 0 |
| *SAV2596* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 0 | 0 | 0 | 1 | 0 | 0 | 0 | 0 | 0 | 0 | 0 | 0 | 1 | 1 | 0 | 0 | 0 |
| *ORF17* | 0 | 0 | 1 | 1 | 0 | 1 | 1 | 1 | 1 | 1 | 1 | 1 | 1 | 1 | 0 | 1 | 1 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 |
| *SAV0868* | 0 | 0 | 1 | 1 | 0 | 1 | 1 | 1 | 1 | 0 | 0 | 0 | 0 | 0 | 0 | 0 | 0 | 0 | 0 | 0 | 1 | 1 | 1 | 0 | 0 | 1 | 0 | 0 | 0 | 0 | 0 | 0 | 0 | 0 | 0 | 0 | 0 | 0 | 0 | 0 | 0 | 1 | 0 | 0 |
| *SA1829* | 1 | 1 | 1 | 1 | 1 | 1 | 1 | 1 | 1 | 0 | 0 | 0 | 0 | 0 | 1 | 0 | 0 | 0 | 1 | 1 | 0 | 0 | 0 | 0 | 0 | 0 | 1 | 1 | 0 | 0 | 0 | 0 | 0 | 1 | 0 | 0 | 0 | 0 | 0 | 0 | 0 | 0 | 0 | 1 |
| *agrC-N315* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 0 | 0 | 0 | 0 | 0 | 0 | 1 | 1 | 1 | 1 | 1 | 1 | 0 | 0 | 0 | 0 | 0 |
| *SAV0847* | 0 | 0 | 0 | 0 | 0 | 0 | 0 | 1 | 1 | 0 | 0 | 0 | 0 | 0 | 0 | 0 | 0 | 0 | 1 | 1 | 1 | 1 | 0 | 1 | 1 | 1 | 0 | 0 | 0 | 0 | 0 | 0 | 0 | 0 | 0 | 1 | 1 | 1 | 1 | 0 | 1 | 1 | 1 | 0 |
| *ORF44* | 1 | 1 | 0 | 0 | 0 | 0 | 1 | 1 | 0 | 1 | 1 | 1 | 1 | 1 | 0 | 1 | 1 | 1 | 0 | 0 | 0 | 0 | 0 | 0 | 0 | 0 | 0 | 1 | 0 | 1 | 0 | 0 | 1 | 1 | 0 | 0 | 0 | 0 | 0 | 1 | 0 | 1 | 1 | 0 |
| *coa-N315* | 0 | 1 | 0 | 0 | 1 | 0 | 0 | 1 | 0 | 0 | 0 | 0 | 0 | 0 | 1 | 0 | 0 | 1 | 1 | 1 | 1 | 1 | 1 | 1 | 1 | 1 | 1 | 0 | 1 | 0 | 0 | 0 | 0 | 0 | 0 | 1 | 1 | 1 | 0 | 1 | 0 | 0 | 0 | 0 |
| *ORF20* | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 1 | 0 | 0 | 0 | 0 | 0 | 0 | 0 | 0 | 0 | 1 | 1 | 1 | 1 | 1 |
| *ORF21* | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 1 | 0 | 0 | 0 | 0 | 0 | 0 | 0 | 0 | 0 | 1 | 1 | 1 | 1 | 1 |
| *agrB-N315* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 0 | 0 | 0 | 0 | 0 | 0 | 1 | 1 | 1 | 1 | 1 | 1 | 0 | 0 | 0 | 0 | 0 |
| *MW0355* | 1 | 1 | 1 | 1 | 1 | 1 | 1 | 0 | 0 | 0 | 0 | 0 | 0 | 0 | 1 | 1 | 1 | 1 | 0 | 0 | 0 | 0 | 0 | 0 | 0 | 0 | 0 | 1 | 1 | 0 | 1 | 1 | 1 | 1 | 0 | 0 | 0 | 0 | 0 | 0 | 0 | 1 | 1 | 0 |
| *set15* | 0 | 0 | 1 | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 0 | 1 | 1 | 0 | 1 | 0 | 1 | 0 | 0 | 0 | 0 | 0 | 0 | 1 | 1 | 1 |
| *MW0063* | 0 | 0 | 0 | 0 | 1 | 0 | 0 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 1 | 0 | 0 | 0 |
| *MW2612* | 1 | 1 | 1 | 1 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 1 | 0 | 1 | 1 | 0 | 0 | 0 | 0 | 0 | 1 | 1 | 1 | 1 | 1 |
| *ORF14* | 0 | 0 | 1 | 0 | 0 | 0 | 0 | 1 | 1 | 1 | 1 | 1 | 1 | 1 | 1 | 0 | 0 | 0 | 0 | 0 | 1 | 1 | 1 | 1 | 0 | 1 | 1 | 0 | 0 | 0 | 0 | 0 | 0 | 0 | 0 | 0 | 0 | 0 | 0 | 0 | 0 | 1 | 0 | 1 |
| *ORF10* | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 1 | 1 | 1 | 1 | 1 | 0 | 1 | 0 | 0 | 0 |
| *sasK(SAV2595)* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 0 | 1 | 1 | 0 | 0 | 0 | 0 | 0 | 1 | 1 | 1 | 1 | 0 |
| *ORF11* | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 1 | 1 | 1 | 1 | 1 | 0 | 1 | 0 | 0 | 0 |
| *SA1640* | 0 | 0 | 0 | 0 | 0 | 0 | 0 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 1 | 1 | 0 | 0 | 0 |
| *cna* | 1 | 1 | 1 | 1 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 1 | 0 | 1 | 1 | 0 | 0 | 0 | 0 | 0 | 1 | 1 | 1 | 1 | 1 |
| *ORF13* | 0 | 0 | 1 | 0 | 0 | 0 | 0 | 1 | 1 | 1 | 1 | 1 | 1 | 1 | 1 | 0 | 0 | 0 | 0 | 0 | 1 | 1 | 1 | 1 | 0 | 1 | 1 | 0 | 0 | 0 | 0 | 0 | 0 | 0 | 0 | 0 | 0 | 0 | 0 | 0 | 0 | 1 | 0 | 1 |
| *SA1636* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 1 | 0 | 0 | 0 |
| *MW1408* | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 1 | 1 | 1 | 1 | 1 | 0 | 1 | 0 | 0 | 0 |
| *MW0053* | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 1 | 0 | 0 | 1 | 1 | 0 | 0 | 0 | 0 | 0 | 0 | 0 | 0 | 1 | 1 | 1 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 | 32 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *aadD* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 |
| *MW1405* | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 1 | 1 | 1 | 1 | 1 | 0 | 1 | 0 | 0 | 0 |
| *ble* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 |
| *qacA* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *SAV0865* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *SA1635* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 0 | 0 | 0 |
| *ccrB1* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *MW0045* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *ccrA1* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *MW1386* | 1 | 0 | 0 | 0 | 0 | 0 | 0 | 1 | 0 | 0 | 0 | 0 | 0 | 0 | 0 | 1 | 1 | 0 | 0 | 0 | 0 | 0 | 0 | 0 | 0 | 1 | 0 | 0 | 1 | 1 | 1 | 0 | 0 | 0 | 1 | 1 | 1 | 1 | 1 | 0 | 1 | 0 | 0 | 0 |
| *ORF7* | 1 | 0 | 0 | 0 | 0 | 0 | 0 | 1 | 0 | 0 | 0 | 0 | 0 | 0 | 0 | 1 | 1 | 0 | 0 | 0 | 0 | 0 | 0 | 0 | 0 | 1 | 0 | 0 | 1 | 1 | 1 | 0 | 0 | 0 | 1 | 1 | 1 | 1 | 1 | 0 | 1 | 0 | 0 | 0 |
| *ORF8* | 1 | 0 | 0 | 0 | 0 | 0 | 0 | 1 | 0 | 0 | 0 | 0 | 0 | 0 | 0 | 1 | 1 | 0 | 0 | 0 | 0 | 0 | 0 | 0 | 0 | 1 | 0 | 0 | 1 | 1 | 1 | 0 | 0 | 0 | 1 | 1 | 1 | 1 | 1 | 0 | 1 | 0 | 0 | 0 |
| *ORF12* | 1 | 0 | 1 | 0 | 0 | 1 | 1 | 0 | 0 | 0 | 0 | 0 | 0 | 0 | 1 | 1 | 1 | 1 | 0 | 0 | 0 | 0 | 0 | 0 | 0 | 1 | 1 | 0 | 1 | 1 | 1 | 0 | 0 | 0 | 1 | 1 | 1 | 1 | 1 | 1 | 1 | 1 | 1 | 0 |
| *MW1393* | 1 | 0 | 0 | 0 | 0 | 0 | 0 | 1 | 0 | 0 | 0 | 0 | 0 | 0 | 0 | 1 | 1 | 0 | 0 | 0 | 0 | 0 | 0 | 0 | 0 | 1 | 0 | 0 | 1 | 1 | 1 | 0 | 0 | 0 | 1 | 1 | 1 | 1 | 1 | 0 | 1 | 0 | 0 | 0 |
| *SAV0909* | 0 | 0 | 1 | 0 | 0 | 0 | 0 | 1 | 1 | 1 | 1 | 1 | 1 | 1 | 1 | 0 | 0 | 0 | 0 | 0 | 1 | 1 | 0 | 1 | 1 | 1 | 1 | 0 | 0 | 0 | 0 | 0 | 0 | 0 | 0 | 0 | 0 | 0 | 0 | 0 | 0 | 1 | 1 | 1 |
| *CZ 040* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *ORF9* | 1 | 0 | 0 | 0 | 0 | 0 | 0 | 1 | 0 | 0 | 0 | 0 | 0 | 0 | 0 | 1 | 1 | 0 | 0 | 0 | 0 | 0 | 0 | 0 | 0 | 1 | 0 | 0 | 1 | 1 | 1 | 0 | 0 | 0 | 1 | 1 | 1 | 1 | 1 | 0 | 1 | 0 | 0 | 0 |
| *CZ 041* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *MW1401* | 1 | 0 | 0 | 0 | 0 | 0 | 0 | 1 | 0 | 0 | 0 | 0 | 0 | 0 | 1 | 1 | 1 | 0 | 0 | 0 | 0 | 0 | 0 | 0 | 0 | 1 | 0 | 0 | 1 | 1 | 1 | 0 | 0 | 0 | 1 | 1 | 1 | 1 | 1 | 0 | 1 | 0 | 0 | 0 |
| *SA1341* | 1 | 1 | 1 | 1 | 1 | 1 | 1 | 0 | 0 | 0 | 0 | 0 | 0 | 0 | 0 | 1 | 1 | 1 | 1 | 1 | 1 | 1 | 1 | 1 | 1 | 1 | 1 | 0 | 1 | 0 | 0 | 0 | 1 | 0 | 1 | 1 | 1 | 1 | 1 | 0 | 0 | 0 | 0 | 0 |
| *SAV0903* | 0 | 0 | 1 | 0 | 0 | 0 | 0 | 1 | 1 | 1 | 1 | 1 | 1 | 1 | 1 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 0 | 1 | 1 | 1 |
| *ORF6* | 1 | 0 | 0 | 0 | 0 | 0 | 0 | 1 | 0 | 0 | 0 | 0 | 0 | 0 | 0 | 1 | 1 | 0 | 0 | 0 | 0 | 0 | 0 | 0 | 0 | 1 | 0 | 0 | 1 | 1 | 1 | 0 | 0 | 0 | 1 | 1 | 1 | 1 | 1 | 1 | 1 | 0 | 0 | 0 |
| *SAV0902* | 0 | 0 | 1 | 0 | 0 | 0 | 0 | 1 | 1 | 1 | 1 | 1 | 1 | 1 | 1 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 0 | 1 | 1 | 1 |
| *SAV0901* | 0 | 0 | 1 | 0 | 0 | 0 | 0 | 1 | 1 | 1 | 1 | 1 | 1 | 1 | 1 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 0 | 1 | 1 | 1 |
| *ORF25* | 0 | 0 | 1 | 0 | 0 | 0 | 0 | 1 | 1 | 1 | 1 | 1 | 1 | 1 | 1 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 0 | 1 | 1 | 1 |
| *SAV0898* | 0 | 0 | 1 | 0 | 0 | 0 | 0 | 1 | 1 | 1 | 1 | 1 | 1 | 1 | 1 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 0 | 1 | 1 | 1 |
| *SAV0890* | 0 | 0 | 1 | 0 | 0 | 0 | 0 | 1 | 1 | 1 | 1 | 1 | 1 | 1 | 1 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 0 | 1 | 1 | 1 |
| *SAV0888* | 0 | 0 | 1 | 0 | 0 | 0 | 0 | 1 | 1 | 1 | 1 | 1 | 1 | 1 | 1 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 0 | 1 | 1 | 1 |
| *MW1400* | 1 | 0 | 0 | 0 | 0 | 0 | 0 | 1 | 0 | 0 | 0 | 0 | 0 | 0 | 1 | 1 | 1 | 0 | 0 | 0 | 0 | 0 | 0 | 0 | 0 | 1 | 0 | 0 | 1 | 1 | 1 | 0 | 0 | 0 | 1 | 1 | 1 | 1 | 1 | 0 | 1 | 0 | 0 | 0 |
| *SA1622* | 1 | 1 | 0 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 0 | 1 | 0 | 0 | 0 | 0 | 0 | 0 | 0 | 0 | 0 | 0 | 1 | 1 | 0 | 0 | 0 |
| *SAV0887* | 0 | 0 | 1 | 0 | 0 | 0 | 0 | 1 | 1 | 1 | 1 | 1 | 1 | 1 | 1 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 0 | 1 | 1 | 1 |
| *pro 8325* | 1 | 0 | 0 | 0 | 0 | 0 | 0 | 1 | 0 | 0 | 0 | 0 | 0 | 0 | 1 | 1 | 1 | 0 | 0 | 0 | 0 | 0 | 0 | 0 | 0 | 1 | 0 | 0 | 1 | 1 | 1 | 1 | 0 | 0 | 1 | 1 | 1 | 1 | 1 | 0 | 1 | 0 | 0 | 0 |
| *SAV0406* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SAV0404* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SAV0414* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SAV0403* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SAV0415* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SAV0413* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 | 32 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *SAV0402* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *repB* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 0 | 1 | 0 | 0 | 0 | 0 | 0 | 0 | 0 | 0 | 0 | 0 | 0 | 0 | 0 | 0 | 0 | 0 | 0 | 0 |
| *SAV0410* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SAV0401* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SAV0409* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SAV0400* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *aacA-aphD* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SAV0408* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SAV0399* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *MW1197* | 0 | 1 | 1 | 0 | 1 | 1 | 1 | 1 | 1 | 1 | 1 | 1 | 1 | 1 | 1 | 1 | 1 | 1 | 0 | 1 | 0 | 0 | 0 | 0 | 0 | 0 | 0 | 1 | 0 | 1 | 0 | 1 | 1 | 1 | 1 | 1 | 1 | 1 | 1 | 0 | 0 | 1 | 0 | 0 |
| *SAV0405* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *Int459(SAV0392)* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *tnpA (Tn554)* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SAV0599* | 1 | 1 | 1 | 1 | 1 | 1 | 1 | 1 | 1 | 1 | 1 | 1 | 1 | 1 | 0 | 0 | 0 | 0 | 1 | 1 | 1 | 1 | 1 | 1 | 1 | 1 | 1 | 1 | 0 | 1 | 0 | 0 | 0 | 0 | 0 | 0 | 0 | 0 | 0 | 1 | 1 | 1 | 1 | 0 |
| *cap8K-like* | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 1 | 1 | 1 | 1 |
| *cap8J* | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 1 | 1 | 1 | 1 |
| *tnp480 (Tn552)* | 1 | 1 | 1 | 0 | 1 | 1 | 1 | 0 | 0 | 0 | 0 | 0 | 0 | 0 | 1 | 0 | 0 | 0 | 1 | 1 | 1 | 0 | 0 | 0 | 0 | 0 | 0 | 1 | 1 | 1 | 1 | 1 | 1 | 0 | 1 | 1 | 1 | 1 | 1 | 1 | 0 | 0 | 0 | 1 |
| *MW2564* | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 1 | 1 | 1 | 1 |
| *cap8I* | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 1 | 1 | 1 | 1 |
| *cap8H* | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 1 | 1 | 1 | 1 |
| *SAV0395* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *ORF22* | 0 | 0 | 1 | 1 | 0 | 1 | 1 | 1 | 1 | 1 | 1 | 1 | 1 | 1 | 0 | 0 | 0 | 0 | 0 | 0 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 1 |
| *spc* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SA0047* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *SA2259* | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 0 | 0 | 1 | 0 | 0 | 1 | 0 | 0 | 0 | 0 | 0 | 0 | 1 | 1 | 0 | 0 | 0 |
| *ORF32* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 0 | 1 | 0 | 0 | 0 | 0 | 0 | 0 | 0 | 0 | 0 | 0 | 0 | 0 | 0 | 0 | 0 | 0 | 0 | 0 |
| *tnpB (Tn554)* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *seg* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 1 | 1 | 0 | 0 | 0 |
| *SA1641* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 1 | 1 | 0 | 0 | 0 |
| *sei* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 1 | 1 | 0 | 0 | 0 |
| *tnp IS 256* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *seo* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 1 | 1 | 0 | 0 | 0 |
| *ermA* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *sem* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 1 | 1 | 0 | 0 | 0 |
| *ORF16* | 0 | 0 | 0 | 0 | 1 | 0 | 0 | 1 | 0 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *sen* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 1 | 1 | 0 | 0 | 0 |
| *SAV0850* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 1 | 0 | 0 | 1 | 0 | 0 | 0 | 0 | 0 | 1 | 1 | 1 | 1 | 1 | 0 | 1 | 0 | 0 | 0 |
| *SA0054* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 0 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 | 32 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *ORF26* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 1 | 0 | 0 | 1 | 1 | 0 | 0 | 0 | 0 | 1 | 1 | 1 | 1 | 1 | 0 | 1 | 0 | 0 | 0 |
| *ORF5* | 0 | 0 | 1 | 1 | 0 | 0 | 0 | 1 | 0 | 0 | 0 | 0 | 0 | 0 | 0 | 1 | 1 | 1 | 0 | 0 | 0 | 0 | 0 | 0 | 0 | 1 | 0 | 0 | 1 | 1 | 1 | 0 | 0 | 0 | 1 | 1 | 1 | 1 | 1 | 1 | 1 | 0 | 0 | 1 |
| *coa-8325* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *MW1749* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 1 |
| *SA0024* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 1 |
| *SAV0905* | 0 | 0 | 0 | 1 | 0 | 0 | 0 | 0 | 0 | 0 | 0 | 0 | 0 | 0 | 1 | 1 | 1 | 1 | 1 | 1 | 1 | 1 | 0 | 1 | 0 | 1 | 0 | 0 | 0 | 0 | 0 | 0 | 0 | 0 | 0 | 0 | 0 | 0 | 0 | 0 | 1 | 0 | 0 | 0 |
| *SA0142* | 1 | 1 | 1 | 1 | 1 | 1 | 1 | 0 | 0 | 0 | 0 | 0 | 0 | 0 | 1 | 1 | 1 | 1 | 1 | 1 | 1 | 1 | 1 | 1 | 1 | 1 | 1 | 0 | 0 | 0 | 0 | 1 | 1 | 1 | 1 | 1 | 1 | 1 | 1 | 1 | 1 | 1 | 1 | 1 |
| *SAV2001* | 1 | 1 | 0 | 1 | 0 | 0 | 0 | 0 | 1 | 0 | 0 | 0 | 0 | 0 | 0 | 0 | 0 | 0 | 1 | 1 | 1 | 1 | 1 | 1 | 1 | 1 | 0 | 1 | 0 | 0 | 0 | 0 | 0 | 1 | 0 | 0 | 0 | 0 | 0 | 0 | 0 | 0 | 0 | 0 |
| *SA0141* | 1 | 1 | 1 | 1 | 1 | 1 | 1 | 0 | 0 | 0 | 0 | 0 | 0 | 0 | 1 | 1 | 1 | 1 | 1 | 1 | 1 | 1 | 1 | 1 | 1 | 1 | 1 | 0 | 0 | 0 | 0 | 1 | 1 | 1 | 1 | 1 | 1 | 1 | 1 | 1 | 1 | 1 | 1 | 1 |
| *tnpIS 1272* | 0 | 0 | 1 | 0 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 1 | 0 | 0 | 0 | 0 | 0 | 0 | 0 | 0 | 0 |
| *SA1826* | 1 | 1 | 1 | 1 | 1 | 1 | 1 | 1 | 1 | 0 | 0 | 0 | 0 | 0 | 1 | 0 | 0 | 0 | 1 | 1 | 0 | 0 | 0 | 0 | 0 | 0 | 1 | 1 | 0 | 0 | 0 | 0 | 1 | 1 | 0 | 0 | 0 | 0 | 0 | 1 | 0 | 0 | 0 | 1 |
| *SAV0039* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 1 |
| *ORF18* | 1 | 1 | 1 | 1 | 0 | 0 | 0 | 1 | 1 | 1 | 1 | 1 | 1 | 1 | 1 | 0 | 0 | 0 | 1 | 1 | 1 | 1 | 1 | 1 | 1 | 1 | 1 | 0 | 0 | 1 | 0 | 0 | 0 | 0 | 0 | 0 | 0 | 0 | 0 | 0 | 0 | 1 | 1 | 1 |
| *mecA* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 1 |
| *SA1822* | 1 | 1 | 1 | 1 | 1 | 1 | 1 | 1 | 1 | 0 | 0 | 0 | 0 | 0 | 1 | 0 | 0 | 0 | 1 | 1 | 0 | 0 | 0 | 0 | 0 | 0 | 1 | 1 | 0 | 0 | 0 | 0 | 1 | 1 | 0 | 0 | 0 | 0 | 0 | 1 | 0 | 0 | 0 | 1 |
| *MW0065* | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *mecR1-2* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 1 |
| *MW0066* | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *MW1750* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 1 |
| *tetM* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 0 | 1 | 1 | 0 | 0 | 0 | 0 | 0 | 0 | 0 | 0 | 0 | 0 | 0 | 0 | 0 | 0 | 0 | 0 | 1 |
| *MW0120* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 0 | 0 | 0 |
| *MW1757* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 1 | 1 |
| *ORF24* | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *ORF29* | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *ORF38* | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 |
| *MW0560* | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 0 | 0 | 1 | 1 | 0 | 0 | 0 | 0 | 0 | 0 | 0 | 0 | 0 | 0 | 0 |
| *entA = entP* | 1 | 1 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 1 | 1 |
| *bsaC* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 | 1 |
| *bsaB* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 | 1 |
| *bsaG* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 | 1 |
| *bsaE* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 | 1 |
| *bsaF* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 | 1 |
| *SA1806* | 1 | 1 | 0 | 1 | 0 | 0 | 0 | 0 | 1 | 0 | 0 | 0 | 0 | 0 | 0 | 1 | 1 | 0 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 | 0 |
| *bsaP* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 | 1 |
| *bsaD* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 | 1 |
| *MW1908* | 0 | 1 | 1 | 1 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 1 | 0 | 0 | 0 | 0 | 0 | 0 | 0 | 0 | 0 | 1 | 1 | 1 | 1 | 1 |
| *MW1898* | 0 | 1 | 1 | 1 | 0 | 1 | 1 | 0 | 0 | 0 | 0 | 0 | 0 | 0 | 0 | 0 | 0 | 0 | 0 | 0 | 0 | 0 | 1 | 0 | 0 | 0 | 1 | 0 | 1 | 1 | 0 | 0 | 0 | 0 | 0 | 0 | 0 | 0 | 0 | 1 | 1 | 1 | 1 | 1 |
| *MW2125* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 1 | 1 | 1 | 1 | 1 | 0 | 0 | 1 | 1 | 0 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 | 32 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *MW2534* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 1 | 1 | 1 | 1 | 1 | 0 | 0 | 1 | 1 | 0 |
| *MW0062* | 0 | 0 | 0 | 0 | 1 | 0 | 0 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 1 | 0 | 0 | 0 |
| *ORF36* | 0 | 0 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 0 |
| *ORF19* | 0 | 1 | 1 | 1 | 0 | 1 | 1 | 0 | 0 | 0 | 0 | 0 | 0 | 0 | 0 | 0 | 0 | 0 | 0 | 0 | 0 | 0 | 1 | 0 | 0 | 0 | 1 | 0 | 1 | 1 | 0 | 0 | 0 | 0 | 0 | 0 | 0 | 0 | 0 | 1 | 1 | 1 | 1 | 1 |
| *CAC3531* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 1 | 1 | 1 | 1 | 1 | 0 | 0 | 0 | 0 | 1 |
| *MW0552* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 0 | 0 | 0 | 0 | 1 |
| *agrB-8325* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 0 | 0 | 0 | 0 | 1 | 1 | 0 | 0 | 1 |
| *ORF39* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 0 | 0 | 0 | 0 | 1 | 1 | 0 | 0 | 1 |
| *ORF28* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 0 | 0 | 0 | 0 | 1 | 1 | 0 | 0 | 1 |
| *ORF40* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 0 | 0 | 0 | 0 | 1 | 1 | 0 | 0 | 1 |
| *MW1744* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 1 | 1 | 1 | 1 | 0 | 0 | 0 | 0 | 0 | 1 | 1 | 1 |
| *sigB* | 1 | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *ORF41* | 1 | 1 | 1 | 1 | 1 | 1 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 1 | 0 | 1 | 1 | 1 | 1 | 1 | 0 | 0 | 0 | 0 | 1 |
| *ORF31* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 |
| *MW0064* | 0 | 0 | 0 | 0 | 1 | 0 | 0 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 1 | 0 | 0 | 0 |
| *MW0558* | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 0 | 0 | 0 | 0 | 1 |
| *SA0392* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 0 | 0 | 0 | 1 | 1 | 0 | 1 | 0 | 0 | 0 | 0 | 0 | 1 | 1 | 0 | 0 | 1 |
| *SAV0804* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 1 | 1 | 0 | 1 | 0 | 0 | 0 | 1 | 1 | 1 | 1 | 1 | 0 | 0 | 1 | 1 | 1 |
| *SAV0803* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 1 | 1 | 0 | 1 | 0 | 0 | 0 | 1 | 1 | 1 | 1 | 1 | 0 | 0 | 1 | 1 | 1 |
| *MW0554* | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 1 | 0 | 0 | 0 | 1 | 1 | 1 | 1 | 1 | 1 | 1 | 0 | 0 | 0 | 0 | 1 |
| *bbp=sdrE* | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 0 | 1 | 1 | 1 | 1 | 1 | 0 | 0 | 1 | 1 | 1 | 1 | 1 | 1 | 1 | 1 | 0 | 1 |
| *MW0553* | 0 | 0 | 0 | 0 | 0 | 0 | 0 | 0 | 0 | 0 | 0 | 0 | 0 | 0 | 1 | 1 | 1 | 1 | 0 | 0 | 0 | 0 | 0 | 0 | 0 | 0 | 0 | 0 | 1 | 0 | 0 | 0 | 1 | 1 | 1 | 1 | 1 | 1 | 1 | 0 | 0 | 0 | 0 | 1 |
| *ORF35* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 1 | 1 | 0 | 1 | 1 | 1 | 1 | 1 | 1 | 1 | 1 | 1 | 1 | 1 | 0 |
| *MW0559* | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 0 | 0 | 0 | 0 | 1 |
| *ORF34* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 1 | 1 | 0 | 1 | 1 | 1 | 1 | 1 | 1 | 1 | 1 | 1 | 1 | 1 | 0 |
| *MW0919* | 1 | 1 | 1 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 1 | 1 | 0 | 1 | 1 | 0 | 0 | 0 | 1 |
| *cap5j* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 0 | 1 | 1 | 1 | 0 | 1 | 1 | 0 | 0 | 0 | 0 | 0 | 0 | 0 | 0 | 0 | 0 | 0 | 0 |
| *ORF45* | 0 | 0 | 0 | 0 | 1 | 0 | 0 | 1 | 0 | 0 | 0 | 0 | 0 | 0 | 1 | 1 | 1 | 1 | 1 | 1 | 1 | 1 | 0 | 1 | 0 | 1 | 0 | 0 | 0 | 0 | 0 | 0 | 0 | 1 | 0 | 1 | 1 | 1 | 1 | 0 | 0 | 1 | 1 | 0 |
| *aml 8325* | 0 | 0 | 1 | 1 | 1 | 1 | 1 | 1 | 1 | 1 | 1 | 1 | 1 | 1 | 1 | 0 | 0 | 0 | 0 | 0 | 1 | 1 | 1 | 1 | 0 | 1 | 1 | 0 | 0 | 0 | 0 | 0 | 0 | 0 | 0 | 0 | 0 | 0 | 0 | 0 | 1 | 1 | 0 | 1 |
| *cap5H* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 1 | 1 | 0 | 0 | 0 | 0 | 0 | 0 | 0 | 0 | 0 | 0 | 0 | 0 |
| *SA2483* | 0 | 0 | 0 | 0 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 0 | 1 | 1 | 1 | 0 | 0 | 0 | 0 | 0 | 0 | 0 | 0 | 0 | 0 | 0 | 0 | 0 |
| *ORF37* | 1 | 1 | 1 | 1 | 1 | 1 | 1 | 1 | 1 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 1 | 1 | 0 | 0 | 0 | 0 | 0 | 0 | 0 | 1 | 1 | 0 |
| *tnp IS 1181* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 0 | 0 | 0 | 0 | 0 | 0 | 0 | 0 | 0 | 0 | 0 | 1 | 1 | 1 | 1 | 1 |
| *phi int12* | 1 | 0 | 1 | 1 | 0 | 1 | 1 | 1 | 0 | 0 | 0 | 0 | 0 | 0 | 1 | 1 | 1 | 0 | 0 | 0 | 0 | 0 | 1 | 0 | 0 | 1 | 0 | 0 | 1 | 1 | 1 | 0 | 0 | 0 | 1 | 1 | 1 | 1 | 1 | 0 | 0 | 0 | 0 | 0 |
| *cap5K* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 1 | 1 | 0 | 0 | 0 | 0 | 0 | 0 | 0 | 0 | 0 | 0 | 0 | 0 |
| *SAV0912* | 0 | 0 | 1 | 1 | 1 | 1 | 1 | 1 | 1 | 1 | 1 | 1 | 1 | 1 | 1 | 0 | 0 | 0 | 0 | 0 | 1 | 1 | 1 | 1 | 0 | 1 | 1 | 0 | 0 | 0 | 0 | 0 | 0 | 0 | 0 | 0 | 0 | 0 | 0 | 0 | 1 | 1 | 0 | 1 |
| *cap5i* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 1 | 1 | 0 | 0 | 0 | 0 | 0 | 0 | 0 | 0 | 0 | 0 | 0 | 0 |
| *SA1791* | 1 | 1 | 1 | 1 | 0 | 0 | 0 | 0 | 1 | 1 | 1 | 1 | 1 | 1 | 0 | 0 | 0 | 1 | 0 | 0 | 1 | 0 | 0 | 0 | 1 | 0 | 1 | 1 | 0 | 1 | 1 | 0 | 0 | 1 | 0 | 0 | 0 | 0 | 0 | 0 | 0 | 1 | 1 | 1 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 | 32 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *ORF3* | 0 | 0 | 0 | 0 | 0 | 0 | 0 | 0 | 0 | 0 | 0 | 0 | 0 | 0 | 1 | 0 | 0 | 0 | 1 | 1 | 1 | 1 | 1 | 1 | 1 | 1 | 1 | 1 | 0 | 0 | 0 | 0 | 0 | 0 | 0 | 0 | 0 | 0 | 0 | 1 | 1 | 1 | 1 | 1 |
| *tnp IS 431* | 1 | 0 | 1 | 1 | 1 | 1 | 1 | 0 | 0 | 0 | 0 | 0 | 0 | 0 | 0 | 1 | 1 | 1 | 1 | 0 | 1 | 0 | 0 | 1 | 0 | 0 | 0 | 1 | 0 | 1 | 0 | 0 | 0 | 0 | 0 | 0 | 0 | 0 | 0 | 1 | 0 | 0 | 0 | 1 |
| *agrC-8325* | 0 | 0 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 1 | 1 | 0 | 1 | 1 | 1 | 0 | 0 | 0 | 0 | 0 | 0 | 0 | 1 | 1 | 1 | 1 |
| *sarH2* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 0 | 1 | 0 | 0 | 0 | 0 | 1 | 1 | 1 | 1 | 1 | 0 | 0 | 1 | 1 | 1 |
| *splE* | 1 | 1 | 1 | 1 | 1 | 1 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 0 | 0 | 1 | 1 | 1 |
| *MW1742* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 1 | 0 | 1 | 0 | 1 | 1 | 1 | 1 | 1 | 1 | 1 | 1 | 1 | 1 | 1 | 1 | 1 |
| *SA1755* | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 0 | 0 | 0 | 0 | 0 | 1 | 1 | 1 | 1 | 1 | 0 | 0 | 0 | 0 | 0 |
| *MW906* | 1 | 1 | 1 | 1 | 1 | 1 | 1 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 1 | 0 | 0 | 1 | 1 | 1 |
| *SA1320* | 0 | 0 | 0 | 0 | 0 | 0 | 0 | 0 | 0 | 0 | 0 | 0 | 0 | 0 | 1 | 1 | 1 | 0 | 1 | 1 | 1 | 1 | 1 | 1 | 1 | 1 | 1 | 1 | 0 | 0 | 1 | 1 | 0 | 0 | 1 | 1 | 1 | 1 | 1 | 0 | 0 | 0 | 0 | 1 |
| *SA0104* | 0 | 0 | 0 | 0 | 0 | 0 | 0 | 1 | 1 | 1 | 1 | 1 | 1 | 1 | 0 | 0 | 0 | 0 | 1 | 1 | 1 | 1 | 1 | 1 | 1 | 1 | 1 | 1 | 1 | 0 | 0 | 0 | 1 | 1 | 0 | 0 | 0 | 0 | 0 | 1 | 1 | 1 | 1 | 0 |
| *lukD* | 0 | 0 | 0 | 0 | 0 | 0 | 0 | 0 | 0 | 0 | 0 | 0 | 0 | 0 | 1 | 0 | 0 | 0 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 |
| *SA0103* | 0 | 0 | 0 | 0 | 0 | 0 | 0 | 1 | 1 | 1 | 1 | 1 | 1 | 1 | 0 | 0 | 0 | 0 | 1 | 1 | 1 | 1 | 1 | 1 | 1 | 1 | 1 | 1 | 1 | 0 | 0 | 0 | 1 | 1 | 0 | 0 | 0 | 0 | 0 | 1 | 1 | 1 | 1 | 0 |
| *splB* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 |
| *SA1768* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 | 1 |
| *splA* | 0 | 0 | 0 | 0 | 0 | 0 | 0 | 0 | 0 | 0 | 0 | 0 | 0 | 0 | 1 | 0 | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 |
| *ORF2* | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 1 | 0 | 1 | 1 | 1 | 1 | 1 | 0 | 0 | 1 | 1 | 1 |
| *SA1766* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 | 1 |
| *SA1778* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 | 1 |
| *SA1777* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 | 1 |
| *ORF42* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 0 | 1 | 0 | 1 | 1 | 1 | 1 | 1 | 0 | 0 | 1 | 1 | 1 |
| *ORF27* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 | 1 |
| *SA1775* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 | 1 |
| *SA1774* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 | 1 |
| *sdrD* | 1 | 1 | 1 | 1 | 1 | 0 | 0 | 0 | 0 | 0 | 0 | 0 | 0 | 0 | 0 | 1 | 1 | 0 | 1 | 1 | 1 | 1 | 1 | 1 | 1 | 1 | 0 | 1 | 1 | 1 | 1 | 1 | 0 | 0 | 1 | 1 | 1 | 1 | 1 | 0 | 1 | 1 | 0 | 1 |
| *set 9* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 |
| *ORF15* | 1 | 1 | 1 | 1 | 0 | 1 | 1 | 1 | 0 | 1 | 1 | 1 | 1 | 1 | 0 | 1 | 1 | 0 | 1 | 1 | 1 | 1 | 1 | 1 | 1 | 1 | 0 | 0 | 0 | 1 | 1 | 0 | 0 | 0 | 0 | 0 | 0 | 0 | 0 | 0 | 0 | 0 | 0 | 1 |
| *set12* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 |
| *SA1795* | 1 | 1 | 1 | 1 | 0 | 0 | 0 | 0 | 1 | 1 | 1 | 1 | 1 | 1 | 0 | 1 | 1 | 1 | 1 | 1 | 1 | 1 | 1 | 1 | 1 | 1 | 1 | 1 | 0 | 1 | 1 | 0 | 0 | 1 | 0 | 0 | 0 | 0 | 0 | 0 | 0 | 1 | 1 | 1 |
| *set14* | 0 | 0 | 0 | 1 | 1 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 0 | 1 | 1 | 0 | 1 | 1 | 1 | 1 | 1 | 1 | 1 | 0 | 0 | 1 | 1 | 1 |
| *lukM* | 0 | 0 | 0 | 1 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 |
| *set 8* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 |
| *MW1769* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 |
| *SA1010* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 |
| *SAV0910* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 1 | 1 | 1 | 1 | 0 | 1 | 1 | 1 | 1 |
| *SA2272* | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 1 | 1 | 1 | 1 | 1 | 1 | 1 | 1 | 1 | 0 |
| *SAV0904* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 1 | 1 | 1 | 1 | 0 | 1 | 1 | 1 | 1 |
| *splD splF* | 1 | 0 | 1 | 1 | 1 | 1 | 1 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 |
| *splC* | 1 | 1 | 1 | 1 | 1 | 1 | 1 | 0 | 0 | 0 | 0 | 0 | 0 | 0 | 1 | 0 | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 | 32 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *phi int13* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 0 | 0 | 1 | 0 | 0 | 0 | 0 | 0 | 1 | 1 | 1 | 1 | 1 |
| *SAV0866* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 0 | 0 | 0 | 0 | 0 | 0 | 1 | 1 | 1 | 1 | 1 | 1 | 1 | 1 | 1 |
| *ORF33* | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 1 | 0 | 1 | 1 | 1 | 1 | 1 | 1 | 1 | 1 | 1 | 1 | 1 | 1 | 1 | 1 | 1 |
| *MW2409* | 1 | 1 | 1 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 1 | 0 | 1 | 1 | 0 | 0 | 1 | 0 | 0 | 0 | 0 | 0 | 0 | 0 | 1 | 1 | 1 |
| *fosB* | 1 | 1 | 1 | 1 | 1 | 1 | 1 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 0 | 0 | 0 | 0 | 1 | 1 | 1 | 1 | 1 | 1 | 1 | 1 | 1 | 1 | 1 |
| *SA1801* | 1 | 1 | 1 | 1 | 1 | 1 | 1 | 0 | 1 | 0 | 0 | 0 | 0 | 0 | 0 | 1 | 1 | 1 | 1 | 1 | 1 | 1 | 1 | 1 | 1 | 1 | 1 | 1 | 1 | 0 | 1 | 0 | 0 | 1 | 0 | 1 | 1 | 1 | 1 | 1 | 1 | 1 | 1 | 1 |
| *SA0378* | 1 | 1 | 1 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 0 | 0 | 0 | 0 | 1 | 0 | 0 | 1 | 1 | 1 | 1 | 1 | 1 | 1 | 0 | 0 | 1 |
| *SA0377* | 1 | 1 | 1 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 0 | 0 | 0 | 0 | 1 | 0 | 0 | 1 | 1 | 1 | 1 | 1 | 1 | 1 | 0 | 0 | 1 |
| *SA2004* | 1 | 1 | 1 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 0 | 1 | 1 | 0 | 1 | 0 | 0 | 1 | 1 | 1 | 1 | 1 | 0 | 0 | 0 | 0 | 1 |
| *map* | 0 | 0 | 1 | 1 | 1 | 0 | 0 | 1 | 1 | 1 | 0 | 0 | 0 | 1 | 0 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 |
| *SA2005* | 1 | 1 | 1 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 0 | 1 | 1 | 1 | 1 | 0 | 0 | 1 | 1 | 1 | 1 | 1 | 0 | 0 | 0 | 0 | 1 |
| *sak* | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 0 | 0 | 1 | 0 | 0 | 0 | 0 | 0 | 1 | 1 | 1 | 1 | 1 |
| *blaZ* | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 1 | 0 | 0 | 1 | 1 | 0 | 1 | 1 | 1 | 1 | 1 | 1 | 1 | 1 | 1 | 1 | 0 | 1 | 1 | 1 |
| *fnbB* | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 1 | 1 | 1 | 1 | 1 |
| *set10* | 0 | 0 | 0 | 0 | 1 | 0 | 0 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 |
| *SA2314* | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *emp* | 1 | 1 | 1 | 1 | 1 | 1 | 1 | 1 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *vwb* | 1 | 1 | 1 | 1 | 1 | 1 | 1 | 0 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SAV1974* | 1 | 1 | 1 | 1 | 1 | 1 | 1 | 1 | 0 | 1 | 1 | 1 | 1 | 1 | 0 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 | 0 | 1 | 1 | 1 |
| *SAV1392* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0276* | 1 | 1 | 1 | 1 | 1 | 1 | 1 | 1 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *vraF(SA0616)* | 1 | 1 | 1 | 1 | 1 | 1 | 1 | 1 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0745* | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *agrC* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 |
| *set13* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 |
| *hlb* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *set 6* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *efb=fib* | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 |
| *set11* | 1 | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *rpoD* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *ssaA (SA2093)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *radA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA1725* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fhuD=fhuG(SA0604)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fmtC (mprF)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fus(SA0505)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fnbA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *nuc SA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 | 32 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| set 7 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| vraR(SA1700) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| geh | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| sdrC | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| SA0022 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| sbi(SA2206) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| dltA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| lexA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| ORF41 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| rsbU | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| dnaK | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| sacP(SA0295) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| agrA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| SA0610 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| hla | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| SA1552 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| srtA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| hySA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| ebpS | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| femB | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| lytS+lytR | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| rnc(SA1076) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| hlgC | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| SA0587 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| hlgA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| aur(SA2430) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| fmtB (mrp) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| sspB (SA0900) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| sspA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| coa | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| SAV2170 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| femA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| SA0675 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| SA0746 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| hlgB | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| lip(SA2463) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| SA0423 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| sodM | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 | 32 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *atl-amidase* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0977* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fabH* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA1970* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA1852* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fhuD2(SA2079)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0102* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *clfA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *atl-glucosamin* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *hsdM* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *clpL* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *clpB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *recA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *agrJ* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA1580* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fhuB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *spa* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0691* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *icaA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *clfB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0704* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *mdr* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *norA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fur* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fhuA=fhuC(SA0602)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *putB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *plc* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *icaC* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *htrA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *ORF30* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |

| Clusters | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|
| Gene | BM12755* | IPF505* | IPF147 | IPF157 | BM12942 | IPF083* | 97233* | 93184* | BM12714* | BM12828* | BM12830* | IPF555* & ° | BM12612* | IPF557* & ° | IPF562* & ° | BM10828* | SpainE1* | 96145* | BM10829* | BM10138* | BM10130* | FinlandE7 | BM10896* | 97130* | BM9290* | BM9343* | BM9586* & ° | BM12184* & ° | BM12188* & ° | BM10888* | BM10872* | BM10761* | BM10759* | BM12152* | BM10914* | BM3364* | |
| *SA1208* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 15 |
| *MW0378* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 16 |
| *SAV2596* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 16 |
| *ORF17* | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 17 |
| *SAV0868* | 0 | 1 | 0 | 1 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 1 | 0 | 0 | 0 | 1 | 0 | 18 |
| *SA1829* | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 18 |
| *agrC-N315* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 19 |
| *SAV0847* | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 19 |
| *ORF44* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 19 |
| *coa-N315* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 19 |
| *ORF20* | 1 | 1 | 0 | 1 | 1 | 0 | 0 | 0 | 0 | 0 | 0 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 19 |
| *ORF21* | 1 | 1 | 0 | 1 | 1 | 0 | 0 | 0 | 0 | 0 | 0 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 19 |
| *agrB-N315* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 19 |
| *MW0355* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 19 |
| *set15* | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 20 |
| *MW0063* | 0 | 0 | 1 | 1 | 1 | 1 | 1 | 0 | 1 | 1 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 21 |
| *MW2612* | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 23 |
| *ORF14* | 1 | 1 | 1 | 0 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 23 |
| *ORF10* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 23 |
| *sasK(SAV2595)* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 23 |
| *ORF11* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 23 |
| *SA1640* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 23 |
| *cna* | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 23 |
| *ORF13* | 1 | 1 | 1 | 0 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 23 |
| *SA1636* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 23 |
| *MW1408* | 0 | 0 | 1 | 1 | 1 | 1 | 0 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 23 |
| *MW0053* | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 23 |

| Clusters | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|
| *aadD* | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 1 | 1 | 1 | 1 | 0 | 24 |
| *MW1405* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 24 |
| *ble* | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 1 | 1 | 1 | 1 | 0 | 24 |
| *qacA* | 0 | 0 | 0 | 0 | 0 | 0 | 0 | 0 | 1 | 0 | 0 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 0 | 24 |
| *SAV0865* | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 24 |
| *SA1635* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 24 |
| *ccrB1* | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 25 |
| *MW0045* | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 25 |
| *ccrA1* | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 25 |
| *MW1386* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 25 |
| *ORF7* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 25 |
| *ORF8* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 25 |
| *ORF12* | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 25 |
| *MW1393* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 25 |
| *SAV0909* | 1 | 1 | 1 | 1 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 25 |
| *CZ 040* | 0 | 1 | 0 | 0 | 0 | 0 | 0 | 0 | 1 | 0 | 0 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 0 | 25 |
| *ORF9* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 25 |
| *CZ 041* | 0 | 1 | 0 | 0 | 0 | 0 | 0 | 0 | 1 | 0 | 0 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 0 | 25 |
| *MW1401* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 26 |
| *SA1341* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 26 |
| *SAV0903* | 1 | 1 | 1 | 1 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 26 |
| *ORF6* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 26 |
| *SAV0902* | 1 | 1 | 1 | 1 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 26 |
| *SAV0901* | 1 | 1 | 1 | 1 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 26 |
| *ORF25* | 1 | 1 | 1 | 1 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 26 |
| *SAV0898* | 1 | 1 | 1 | 1 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 26 |
| *SAV0890* | 1 | 1 | 1 | 1 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 26 |
| *SAV0888* | 1 | 1 | 1 | 1 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 26 |
| *MW1400* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 26 |
| *SA1622* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 26 |
| *SAV0887* | 1 | 1 | 1 | 1 | 0 | 0 | 0 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 26 |
| *pro 8325* | 0 | 0 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 27 |
| *SAV0406* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 27 |
| *SAV0404* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *SAV0414* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *SAV0403* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *SAV0415* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *SAV0413* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |

| Clusters | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|
| *SAV0402* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *repB* | 0 | 0 | 0 | 0 | 0 | 1 | 1 | 1 | 1 | 1 | 1 | 1 | 1 | 0 | 0 | 1 | 1 | 1 | 1 | 1 | 1 | 1 | 1 | 1 | 0 | 0 | 0 | 0 | 0 | 0 | 0 | 1 | 1 | 1 | 1 | 0 | 28 |
| *SAV0410* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *SAV0401* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *SAV0409* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *SAV0400* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *aacA-aphD* | 1 | 1 | 0 | 0 | 0 | 0 | 0 | 0 | 1 | 0 | 1 | 0 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 28 |
| *SAV0408* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *SAV0399* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *MW1197* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 28 |
| *SAV0405* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *Int459(SAV0392)* | 1 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 28 |
| *tnpA (Tn554)* | 1 | 1 | 0 | 0 | 0 | 0 | 0 | 0 | 1 | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 29 |
| *SAV0599* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 29 |
| *cap8K-like* | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 29 |
| *cap8J* | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 29 |
| *tnp480 (Tn552)* | 1 | 1 | 0 | 1 | 1 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 29 |
| *MW2564* | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 29 |
| *cap8I* | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 29 |
| *cap8H* | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 29 |
| *SAV0395* | 1 | 1 | 0 | 0 | 1 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 29 |
| *ORF22* | 1 | 1 | 0 | 0 | 1 | 0 | 0 | 0 | 0 | 0 | 1 | 1 | 1 | 1 | 1 | 0 | 0 | 0 | 0 | 0 | 0 | 1 | 1 | 0 | 0 | 0 | 0 | 0 | 0 | 0 | 0 | 0 | 0 | 0 | 1 | 1 | 30 |
| *spc* | 1 | 1 | 0 | 0 | 0 | 0 | 0 | 0 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 30 |
| *SA0047* | 1 | 1 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 30 |
| *SA2259* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 30 |
| *ORF32* | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 1 | 1 | 1 | 1 | 0 | 30 |
| *tnpB (Tn554)* | 1 | 1 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 31 |
| *seg* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *SA1641* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *sei* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *tnp IS 256* | 1 | 1 | 0 | 0 | 0 | 0 | 0 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 31 |
| *seo* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *ermA* | 1 | 1 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 31 |
| *sem* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *ORF16* | 1 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 1 | 0 | 31 |
| *sen* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *SAV0850* | 0 | 0 | 1 | 0 | 1 | 0 | 0 | 0 | 0 | 0 | 0 | 1 | 1 | 1 | 1 | 1 | 0 | 0 | 1 | 1 | 1 | 0 | 0 | 1 | 1 | 1 | 1 | 1 | 1 | 1 | 1 | 1 | 1 | 0 | 0 | 0 | 31 |
| *SA0054* | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 32 |

| Clusters | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|
| *ORF26* | 0 | 0 | 1 | 1 | 1 | 0 | 0 | 0 | 0 | 0 | 0 | 1 | 1 | 1 | 1 | 0 | 1 | 1 | 1 | 1 | 1 | 0 | 0 | 1 | 1 | 1 | 1 | 1 | 1 | 0 | 0 | 1 | 1 | 0 | 0 | 0 | 32 |
| *ORF5* | 0 | 0 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 1 | 1 | 33 |
| *coa-8325* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 34 |
| *MW1749* | 1 | 1 | 1 | 1 | 1 | 0 | 1 | 1 | 1 | 1 | 1 | 1 | 1 | 1 | 1 | 1 | 1 | 1 | 1 | 1 | 0 | 1 | 1 | 0 | 1 | 1 | 1 | 1 | 1 | 1 | 1 | 0 | 0 | 1 | 1 | 1 | 34 |
| *SA0024* | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 34 |
| *SAV0905* | 0 | 0 | 0 | 0 | 0 | 0 | 0 | 0 | 0 | 0 | 0 | 1 | 1 | 1 | 1 | 1 | 1 | 1 | 1 | 1 | 1 | 0 | 1 | 0 | 1 | 1 | 1 | 1 | 1 | 1 | 1 | 1 | 1 | 1 | 1 | 0 | 34 |
| *SA0142* | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 35 |
| *SAV2001* | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 0 | 35 |
| *SA0141* | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 35 |
| *tnpIS 1272* | 0 | 0 | 0 | 0 | 0 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 35 |
| *SA1826* | 0 | 0 | 0 | 1 | 0 | 1 | 1 | 1 | 0 | 0 | 0 | 0 | 0 | 0 | 0 | 0 | 0 | 0 | 1 | 1 | 1 | 1 | 1 | 0 | 1 | 1 | 1 | 1 | 1 | 0 | 0 | 1 | 1 | 0 | 1 | 1 | 36 |
| *SAV0039* | 1 | 1 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 36 |
| *ORF18* | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 36 |
| *mecA* | 1 | 1 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 36 |
| *SA1822* | 0 | 0 | 0 | 1 | 0 | 1 | 1 | 1 | 0 | 0 | 0 | 0 | 0 | 0 | 0 | 0 | 0 | 0 | 1 | 1 | 1 | 1 | 1 | 0 | 1 | 1 | 1 | 1 | 1 | 0 | 0 | 1 | 1 | 0 | 1 | 1 | 36 |
| *MW0065* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 36 |
| *mecR1-2* | 1 | 1 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 36 |
| *MW0066* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 36 |
| *MW1750* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 37 |
| *tetM* | 1 | 1 | 0 | 1 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 37 |
| *MW0120* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 37 |
| *MW1757* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 38 |
| *ORF24* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 39 |
| *ORF29* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 39 |
| *ORF38* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 39 |
| *MW0560* | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 40 |
| *entA = entP* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 41 |
| *bsaC* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| *bsaB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| *bsaG* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| *bsaE* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| *bsaF* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| *SA1806* | 0 | 0 | 0 | 0 | 0 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 41 |
| *bsaP* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| *bsaD* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| *MW1908* | 1 | 1 | 0 | 1 | 1 | 0 | 0 | 0 | 0 | 0 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 42 |
| *MW1898* | 1 | 1 | 0 | 0 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 43 |
| *MW2125* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 43 |

| Clusters | 33 | 33 | 34 | 35 | 36 | 37 | 37 | 38 | 39 | 40 | 40 | 41' | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|
| *MW2534* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 43 |
| *MW0062* | 0 | 0 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 0 | 1 | 1 | 1 | 1 | 1 | 44 |
| *ORF36* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 44 |
| *ORF19* | 1 | 1 | 0 | 1 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 44 |
| *CAC3531* | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 44 |
| *MW0552* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |
| *agrB-8325* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |
| *ORF39* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |
| *ORF28* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |
| *ORF40* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |
| *MW1744* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |
| *sigB* | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 46 |
| *ORF41* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 1 | 1 | 1 | 1 | 0 | 46 |
| *ORF31* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 46 |
| *MW0064* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 46 |
| *MW0558* | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 46 |
| *SA0392* | 1 | 1 | 1 | 1 | 1 | 1 | 1 | 1 | 1 | 1 | 1 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 47 |
| *SAV0804* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 1 | 47 |
| *SAV0803* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 1 | 47 |
| *MW0554* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 47 |
| *bbp=sdrE* | 1 | 1 | 1 | 1 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 47 |
| *MW0553* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 48 |
| *ORF35* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 49 |
| *MW0559* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 49 |
| *ORF34* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 49 |
| *MW0919* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 49 |
| *cap5j* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 50 |
| *ORF45* | 1 | 1 | 0 | 0 | 0 | 0 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 50 |
| *ami 8325* | 1 | 1 | 1 | 1 | 1 | 0 | 0 | 0 | 0 | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 0 | 50 |
| *cap5H* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *SA2483* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *ORF37* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *tnp IS 1181* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *phi int12* | 0 | 0 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 0 | 1 | 1 | 1 | 1 | 1 | 1 | 1 | 1 | 1 | 1 | 51 |
| *cap5K* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *SAV0912* | 1 | 1 | 1 | 1 | 1 | 0 | 0 | 0 | 0 | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 51 |
| *cap5i* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *SA1791* | 0 | 0 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 52 |

| Clusters | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|
| *ORF3* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 52 |
| *tnp IS 431* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 52 |
| *agrC-8325* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 52 |
| *sarH2* | 1 | 1 | 1 | 1 | 0 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 53 |
| *splE* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 54 |
| *MW1742* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 54 |
| *SA1755* | 0 | 0 | 0 | 0 | 0 | 0 | 0 | 0 | 0 | 0 | 0 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 0 | 1 | 0 | 55 |
| *MW906* | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 55 |
| *SA1320* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 57 |
| *SA0104* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 57 |
| *lukD* | 1 | 0 | 1 | 1 | 0 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 58 |
| *SA0103* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 58 |
| *splB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 58 |
| *SA1768* | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 59 |
| *splA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 60 |
| *ORF2* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 60 |
| *SA1766* | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *SA1778* | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *SA1777* | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *ORF42* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 60 |
| *ORF27* | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *SA1775* | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *SA1774* | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *sdrD* | 1 | 1 | 1 | 1 | 1 | 1 | 1 | 1 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 61 |
| *set 9* | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 61 |
| *ORF15* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 61 |
| *set12* | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 62 |
| *SA1795* | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 62 |
| *set14* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 63 |
| *lukM* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 1 | 63 |
| *set 8* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 63 |
| *MW1769* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 63 |
| *SA1010* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 64 |
| *SAV0910* | 1 | 0 | 1 | 1 | 1 | 0 | 0 | 0 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 64 |
| *SA2272* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 64 |
| *SAV0904* | 1 | 1 | 1 | 1 | 1 | 0 | 0 | 0 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 0 | 64 |
| *splD splF* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 65 |
| *splC* | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 0 | 0 | 1 | 1 | 1 | 66 |

| Clusters | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|
| *phl Int13* | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 0 | 66 |
| *SAV0866* | 1 | 1 | 0 | 1 | 1 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 66 |
| *ORF33* | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 66 |
| *MW2409* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 67 |
| *fosB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 68 |
| *SA1801* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 68 |
| *SA0378* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 69 |
| *SA0377* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 69 |
| *SA2004* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 69 |
| *map* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 70 |
| *SA2005* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 70 |
| *sak* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 70 |
| *blaZ* | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 70 |
| *fnbB* | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 72 |
| *set10* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 72 |
| *SA2314* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 73 |
| *emp* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 74 |
| *vwb* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 74 |
| *SAV1974* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 75 |
| *SAV1392* | 0 | 0 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 1 | 75 |
| *SA0276* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 75 |
| *vraF(SA0616)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 75 |
| *SA0745* | 1 | 1 | 1 | 1 | 1 | 1 | 1 | 1 | 1 | 1 | 1 | 1 | 0 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 76 |
| *agrC* | 1 | 1 | 1 | 1 | 1 | 1 | 0 | 0 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 76 |
| *set13* | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 77 |
| *hlb* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 1 | 77 |
| *set 6* | 1 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 78 |
| *efb=fib* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 78 |
| *set11* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 78 |
| *rpoD* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *ssaA (SA2093)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *radA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA1725* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fhuD=fhuG(SA0604)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fmtC (mprF)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fus(SA0505)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fnbA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *nuc SA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |

| Clusters | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|
| *set 7* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *vraR(SA1700)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *geh* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *sdrC* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA0022* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *sbi(SA2206)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *dltA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *lexA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *ORF41* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *rsbU* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *dnaK* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *sacP(SA0295)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *agrA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA0610* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *hla* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA1552* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *srtA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *hySA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *ebpS* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *femB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *lytS+lytR* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *rnc(SA1076)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *hlgC* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA0587* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *hlgA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *aur(SA2430)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fmtB (mrp)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *sspB (SA0900)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *sspA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *coa* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SAV2170* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *femA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA0675* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA0746* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *hlgB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *lip(SA2463)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA0423* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *sodM* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |

| Clusters | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|
| atl-amidase | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA0977 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| fabH | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA1970 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA1852 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| fhuD2(SA2079) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA0102 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| clfA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| atl-glucosamin | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| hsdM | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| clpL | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| clpB | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| recA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| agrJ | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA1580 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| fhuB | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| spa | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA0691 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| icaA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| clfB | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA0704 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| mdr | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| norA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| fur | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| fhuA=fhuC(SA0602) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| putB | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| plc | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| icaC | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| htrA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| ORF30 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |

Table T60: Gene content of each of the 80 *S. aureus* isolates tested. mecA+ isolates are marked by an asterisk. The gene was scored as present (1) or absent (0). The cluster numbers are those reported in Figure 1.

° Outbreak related isolates

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 |
|---|---|---|---|---|---|---|---|---|---|---|---|---|---|---|---|---|---|---|---|---|---|---|
| Gene | BM12987 (O24) | IPF143 | BM12685 | BM12947 | IPF493 | BM12286 | BM18287 | IPF140 | IPF150 | IPF735° | IPF736° | IPF741° | IPF743° | IPF738° | IPF511 | BM12633 | BM12623 | BM12863 | BM12718 | BM12771 | BM12889 | IPF497 |
| *tnpB (Tn554)* | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *seg* | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA1641* | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *sei* | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *tnp IS 256* | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *seo* | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *ermA* | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *sem* | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *ORF16* | 0 | 0 | 0 | 0 | 1 | 0 | 0 | 1 | 0 | 1 | 1 | 1 | 1 | 1 | 0 | 0 | 0 | 0 | 0 | 0 | 0 | 0 |
| *sen* | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SAV0850* | 0 | 0 | 0 | 0 | 0 | 0 | 0 | 0 | 0 | 0 | 0 | 0 | 0 | 0 | 0 | 1 | 1 | 1 | 0 | 0 | 0 | 0 |
| *SA0054* | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *ORF26* | 0 | 0 | 0 | 0 | 0 | 0 | 0 | 0 | 0 | 0 | 0 | 0 | 0 | 0 | 0 | 1 | 1 | 1 | 0 | 0 | 0 | 0 |
| *ORF5* | 0 | 0 | 1 | 1 | 0 | 0 | 0 | 1 | 0 | 0 | 0 | 0 | 0 | 0 | 0 | 1 | 1 | 1 | 0 | 0 | 0 | 0 |
| *coa-8325* | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *MW1749* | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *SA0024* | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *SAV0905* | 0 | 0 | 0 | 1 | 0 | 0 | 0 | 0 | 0 | 0 | 0 | 0 | 0 | 0 | 1 | 1 | 1 | 1 | 1 | 1 | 1 | 1 |
| *SA0142* | 1 | 1 | 1 | 1 | 1 | 1 | 1 | 0 | 0 | 0 | 0 | 0 | 0 | 0 | 1 | 1 | 1 | 1 | 1 | 1 | 1 | 1 |
| *SAV2001* | 1 | 1 | 0 | 1 | 0 | 0 | 0 | 0 | 1 | 0 | 0 | 0 | 0 | 0 | 0 | 0 | 0 | 0 | 1 | 1 | 1 | 1 |
| *SA0141* | 1 | 1 | 1 | 1 | 1 | 1 | 1 | 0 | 0 | 0 | 0 | 0 | 0 | 0 | 1 | 1 | 1 | 1 | 1 | 1 | 1 | 1 |
| *tnpIS 1272* | 0 | 0 | 1 | 0 | 1 | 0 | 0 | 0 | 0 | 0 | 0 | 0 | 0 | 0 | 0 | 0 | 0 | 0 | 0 | 0 | 0 | 0 |
| *SA1826* | 1 | 1 | 1 | 1 | 1 | 1 | 1 | 1 | 1 | 0 | 0 | 0 | 0 | 0 | 1 | 0 | 0 | 0 | 1 | 1 | 0 | 0 |
| *SAV0039* | 0 | 0 | 0 | 0 | 0 | 0 | 0 | 0 | 0 | 0 | 0 | 0 | 0 | 0 | 0 | 0 | 0 | 0 | 0 | 0 | 0 | 0 |

| Clusters | 15 | 16 | 17 | 18 | 19 | 20 | 21 | 22 | 23 | 24 | 25 | 26 | 27 | 28 | 28 | 28 | 28 | 29 | 30 | 31 | 31 |
|---|---|---|---|---|---|---|---|---|---|---|---|---|---|---|---|---|---|---|---|---|---|
| Gene | BM12766 | BM12816* | IPF139 | IPF166 | BM12764 | IPF524 | BM12881 | IPF161* | IPF498 | IPF488 | IPF494 | BM12666 | IPF153 | IPF308° | IPF310° | IPF323° | IPF311° | IPF145 | IPF520 | IPF490 | IPF159 |
| *tnpB (Tn554)* | 0 | 0 | 0 | 0 | 0 | 0 | 0 | 0 | 0 | 0 | 0 | 0 | 0 | 0 | 0 | 0 | 0 | 0 | 0 | 0 | 0 |
| *seg* | 1 | 1 | 1 | 1 | 1 | 1 | 1 | 0 | 0 | 0 | 0 | 0 | 0 | 0 | 0 | 0 | 0 | 1 | 1 | 0 | 0 |
| *SA1641* | 1 | 1 | 1 | 1 | 1 | 1 | 1 | 0 | 0 | 0 | 0 | 0 | 0 | 0 | 0 | 0 | 0 | 1 | 1 | 0 | 0 |
| *sei* | 1 | 1 | 1 | 1 | 1 | 1 | 1 | 0 | 0 | 0 | 0 | 0 | 0 | 0 | 0 | 0 | 0 | 1 | 1 | 0 | 0 |
| *tnp IS 256* | 0 | 0 | 0 | 0 | 0 | 0 | 0 | 0 | 0 | 0 | 0 | 0 | 0 | 0 | 0 | 0 | 0 | 0 | 0 | 0 | 0 |
| *seo* | 1 | 1 | 1 | 1 | 1 | 1 | 1 | 0 | 0 | 0 | 0 | 0 | 0 | 0 | 0 | 0 | 0 | 1 | 1 | 0 | 0 |
| *ermA* | 0 | 0 | 0 | 0 | 0 | 0 | 0 | 0 | 0 | 0 | 0 | 0 | 0 | 0 | 0 | 0 | 0 | 0 | 0 | 0 | 0 |
| *sem* | 1 | 1 | 1 | 1 | 1 | 1 | 1 | 0 | 0 | 0 | 0 | 0 | 0 | 0 | 0 | 0 | 0 | 1 | 1 | 0 | 0 |
| *ORF16* | 0 | 0 | 0 | 0 | 0 | 0 | 0 | 0 | 0 | 0 | 0 | 0 | 0 | 0 | 0 | 0 | 0 | 0 | 0 | 0 | 0 |
| *sen* | 1 | 1 | 1 | 1 | 1 | 1 | 1 | 0 | 0 | 0 | 0 | 0 | 0 | 0 | 0 | 0 | 0 | 1 | 1 | 0 | 0 |
| *SAV0850* | 0 | 0 | 0 | 1 | 0 | 0 | 1 | 0 | 0 | 0 | 0 | 0 | 1 | 1 | 1 | 1 | 1 | 0 | 1 | 0 | 0 |
| *SA0054* | 0 | 1 | 0 | 0 | 0 | 0 | 0 | 1 | 0 | 0 | 0 | 0 | 0 | 0 | 0 | 0 | 0 | 0 | 0 | 0 | 0 |
| *ORF26* | 0 | 0 | 0 | 1 | 0 | 0 | 1 | 1 | 0 | 0 | 0 | 0 | 1 | 1 | 1 | 1 | 1 | 0 | 1 | 0 | 0 |
| *ORF5* | 0 | 0 | 0 | 1 | 0 | 0 | 1 | 1 | 1 | 0 | 0 | 0 | 1 | 1 | 1 | 1 | 1 | 1 | 1 | 0 | 0 |
| *coa-8325* | 0 | 0 | 0 | 0 | 0 | 0 | 0 | 0 | 0 | 0 | 0 | 0 | 0 | 0 | 0 | 0 | 0 | 0 | 0 | 0 | 0 |
| *MW1749* | 0 | 0 | 0 | 0 | 0 | 0 | 0 | 0 | 0 | 0 | 0 | 0 | 0 | 0 | 0 | 0 | 0 | 0 | 0 | 1 | 1 |
| *SA0024* | 0 | 1 | 0 | 0 | 0 | 0 | 0 | 1 | 0 | 0 | 0 | 0 | 0 | 0 | 0 | 0 | 0 | 0 | 0 | 0 | 0 |
| *SAV0905* | 0 | 1 | 0 | 1 | 0 | 0 | 0 | 0 | 0 | 0 | 0 | 0 | 0 | 0 | 0 | 0 | 0 | 0 | 1 | 0 | 0 |
| *SA0142* | 1 | 1 | 1 | 1 | 1 | 0 | 0 | 0 | 0 | 1 | 1 | 1 | 1 | 1 | 1 | 1 | 1 | 1 | 1 | 1 | 1 |
| *SAV2001* | 1 | 1 | 1 | 1 | 0 | 1 | 0 | 0 | 0 | 0 | 0 | 1 | 0 | 0 | 0 | 0 | 0 | 0 | 0 | 0 | 0 |
| *SA0141* | 1 | 1 | 1 | 1 | 1 | 0 | 0 | 0 | 0 | 1 | 1 | 1 | 1 | 1 | 1 | 1 | 1 | 1 | 1 | 1 | 1 |
| *tnpIS 1272* | 0 | 1 | 0 | 0 | 0 | 0 | 0 | 1 | 0 | 0 | 0 | 0 | 1 | 0 | 0 | 0 | 0 | 0 | 0 | 0 | 0 |
| *SA1826* | 0 | 0 | 0 | 0 | 1 | 1 | 0 | 0 | 0 | 0 | 1 | 1 | 0 | 0 | 0 | 0 | 0 | 1 | 0 | 0 | 0 |
| *SAV0039* | 0 | 1 | 0 | 0 | 0 | 0 | 0 | 1 | 0 | 0 | 0 | 0 | 0 | 0 | 0 | 0 | 0 | 0 | 0 | 0 | 0 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *ORF18* | 1 | 1 | 1 | 1 | 0 | 0 | 0 | 1 | 1 | 1 | 1 | 1 | 1 | 1 | 1 | 0 | 0 | 0 | 1 | 1 | 1 | 1 | 1 | 1 | 1 | 1 | 1 | 0 | 0 | 1 | 0 | 0 | 0 | 0 | 0 | 0 | 0 | 0 | 0 | 0 | 0 | 1 | 1 |
| *mecA* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | 0 |
| *SA1822* | 1 | 1 | 1 | 1 | 1 | 1 | 1 | 1 | 1 | 0 | 0 | 0 | 0 | 0 | 1 | 0 | 0 | 0 | 1 | 1 | 0 | 0 | 0 | 0 | 0 | 0 | 1 | 1 | 0 | 0 | 0 | 0 | 1 | 1 | 0 | 0 | 0 | 0 | 0 | 1 | 0 | 0 | 0 |
| *MW0065* | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *mecR1-2* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | 0 |
| *MW0066* | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *MW1750* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 |
| *tetM* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 0 | 1 | 1 | 0 | 0 | 0 | 0 | 0 | 0 | 0 | 0 | 0 | 0 | 0 | 0 | 0 | 0 | 0 | 0 |
| *MW0120* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 0 | 0 |
| *MW1757* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 1 |
| *ORF24* | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *ORF29* | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *ORF38* | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| *MW0560* | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 0 | 0 | 1 | 1 | 0 | 0 | 0 | 0 | 0 | 0 | 0 | 0 | 0 | 0 |
| *entA = entP* | 1 | 1 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 1 |
| *bsaC* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 |
| *bsaB* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 |
| *bsaG* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 |
| *bsaE* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 |
| *bsaF* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 |
| *SA1806* | 1 | 1 | 0 | 1 | 0 | 0 | 0 | 0 | 1 | 0 | 0 | 0 | 0 | 0 | 0 | 1 | 1 | 0 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 |
| *bsaP* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 |
| *bsaD* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 1 | 1 |
| *MW1908* | 0 | 1 | 1 | 1 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 1 | 0 | 0 | 0 | 0 | 0 | 0 | 0 | 0 | 0 | 1 | 1 | 1 | 1 |
| *MW1898* | 0 | 1 | 1 | 1 | 0 | 1 | 1 | 0 | 0 | 0 | 0 | 0 | 0 | 0 | 0 | 0 | 0 | 0 | 0 | 0 | 0 | 0 | 1 | 0 | 0 | 0 | 1 | 0 | 1 | 1 | 0 | 0 | 0 | 0 | 0 | 0 | 0 | 0 | 0 | 1 | 1 | 1 | 1 |
| *MW2125* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 1 | 1 | 1 | 1 | 1 | 0 | 0 | 1 | 1 |
| *MW2534* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 1 | 1 | 1 | 1 | 1 | 0 | 0 | 1 | 1 |
| *MW0062* | 0 | 0 | 0 | 0 | 1 | 0 | 0 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 1 | 0 | 0 |
| *ORF36* | 0 | 0 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 |
| *ORF19* | 0 | 1 | 1 | 1 | 0 | 1 | 1 | 0 | 0 | 0 | 0 | 0 | 0 | 0 | 0 | 0 | 0 | 0 | 0 | 0 | 0 | 0 | 1 | 0 | 0 | 0 | 1 | 0 | 1 | 1 | 0 | 0 | 0 | 0 | 0 | 0 | 0 | 0 | 0 | 1 | 1 | 1 | 1 |
| *CAC3531* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 1 | 1 | 1 | 1 | 1 | 0 | 0 | 0 | 0 |
| *MW0552* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 0 | 0 | 0 | 0 |
| *agrB-8325* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 0 | 0 | 0 | 0 | 1 | 1 | 0 | 0 |
| *ORF39* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 0 | 0 | 0 | 0 | 1 | 1 | 0 | 0 |
| *ORF28* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 0 | 0 | 0 | 0 | 1 | 1 | 0 | 0 |
| *ORF40* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 1 | 1 | 0 | 0 | 0 | 0 | 0 | 0 | 0 | 1 | 1 | 0 | 0 |
| *MW1744* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 1 | 1 | 1 | 1 | 0 | 0 | 0 | 0 | 0 | 1 | 1 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *sigB* | 1 | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *ORF41* | 1 | 1 | 1 | 1 | 1 | 1 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 1 | 0 | 1 | 1 | 1 | 1 | 1 | 0 | 0 | 0 | 0 |
| *ORF31* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 |
| *MW0064* | 0 | 0 | 0 | 0 | 1 | 0 | 0 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 1 | 0 | 0 |
| *MW0558* | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 0 | 0 | 0 | 0 |
| *SA0392* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 0 | 0 | 0 | 1 | 1 | 0 | 1 | 0 | 0 | 0 | 0 | 0 | 1 | 1 | 0 | 0 |
| *SAV0804* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 1 | 1 | 0 | 1 | 0 | 0 | 0 | 1 | 1 | 1 | 1 | 1 | 0 | 0 | 1 | 1 |
| *SAV0803* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 1 | 1 | 0 | 1 | 0 | 0 | 0 | 1 | 1 | 1 | 1 | 1 | 0 | 0 | 1 | 1 |
| *MW0554* | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 1 | 0 | 0 | 0 | 1 | 1 | 1 | 1 | 1 | 1 | 1 | 0 | 0 | 0 | 0 |
| *bbp=sdrE* | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 0 | 1 | 1 | 1 | 1 | 1 | 0 | 0 | 1 | 1 | 1 | 1 | 1 | 1 | 1 | 1 | 0 |
| *MW0553* | 0 | 0 | 0 | 0 | 0 | 0 | 0 | 0 | 0 | 0 | 0 | 0 | 0 | 0 | 1 | 1 | 1 | 1 | 0 | 0 | 0 | 0 | 0 | 0 | 0 | 0 | 0 | 0 | 1 | 0 | 0 | 0 | 1 | 1 | 1 | 1 | 1 | 1 | 1 | 0 | 0 | 0 | 0 |
| *ORF35* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 1 | 1 | 0 | 1 | 1 | 1 | 1 | 1 | 1 | 1 | 1 | 1 | 1 | 1 |
| *MW0559* | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 0 | 0 | 0 | 0 |
| *ORF34* | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 1 | 1 | 0 | 1 | 1 | 1 | 1 | 1 | 1 | 1 | 1 | 1 | 1 | 1 |
| *MW0919* | 1 | 1 | 1 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 1 | 1 | 0 | 1 | 1 | 0 | 0 | 0 |
| *cap5j* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 0 | 1 | 1 | 1 | 0 | 1 | 1 | 0 | 0 | 0 | 0 | 0 | 0 | 0 | 0 | 0 | 0 | 0 |
| *ORF45* | 0 | 0 | 0 | 0 | 1 | 0 | 0 | 1 | 0 | 0 | 0 | 0 | 0 | 0 | 1 | 1 | 1 | 1 | 1 | 1 | 1 | 1 | 0 | 1 | 0 | 1 | 0 | 0 | 0 | 0 | 0 | 0 | 0 | 1 | 0 | 1 | 1 | 1 | 1 | 0 | 0 | 1 | 1 |
| *ami 8325* | 0 | 0 | 1 | 1 | 1 | 1 | 1 | 1 | 1 | 1 | 1 | 1 | 1 | 1 | 1 | 0 | 0 | 0 | 0 | 0 | 1 | 1 | 1 | 1 | 0 | 1 | 1 | 0 | 0 | 0 | 0 | 0 | 0 | 0 | 0 | 0 | 0 | 0 | 0 | 0 | 1 | 1 | 0 |
| *cap5H* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 1 | 1 | 0 | 0 | 0 | 0 | 0 | 0 | 0 | 0 | 0 | 0 | 0 |
| *SA2483* | 0 | 0 | 0 | 0 | 1 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 0 | 1 | 1 | 1 | 0 | 0 | 0 | 0 | 0 | 0 | 0 | 0 | 0 | 0 | 0 | 0 |
| *ORF37* | 1 | 1 | 1 | 1 | 1 | 1 | 1 | 1 | 1 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 1 | 1 | 0 | 0 | 0 | 0 | 0 | 0 | 0 | 1 | 1 |
| *tnp IS 1181* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 0 | 0 | 0 | 0 | 0 | 0 | 0 | 0 | 0 | 0 | 0 | 1 | 1 | 1 | 1 |
| *phi int12* | 1 | 0 | 1 | 1 | 0 | 1 | 1 | 1 | 0 | 0 | 0 | 0 | 0 | 0 | 1 | 1 | 1 | 0 | 0 | 0 | 0 | 0 | 1 | 0 | 0 | 1 | 0 | 0 | 1 | 1 | 1 | 0 | 0 | 0 | 1 | 1 | 1 | 1 | 1 | 0 | 0 | 0 | 0 |
| *cap5K* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 1 | 1 | 0 | 0 | 0 | 0 | 0 | 0 | 0 | 0 | 0 | 0 | 0 |
| *SAV0912* | 0 | 0 | 1 | 1 | 1 | 1 | 1 | 1 | 1 | 1 | 1 | 1 | 1 | 1 | 1 | 0 | 0 | 0 | 0 | 0 | 1 | 1 | 1 | 1 | 0 | 1 | 1 | 0 | 0 | 0 | 0 | 0 | 0 | 0 | 0 | 0 | 0 | 0 | 0 | 0 | 1 | 1 | 0 |
| *cap5i* | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 1 | 1 | 0 | 0 | 0 | 0 | 0 | 0 | 0 | 0 | 0 | 0 | 0 |
| *SA1791* | 1 | 1 | 1 | 1 | 0 | 0 | 0 | 0 | 1 | 1 | 1 | 1 | 1 | 1 | 0 | 0 | 0 | 1 | 0 | 0 | 1 | 0 | 0 | 0 | 1 | 0 | 1 | 1 | 0 | 1 | 1 | 0 | 0 | 1 | 0 | 0 | 0 | 0 | 0 | 0 | 0 | 1 | 1 |
| *ORF3* | 0 | 0 | 0 | 0 | 0 | 0 | 0 | 0 | 0 | 0 | 0 | 0 | 0 | 0 | 1 | 0 | 0 | 0 | 1 | 1 | 1 | 1 | 1 | 1 | 1 | 1 | 1 | 1 | 0 | 0 | 0 | 0 | 0 | 0 | 0 | 0 | 0 | 0 | 0 | 1 | 1 | 1 | 1 |
| *tnp IS 431* | 1 | 0 | 1 | 1 | 1 | 1 | 1 | 0 | 0 | 0 | 0 | 0 | 0 | 0 | 0 | 1 | 1 | 1 | 1 | 0 | 1 | 0 | 0 | 1 | 0 | 0 | 0 | 1 | 0 | 1 | 0 | 0 | 0 | 0 | 0 | 0 | 0 | 0 | 0 | 1 | 0 | 0 | 0 |
| *agrC-8325* | 0 | 0 | 0 | 0 | 0 | 0 | 0 | 1 | 1 | 1 | 1 | 1 | 1 | 1 | 0 | 0 | 0 | 0 | 0 | 0 | 0 | 0 | 0 | 0 | 0 | 0 | 0 | 1 | 1 | 0 | 1 | 1 | 1 | 0 | 0 | 0 | 0 | 0 | 0 | 0 | 1 | 1 | 1 |
| *sarH2* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 0 | 1 | 0 | 0 | 0 | 0 | 1 | 1 | 1 | 1 | 1 | 0 | 0 | 1 | 1 |
| *splE* | 1 | 1 | 1 | 1 | 1 | 1 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 0 | 0 | 1 | 1 |
| *MW1742* | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 1 | 0 | 1 | 0 | 1 | 1 | 1 | 1 | 1 | 1 | 1 | 1 | 1 | 1 | 1 | 1 |
| *SA1755* | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 0 | 0 | 0 | 0 | 0 | 1 | 1 | 1 | 1 | 1 | 0 | 0 | 0 | 0 |
| *MW906* | 1 | 1 | 1 | 1 | 1 | 1 | 1 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 1 | 1 | 1 | 1 | 1 | 1 | 1 | 1 | 1 | 0 | 0 | 1 | 1 |
| *SA1320* | 0 | 0 | 0 | 0 | 0 | 0 | 0 | 0 | 0 | 0 | 0 | 0 | 0 | 0 | 1 | 1 | 1 | 0 | 1 | 1 | 1 | 1 | 1 | 1 | 1 | 1 | 1 | 1 | 0 | 0 | 1 | 1 | 0 | 0 | 1 | 1 | 1 | 1 | 1 | 0 | 0 | 0 | 0 |
| *SA0104* | 0 | 0 | 0 | 0 | 0 | 0 | 0 | 1 | 1 | 1 | 1 | 1 | 1 | 1 | 0 | 0 | 0 | 0 | 1 | 1 | 1 | 1 | 1 | 1 | 1 | 1 | 1 | 1 | 1 | 0 | 0 | 0 | 1 | 1 | 0 | 0 | 0 | 0 | 0 | 1 | 1 | 1 | 1 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *lukD* | 0 | 0 | 0 | 0 | 0 | 0 | 0 | 0 | 0 | 0 | 0 | 0 | 0 | 0 | 1 | 0 | 0 | 0 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 |
| *SA0103* | 0 | 0 | 0 | 0 | 0 | 0 | 0 | 1 | 1 | 1 | 1 | 1 | 1 | 1 | 0 | 0 | 0 | 0 | 1 | 1 | 1 | 1 | 1 | 1 | 1 | 1 | 1 | 1 | 1 | 0 | 0 | 0 | 1 | 1 | 0 | 0 | 0 | 0 | 0 | 1 | 1 | 1 | 1 |
| *splB* | 0 | 0 | 0 | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 |
| *SA1768* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 |
| *splA* | 0 | 0 | 0 | 0 | 0 | 0 | 0 | 0 | 0 | 0 | 0 | 0 | 0 | 0 | 1 | 0 | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 |
| *ORF2* | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 1 | 0 | 1 | 1 | 1 | 1 | 1 | 0 | 0 | 1 | 1 |
| *SA1766* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 |
| *SA1778* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 |
| *SA1777* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 |
| *ORF42* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 0 | 1 | 0 | 1 | 1 | 1 | 1 | 1 | 0 | 0 | 1 | 1 |
| *ORF27* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 |
| *SA1775* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 |
| *SA1774* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 0 | 1 | 0 | 0 | 1 | 0 | 0 | 1 | 0 | 0 | 0 | 0 | 0 | 0 | 0 | 0 | 0 |
| *sdrD* | 1 | 1 | 1 | 1 | 1 | 0 | 0 | 0 | 0 | 0 | 0 | 0 | 0 | 0 | 0 | 1 | 1 | 0 | 1 | 1 | 1 | 1 | 1 | 1 | 1 | 1 | 0 | 1 | 1 | 1 | 1 | 1 | 0 | 0 | 1 | 1 | 1 | 1 | 1 | 0 | 1 | 1 | 0 |
| *set 9* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 |
| *ORF15* | 1 | 1 | 1 | 1 | 0 | 1 | 1 | 1 | 0 | 1 | 1 | 1 | 1 | 1 | 0 | 1 | 1 | 0 | 1 | 1 | 1 | 1 | 1 | 1 | 1 | 1 | 0 | 0 | 0 | 1 | 1 | 0 | 0 | 0 | 0 | 0 | 0 | 0 | 0 | 0 | 0 | 0 | 0 |
| *set12* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 |
| *SA1795* | 1 | 1 | 1 | 1 | 0 | 0 | 0 | 0 | 1 | 1 | 1 | 1 | 1 | 1 | 0 | 1 | 1 | 1 | 1 | 1 | 1 | 1 | 1 | 1 | 1 | 1 | 1 | 1 | 0 | 1 | 1 | 0 | 0 | 1 | 0 | 0 | 0 | 0 | 0 | 0 | 0 | 1 | 1 |
| *set14* | 0 | 0 | 0 | 1 | 1 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 0 | 1 | 1 | 0 | 1 | 1 | 1 | 1 | 1 | 1 | 1 | 0 | 0 | 1 | 1 |
| *lukM* | 0 | 0 | 0 | 1 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 |
| *set 8* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 |
| *MW1769* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 |
| *SA1010* | 0 | 0 | 0 | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 |
| *SAV0910* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 1 | 1 | 1 | 1 | 0 | 1 | 1 | 1 |
| *SA2272* | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 1 | 1 | 1 | 1 | 1 | 1 | 1 | 1 | 1 |
| *SAV0904* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 1 | 1 | 1 | 1 | 0 | 1 | 1 | 1 |
| *splD splF* | 1 | 0 | 1 | 1 | 1 | 1 | 1 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 |
| *splC* | 1 | 1 | 1 | 1 | 1 | 1 | 1 | 0 | 0 | 0 | 0 | 0 | 0 | 0 | 1 | 0 | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 |
| *phi int13* | 1 | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 0 | 0 | 1 | 0 | 0 | 0 | 0 | 0 | 1 | 1 | 1 | 1 |
| *SAV0866* | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 0 | 0 | 0 | 0 | 0 | 0 | 1 | 1 | 1 | 1 | 1 | 1 | 1 | 1 |
| *ORF33* | 1 | 1 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 1 | 0 | 1 | 1 | 1 | 1 | 1 | 1 | 1 | 1 | 1 | 1 | 1 | 1 | 1 | 1 |
| *MW2409* | 1 | 1 | 1 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 1 | 0 | 1 | 1 | 0 | 0 | 1 | 0 | 0 | 0 | 0 | 0 | 0 | 0 | 1 | 1 |
| *fosB* | 1 | 1 | 1 | 1 | 1 | 1 | 1 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 0 | 0 | 0 | 0 | 1 | 1 | 1 | 1 | 1 | 1 | 1 | 1 | 1 | 1 |
| *SA1801* | 1 | 1 | 1 | 1 | 1 | 1 | 1 | 0 | 1 | 0 | 0 | 0 | 0 | 0 | 0 | 1 | 1 | 1 | 1 | 1 | 1 | 1 | 1 | 1 | 1 | 1 | 1 | 1 | 1 | 0 | 1 | 0 | 0 | 1 | 0 | 1 | 1 | 1 | 1 | 1 | 1 | 1 | 1 |
| *SA0378* | 1 | 1 | 1 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 0 | 0 | 0 | 0 | 1 | 0 | 0 | 1 | 1 | 1 | 1 | 1 | 1 | 1 | 0 | 0 |
| *SA0377* | 1 | 1 | 1 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 0 | 0 | 0 | 0 | 1 | 0 | 0 | 1 | 1 | 1 | 1 | 1 | 1 | 1 | 0 | 0 |
| *SA2004* | 1 | 1 | 1 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 0 | 1 | 1 | 0 | 1 | 0 | 0 | 1 | 1 | 1 | 1 | 1 | 0 | 0 | 0 | 0 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *map* | 0 | 0 | 1 | 1 | 1 | 0 | 0 | 1 | 1 | 1 | 0 | 0 | 0 | 1 | 0 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 |
| *SA2005* | 1 | 1 | 1 | 1 | 1 | 1 | 1 | 1 | 1 | 1 | 1 | 1 | 1 | 1 | 1 | 0 | 0 | 0 | 1 | 1 | 1 | 1 | 1 | 1 | 1 | 1 | 1 | 0 | 1 | 1 | 1 | 1 | 0 | 0 | 1 | 1 | 1 | 1 | 1 | 0 | 0 | 0 | 0 |
| *sak* | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 0 | 0 | 1 | 0 | 0 | 0 | 0 | 0 | 1 | 1 | 1 | 1 |
| *blaZ* | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 1 | 0 | 0 | 1 | 1 | 0 | 1 | 1 | 1 | 1 | 1 | 1 | 1 | 1 | 1 | 1 | 0 | 1 | 1 |
| *fnbB* | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 1 | 1 | 1 | 1 |
| *set10* | 0 | 0 | 0 | 0 | 1 | 0 | 0 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 |
| *SA2314* | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *emp* | 1 | 1 | 1 | 1 | 1 | 1 | 1 | 1 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *vwb* | 1 | 1 | 1 | 1 | 1 | 1 | 1 | 0 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SAV1974* | 1 | 1 | 1 | 1 | 1 | 1 | 1 | 1 | 0 | 1 | 1 | 1 | 1 | 1 | 0 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 | 0 | 1 | 1 |
| *SAV1392* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0276* | 1 | 1 | 1 | 1 | 1 | 1 | 1 | 1 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *vraF(SA0616)* | 1 | 1 | 1 | 1 | 1 | 1 | 1 | 1 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0745* | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *agrC* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 |
| *set13* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 |
| *hlb* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *set 6* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *efb=fib* | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 |
| *set11* | 1 | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *rpoD* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *ssaA (SA2093)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *radA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA1725* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fhuD=fhuG(SA0604)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fmtC (mprF)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fus(SA0505)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fnbA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *nuc SA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *set 7* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *vraR(SA1700)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *geh* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *sdrC* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0022* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *sbi(SA2206)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *dltA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *lexA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *ORF41* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *rsbU* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *dnaK* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *sacP(SA0295)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *agrA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0610* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *hla* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA1552* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *srtA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *hysA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *ebpS* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *femB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *lytS+lytR* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *rnc(SA1076)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *hlgC* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0587* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *hlgA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *aur(SA2430)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fmtB (mrp)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *sspB (SA0900)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *sspA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *coa* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SAV2170* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *femA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0675* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0746* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *hlgB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *lip(SA2463)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0423* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *sodM* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *atl-amidase* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0977* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fabH* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA1970* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA1852* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fhuD2(SA2079)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0102* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |

| Clusters | 1 | 2 | 3 | 4 | 5 | 6 | 6 | 7 | 8 | 9 | 9 | 9 | 9 | 9 | 10 | 11 | 11 | 12 | 13 | 13 | 14 | 14 | 15 | 16 | 17 | 18 | 19 | 20 | 21 | 22 | 23 | 24 | 25 | 26 | 27 | 28 | 28 | 28 | 28 | 29 | 30 | 31 | 31 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| *clfA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *atl-glucosamin* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *hsdM* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *clpL* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *clpB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *recA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *agrJ* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA1580* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fhuB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *spa* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0691* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *icaA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *clfB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *SA0704* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *mdr* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *norA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fur* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *fhuA=fhuC(SA0602)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *putB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *plc* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *icaC* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *htrA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| *ORF30* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |

| Clusters | 32 | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|---|
| Gene | BM12681* | BM12755* | IPF505* | IPF147 | IPF157 | BM12942 | IPF083* | 97233* | 93184* | BM12714* | BM12828* | BM12830* | IPF555* & ° | BM12612* | IPF557* & ° | IPF562* & ° | BM10828* | SpainE1* | 96145* | BM10829* | BM10138* | BM10130* | FinlandE7 | BM10896* | 97130* | BM9290* | BM9343* | BM9586* & ° | BM12184* & ° | BM12188* & ° | BM10888* | BM10872* | BM10761* | BM10759* | BM12152* | BM10914* | BM3364* | |
| *tnpB (Tn554)* | 1 | 1 | 1 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 31 |
| *seg* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *SA1641* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *sei* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *tnp IS 256* | 1 | 1 | 1 | 0 | 0 | 0 | 0 | 0 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 31 |
| *seo* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *ermA* | 1 | 1 | 1 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 31 |
| *sem* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *ORF16* | 1 | 1 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 1 | 0 | 31 |
| *sen* | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 31 |
| *SAV0850* | 0 | 0 | 0 | 1 | 0 | 1 | 0 | 0 | 0 | 0 | 0 | 0 | 1 | 1 | 1 | 1 | 1 | 0 | 0 | 1 | 1 | 1 | 0 | 0 | 1 | 1 | 1 | 1 | 1 | 1 | 1 | 1 | 1 | 1 | 0 | 0 | 0 | 31 |
| *SA0054* | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 32 |
| *ORF26* | 0 | 0 | 0 | 1 | 1 | 1 | 0 | 0 | 0 | 0 | 0 | 0 | 1 | 1 | 1 | 1 | 0 | 1 | 1 | 1 | 1 | 1 | 0 | 0 | 1 | 1 | 1 | 1 | 1 | 1 | 0 | 0 | 1 | 1 | 0 | 0 | 0 | 32 |
| *ORF5* | 1 | 0 | 0 | 1 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 1 | 1 | 33 |
| *coa-8325* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 34 |
| *MW1749* | 1 | 1 | 1 | 1 | 1 | 1 | 0 | 1 | 1 | 1 | 1 | 1 | 1 | 1 | 1 | 1 | 1 | 1 | 1 | 1 | 1 | 0 | 1 | 1 | 0 | 1 | 1 | 1 | 1 | 1 | 1 | 1 | 0 | 0 | 1 | 1 | 1 | 34 |
| *SA0024* | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 34 |
| *SAV0905* | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 0 | 1 | 0 | 1 | 1 | 1 | 1 | 1 | 1 | 1 | 1 | 1 | 1 | 1 | 0 | 34 |
| *SA0142* | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 35 |
| *SAV2001* | 0 | 0 | 0 | 0 | 0 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 0 | 35 |
| *SA0141* | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 35 |
| *tnpIS 1272* | 0 | 0 | 0 | 0 | 0 | 0 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 35 |
| *SA1826* | 1 | 0 | 0 | 0 | 1 | 0 | 1 | 1 | 1 | 0 | 0 | 0 | 0 | 0 | 0 | 0 | 0 | 0 | 0 | 1 | 1 | 1 | 1 | 1 | 0 | 1 | 1 | 1 | 1 | 1 | 0 | 0 | 1 | 1 | 0 | 1 | 1 | 36 |
| *SAV0039* | 1 | 1 | 1 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 36 |

| Clusters | 32 | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|---|
| ORF18 | 1 | 1 | 1 | 1 | 1 | 1 | 1 | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 36 |
| mecA | 1 | 1 | 1 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 36 |
| SA1822 | 1 | 0 | 0 | 0 | 1 | 0 | 1 | 1 | 1 | 0 | 0 | 0 | 0 | 0 | 0 | 0 | 0 | 0 | 0 | 1 | 1 | 1 | 1 | 1 | 0 | 1 | 1 | 1 | 1 | 1 | 0 | 0 | 1 | 1 | 0 | 1 | 1 | 36 |
| MW0065 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 36 |
| mecR1-2 | 1 | 1 | 1 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 36 |
| MW0066 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 36 |
| MW1750 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 36 |
| tetM | 1 | 1 | 1 | 0 | 1 | 0 | 0 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 37 |
| MW0120 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 37 |
| MW1757 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 37 |
| ORF24 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 38 |
| ORF29 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 39 |
| ORF38 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 39 |
| MW0560 | 0 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 40 |
| entA = entP | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 41 |
| bsaC | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| bsaB | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| bsaG | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| bsaE | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| bsaF | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| SA1806 | 0 | 0 | 0 | 0 | 0 | 0 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 41 |
| bsaP | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| bsaD | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 41 |
| MW1908 | 1 | 1 | 1 | 0 | 1 | 1 | 0 | 0 | 0 | 0 | 0 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 42 |
| MW1898 | 1 | 1 | 1 | 0 | 0 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 43 |
| MW2125 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 43 |
| MW2534 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 43 |
| MW0062 | 0 | 0 | 0 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 0 | 1 | 1 | 1 | 1 | 1 | 44 |
| ORF36 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 44 |
| ORF19 | 1 | 1 | 1 | 0 | 1 | 1 | 0 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 44 |
| CAC3531 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 44 |
| MW0552 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |
| agrB-8325 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |
| ORF39 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |
| ORF28 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |
| ORF40 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |
| MW1744 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 45 |

| Clusters | 32 | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|---|
| *sigB* | 1 | 1 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 46 |
| *ORF41* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 0 | 1 | 1 | 1 | 1 | 0 | 46 |
| *ORF31* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 46 |
| *MW0064* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 46 |
| *MW0558* | 1 | 1 | 1 | 1 | 1 | 1 | 0 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 46 |
| *SA0392* | 1 | 1 | 1 | 1 | 1 | 1 | 1 | 1 | 1 | 1 | 1 | 1 | 0 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 46 |
| *SAV0804* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 1 | 47 |
| *SAV0803* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 1 | 47 |
| *MW0554* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 47 |
| *bbp=sdrE* | 1 | 1 | 1 | 1 | 1 | 0 | 1 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 47 |
| *MW0553* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 48 |
| *ORF35* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 49 |
| *MW0559* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 49 |
| *ORF34* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 49 |
| *MW0919* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 49 |
| *cap5j* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 50 |
| *ORF45* | 0 | 1 | 1 | 0 | 0 | 0 | 0 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 50 |
| *ami 8325* | 1 | 1 | 1 | 1 | 1 | 1 | 0 | 0 | 0 | 0 | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 0 | 50 |
| *cap5H* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *SA2483* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *ORF37* | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *tnp IS 1181* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *phi int12* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 0 | 1 | 1 | 1 | 1 | 1 | 1 | 1 | 1 | 1 | 1 | 51 |
| *cap5K* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *SAV0912* | 1 | 1 | 1 | 1 | 1 | 1 | 0 | 0 | 0 | 0 | 0 | 0 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 51 |
| *cap5i* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 51 |
| *SA1791* | 1 | 0 | 0 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 52 |
| *ORF3* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 52 |
| *tnp IS 431* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 52 |
| *agrC-8325* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 52 |
| *sarH2* | 1 | 1 | 1 | 1 | 1 | 0 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 53 |
| *splE* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 54 |
| *MW1742* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 54 |
| *SA1755* | 0 | 0 | 0 | 0 | 0 | 0 | 0 | 0 | 0 | 0 | 0 | 0 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 0 | 1 | 0 | 55 |
| *MW906* | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 55 |
| *SA1320* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 57 |
| *SA0104* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 57 |

| Clusters | 32 | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|---|
| *lukD* | 1 | 1 | 0 | 1 | 1 | 0 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 58 |
| *SA0103* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 58 |
| *splB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 58 |
| *SA1768* | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 0 | 59 |
| *splA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 60 |
| *ORF2* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 60 |
| *SA1766* | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *SA1778* | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *SA1777* | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *ORF42* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 60 |
| *ORF27* | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *SA1775* | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *SA1774* | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 60 |
| *sdrD* | 1 | 1 | 1 | 1 | 1 | 1 | 1 | 1 | 1 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 61 |
| *set 9* | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 61 |
| *ORF15* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 61 |
| *set12* | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 62 |
| *SA1795* | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 62 |
| *set14* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 63 |
| *lukM* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 1 | 63 |
| *set 8* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 63 |
| *MW1769* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 63 |
| *SA1010* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 64 |
| *SAV0910* | 1 | 1 | 0 | 1 | 1 | 1 | 0 | 0 | 0 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 64 |
| *SA2272* | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 64 |
| *SAV0904* | 1 | 1 | 1 | 1 | 1 | 1 | 0 | 0 | 0 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 0 | 64 |
| *splD splF* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 1 | 65 |
| *splC* | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 0 | 0 | 1 | 1 | 1 | 66 |
| *phi int13* | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 0 | 66 |
| *SAV0866* | 1 | 1 | 1 | 0 | 1 | 1 | 0 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 66 |
| *ORF33* | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 66 |
| *MW2409* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 67 |
| *fosB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 68 |
| *SA1801* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 68 |
| *SA0378* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 69 |
| *SA0377* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 69 |
| *SA2004* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 69 |

| Clusters | 32 | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|---|
| *map* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 70 |
| *SA2005* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 70 |
| *sak* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 70 |
| *blaZ* | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 0 | 1 | 1 | 1 | 1 | 1 | 1 | 1 | 70 |
| *fnbB* | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 72 |
| *set10* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 72 |
| *SA2314* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 73 |
| *emp* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 74 |
| *vwb* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 74 |
| *SAV1974* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 75 |
| *SAV1392* | 1 | 0 | 0 | 1 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 1 | 0 | 1 | 75 |
| *SA0276* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 75 |
| *vraF(SA0616)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 75 |
| *SA0745* | 1 | 1 | 1 | 1 | 1 | 1 | 1 | 1 | 1 | 1 | 1 | 1 | 1 | 0 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 76 |
| *agrC* | 1 | 1 | 1 | 1 | 1 | 1 | 1 | 0 | 0 | 1 | 1 | 1 | 1 | 1 | 1 | 1 | 1 | 1 | 1 | 0 | 1 | 1 | 1 | 1 | 1 | 1 | 1 | 1 | 1 | 1 | 1 | 1 | 1 | 1 | 1 | 1 | 1 | 76 |
| *set13* | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 77 |
| *hlb* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 0 | 0 | 1 | 1 | 0 | 1 | 1 | 77 |
| *set 6* | 1 | 1 | 1 | 1 | 1 | 1 | 1 | 1 | 1 | 1 | 1 | 1 | 1 | 1 | 0 | 0 | 1 | 1 | 1 | 1 | 1 | 1 | 1 | 1 | 1 | 1 | 1 | 1 | 1 | 1 | 1 | 1 | 1 | 1 | 1 | 1 | 1 | 78 |
| *efb=fib* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 78 |
| *set11* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 78 |
| *rpoD* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *ssaA (SA2093)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *radA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA1725* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fhuD=fhuG(SA0604)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fmtC (mprF)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fus(SA0505)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fnbA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *nuc SA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *set 7* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *vraR(SA1700)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *geh* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *sdrC* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA0022* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *sbi(SA2206)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *dltA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *lexA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |

| Clusters | 32 | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|---|
| ORF41 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| rsbU | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| dnaK | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| sacP(SA0295) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| agrA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA0610 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| hla | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA1552 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| srtA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| hysA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| ebpS | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| femB | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| lytS+lytR | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| rnc(SA1076) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| hlgC | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA0587 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| hlgA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| aur(SA2430) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| fmtB (mrp) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| sspB (SA0900) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| sspA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| coa | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SAV2170 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| femA | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA0675 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA0746 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| hlgB | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| lip(SA2463) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA0423 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| sodM | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| atl-amidase | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA0977 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| fabH | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA1970 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA1852 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| fhuD2(SA2079) | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| SA0102 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |

| Clusters | 32 | 33 | 33 | 34 | 35 | 36 | 37 | 37 | 38 | 39 | 40 | 40 | 41 | 41 | 41 | 41 | 42 | 42 | 42 | 43 | 43 | 43 | 44 | 44 | 45 | 46 | 46 | 46 | 46 | 47 | 48 | 48 | 49 | 49 | 50 | 51 | 52 | |
|---|---|---|---|---|---|---|---|---|---|---|---|---|---|---|---|---|---|---|---|---|---|---|---|---|---|---|---|---|---|---|---|---|---|---|---|---|---|---|
| *clfA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *atl-glucosamin* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *hsdM* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *clpL* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *clpB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *recA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *agrJ* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA1580* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fhuB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *spa* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA0691* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *icaA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *clfB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *SA0704* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *mdr* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *norA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fur* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *fhuA=fhuC(SA0602)* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *putB* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *plc* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *icaC* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *htrA* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |
| *ORF30* | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 80 |

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 1. | *190* | *nuc SA** | Thermonuclease | 7 / 7. | N315 | Kuroda et al. 2001 (15) | BA00018* | 1321407 | 1321831 | SEQ ID No. : 1 AAGTCAAATAAATCGCTTGC | SEQ ID No. : 2 CCCTTTTTCCACTAATTCCTT |
| 2. | *156* | *sodM** | Superoxide dismutase | 7 / 7. | NCTC8325 | | ftp://ftp.genome.ou.edu/pub/staph/ | | 2722342 | SEQ ID No. : 3 TTCATCACGACAAACATCAC | SEQ ID No. : 4 ATGTTCCAAAATGCAGTCAT |
| 3. | *189* | *nuc SI** | *S. intermedius* thermonuclease | 0 / 7. | *S. intermedius* LRA076 | Chesneau *et al.* 1992 (9) | X67678# | 694 | 999 | SEQ ID No. : 5 GGTGTGGATACACCAGAAC | SEQ ID No. : 6 GCGTTCCCAAATGTTCAGCTG |

TABLE I

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 1. | 5 | *aadE* | Streptomycin adenyltransferase: AAD6' | 0 / 7. | BM3121(Tn*5405*) | Derbise et al. 1996 (11) | AF330699# | 75 | 530 | SEQ ID No. : 7 TGAACGTATTCGAATTGTGA | SEQ ID No. : 8 CGGCACAATCCTTTAATAAC |
| 2. | 9 | *aphA-3* | Aminoside phosphotransferase: APH3'-III | 0 / 7. | BM3121(Tn*5405*) | Derbise et al. 1996 (11) | U51474# | 1106 | 1549 | SEQ ID No. : 9 TAAAGGGACCACCTATGATG | SEQ ID No. : 10 ACAAAGATGTTGCTGTCTCC |
| 3. | 12 | *ermB* | ARNr 23S methylase: MLS resistance | 0 / 7. | *E.coli* TG1(pEC3) | Brückner, 1997 (8) | Y13600# | 721 | 1079 | SEQ ID No. : 11 GTTTACGAAATTGGAACAGG | SEQ ID No. : 12 TGAATCGAGACTTGAGTGTG |
| 4. | 13 | *ermC* | ARNr 23S methylase: MLS resistance | 0 / 7. | RN450(pE194) | | V01278# | 2077 | 2520 | SEQ ID No. : 13 TGATCACGATAATTTCCAAGT | SEQ ID No. : 14 ATTCCTGCATGTTTTAAGGA |
| 5. | 14 | *ermY* | ARNr 23S methylase: MLS resistance | 0 / 7. | RN4220(pMS97) | Matsuoka *et al.* 2002 (17) | AB014481# | 680 | 1065 | SEQ ID No. : 15 GGGCATTTCACACTAGAACT | SEQ ID No. : 16 TTAATTTTAGGCTTTGGATG |
| 6. | 15 | *mphC* | Erythromycine phosphotransferase | 0 / 7. | RN4220(pMS97) | Matsuoka *et al.* 2002 (17) | AF167161# | 5866 | 6298 | SEQ ID No. : 17 ATTTGAAATACCGAAGTGGA | SEQ ID No. : 18 CGTTTGCTTGGTTATCTACC |
| 7. | 16 | *msrA* | ABC protein : MS resistance | 0 / 7. | RN4220(pMS97) | Matsuoka *et al.* 2002 (17) | X52085# | 440 | 846 | SEQ ID No. : 19 AAATAGCACTTATTGGGGGT | SEQ ID No. : 20 TGTGTCAGCAATTTGGTCTA |
| 8. | 17 | *msrC* | ABC protein : MS resistance | 0 / 7. | *E.faecium* CIP107387 | | AY004350# | 680 | 1047 | SEQ ID No. : 21 TCCAAACAGAGATCACCTTC | SEQ ID No. : 22 ATAATTCCCCGAAAACTCTC |
| 9. | 18 | *lnuA* | Lincosamide nucleotidyltransferase | 0 / 7. | *S.epidermidis* BM12911 | This study | J03947# | 715 | 1075 | SEQ ID No. : 23 ACTCATTGGTTAGATGGAGG | SEQ ID No. : 24 AACCGGAATGAAAAAGAAGT |
| 10. | 19 | *lnuB* | Lincosamide nucleotidyltransferase | 0 / 7. | *E. faecium* HM1025 | Bozdogan *et al.* 1999 (7) | AF110130# | 380 | 795 | SEQ ID No. : 25 CTTATACGTGGGGAATTTCA | SEQ ID No. : 26 TCTAATCGAGCAGTGGTCTT |
| 11. | 20 | *lsa* | ABC protein: streptogramin A and lincosamide resistance | 0 / 7. | *E. faecalis* V 583 | This study | AE016830# | 2631318 | 2631705 | SEQ ID No. : 27 CGTCTAGGCTACGAGAAAAA | SEQ ID No. : 28 11AAAGCTCTGCTGATTGAGAC |
| 12. | 21 | *vga* | ABC protein: Streptogramin A resistance | 0 / 7. | BM3093 (pIP680) | Allignet & El Solh, 1999 (1) | M90056# | 964 | 1374 | SEQ ID No. : 29 TGTTGAACATAAATCGCCTA | SEQ ID No. : 30 CAGTTATTCTTCCCTCGTCA |
| 13. | 22 | *vgaB** | ABC protein: Streptogramin A resistance | 0 / 7. | BM3385 (pIP1156) | Haroche *et al.* 2002 (14) | U82085# | 842 | 1213 | SEQ ID No. : 31 TCTCTCAATTAGAAGAACCACA | SEQ ID No. : 32 TTGCTTTCGTAGAAGCATTT |
| 14. | 23 | *vgav* | ABC protein: Streptogramin A resistance | 0 / 7. | BM3327 | Haroche *et al.* 2002 (14) | AF186237# | 5153 | 5545 | SEQ ID No. : 33 ATGACCGAATTGGGTTAGTT | SEQ ID No. : 34 AGTTCCCTTTGTATTCGGTT |
| 15. | 24 | *vatA* | Streptogramin A acetyl transferase | 0 / 7. | BM3093(pIP680) | Allignet & El Solh, 1999 (1) | L07778# | 390 | 746 | SEQ ID No. : 35 AGTAAGCGAGGAGAATCCTT | SEQ ID No. : 36 TCCACCGACAATAGAATAGG |
| 16. | 25 | *vatB* | Streptogramin A acetyl transferase | 0 / 7. | BM3385 (pIP1156) | Haroche *et al.* 2002 (14) | U19459# | 72 | 495 | SEQ ID No. : 37 ATATGGCCCTGATCCAAATA | SEQ ID No. : 38 AACAATTGCTCCATCTCCTA |
| 17. | 26 | *vatC* | Streptogramin A acetyl transferase | 0 / 7. | BM10711( (pIP1714) | Allignet *et al.* 1998 (1) | AF015628# | 1388 | 1741 | SEQ ID No. : 39 ACCATCTATAACAAAGCCCA | SEQ ID No. : 40 ATAATAGCCCCGTTTCCTAT |
| 18. | 27 | *vatD* | Streptogramin A acetyl transferase | 0 / 7. | *E.faecium* KH4 | Haroche et *al.* 2000 (13) | L12033# | 189 | 782 | SEQ ID No. : 41 CCTATAGAAGGAAACAAATCAG | SEQ ID No. : 42 CCATATGACTTCTCTAATGATGC |
| 19. | 28 | *vatE* | Streptogramin A acetyl transferase | 0 / 7. | *E.faecium*K14 | Haroche et *al.* 2000 (13) | AF153312# | 414 | 832 | SEQ ID No. : 43 GAAACACGTTACCCATCACT | SEQ ID No. : 44 TATCTTTATTCCACCATGCC |
| 20. | 29 | *vgb* | Streptogramine B lyase | 0 / 7. | BM3093(pIP680) | Allignet & El Solh, 1999 (1) | M20129# | 938 | 1333 | SEQ ID No. : 45 GCACCCTACGGTATTACAGA | SEQ ID No. : 46 AGTAATTGCATGAGGTCGAG |
| 21. | 30 | *vgbB* | Streptogramine B lyase | 0 / 7. | BM10711 | Allignet *et al.* 1998 (3) | AF015628# | 625 | 1019 | SEQ ID No. : 47 GTGCAAATAAAATCGGAAAG | SEQ ID No. : 48 ACCTATTTTGTTGCCCATAA |

TABLE II

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 22. | 32 | *tetL* | Tetracycline resistance | 0 / 7. | *E.coli*BM8610 | Schwarz *et al.* 1992 (20) | X60828# | 977 | 1413 | SEQ ID No. : 49 GAACAGTAGCAGGGTTTGTC | SEQ ID No. : 50 TGGATCAAGTAAGGGTATGG |
| 23. | 34 | *cat* | Chloramphenicol resistance | 0 / 7. | RN450(pC194) | | V01277# | 1469 | 1858 | SEQ ID No. : 51 TTACAATAGCGACGGAGAGT | SEQ ID No. : 52 AACAATCCTGCATGATAACC |
| 24. | 35 | *far1* | Fucidic acid resistance | 0 / 7. | WBG1576(pIB101) | O'Brien *et al.* 2002 (18) | AF468686# | 1762 | 2195 | SEQ ID No. : 53 TCAAAGTTATTCAATCGGAAA | SEQ ID No. : 54 CGAATTGGTTTTTGACTTTT |
| 25. | 36 | *mupA* | Mupirocin resistance | 0 / 7. | IPF874 | This study | X75439# | 3064 | 3415 | SEQ ID No. : 55 CTTCTGTGGGACCAAAACTA | SEQ ID No. : 56 AATAATTCCCCAGTTACACC |
| 26. | 37 | *dfrA* | Triméthoprime resistance | 0 / 7. | *S.epidermidis* BM10393(pIP1630) | Aubert *et al.* 1998 (4) | AF045472# | 5876 | 6271 | SEQ ID No. : 57 AATTGTCGCTCACGATAAAC | SEQ ID No. : 58 GAAGATTCGACTTCCCAGTT |
| 27. | 38 | *fos* | Fosfomycin resistance | 0 / 7. | *S.epidermidis* IPF116 | This study | X54227# | 729 | 1076 | SEQ ID No. : 59 AAATCATATTACTTATTCGGTTTC | SEQ ID No. : 60 AAACTTCCTGTATGCAATTCTAA |
| 28. | 40 | *vanA* | Resistance to vancomycin | 0 / 7. | *E.faecium* CIP107387 | | M97297# | 7562 | 7939 | SEQ ID No. : 61 AATCGGCAAGACAATATGAC | SEQ ID No. : 62 TACGGGGATAACGACTGTAT |
| 29. | 41 | *vanB* | Resistance to vancomycin | 0 / 7. | *E. faecalis* CIP104676 | | U00456# | 73 | 472 | SEQ ID No. : 63 AAAAGTCGCAATTATCTTCG | SEQ ID No. : 64 AATGTAGGCCAGTGATTTGT |
| 30. | 42 | *vanC* | Resistance to vancomycin | 0 / 7. | *E. gallinarum* CIP105985 | | AF162694# | 1499 | 1918 | SEQ ID No. : 65 ACCCGCTGAAATATGAAGTA | SEQ ID No. : 66 GGAATCCATGGTCTTGAATA |
| 31. | 43 | *sat4* | Streptothricine-acetyl-transferase | 0 / 7. | BM3121(Tn*5405*) | Derbise et al. 1996 (11) | U73026# | 202 | 559 | SEQ ID No. : 67 GAACCATTTGAGGTGATAGG | SEQ ID No. : 68 AGCATTAGTCCATGCAAGTT |
| 32. | 44 | *qacA* | Antiseptic resistance | 0 / 7. | SK2355(pSK1) | Paulsen *et al.* 1998 (19) | AF053772# | 1259 | 1648 | SEQ ID No. : 69 TTATGGCTTTACCGGAATTA | SEQ ID No. : 70 CAAGTAAAGCTCCTCCGATA |
| 33. | 45 | *smr(qacC)** | Antiseptic resistance | 0 / 7. | SK5428(pSK41) | Berg *et al.* 1998 (6) | U50077# | 1589 | 1868 | SEQ ID No. : 71 CTGAAGTTATTGGAAGTGCAT | SEQ ID No. : 72 GTTCCGAAAATGTTTAACGA |
| 34. | 48 | *CZ040* | SSCmecIII Organomercurial lyase | 0 / 7. | IFP805(SCCmecIII) | | AB037671# | 37755 | 38199 | SEQ ID No. : 73 TATTTCAGAATTCTCAGCCC | SEQ ID No. : 74 TGGTGTAACAATCGAGACAA |
| 35. | 49 | *CZ041* | SSCmecIII Mercuric reductase | 0 / 7. | IFP805(SCCmecIII) | | AB037671# | 39293 | 39704 | SEQ ID No. : 75 AGTGTAGATTTCAATCGGGA | SEQ ID No. : 76 TAGACGTGATGTTTTGTCCA |
| 36. | 59 | *entD* | Enterotoxin D | 0 / 7. | FR1171 (pIB485) | Zhang *et al.* 1998 (23) | M28521# | 450 | 901 | SEQ ID No. : 77 TTCTTATGCAGATAAAAATCCA | SEQ ID No. : 78 TTTTTCCTCCGAGAGTATCA |
| 37. | 63 | *sej* | Enterotoxin J | 0 / 7. | FR1171 (pIB485) | Zhang *et al.* 1998 (23) | AF053140# | 1081 | 1560 | SEQ ID No. : 79 CGAAAAGGGTATCTCTGAAA | SEQ ID No. : 80 AACGGTTCTTTTGAGGTATG |
| 38. | 82 | *eta* | Exfoliative toxin A | 0 / 7. | IPF529 | This Study | L25372# | 511 | 916 | SEQ ID No. : 81 AAAACATGAAGAGAAATGGAA | SEQ ID No. : 82 TTTTGCTGGCGATATTTTAT |
| 39. | 83 | *etd* | Exfoliative toxin D | 0 / 7. | TY114 | Yamaguchi *et al.* 2002 (22) | AB057421# | 5457 | 5885 | SEQ ID No. : 83 ATATTTCTCTCCCCGTTGAT | SEQ ID No. : 84 TCCGTCTTTATTGGGTTTTA |
| 40. | 96 | *edinB* | Epidermall cellular diferenciation inhibitor B | 0 / 7. | TY114 | Yamaguchi *et al.* 2002 (22) | AB057421# | 7254 | 7720 | SEQ ID No. : 85 TAGCTGCCGAGACTAAAAAT | SEQ ID No. : 86 TAATTCAATTGGCCTACCTG |
| 41. | 130 | *sdrG* | Fibrinogen-binding Serine-Aspartate protein | 0 / 7. | *S.epidermidis* RP62A | | AF245042# | 143 | 554 | SEQ ID No. : 87 CCAAAGCTGAGGAGAATACA | SEQ ID No. : 88 CGGGAATTTTCTTCATTATC |
| 42. | 131 | *sdrH** | Serine-Aspartate protein | 0 / 7. | *S.epidermidis* RP62A | | AF245043# | 22* | 206* | SEQ ID No. : 89 CATTCATTTATGCTTACGGG | SEQ ID No. : 90 TTCATACTAATTCCTGTGCG |
| 43. | 132 | *sdrY* | Serine-Aspartate protein | 0 / 7. | *S.caprae* 96007 | Allignet *et al.* | AY048594# | 115 | 532 | SEQ ID No. : 91 | SEQ ID No. : 92 |

TABLE II

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| | | | | | | 1999 (2) | | | | AAACAACCATAACGTCCAAC | GCGATGTTTGTAACTGTGAA |
| 44. | 133 | *sdrZ** | Serine-Aspartate protein | 0 / 7. | *S.caprae* 96007 | Allignet *et al.* 1999 (2) | AY048595# | 698* | 933* | SEQ ID No. : 93 TTGTATTAACAGGCTTTGCC | SEQ ID No. : 94 ACCTCACTCGTTGTATTTGC |
| 45. | 143 | *bap* | Biofilm associated surface protein | 0 / 7. | V329 | Cucarella *et al.* 2001 (10) | AF288402# | 6460 | 6868 | SEQ ID No. : 95 GGCAATACTTCTGAAAGTACC | SEQ ID No. : 96 TTCTACGACGTTTGGTAAATA ATA |
| 46. | 146 | *cap1G* | Capsular polysaccharide synthesis Cap1G | 0 / 7. | M strain | Luong *et al.* 2002 (16) | U10927# | 17901 | 18305 | SEQ ID No. : 97 GATAAGATAAGGAAATTGGGT G | SEQ ID No. : 98 TGTGTAATCCGCATTTTCTA |
| 47. | 193 | tnpA (Tn*5406*)* | Tn *5406A* transposase | 0 / 7. | BM3327 (Tn5406) | | AF186237# | 1913 | 2276 | SEQ ID No. : 99 AAATCGTAGAAGCCTGTCAC | SEQ ID No. : 100 CCTGTTCTTTTATTCAACCG |
| 48. | 194 | tnpB (Tn*5406*)* | Tn *5406B* transposase | 0 / 7. | BM3327 " | - | AF186237# | 2541 | 2922 | SEQ ID No. : 101 TTAGAAGGATATTGGGCTGA | SEQ ID No. : 102 TTCTTTCTTGTTTGGCATTT |
| 49. | 196 | tnp IS *256* | IS *256* transposase | 0 / 7. | BM3121 (IS5256) | Derbise et al. 1996 (11) | AJ416751# | 182 | 557 | SEQ ID No. : 103 GAAAAGCGAAGAGATTCAAA | SEQ ID No. : 104 AGAGACGGATTTACCACAAA |
| 50. | 199 | tnp IS *1182* | IS *1182* transposase | 0 / 7. | BM3121 (IS1182) | Derbise et al. 1996 (11) | L43082# | 2219 | 2586 | SEQ ID No. : 105 TGTCCCAATCAGAAAAGAGT | SEQ ID No. : 106 AAATTGTGCGCAAGAAATAC |
| 51. | 207 | *orfX* | Encoded protein Tn*5405* | 0 / 7. | BM3121 | Derbise et al. 1996 (11) | U73025# | 1428 | 1822 | SEQ ID No. : 107 CCATACAGAGGATTCGGATA | SEQ ID No. : 108 AACCCTGCTTCAAACATAAA |
| 52. | 208 | *orfZ* | Encoded protein Tn*5405* | 0 / 7. | BM3121 | Derbise et al. 1996 (11) | U73027# | 182 | 557 | SEQ ID No. : 109 GCTTTGATAATCCGGAAGTT | SEQ ID No. : 110 TGTCTGTCCGCATGTATTTA |
| 53. | 211 | *hsdS*-TY114 | Restriction modification enzyme subunit HsdS | 0 / 7. | TY114 | Yamaguchi *et al.* 2002 (22) | AB057421# | 2401 | 2900 | SEQ ID No. : 111 AACAAGGTTTGATTCGAAAA | SEQ ID No. : 112 ACATCGATTGAAGTAAGCCT |
| 54. | 251 | *rep pC194** | pC194 Rep protein | 0 / 7. | NCTC8325 (pC194) | | V01277# | 714 | 1041 | SEQ ID No. : 113 TTGATTCTCTCCAATATGACG | SEQ ID No. : 114 TTAAAGGATTTGAGCGTAGC |
| 55. | 252 | *rep1*pIP 1629* | pIP1629 replication protein | 0 / 7. | *S.epidermidis* BM10385 | Aubert *et al.* 1998 (4) | AF045240# | 3952 | 4320 | SEQ ID No. : 115 AACTTAGAGAAATGACACACC TT | SEQ ID No. : 116 CCAACCATTTTGGTGTTTT |
| 56. | 253 | *traA* ( *pSK1)* | Transfert protein from pSK1 plasmid | 0 / 7. | SK5428(pSK41) | Firth *et al.* 1993 (12) | AF051917# | 24286 | 24643 | SEQ ID No. : 117 AATAAGTTAGTCATGGCCGA | SEQ ID No. : 118 ATGCTGTTGCTGTAAGTCCT |
| 57. | 254 | *traC* ( *pSK1)* | Transfert protein from pSK1 plasmid | 0 / 7. | SK5428(pSK41) | Firth *et al.* 1993 (12) | AF051917# | 25493 | 25897 | SEQ ID No. : 119 TGTCTGTTGAGCAAGAAGTT | SEQ ID No. : 120 AAACATCATCATCACCCAAT |
| 58. | 255 | *traD* ( *pSK1)* | Transfert protein from pSK1 plasmid | 0 / 7. | SK5428(pSK41) | Firth *et al.* 1993 (12) | AF051917# | 25953 | 26325 | SEQ ID No. : 121 GATATATAAAAGGGGACGGA | SEQ ID No. : 122 CCTTTTGCTAGCTTTTCTTG |
| 59. | 256 | *traE* ( *pSK1)* | Transfert protein from pSK1 plasmid | 0 / 7. | SK5428(pSK41) | Firth *et al.* 1993 (12) | AF051917# | 28053 | 28423 | SEQ ID No. : 123 CAGTTTCTTATGGACGAAA | SEQ ID No. : 124 ATGCTCTTTGTGTTCCGTAT |
| 60. | 257 | *traF* ( *pSK1)* | Transfert protein from pSK1 plasmid | 0 / 7. | SK5428(pSK41) | Firth *et al.* 1993 (12) | AF051917# | 28638 | 28993 | SEQ ID No. : 125 GGAACATTGGTAGGAAAACA | SEQ ID No. : 126 GGTAAATATTCATGGCCTTT |
| 61. | 258 | *traG* ( *pSK1)* | Transfert protein from pSK1 plasmid | 0 / 7. | SK5428(pSK41) | Firth *et al.* 1993 (12) | AF051917# | 30085 | 30473 | SEQ ID No. : 127 AATGAAAGAGGCTGAATCAA | SEQ ID No. : 128 CATTACTAGCGCCTACTGGT |
| 62. | 259 | *traH* ( *pSK1)* | Transfert protein from pSK1 plasmid | 0 / 7. | SK5428(pSK41) | Firth *et al.* 1993 (12) | AF051917# | 31026 | 31405 | SEQ ID No. : 129 TATTCACTTTGGTTCTGGCT | SEQ ID No. : 130 CGTTAATTCAATTTTCCCAT |
| 63. | 260 | *traI* ( | Transfert protein from pSK1 | 0 / 7. | SK5428(pSK41) | Firth *et al.* 1993 | AF051917# | 31621 | 32000 | SEQ ID No. : 131 | SEQ ID No. : 132 |

TABLE II

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| | | *pSK1)* | plasmid | | | (12) | | | | CTTTTTAACTTGGGCAGTTG | TGCCCTCTTTGTAAAATCC |
| 64. | 261 | *traJ* ( *pSK1)* | Transfert protein from pSK1 plasmid | 0 / 7. | SK5428(pSK41) | Firth *et al.* 1993 (12) | AF051917# | 33637 | 33986 | SEQ ID No. : 133 TTTAACTGGTATTATTTGGCTAGT | SEQ ID No. : 134 CTTCTGAAAACAATTCGCTA |
| 65. | 262 | *traK* ( *pSK1)* | Transfert protein from pSK1 plasmid | 0 / 7. | SK5428(pSK41) | Firth *et al.* 1993 (12) | AF051917# | 34276 | 34660 | SEQ ID No. : 135 AGTGGCGAAGTTTATGAAAA | SEQ ID No. : 136 CTCTAGCTGGGTGGTCTTTA |
| 66. | 263 | *traL* ( *pSK1)* | Transfert protein from pSK1 plasmid | 0 / 7. | SK5428(pSK41) | Firth *et al.* 1993 (12) | AF051917# | 36272 | 36623 | SEQ ID No. : 137 AGGTTCTATGGGGACTATGG | SEQ ID No. : 138 TGATCTAATAAAGGAAGGCG |
| 67. | 264 | *traM* ( *pSK1)* | Transfert protein from pSK1 plasmid | 0 / 7. | SK5428(pSK41) | Firth *et al.* 1993 (12) | AF051917# | 36748 | 37080 | SEQ ID No. : 139 TATAGTGGACGTATCGCAAC | SEQ ID No. : 140 ACTATCTTCTGCTGGGCTTA |

TABLE II

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 1. | 50 | *fmtB(mrp)* | FmtB : Beta-lactam resistance regulator | 7 / 7. | N315 | Kuroda et al. 2001 (15) | BA00018* | 2224911 | 2225407 | SEQ ID No. : 141 ATGCCAATACACAGCCTAAC | SEQ ID No. : 142 ATCGCTTCGTCTTAATTCTG |
| 2. | 51 | *fmtC(mrpF)* | FmtC : Beta-lactam resistance regulator | 7 / 7. | N315 | Kuroda et al. 2001 (15) | BA00018* | 1363771 | 1364209 | SEQ ID No. : 143 GGCATCGCTTGTTATTCTAT | SEQ ID No. : 144 CCATTCAACACACGACACTA |
| 3. | 52 | *dltA* | D-alanine-D-alanyl carrier protein ligase | 7 / 7. | N315 | Kuroda et al. 2001 (15) | BA00018* | 898568 | 898974 | SEQ ID No. : 145 GAAACCGATGATTTTATTCG | SEQ ID No. : 146 CTTGGTTAAGCCATTGTTGT |
| 4. | 54 | *femB* | Beta-lactam resistance regulator | 7 / 7. | NCTC8325 | | ftp://ftp.genome.ou.edu/pub/staph/ | 1502954 | 1503386 | SEQ ID No. : 147 CAGCAAGCCTTTTCTCTAAA | SEQ ID No. : 148 CGTTCAAGGAATCTGACTTT |
| 5. | 55 | *femA* | Beta-lactam resistance regulator | 7 / 7. | NCTC8325 | | ftp://ftp.genome.ou.edu/pub/staph/ | 1504363 | 1504812 | SEQ ID No. : 149 ACAGCTAAAGAGTTTGGTGC | SEQ ID No. : 150 ACGAATTTGTAGCACAGGAT |
| 6. | 70 | *set6** | Exotoxin 6 | 7 / 7. | N315 | Kuroda et al. 2001 (15) | BA00018* | 441064 | 441489 | SEQ ID No. : 151 TTGGGGCTAAAAATTATGAA | SEQ ID No. : 152 TTCAAGGATTGGTGTGTGTA |
| 7. | 71 | *set7* | Exotoxin 7 | 7 / 7. | N315 | Kuroda et al. 2001 (15) | BA00018* | 442299 | 442701 | SEQ ID No. : 153 AATTGGCAAAGATAAGCAAC | SEQ ID No. : 154 TGTGACATTGATAACATCGG |
| 8. | 78 | *set13** | Exotoxin 13 | 7 / 7. | N315 | Kuroda et al. 2001 (15) | BA00018* | 449130 | 449530 | SEQ ID No. : 155 TGAATCAACAAACATTAGCG | SEQ ID No. : 156 TATCTGACGCGCCTTTATAC |
| 9. | 79 | *set14* | Exotoxin 14 | 7 / 7. | N315 | Kuroda et al. 2001 (15) | BA00018* | 450110 | 450593 | SEQ ID No. : 157 AGGTCATGCAAAACAAAATC | SEQ ID No. : 158 TTTGACCCTACCATCTTTTG |
| 10. | 84 | *hlb* | Beta-hemolysin | 7 / 7. | COL | | http://www.tigr.org/tigr-scripts/CMR2/GenomePage3.spl?database=gsa : SA2003 | 157 | 597 | SEQ ID No. : 159 TTATCGACCGTTTTGTATCC | SEQ ID No. : 160 AATTTTTCGATCATGTCCAG |
| 11. | 85 | *hla* | Alpha-hemolysin | 7 / 7. | N315 | Kuroda et al. 2001 (15) | BA00018* | 1140980 | 1141442 | SEQ ID No. : 161 AATGAATCCTGTCGCTAATG | SEQ ID No. : 162 TTTCAGTGTATGACCAATCG |
| 12. | 86 | *hlgA* | Gamma-hemolysin component A | 7 / 7. | N315 | Kuroda et al. 2001 (15) | BA00018* | 2478121 | 2478563 | SEQ ID No. : 163 TGCCCTAGTTGTTAAGATGC | SEQ ID No. : 164 CGAAATAGTCTCTTGCTGCT |
| 13. | 87 | *hlgB* | Gamma-hemolysin component B | 7 / 7. | N315 | Kuroda et al. 2001 (15) | BA00018* | 2480769 | 2481204 | SEQ ID No. : 165 GCTATACATTTGGTGGTGACA | SEQ ID No. : 166 TAGAAGCCATTCCAACGAA |
| 14. | 88 | *hlgC* | Gamma-hemolysin component C | 7 / 7. | MW2 | Baba et al. 2002 (5) | BA00033* | 2493193 | 2493643 | SEQ ID No. : 167 AATCATGTTAAAGCTATGCGA | SEQ ID No. : 168 CATGAGATACTGTGGCGATA |
| 15. | 89 | SAV2170* | Putative hemolysin | 7 / 7. | Mu50 | Kuroda et al. 2001 (15) | BA00017* | 2311201 | 2311623 | SEQ ID No. : 169 GAGAAGAAATTGGTAATGCG | SEQ ID No. : 170 AGATAATACCTACCCAGCCC |
| 16. | 93 | *lukM*(SA1813) | Putative leucocidine | 7 / 7. | N315 | Kuroda et al. 2001 (15) | BA00018* | 2051768 | 2052257 | SEQ ID No. : 171 AATATTAGACCAATTGCCGA | SEQ ID No. : 172 TTCTAAAATTGGAGGTGCAT |
| 17. | 94 | *hySA** | Hyaluronate lyase precursor | 7 / 7. | NCTC8325 | | ftp://ftp.genome.ou.edu/pub/staph/ | 535974 | 535555 | SEQ ID No. : 173 ATGTTCAAACGCCAGATTAT | SEQ ID No. : 174 GTTCCAATTTCATAATCCCA |
| 18. | 95 | *aur*(SA2430) | Zinc metalloproteinase aureolysin | 7 / 7. | N315 | Kuroda et al. 2001 (15) | BA00018* | 2727848 | 2728327 | SEQ ID No. : 175 CAAGTATGGCAGCAGTAACA | SEQ ID No. : 176 TACTGTCACCATCGATTTCA |
| 19. | 98 | *spa* | Immunoglobulin G binding protein A precursor | 7 / 7. | N315 | Kuroda et al. 2001 (15) | BA00018* | 123374 | 123869 | SEQ ID No. : 177 CGCAACACGATGAAGCTC | SEQ ID No. : 178 TTAGCTTCTGACAATAGGTTAGC |
| 20. | 99 | *putB* | High affinity proline permease | 7 / 7. | N315 | Kuroda et al. 2001 (15) | BA00018* | 1965751 | 1966117 | SEQ ID No. : 179 CAACTGGTAACCTAAGCGAG | SEQ ID No. : 180 TTACCACCAGATACGAAACC |

TABLE III

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 21. | 100 | *sspA** | Staphylococcal serine protease V8 | 7 / 7. | NCTC8325 | | ftp://ftp.genome.ou.edu/pub/staph/ | 1861638 | 1862002 | SEQ ID No. : 181 AAAAGGCGGTAACCTTAAAC | SEQ ID No. : 182 GTTTGTTTTGCTCATTAGGG |
| 22. | 102 | *splC** | Serine protease SplC | 7 / 7. | NCTC8325 | | ftp://ftp.genome.ou.edu/pub/staph/ | 976188 | 976646 | SEQ ID No. : 183 CAGCATTAGCCATTCTAACC | SEQ ID No. : 184 TATTTTGAGCAGGTAATGGG |
| 23. | 104 | *splD = splF* | Serine protease SplD, splF | 7 / 7. | N315 | Kuroda et al. 2001 (15) | BA00018* | 1862021 | 1862520 | SEQ ID No. : 185 ATTAATTACCAACACGAATGT | SEQ ID No. : 186 ACATAACACCAATTGCTTCTC |
| 24. | 106 | *htrA* | Serine protease HtrA | 7 / 7. | N315 | Kuroda et al. 2001 (15) | BA00018* | 997307 | 997776 | SEQ ID No. : 187 GAACAGAGAAACCGTGATGT | SEQ ID No. : 188 TCATCAGATTCCGACAATTT |
| 25. | 108 | *clpB* | ClpB chaperone protein | 7 / 7. | NCTC8325 | | ftp://ftp.genome.ou.edu/pub/staph/ | 1936815 | 1937301 | SEQ ID No. : 189 ATATTGGACATGATGAAGGG | SEQ ID No. : 190 CCGTATTGAGGTTCATAAGC |
| 26. | 109 | *clpL** | ATP-dependent Clp proteinase chain clpL | 7 / 7. | NCTC8325 | | ftp://ftp.genome.ou.edu/pub/staph/ | 186918 | 187405 | SEQ ID No. : 191 GAAGAACTAGCGCAACTCAC | SEQ ID No. : 192 CCGATAATTTGATGGATTTC |
| 27. | 110 | SA1725 | Cysteine protease : staphylopain | 7 / 7. | N315 | Kuroda et al. 2001 (15) | BA00018* | 1976670 | 1977131 | SEQ ID No. : 193 TGTAACGCAACTAAAGCAAA | SEQ ID No. : 194 TCTAATTTAGCGTTCCCAAC |
| 28. | 111 | *sspB**(SA0900) | Cysteine protease | 7 / 7. | N315 | Kuroda et al. 2001 (15) | BA00018* | 1021027 | 1021439 | SEQ ID No. : 195 AAGCCGATTCACACTCTAAA | SEQ ID No. : 196 TTTGCCATCTTCTTCAAAAT |
| 29. | 112 | *srtA* | Sortase | 7 / 7. | N315 | Kuroda et al. 2001 (15) | BA00018* | 2599922 | 2600316 | SEQ ID No. : 197 AATCGATTAATGACAATCGC | SEQ ID No. : 198 TTGGCTGCTTTAAGATTTGT |
| 30. | 113 | *vwb** | Secreted von Willebrand factor-binding protein | 7 / 7. | NCTC8325 | | ftp://ftp.genome.ou.edu/pub/staph/ | 2024553 | 2025052 | SEQ ID No. : 199 AAAATCACAAACCTGCATCT | SEQ ID No. : 200 AATTACACGCTTCTCACGTT |
| 31. | 114 | *fnbA* | Fibronectin-binding protein | 7 / 7. | NCTC8325 | | ftp://ftp.genome.ou.edu/pub/staph/ | 237031 | 237514 | SEQ ID No. : 201 GCATCAGAACAAAGACAAC | SEQ ID No. : 202 TTACATCTGTACCCGTTTCC |
| 32. | 115 | *fnbB* | Fibronectin binding protein B | 7 / 7. | NCTC8325 | | ftp://ftp.genome.ou.edu/pub/staph/ | 240820 | 241284 | SEQ ID No. : 203 GAAAGTAAAGCAGCGAAAC | SEQ ID No. : 204 AATTCCTTCTCCAAATTTCC |
| 33. | 116 | *efb=fib* | Fibrinogen-binding Serine-Aspartate protein | 7 / 7. | N315 | Kuroda et al. 2001 (15) | BA00018* | 1135800 | 1136161 | SEQ ID No. : 205 CTACAATTGCGTCAACAGC | SEQ ID No. : 206 CGAAACTAAGTTGACTGCCT |
| 34. | 117 | *coa** | Staphylocoagulase (conserved domain) | 7 / 7. | N315 | Kuroda et al. 2001 (15) | BA00018* | 265638 | 266105 | SEQ ID No. : 207 GAAACCAGCAAGAGGTTAAA | SEQ ID No. : 208 CGTTGTATTCACGGATACCT |
| 35. | 120 | *ebpS* | Cell surface elastin binding protein EbpS | 7 / 7. | NCTC8325 | | ftp://ftp.genome.ou.edu/pub/staph/ | 1366823 | 1367261 | SEQ ID No. : 209 AGACCATACGGAAGATGTTG | SEQ ID No. : 210 TGGTTTAGGTTGCTGAGATT |
| 36. | 121 | *ssaA*(SA2093) | Secretory antigen precursor SsaA homolog | 7 / 7. | NCTC8325 | | ftp://ftp.genome.ou.edu/pub/staph/ | 458370 | 458868 | SEQ ID No. : 211 ACGACCCAACATCATATAGC | SEQ ID No. : 212 ACTGTGTAACCAGCTCTTGC |
| 37. | 122 | *map** | Cell surface binding protein | 7 / 7. | NCTC8325 | | ftp://ftp.genome.ou.edu/pub/staph/ | 790624 | 791042 | SEQ ID No. : 213 ATAATAATGAAGCGTCTGCC | SEQ ID No. : 214 TGGAACTTGGTAATTTGCTT |
| 38. | 123 | *emp* | Plasma binding protein | 7 / 7. | N315 | Kuroda et al. 2001 (15) | BA00018* | 853958 | 854406 | SEQ ID No. : 215 CATTACTACGCAAACCCACT | SEQ ID No. : 216 AACCATTTGATTGTGAAAATG |
| 39. | 127 | *clfB** | Serine- Aspartate Clumping factor B | 7 / 7. | NCTC8325 | | ftp://ftp.genome.ou.edu/pub/staph/ | 95930 | 96341 | SEQ ID No. : 217 ATAATGCAAGTGCGATTCC | SEQ ID No. : 218 GCTCTCGTTCTAACACTTGG |
| 40. | 128 | *sdrC* | Serine-Aspartate protein | 7 / 7. | COL | | http://www.tigr.org/tigr-scripts/CMR2/GenomePage3.spl?database=gsa : SA0608 | 115 | 552 | SEQ ID No. : 219 TTGATTTTTGGGTTAAGTGG | SEQ ID No. : 220 ATTTGTTCCTTGTTGTGGAG |
| 41. | 135 | *fus*(SA0505) | Elongation factor G and vitronectin-binding protein | 7 / 7. | N315 | Kuroda et al. 2001 (15) | BA00018* | 589898 | 590373 | SEQ ID No. : 221 CTCCAATGGTTTCATATCGT | SEQ ID No. : 222 AGGTTCCATACCATCAACAC |

TABLE III

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 42. | 136 | *sbi*(SA2206) | IgG-binding protein Sbi | 7 / 7. | N315 | Kuroda et al. 2001 (15) | BA00018* | 2476152 | 2476593 | SEQ ID No. : 223 CACGCAACAAACTTCAACTA | SEQ ID No. : 224 AGCTTCATATGCTGCTGATT |
| 43. | 137 | *atl-glucosamin* | Atl : autolysin glucosaminidase domain | 7 / 7. | NCTC8325 | | ftp://ftp.genome.ou.edu/pub/staph/ | 1856435 | 1856843 | SEQ ID No. : 225 ACACTGGTATTCGTGCTTCT | SEQ ID No. : 226 GGTGCAGTTAAATCTTTTGC |
| 44. | 138 | *atl-amidase* | Atl : autolysin amidase domain | 7 / 7. | NCTC8325 | | ftp://ftp.genome.ou.edu/pub/staph/ | 1854856 | 1855332 | SEQ ID No. : 227 TCGACGATAAATGGTGAAAT | SEQ ID No. : 228 TTTTGATGGTGTAGTAGGGG |
| 45. | 140 | SA 0423 | Putative autolysin | 7 / 7. | N315 | Kuroda et al. 2001 (15) | BA00018* | 484936 | 485345 | SEQ ID No. : 229 GCAGCTATTATTGGGACAAG | SEQ ID No. : 230 GCGTTACTTGAGCTAGCAGT |
| 46. | 141 | SA0977 | Cell surface protein Q99UX4 | 7 / 7. | N315 | Kuroda et al. 2001 (15) | BA00018* | 1107246 | 1107726 | SEQ ID No. : 231 TGTATACATAGGCGCAGACA | SEQ ID No. : 232 CGGTTTAACATTGTTTGGTT |
| 47. | 144 | *icaA* | Intercellular adhesion protein A | 7 / 7. | N315 | Kuroda et al. 2001 (15) | BA00018* | 2769525 | 2769943 | SEQ ID No. : 233 TGTTCTTGCACTCAAATACG | SEQ ID No. : 234 ATAGAGTGAAGACACCCGAA |
| 48. | 145 | *icaC* | Intercellular adhesion protein C | 7 / 7. | N315 | Kuroda et al. 2001 (15) | BA00018* | 2771838 | 2772209 | SEQ ID No. : 235 TTTGGTACACCTTGCTTTATT | SEQ ID No. : 236 GGTATCGTGAAACGCTGT |
| 49. | 157 | *putB* | High affinity proline permease | 7 / 7. | N315 | Kuroda et al. 2001 (15) | BA00018* | 1965751 | 1966117 | SEQ ID No. : 237 CAACTGGTAACCTAAGCGAG | SEQ ID No. : 238 TTACCACCAGATACGAAACC |
| 50. | 158 | *recA* | Recombinase RecA | 7 / 7. | Mu50 | Kuroda et al. 2001 (15) | BA00017* | 1357180 | 1357628 | SEQ ID No. : 239 GGCTATCCTAAAGGACGAAT | SEQ ID No. : 240 CCGAACATAACACCAACTTT |
| 51. | 159 | *lexA* | SOS regulatory LexA protein | 7 / 7. | NCTC8325 | | ftp://ftp.genome.ou.edu/pub/staph/ | 1337271 | 1337668 | SEQ ID No. : 241 AAAGGTTATCCGCCTAGTGT | SEQ ID No. : 242 TCTCCATTTTCTGCTATGGT |
| 52. | 160 | *agrA* | Accessory gene regulator (Agr) protein A | 7 / 7. | N315 | Kuroda et al. 2001 (15) | BA00018* | 2081482 | 2081918 | SEQ ID No. : 243 GAAGACGATCCAAAACAAAG | SEQ ID No. : 244 ACTGAATTACTGCCACGTTT |
| 53. | 164 | *agrC** | AgrC (N terminal conserved domain) | 7 / 7. | N315 | Kuroda et al. 2001 (15) | BA00018* | 2080676 | 2081110 | SEQ ID No. : 245 ACGGTGATAGCTTAATTCCA | SEQ ID No. : 246 TTACTTCATCGGGTATTTCG |
| 54. | 168 | *agrJ* | AgrJ | 7 / 7. | NCTC8325 | | ftp://ftp.genome.ou.edu/pub/staph/ | 2662057 | 2662521 | SEQ ID No. : 247 TTTGGGTGGATTTACTCATC | SEQ ID No. : 248 ATTTGGGTGAATCATACCTG |
| 55. | 169 | *norA** | Quinolones transporter NorA | 7 / 7. | NCTC8325 | | ftp://ftp.genome.ou.edu/pub/staph/ | 2133927 | 2134331 | SEQ ID No. : 249 CAAATGATTATATCGCCGTT | SEQ ID No. : 250 TGTCGTAGACTTTTTCGGAT |
| 56. | 170 | *radA* | DNA repair protein | 7 / 7. | NCTC8325 | | ftp://ftp.genome.ou.edu/pub/staph/ | 2313171 | 2313638 | SEQ ID No. : 251 GGAAATTGTTGAAAAAGCAG | SEQ ID No. : 252 ACTTGTGAAACAGAACCTGG |
| 57. | 171 | *rnc*(SA1076) | Ribonuclease III | 7 / 7. | N315 | Kuroda et al. 2001 (15) | BA00018* | 1216494 | 1216936 | SEQ ID No. : 253 GGAATTTAACAAAAATGCGT | SEQ ID No. : 254 AGCACGTTGTTCTGATTCTT |
| 58. | 172 | *rpoD** | RNA polymerase sigma factor rpoD | 7 / 7. | Mu50 | Kuroda et al. 2001 (15) | BA00017* | 1672557 | 1672985 | SEQ ID No. : 255 ACAAACAATTGATCCGACAT | SEQ ID No. : 256 CGACCTACGTATCTTTTAGCA |
| 59. | 173 | *sigB** | Sigma factor B | 7 / 7. | Mu50 | Kuroda et al. 2001 (15) | BA00017* | 2194912 | 2195322 | SEQ ID No. : 257 TAAAGGACAATCACATCACG | SEQ ID No. : 258 TCTTGTTGCCCCATAATATC |
| 60. | 174 | *rsbU** | SigmaB regulation protein RsbU | 7 / 7. | Mu50 | Kuroda et al. 2001 (15) | BA00017* | 2196900 | 2197379 | SEQ ID No. : 259 ATCGTGGAAGAATTTAAGCA | SEQ ID No. : 260 TGGTATACCTTTCCCAATGA |
| 61. | 175 | *mdr** | Putative multidrug resistance protein | 7 / 7. | NCTC8325 | | ftp://ftp.genome.ou.edu/pub/staph/ | 401655 | 402070 | SEQ ID No. : 261 AAATGGCGATCAAATTAAAA | SEQ ID No. : 262 CATCACTATTTGATGATGGC |
| 62. | 176 | *vraR* (SA1700) | Two-component response regulator | 7 / 7. | NCTC8325 | | ftp://ftp.genome.ou.edu/pub/staph/ | 848336 | 848774 | SEQ ID No. : 263 GTGGATGATCATGAAATGGT | SEQ ID No. : 264 CCATTTCTCGTTCTGTAAGC |
| 63. | 177 | *dnaK* | Heat shock chaperone protein dnaK | 7 / 7. | NCTC8325 | | ftp://ftp.genome.ou.edu/pub/staph/ | 1227557 | 1228019 | SEQ ID No. : 265 TCACGTACAACACCATCTGT | SEQ ID No. : 266 CGAATACACCGTCACCTAAT |

TABLE III

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 64. | 178 | *lytS+lytR* | TWO-component response regulator | 7 / 7. | NCTC8325 | | ftp://ftp.genome.ou.edu/pub/staph/ | 2568088 | 2568578 | SEQ ID No. : 267 AAATGAAATTGGTGGTTTTG | SEQ ID No. : 268 GATTCAATGGCTCTGTTGTT |
| 65. | 180 | *geh* | Glycerol ester hydrolase | 7 / 7. | NCTC8325 | | ftp://ftp.genome.ou.edu/pub/staph/ | 2507130 | 2507539 | SEQ ID No. : 269 GTATTCAATAGGCGTGGTGT | SEQ ID No. : 270 GTTCAGCATGATGGGTATTT |
| 66. | 181 | *sacP* (SA0295) | Acidic phosphatase | 7 / 7. | NCTC8325 | | ftp://ftp.genome.ou.edu/pub/staph/ | 2521991 | 2522425 | SEQ ID No. : 271 GGCTTGGTATCAAAATTCAG | SEQ ID No. : 272 ATTTGTCTGCGTGATTCTTT |
| 67. | 182 | *lip*(SA2463) | Triacylglycerol lipase | 7 / 7. | N315 | Kuroda et al. 2001 (15) | BA00018* | 2774676 | 2775077 | SEQ ID No. : 273 GTGTAGGTGCATCTTCCATT | SEQ ID No. : 274 GTTTGCCATCATTAATAGCC |
| 68. | 183 | SA0587 | Putative lipoprotein | 7 / 7. | N315 | Kuroda et al. 2001 (15) | BA00018* | 681046 | 681464 | SEQ ID No. : 275 CTGTTGGTCAAGATCCTCAT | SEQ ID No. : 276 ACGTTGTTCTTTTGGAATGT |
| 69. | 184 | SA0691 | Protein similar to ferrichrome ABC transporter | 7 / 7. | N315 | Kuroda et al. 2001 (15) | BA00018* | 789862 | 790319 | SEQ ID No. : 277 TGCGGTAACAATTCTGATAA | SEQ ID No. : 278 TTTTCATCTGCACCAACATA |
| 70. | 185 | *plc* | Phosphatidylinositol phosphodiesterase SA091 | 7 / 7. | N315 | Kuroda et al. 2001 (15) | BA00018* | 102766 | 103237 | SEQ ID No. : 279 TGAGATTAATATGCCAGGCT | SEQ ID No. : 280 GTCAGCACCATAACCACTCT |
| 71. | 188 | SA0746* | Staphylococcal nuclease | 7 / 7. | N315 | Kuroda et al. 2001 (15) | BA00018* | 855825 | 856260 | SEQ ID No. : 281 AGAGGTTTTTCTTTTTCGCT | SEQ ID No. : 282 TTTACCATTTTTCCATCAGC |
| 72. | 212 | *hsdM* | Restriction modification enzyme subunit HsdM | 7 / 7. | N315 | Kuroda et al. 2001 (15) | BA00018* | 1860336 | 1860820 | SEQ ID No. : 283 AATAACAATTGCGCCACTAA | SEQ ID No. : 284 GCCGTCGCTAGATGTTCT |
| 73. | 265 | *fur** | Iron uptake regulatory protein | 7 / 7. | Mu50 | Kuroda et al. 2001 (15) | BA00017* | 1613567 | 1613998 | SEQ ID No. : 285 ATTAAATCGCGTTAAGCAAC | SEQ ID No. : 286 TTACCTTTAGCTTGGCATGT |
| 74. | 266 | *fhuD2* (SA2079) | Ferric hydroxamatereceptor 2 | 7 / 7. | N315 | Kuroda et al. 2001 (15) | BA00018* | 2339296 | 2339793 | SEQ ID No. : 287 GTTGCTAAAGAAAAGCCAGA | SEQ ID No. : 288 AGCTTTCAAATTCTTCCACA |
| 75. | 267 | *fhuD=fhuG**( SA0604) | Ferrichrome transport permease FhuG | 7 / 7. | N315 | Kuroda et al. 2001 (15) | BA00018* | 699364 | 699802 | SEQ ID No. : 289 TGATTACTATTTTGGCTGGC | SEQ ID No. : 290 ATAATAATCAACACCCACGG |
| 76. | 268 | *fhuB* | Ferrichrome transport permease FhuB | 7 / 7. | N315 | Kuroda et al. 2001 (15) | BA00018* | 698260 | 698661 | SEQ ID No. : 291 TTAATAGGTGACGCCAAAAT | SEQ ID No. : 292 CATAGCACTTACTGCTGCAC |
| 77. | 269 | *fhuA=fhuC* (SA0602) | Ferrichrome transport ATP-binding protein FhuA (FhuC) | 7 / 7. | N315 | Kuroda et al. 2001 (15) | BA00018* | 697638 | 698122 | SEQ ID No. : 293 GTTTTCCACATCAAAAAGGA | SEQ ID No. : 294 AGCTCTGCGACATAAGTCAT |
| 78. | 270 | SA0675 | ABC protein | 7 / 7. | N315 | Kuroda et al. 2001 (15) | BA00018* | 769798 | 770212 | SEQ ID No. : 295 GTACACCAGCCAAATGTTCT | SEQ ID No. : 296 TTTTCCCATAATCTGCACTT |
| 79. | 271 | SA1852 | ABC protein | 7 / 7. | N315 | Kuroda et al. 2001 (15) | BA00018* | 2091748 | 2092157 | SEQ ID No. : 297 CTCCAACATTTTGAAGGAAC | SEQ ID No. : 298 TCGCTATCTTGTTTTTGGAT |
| 80. | 272 | SAV1392* | ABC protein | 7 / 7. | Mu50 | Kuroda et al. 2001 (15) | BA00018* | 1474685 | 1475111 | SEQ ID No. : 299 TCAGAGTTCTTTGAGGGTGT | SEQ ID No. : 300 ATTTTAAAACGCCGATTTCT |
| 81. | 273 | SA1580* | ABC protein | 7 / 7. | N315 | Kuroda et al. 2001 (15) | BA00018* | 1819572 | 1819922 | SEQ ID No. : 301 ACTGTTGCTGGTTTAGTGCT | SEQ ID No. : 302 AAAGCTAAATTCTGCGACAA |
| 82. | 279 | *fabH* | FabH protein | 7 / 7. | NCTC8325 | | ftp://ftp.genome.ou.edu/pub/staph/ | 1930239 | 1930731 | SEQ ID No. : 303 TATGCGCCAGAAAAGATTAT | SEQ ID No. : 304 TACCTCTGCCATCTGAAACT |
| 83. | 281 | ORF30 | Putative Cyclase hisF Q8NUI3 | 7 / 7. | NCTC8325 | | ftp://ftp.genome.ou.edu/pub/staph/ | 37874 | 38328 | SEQ ID No. : 305 AGGATTAAGGGATATTGGGA | SEQ ID No. : 306 CCATACTTGTAACGAGGAGC |
| 84. | 293 | SA0102 | Putative protein Q99XA7 | 7 / 7. | N315 | Kuroda et al. 2001 (15) | BA00018* | 116622 | 117047 | SEQ ID No. : 307 AAAGCAGGTGGTAGTCTTGA | SEQ ID No. : 308 ATAACGACGCATTTCCATAG |
| 85. | 294 | SA0610* | Putative protein Q99VW6 | 7 / 7. | N315 | Kuroda et al. 2001 (15) | BA00018* | 704721 | 705193 | SEQ ID No. : 309 GCATTTCATCTGAAACAACA | SEQ ID No. : 310 AAGCGATCATTCGTCTGTAT |

TABLE III

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 86. | 295 | SA 1970 | Putative protein Q99S99 | 7 / 7. | NCTC8325 | | ftp://ftp.genome.ou.edu/pub/staph/ | 574302 | 574774 | SEQ ID No. : 311 GAACATTTGCATTATGTACGA | SEQ ID No. : 312 TTTTGCACCAAAACTCATTA |
| 87. | 296 | SA0022 | Putative protein Q99XE9 | 7 / 7. | N315 | Kuroda et al. 2001 (15) | BA00018* | 30983 | 31412 | **SEQ ID No. : 313** TGACAGTTAATGGAGGGAAC | SEQ ID No. : 314 GGTGAAAGTGATGCTGTTTT |
| 88. | 297 | SA0276 | Putative diarrheal toxin-like protein Q99WT9 | 7 / 7. | N315 | Kuroda et al. 2001 (15) | BA00018* | 332583 | 333045 | SEQ ID No. : 315 CGTGCAGATATTACGTTGAA | SEQ ID No. : 316 TTAATGACGATGCCACTGTA |
| 89. | 298 | SA1552 | Putative protein Q99TD3 | 7 / 7. | N315 | Kuroda et al. 2001 (15) | BA00018* | 1777340 | 1777762 | SEQ ID No. : 317 TGCAACTACAACTCAGCAAC | SEQ ID No. : 318 ATTTTCAATTCTTGTGTGCC |
| 90. | 331 | MW2409 | Q8NUV5 | 7 / 7. | NCTC8325 | | ftp://ftp.genome.ou.edu/pub/staph/ | 256388 | 256828 | SEQ ID No. : 319 CCAGCTTAAAGGCTCAACTA | SEQ ID No. : 320 TATCAGCTAGCAGCATTTGA |
| ***91.*** | ***126*** | ***clfA**** | **Serine-Aspartate Clumping factor A** | 7 / 7 | **NCTC8325** | | ftp://ftp.genome.ou.edu/pub/staph/ | **2028255** | **2028723** | **SEQ ID No. : 321 TAAAACAGACACACAAACG** | **SEQ ID No. : 322 AGCTACTGCCGCTAAACTAA** |

TABLE III

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 1. | 8 | *aacA-aphD* | Acetyl transferase-phosphotransferase: AAC6'-APH2" | 1 / 7. | BM3121(Tn*4001*) | Derbise et al. 1996 (11) | M18086# | 1872 | 2221 | SEQ ID No. : 323 TATACAGAGCCTTGGGAAGA | SEQ ID No. : 324 TCGTGTAATTCATGTTCTGG |
| 2. | 31 | *tetK* | Tetracycline resistance | 1 / 7. | RN450(pT181) | | S67449# | 759 | 1205 | SEQ ID No. : 325 GAAAAACATTCCGTTTATGC | SEQ ID No. : 326 GCTATACCTGTTCCCTCTGA |
| 3. | 33 | *tetM* | Tetracycline resistance | 1 / 7. | Mu50 | Kuroda et al. 2001 (15) | BA00017* | 441266 | 441648 | SEQ ID No. : 327 ACGCTTTTAGAACGTCAGAG | SEQ ID No. : 328 TCAGATTCGGTAAAGTTCGT |
| 4. | 57 | *entB* | Enterotoxin B | 1 / 7. | COL | | http://www.tigr.org/tigr-scripts/CMR2/GenomePage3.spl?database=gsa : SA0907 | 113 | 591 | SEQ ID No. : 329 TGCACAAATCGAGTAAATTC | SEQ ID No. : 330 TTTCACCAAATAGTGACGAGT |
| 5. | 90 | *lukS* | Leucocidin S | 1 / 7. | MW2 | Baba et al. 2002 (5) | BA00033* | 1531011 | 1530602 | SEQ ID No. : 331 TATAAAAGCAATGAGGTGGC | SEQ ID No. : 332 CACTGTGTACTAATGGGGGT |
| 6. | 92 | *lukS+F** | Leucocidins F + S | 1 / 7. | MW2 | Baba et al. 2002 (5) | BA00033* | 1530288 | 1530720 | SEQ ID No. : 333 ATCATTAGGTAAAATGTCTGGACATGATCCA | SEQ ID No. : 334 GCATCAASTGTATTGGATAGCAAAAGC |
| 7. | 201 | SAV 0415 | Putative transposase | 1 / 7. | Mu50 | Kuroda et al. 2001 (15) | BA00017* | 460876 | 461364 | SEQ ID No. : 335 AATTATTCCGTGTCCATTTG | SEQ ID No. : 336 CTGCGATATGAATAGCTTCC |
| 8. | 215 | SAV0847 | Putative integrase | 1 / 7. | Mu50 | Kuroda et al. 2001 (15) | BA00017* | 918085 | 918485 | SEQ ID No. : 337 TGAAGCTAAGAGAGCAGAGG | SEQ ID No. : 338 CTTCAAATTCTTCACGCATC |
| 9. | 216 | SAV2028 | Putative integrase | 1 / 7. | Mu50 | Kuroda et al. 2001 (15) | BA00017* | 2147543 | 2147961 | SEQ ID No. : 339 AGCTTTTTCCAAATAAAGGC | SEQ ID No. : 340 ATTGGTTTGTTCTTAAAGCG |
| 10. | 217 | *Int459* (SAV0392) | Putative integrase Int459 Q9AGN0 | 1 / 7. | Mu50 | Kuroda et al. 2001 (15) | BA00017* | 436798 | 437243 | SEQ ID No. : 341 AAAAGCAAAGAATGGAACCT | SEQ ID No. : 342 TAGATGGATTTGTGGTGGTT |
| 11. | 218 | *phi int11* | Integrase phage phi11 | 1 / 7. | NCTC8325 | | ftp://ftp.genome.ou.edu/pub/staph/ | 855544 | 856033 | SEQ ID No. : 343 GAGAAGCAAAATGCCAGTAT | SEQ ID No. : 344 TGGTTTTGTTCTGGGAATAG |
| 12. | 234 | ORF15 | DUT Pase from phage phiPVL O80091 | 1 / 7. | NCTC8325 | | ftp://ftp.genome.ou.edu/pub/staph/ | 869364 | 869783 | SEQ ID No. : 345 GAACGAAATCATAAGACGGA | SEQ ID No. : 346 TGAAACACTTTCGAATTCCT |
| 13. | 236 | ORF17 | Ssb from phage phi 11 Q8SDW4 | 1 / 7. | NCTC8325 | | ftp://ftp.genome.ou.edu/pub/staph/ | 863365 | 863772 | SEQ ID No. : 347 AAAGTTGCAAATGTCGAGTT | SEQ ID No. : 348 GAATGGGTTTGATTGTTGAC |
| 14. | 241 | ORF22* | Orf178 from phage phiSLT Q9B0G4 | 1 / 7. | NCTC8325 | | ftp://ftp.genome.ou.edu/pub/staph/ | 862004 | 862412 | SEQ ID No. : 349 TGGGTTTTTAAGAAACTGGA | SEQ ID No. : 350 TAGCACCCTCTAAAACTTCG |
| 15. | 242 | ORF23* | PV83 Orf 3 from phage phi 11Q8SDX4 | 1 / 7. | NCTC8325 | | ftp://ftp.genome.ou.edu/pub/staph/ | 856395 | 856836 | SEQ ID No. : 351 TCAAATGTTTAACTTGAGCG | SEQ ID No. : 352 AGCAGCTTTGACTGAATGTT |
| 16. | 243 | ORF24* | DnaC from phage phi 13 Q9B0F8 | 1 / 7. | NCTC8325 | | ftp://ftp.genome.ou.edu/pub/staph/ | 758138 | 758615 | SEQ ID No. : 353 TCAAACGTTAATCCGTCTTT | SEQ ID No. : 354 AATTGATACGTTGCCAGTTC |
| 17. | 250 | *repC** | pT181 RepC protein (polypeptide A) | 1 / 7. | NCTC8325 (pT181) | | J01764# | 1-379 | 4396-4439 | SEQ ID No. : 355 TGTGCATATCTGATCCAAAA | SEQ ID No. : 356 TTTGTTTCTGGCTTACCATT |
| 18. | 284 | SAV1998* | Repressor homolog Q931J0 | 1 / 7. | Mu50 | Kuroda et al. 2001 (15) | BA00017* | 2122797 | 2123204 | SEQ ID No. : 357 TCTGAACGGAAGATATCTCAA | SEQ ID No. : 358 AACCATTTCGGTAAACAATG |
| 19. | 285 | MW0751 | Putative primase Q8NXK4 | 1 / 7. | MW2 | Baba et al. 2002 (5) | BA00033* | 842774 | 843192 | SEQ ID No. : 359 TAATTGGGTATTATGGCGAG | SEQ ID No. : 360 TTGTGCATCCTGTAACAGTC |
| 20. | 351 | SA1808 | Q99SP1 | 1 / 7. | N315 | Kuroda et al. 2001 (15) | BA00018* | 2047292 | 2047705 | SEQ ID No. : 361 TGACAATGGTGAGACTTTGA | SEQ ID No. : 362 TGGTCTTCAGCAGTTTTAGA |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| | | | | | | | | | | | G |
| 21. | 354 | SA1794* | Q99SQ7 | 1 / 7. | N315 | Kuroda et al. 2001 (15) | BA00018* | 2038243 | 2038662 | SEQ ID No. : 363 CCTGCTAAGAATCAAGGCTA | SEQ ID No. : 364 AATCTTTTTACATGCACGGT |
| 22. | 398 | MW0757 | Q8NXJ8 | 1 / 7. | MW2 | Baba et al. 2002 (5) | BA00033* | 849682 | 850130 | SEQ ID No. : 365 AAAGAGAAATTAGGAACTGGTTT | SEQ ID No. : 366 AAGATAATGTGTACCTTTTTATTG |
| 23. | 399 | MW0756 | Q8NXJ9 | 1 / 7. | MW2 | Baba et al. 2002 (5) | BA00033* | 848651 | 849122 | SEQ ID No. : 367 CGCTTGATGATGTTGAAAA | SEQ ID No. : 368 TTCCCTAATTTAAGTCCATCC |
| 24. | 400 | MW0755 | Q8NXK0 | 1 / 7. | MW2 | Baba et al. 2002 (5) | BA00033* | 847753 | 848190 | SEQ ID No. : 369 TCTGACTTTGAAAGCAATGAT | SEQ ID No. : 370 TCGATGTAATTCTTCTCGCT |
| 25. | 401 | MW0754 | Q8NXK1 | 1 / 7. | MW2 | Baba et al. 2002 (5) | BA00033* | 846616 | 847102 | SEQ ID No. : 371 TGATTGAACAGGGGTTAAAG | SEQ ID No. : 372 TCATATGCGACACACTCATT |
| 26. | 402 | MW0746 | Q8NXK9 | 1 / 7. | MW2 | Baba et al. 2002 (5) | BA00033* | 840975 | 841402 | SEQ ID No. : 373 AAATGTTGATGTTGCAGATTT | SEQ ID No. : 374 CTGGGAATGAGGAAAGAATA |
| 27. | 415 | MW0047 | Q8VUW0 | 1 / 7. | MW2 | Baba et al. 2002 (5) | BA00033* | 57380 | 57835 | SEQ ID No. : 375 CGAACATCTTGTAAAGGCAT | SEQ ID No. : 376 ATGATTTCTTCATCAAACCG |
| 28. | 417 | MW0043 | Q8VUV8 | 1 / 7. | MW2 | Baba et al. 2002 (5) | BA00033* | 53645 | 54047 | SEQ ID No. : 377 GGTGTTGAAGATCCAGAGAA | SEQ ID No. : 378 AATCCAGTTGGCTCATATTC |
| 29. | 418 | MW0042 | Q8VUV7 | 1 / 7. | MW2 | Baba et al. 2002 (5) | BA00033* | 51771 | 52217 | SEQ ID No. : 379 ATTATGATTAGACGCGGAAC | SEQ ID No. : 380 TTGCGTTTTGTTTATTTCTG |
| 30. | 422 | SAV1999 | Q931I9 | 1 / 7. | Mu50 | Kuroda et al. 2001 (15) | BA00017* | 2123545 | 2124020 | SEQ ID No. : 381 ACCAATAATCAGCGACAGTT | SEQ ID No. : 382 GTTCATTCCCTTTCAAATCA |
| 31. | 423 | SAV0916* | Q931V6 | 1 / 7. | Mu50 | Kuroda et al. 2001 (15) | BA00017* | 960546 | 960975 | SEQ ID No. : 383 TTTTAAAAGAGGAGGGAGAAA | SEQ ID No. : 384 AAAACTAAGTTGCGGATTGA |
| 32. | 436 | SAV0869 | Q932A3 | 1 / 7. | Mu50 | Kuroda et al. 2001 (15) | BA00017* | 928236 | 928696 | SEQ ID No. : 385 AAGCGTTTGATTTGAAATGT | SEQ ID No. : 386 GGTCATAGCAGTTTGGTTGT |
| 33. | 437 | SAV0868 | Q932A4 | 1 / 7. | Mu50 | Kuroda et al. 2001 (15) | BA00017* | 927554 | 927992 | SEQ ID No. : 387 TACTGCACAGCAAGTAATGG | SEQ ID No. : 388 CTCGAGTACCTTGCATGATT |
| 34. | 439 | SAV0865 | Q932A7 | 1 / 7. | Mu50 | Kuroda et al. 2001 (15) | BA00017* | 925750 | 926180 | SEQ ID No. : 389 GTTACGAGGGCATATACGAA | SEQ ID No. : 390 TCTGCCTAAGAATTTTCCTG |
| 35. | 441 | SAV0858 | Q932B4 | 1 / 7. | Mu50 | Kuroda et al. 2001 (15) | BA00017* | 922799 | 923217 | SEQ ID No. : 391 CGTATGTTATGAACGCTCTG | SEQ ID No. : 392 CGGAGATGTTACTCCGATAG |
| 36. | 443 | SAV0849 | Q932C1 | 1 / 7. | Mu50 | Kuroda et al. 2001 (15) | BA00017* | 919275 | 919689 | SEQ ID No. : 393 TCCCATTCATGAGAAAAGAC | SEQ ID No. : 394 AAATCTAAGTGGTTGAACGG |
| 37. | 449 | SAV0784 | Q932E4 | 1 / 7. | Mu50 | Kuroda et al. 2001 (15) | BA00017* | 869773 | 870197 | SEQ ID No. : 395 GATGGAAGAGTTTGGTGAAA | SEQ ID No. : 396 ATTCTTGCGCATTTATTGTT |
| 38. | 452 | SAV0414 | Q932H2 | 1 / 7. | Mu50 | Kuroda et al. 2001 (15) | BA00017* | 459540 | 460010 | SEQ ID No. : 397 GTGAGAATGATTCAGGGAAA | SEQ ID No. : 398 CATTGAGTCTTCCTACCAATTT |
| 39. | 453 | SAV0413 | Q932H3 | 1 / 7. | Mu50 | Kuroda et al. 2001 (15) | BA00017* | 457278 | 457708 | SEQ ID No. : 399 GAAGAAATTAAGGAACGCCT | SEQ ID No. : 400 AATTCATCCACATAAATCGC |
| 40. | 454 | SAV0410 | Q932H6 | 1 / 7. | Mu50 | Kuroda et al. 2001 (15) | BA00017* | 454619 | 455024 | SEQ ID No. : 401 ACAAACCATACGCAAACTCT | SEQ ID No. : 402 TCCATTGATTTTCTCAATCC |
| 41. | 455 | SAV0409 | Q932H7 | 1 / 7. | Mu50 | Kuroda et al. | BA00017* | 453904 | 45437 | SEQ ID No. : 403 | SEQ ID No. : 404 |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| | | | | | | 2001 (15) | | | 4 | GAATCAAAGCCAACGATAAG | CTAGGTGCAAAAGCTGATCT |
| 42. | 456 | SAV0408 | Q932H8 | 1 / 7. | Mu50 | Kuroda et al. 2001 (15) | BA00017* | 452331 | 452765 | SEQ ID No. : 405 ATGGCGTTTCTCAAAATAAA | SEQ ID No. : 406 TATGTTCCATGCAATCTTGA |
| 43. | 457 | SAV0406 | Q932I0 | 1 / 7. | Mu50 | Kuroda et al. 2001 (15) | BA00017* | 450417 | 450829 | SEQ ID No. : 407 GATTTGATTACCCAGACAGC | SEQ ID No. : 408 TGCCTATTTCTTGCTTCATT |
| 44. | 458 | SAV0405 | Q932I1 | 1 / 7. | Mu50 | Kuroda et al. 2001 (15) | BA00017* | 449805 | 450207 | SEQ ID No. : 409 ATGAAGGTGAATTAGTCGGA | SEQ ID No. : 410 TATCACGTCCCAAAGATTG |
| 45. | 459 | SAV0404 | Q932I2 | 1 / 7. | Mu50 | Kuroda et al. 2001 (15) | BA00017* | 449170 | 449646 | SEQ ID No. : 411 AAAGATAAAGGCTGGGTGAT | SEQ ID No. : 412 GCTGGGTCTATACAATCTCG |
| 46. | 460 | SAV0403 | Q932I3 | 1 / 7. | Mu50 | Kuroda et al. 2001 (15) | BA00017* | 448598 | 449046 | SEQ ID No. : 413 CATTGAAAGAGCAATCACCT | SEQ ID No. : 414 GCCATCTAGTTGTTCACTCC |
| 47. | 461 | SAV0402 | Q932I4 | 1 / 7. | Mu50 | Kuroda et al. 2001 (15) | BA00017* | 447955 | 448373 | SEQ ID No. : 415 AAAAAGAAATCACTGGACGA | SEQ ID No. : 416 TTCGTATCGTTTCGGTAAAT |
| 48. | 462 | SAV0401 | Q932I5 | 1 / 7. | Mu50 | Kuroda et al. 2001 (15) | BA00017* | 445623 | 446099 | SEQ ID No. : 417 TGTGGGCGTTATTTTACTTT | SEQ ID No. : 418 ACAGCTCGTATCACTTTCGT |
| 49. | 463 | SAV0400 | Q932I6 | 1 / 7. | Mu50 | Kuroda et al. 2001 (15) | BA00017* | 443296 | 443755 | SEQ ID No. : 419 CAGAACAACAAGGAGTGGAT | SEQ ID No. : 420 TCCATAGAGATTTCTTGCGT |
| 50. | 464 | SAV0399 | Q932I7 | 1 / 7. | Mu50 | Kuroda et al. 2001 (15) | BA00017* | 442465 | 442897 | SEQ ID No. : 421 GTCCGTGAAACAGAAATCAT | SEQ ID No. : 422 CTAGCTGTTTGGGTTGGTAG |
| 51. | 465 | SAV0395 | Q932J0 | 1 / 7. | Mu50 | Kuroda et al. 2001 (15) | BA00017* | 438277 | 438707 | SEQ ID No. : 423 GGGGTTTAGTATGAAGGGAG | SEQ ID No. : 424 AAACAGTCGAACGATTCAAA |
| 52. | *1* | *blaZ* | Beta-lactamase | 2 / 7. | BM3121 (pIP1066) | Derbise *et al.* 1996 (11) | X52734# | 5788 | 6238 | SEQ ID No. : 425 TAAAAGAAATCGGTGGAATC | SEQ ID No. : 426 TCCTTCATTACACTCTTGGC |
| 53. | *46* | *ccrB1* | SSCmecl Recombinase B1 | 2 / 7. | COL (SCCmecl) | | http://www.tigr.org/tigr-scripts/CMR2/GenomePage3.spl?database=gsa : SA0041 | 23 | 484 | SEQ ID No. : 427 TTGTAGGAGGCTACATTCGT | SEQ ID No. : 428 ATGGAAGATTGCCTTGATAA |
| 54. | *47* | *ccrA1* | SSCmecl Recombinase A1 | 2 / 7. | COL (SCCmecl) | | http://www.tigr.org/tigr-scripts/CMR2/GenomePage3.spl?database=gsa : SA0042 | 731 | 1215 | SEQ ID No. : 429 CTCAAGTTACCCGAACTCAG | SEQ ID No. : 430 TTGTTGAATGATGTTTTGGA |
| 55. | *53* | SA1633 | Beta-lactamase Q99T58 | 2 / 7. | N315 | Kuroda et al. 2001 (15) | BA00018* | 1866168 | 1866573 | SEQ ID No. : 431 AAAACAGCAAAAGCAGAAGA | SEQ ID No. : 432 CTGCTCCTACTTTACCTGAAA |
| 56. | *61* | *seh** | Enterotoxin H | 2 / 7. | MW2 | Baba et al. 2002 (5) | BA00033* | 61734 | 62144 | SEQ ID No. : 433 GGTGATAGTGGCAATGATTT | SEQ ID No. : 434 TCCTTTTAAATCATAAATGTCG |
| 57. | *81* | *tst* | Toxic shock syndrome toxin-1 | 2 / 7. | N315 | Kuroda et al. 2001 (15) | BA00018* | 2061154 | 2061589 | SEQ ID No. : 435 AATCAAAACTGCAAAAGCAT | SEQ ID No. : 436 TTGAGTTAGCTGATGACGAA |
| 58. | *118* | *coa-N315** | Staphylocoagulase (N315 variable domain) | 2 / 7. | N315 | Kuroda et al. 2001 (15) | BA00018* | 264708 | 265163 | SEQ ID No. : 437 CATGGGATAACAAAGCAGAT | SEQ ID No. : 438 CAAACTGCTTCAAGTCAACA |
| 59. | *119* | *coa-8325* | Staphylocoagulase (NCTC8325 variable domain) | 2 / 7. | NCTC8325 | | ftp://ftp.genome.ou.edu/pub/staph/ | 2609636 | 2610046 | SEQ ID No. : 439 AAATGGGACATTAATAGATAGCA | SEQ ID No. : 440 TCTTCTGCTGCATTAAAAGTT |
| 60. | *139* | *ami 8325* | Putative amidase from bacteriophage phi 11 | 2 / 7. | NCTC8325 | | ftp://ftp.genome.ou.edu/pub/staph/ | 895853 | 896269 | SEQ ID No. : 441 TTGAAAACTTCTGAGGGAAA | SEQ ID No. : 442 TTTAAAATTCGGACGGATAA |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 61. | 162 | *agrB-N315* | AgrB (N315 variable domain) | **2 / 7.** | N315 | Kuroda et al. 2001 (15) | BA00018* | 2079509 | 2079985 | SEQ ID No. : 443 ACGTTTAGGGATGCAAATTA | SEQ ID No. : 444 TAATCCTCCTTAGGGAAAAA |
| 62. | 165 | *agrC-8325* | AgrC (NCTC8325 variable domain) | **2 / 7.** | NCTC8325 | | ftp://ftp.genome.ou.edu/pub/staph/ | 726687 | 727138 | SEQ ID No. : 445 ATAGCGCGTCCTTAATCATA | SEQ ID No. : 446 AAGGAGAAATTGAGAAATAACAA |
| 63. | 167 | *agrC-N315** | AgrC (N315 variable domain) | **2 / 7.** | N315 | Kuroda et al. 2001 (15) | BA00018* | 2080313 | 2080774 | SEQ ID No. : 447 ATGGAAGTAGAGCCGTATTG | SEQ ID No. : 448 TCTCTTTAAGGGTGAAAAGC |
| 64. | 195 | tnp 480 (*Tn552*) | Tn*552 Tnp 480* transposase | **2 / 7.** | BM3121 (Tn552) | Derbise et al. 1996 (11) | X52734# | 1850 | 2300 | SEQ ID No. : 449 TGAAGCCAAACGTAAAGAAG | SEQ ID No. : 450 TATTTCATTGGGTCTTGAGG |
| 65. | 200 | tnp IS *1272* | IS *1272* transposase | **2 / 7.** | MW2 (IS1272) | Baba et al. 2002 (5) | BA00033* | 43712 | 44111 | SEQ ID No. : 451 GGATTCTAAAGCCCTCTACC | SEQ ID No. : 452 TTGGGAATTTGTTGCTATTT |
| 66. | 210 | SA1818 | Tn*557* putative protein | **2 / 7.** | N315 | Kuroda et al. 2001 (15) | BA00018* | 2059674 | 2047705 | SEQ ID No. : 453 GGTTCAGGTTTTCATTCTTCT | SEQ ID No. : 454 CAGTCTCATCTTTAGGGTCTGT |
| 67. | 231 | ORF12 | Na/K ATPase from phage phi 12, 13 Q8SBD6 | **2 / 7.** | NCTC8325 | | ftp://ftp.genome.ou.edu/pub/staph/ | 1314738 | 1315186 | SEQ ID No. : 455 AGATGACTCAATTTCTAGGGG | SEQ ID No. : 456 TTCACCCTCTTCAATGATTC |
| 68. | 232 | ORF13 | Small terminase from phage phi 11 Q8SDV0 | **2 / 7.** | NCTC8325 | | ftp://ftp.genome.ou.edu/pub/staph/ | 871321 | 871741 | SEQ ID No. : 457 TCGCAATTAGGAATAAAGGA | SEQ ID No. : 458 CAAAAGTTGGTGTGATTGTG |
| 69. | 233 | ORF14 | Terminase from phage phi 11 Q8SDU9 | **2 / 7.** | Mu50 | Kuroda et al. 2001 (15) | BA00017* | 933967 | 934384 | SEQ ID No. : 459 GGTGAGTACGATGACGAAAG | SEQ ID No. : 460 CGCTTCTTCCATGACTATGT |
| 70. | 235 | ORF16 | Helicase DnaB from phage phi 11 Q8SDW1 | **2 / 7.** | NCTC8325 | | ftp://ftp.genome.ou.edu/pub/staph/ | 865785 | 866270 | SEQ ID No. : 461 AATTATGTAATGGACGTCGG | SEQ ID No. : 462 TCCATTTTGTGCTATGTTCA |
| 71. | 244 | ORF25 | Hypothetical protein from phage phi 11 Q8SDT8 | **2 / 7.** | Mu50 | Kuroda et al. 2001 (15) | BA00017* | 942411 | 942815 | SEQ ID No. : 463 CGGATATTTTAACGGAGTTG | SEQ ID No. : 464 TCATCACGTTCGCTACTAAA |
| 72. | 247 | ORF28 | Hypothetical protein from plasmid pSV41 O87359 | **2 / 7.** | NCTC8325 | | ftp://ftp.genome.ou.edu/pub/staph/ | 606936 | 607351 | SEQ ID No. : 465 AAGCTAGTGAGCGTAAATGC | SEQ ID No. : 466 TAAGCACGCTCAAGAGAAAT |
| 73. | 280 | ORF29 | Putative ATP/GTP-binding protein Q935V0 | **2 / 7.** | NCTC8325 | | ftp://ftp.genome.ou.edu/pub/staph/ | 524490 | 524934 | SEQ ID No. : 467 CAATATCATAGTGCGAGCAA | SEQ ID No. : 468 GGCTAATTCTGTTTTTCCAA |
| 74. | 305 | ORF34* | Q9RL82 | **2 / 7.** | NCTC8325 | | ftp://ftp.genome.ou.edu/pub/staph/ | 2643225 | 2643656 | SEQ ID No. : 469 TTGCAATACTGTGGTGAAAA | SEQ ID No. : 470 TGCTACGTATCCATCTTCCT |
| 75. | 306 | ORF35 | Putative protein | **2 / 7.** | NCTC8325 | | ftp://ftp.genome.ou.edu/pub/staph/ | 2641973 | 2642449 | SEQ ID No. : 471 GGATTTTGTGAGAGCTTGAA | SEQ ID No. : 472 GCTTGGATTTGTTATTCCTTT |
| 76. | 307 | ORF36 | Putative protein | **2 / 7.** | NCTC8325 | | ftp://ftp.genome.ou.edu/pub/staph/ | 2548087 | 2548532 | SEQ ID No. : 473 TGGAATTGAAAGTCGATGAT | SEQ ID No. : 474 GGCATTAACTGCAGGATTAG |
| 77. | 330 | ORF38 | Q935U9 | **2 / 7.** | NCTC8325 | | ftp://ftp.genome.ou.edu/pub/staph/ | 523734 | 524138 | SEQ ID No. : 475 AAAGTGAACATGGAATTTGG | SEQ ID No. : 476 TCTGTTTTCATTCCCTCTTG |
| 78. | 332 | ORF39 | Putative protein | **2 / 7.** | NCTC8325 | | ftp://ftp.genome.ou.edu/pub/staph/ | 224236 | 224636 | SEQ ID No. : 477 ATACACGCCTATTATTCCGA | SEQ ID No. : 478 CCCATCTTGCACCTAAAGTA |
| 79. | 333 | ORF40 | Putative protein | **2 / 7.** | NCTC8325 | | ftp://ftp.genome.ou.edu/pub/staph/ | 223693 | 224103 | SEQ ID No. : 479 AGAGGAGGAATAATAGTGGATATG | SEQ ID No. : 480 CATTTCATCATCATCAAAGAC |
| 80. | 341 | SA2012 | Q99S58 | **2 / 7.** | N315 | Kuroda et al. 2001 (15) | BA00018* | 2284667 | 2285129 | SEQ ID No. : 481 TCGGAAAATTTCTAAGTCAAA | SEQ ID No. : 482 ATTGCATCAATAAAGTCGCT |
| 81. | 342 | SA2011 | Q99S59 | **2 / 7.** | N315 | Kuroda et al. 2001 (15) | BA00018* | 2283981 | 2284398 | SEQ ID No. : 483 CTCCAAATAATACACTGAGC | SEQ ID No. : 484 TTCTGCTGATGTAATCCGTT |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| | | | | | | | | | | C | |
| 82. | 343 | SA2010 | Q99S60 | 2 / 7. | N315 | Kuroda et al. 2001 (15) | BA00018* | 2283224 | 2283713 | SEQ ID No. : 485 TGGAGAAAATTGAGAATGAA | SEQ ID No. : 486 ATATATGGACTGGTTGGTGC |
| 83. | 346 | SA1834 | Q99SL9 | 2/ 7. | N315 | Kuroda et al. 2001 (15) | BA00018* | 2070443 | 2070886 | SEQ ID No. : 487 CGTGTTGCAAAAGATGTAAA | SEQ ID No. : 488 GTTTTTAAGGCCTGCAGATA |
| 84. | 364 | SA1640 | Q99T51 | 2 / 7. | N315 | Kuroda et al. 2001 (15) | BA00018* | 1874118 | 1874583 | SEQ ID No. : 489 GGAGATAACAGCATGAGGAG | SEQ ID No. : 490 TTTGGTCGCTATTTTCTTGT |
| 85. | 365 | SA1636 | Q99T55 | 2 / 7. | N315 | Kuroda et al. 2001 (15) | BA00018* | 1868168 | 1868617 | SEQ ID No. : 491 CCTATCGTCGATTATTTTGG | SEQ ID No. : 492 CTCTGTAGCTTCTCCTGGAA |
| 86. | 366 | SA1635 | Q99T56 | 2 / 7. | N315 | Kuroda et al. 2001 (15) | BA00018* | 1867590 | 1867970 | SEQ ID No. : 493 TTTGAAACTGAACGATGAAG | SEQ ID No. : 494 ACAAAATCAGAGCTATCGTCA |
| 87. | 392 | MW2292 | Q8NV38 | 2 / 7. | MW2 | Baba et al. 2002 (5) | BA00033* | 2440455 | 2440950 | SEQ ID No. : 495 AGTTTTACAATGGACGCAAA | SEQ ID No. : 496 AGGATTATTTCTTGCAGGGT |
| 88. | 395 | MW1197 | Q8NWX1 | 2 / 7. | MW2 | Baba et al. 2002 (5) | BA00033* | 1313494 | 1313953 | SEQ ID No. : 497 AGATGACACAACCAAAGATG | SEQ ID No. : 498 TGATTTGCAATATTCTTTTGTC |
| 89. | 397 | MW0758 | Q8NXJ7 | 2 / 7. | MW2 | Baba et al. 2002 (5) | BA00033* | 850529 | 850940 | SEQ ID No. : 499 GGTTTTCATTCTTCTTCAAATTA | SEQ ID No. : 500 TAGCTGAAATTGGGGGAT |
| 90. | 405 | MW0379* | Q8NY57 | 2 / 7. | MW2 | Baba et al. 2002 (5) | BA00033* | 426649 | 427127 | SEQ ID No. : 501 GGAAAAGTTACAGCGAAAGA | SEQ ID No. : 502 GGTACAAAATCGCTATCACC |
| 91. | 406 | MW0378* | Q8NY58 | 2 / 7. | MW2 | Baba et al. 2002 (5) | BA00033* | 425920 | 426368 | SEQ ID No. : 503 CGTGTTTGATAGGCGATAAT | SEQ ID No. : 504 CGTTTCATTCATGTTTTGGT |
| 92. | 407 | ORF44* | Putative protein | 2 / 7. | MW2 | Baba et al. 2002 (5) | BA00033* | 424483 | 424903 | SEQ ID No. : 505 AATTTAATAAAGGACAACGC | SEQ ID No. : 506 TTATTTCGCCAGTTTCTTTG |
| 93. | 408 | MW0374* | Q8NY62 | 2 / 7. | MW2 | Baba et al. 2002 (5) | BA00033* | 421394 | 421845 | SEQ ID No. : 507 CAAGAATTTTTCTCAACGCT | SEQ ID No. : 508 CCTTCAAAGAAGATACACGG |
| 94. | 409 | MW0372* | Q8NY65 | 2 / 7. | MW2 | Baba et al. 2002 (5) | BA00033* | 418466 | 418902 | SEQ ID No. : 509 GTCTTGACTGCCGTTTTATT | SEQ ID No. : 510 GCAATGGCTTCTTTGACTAC |
| 95. | 410 | MW0368* | Q8NY68 | 2 / 7. | MW2 | Baba et al. 2002 (5) | BA00033* | 416813 | 417247 | SEQ ID No. : 511 TTCTGCCAAAAAGCTAAAAG | SEQ ID No. : 512 AAAAATGTAACTAACCTTTGCTG |
| 96. | 413 | MW0074 | Q8NYT9 | 2 / 7. | MW2 | Baba et al. 2002 (5) | BA00033* | 85207 | 85607 | SEQ ID No. : 513 GTGGGAATTTTCCTAAAAAC | SEQ ID No. : 514 GATGTTATAACGAAGCCCAA |
| 97. | 416 | MW0045 | Q8VUV9 | 2 / 7. | MW2 | Baba et al. 2002 (5) | BA00033* | 56035 | 56533 | SEQ ID No. : 515 TACTCATGCAGCTGAAACAC | SEQ ID No. : 516 ATAGCCAAGCGTAGATGTTG |
| 98. | 419 | SAV2596 | Q931E7 | 2 / 7. | Mu50 | Kuroda et al. 2001 (15) | BA00017* | 2740569 | 2741019 | SEQ ID No. : 517 GATTTCCAGGAGACAAGTCA | SEQ ID No. : 518 TCATTGCTATTAAAGTAACCCA |
| 99. | 420 | SAV2025 | Q931I0 | 2 / 7. | Mu50 | Kuroda et al. 2001 (15) | BA00017* | 2146163 | 2146583 | SEQ ID No. : 519 GAGGCATAAACCAATGTTCA | SEQ ID No. : 520 ATGAACGCTAAAAGCAACAT |
| 100. | 421 | SAV2001 | Q931I8 | 2 / 7. | Mu50 | Kuroda et al. 2001 (15) | BA00017* | 2124654 | 2125051 | SEQ ID No. : 521 AAGCAAAGGAGAAATGAGAA | SEQ ID No. : 522 CTTCAATTTGTTTTTGAGCC |
| 101. | 424 | SAV0912* | Q931W0 | 2 / 7. | Mu50 | Kuroda et al. | BA00017* | 957596 | 95800 | SEQ ID No. : 523 | SEQ ID No. : 524 |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| | | | | | | 2001 (15) | | | 1 | AAGGAGCAAACAAATGGATA | ATTCACATCAAAGCCAACAT |
| 102. | 425 | SAV0910* | Q931W2 | 2 / 7. | Mu50 | Kuroda et al. 2001 (15) | BA00017* | 956087 | 956582 | SEQ ID No. : 525 TTTGTTTATGGAAGATGGCT | SEQ ID No. : 526 ATCAATTCGCCACATTTTAG |
| 103. | 426 | SAV0909* | Q931W3 | 2 / 7. | Mu50 | Kuroda et al. 2001 (15) | BA00017* | 954186 | 954685 | SEQ ID No. : 527 GATGCTATGGCTCAAAAATC | SEQ ID No. : 528 ATCGTTTGTGCGTTTCTAAT |
| 104. | 427 | SAV0905* | Q931W7 | 2 / 7. | Mu50 | Kuroda et al. 2001 (15) | BA00017* | 951322 | 951751 | SEQ ID No. : 529 TGCGATGACTAGTATTGTGC | SEQ ID No. : 530 AATCATCTGTACCGCTTCAT |
| 105. | 428 | SAV0904* | Q931W8 | 2 / 7. | Mu50 | Kuroda et al. 2001 (15) | BA00017* | 949217 | 949664 | SEQ ID No. : 531 ACGGATAGAAAAGGAGGAAG | SEQ ID No. : 532 TCGGTATTCTGTAGCTCGAT |
| 106. | 429 | SAV0903 | Q931W9 | 2 / 7. | Mu50 | Kuroda et al. 2001 (15) | BA00017* | 947495 | 947954 | SEQ ID No. : 533 AAGGTGAAAACGACAAGAAA | SEQ ID No. : 534 TATCCTCTAATGAAGGTGGC |
| 107. | 430 | SAV0902 | Q931X0 | 2 / 7. | Mu50 | Kuroda et al. 2001 (15) | BA00017* | 946541 | 947002 | SEQ ID No. : 535 GTTCCTTGTTTTTGTGTGGT | SEQ ID No. : 536 ACCTTTGCTCAAATTTTCAA |
| 108. | 431 | SAV0901 | Q931X1 | 2 / 7. | Mu50 | Kuroda et al. 2001 (15) | BA00017* | 942879 | 943305 | SEQ ID No. : 537 GAGGTGTTAATGTGACGGAA | SEQ ID No. : 538 TTACTGTTATCAGCATTGACG |
| 109. | 432 | SAV0898 | Q931X4 | 2 / 7. | Mu50 | Kuroda et al. 2001 (15) | BA00017* | 941525 | 941971 | SEQ ID No. : 539 TTTTGTTTAGAAAAGCTGGC | SEQ ID No. : 540 ATCTGTATCCAGTCGCCTTA |
| 110. | 433 | SAV0890 | Q931Y2 | 2 / 7. | Mu50 | Kuroda et al. 2001 (15) | BA00017* | 938259 | 938722 | SEQ ID No. : 541 ATAACAACGATGATGAAGGC | SEQ ID No. : 542 AAATGAATCTCCACCAGTCA |
| 111. | 434 | SAV0888 | Q931Y4 | 2 / 7. | Mu50 | Kuroda et al. 2001 (15) | BA00017* | 936917 | 937364 | SEQ ID No. : 543 TATGATGATTAAGCGCATTG | SEQ ID No. : 544 CATTGCTTCGGTATTAGTCC |
| 112. | 435 | SAV0887 | Q931Y5 | 2 / 7. | Mu50 | Kuroda et al. 2001 (15) | BA00017* | 935290 | 935770 | SEQ ID No. : 545 CAGATACAGATCTACGGGGA | SEQ ID No. : 546 GCATCACTCTTGTATAAACGC |
| 113. | 440 | ORF45 | Putative protein | 2 / 7. | Mu50 | Kuroda et al. 2001 (15) | BA00017* | 924749 | 925188 | SEQ ID No. : 547 ATGGTCATATGCACATCAAG | SEQ ID No. : 548 TTTTTAGCCATCTCATCGTT |
| 114. | 446 | SAV0801 | Q932D1 | 2 / 7. | Mu50 | Kuroda et al. 2001 (15) | BA00017* | 878894 | 879322 | SEQ ID No. : 549 TCGGGTTATGGCTACTTTTA | SEQ ID No. : 550 AACCATTTTCACGATGCTAT |
| 115. | 447 | SAV0792 | Q932D6 | 2 / 7. | Mu50 | Kuroda et al. 2001 (15) | BA00017* | 874223 | 874618 | SEQ ID No. : 551 GAAATGGGACACCTATCAAA | SEQ ID No. : 552 GACACACTCCGTTTCTTTCT |
| 116. | 448 | SAV0791 | Q932D7 | 2 / 7. | Mu50 | Kuroda et al. 2001 (15) | BA00017* | 872672 | 873126 | SEQ ID No. : 553 GGGCTAGTACAATATGACGC | SEQ ID No. : 554 TGGTTATACCAATGACCTCC |
| 117. | 450 | SAV0783 | Q932E5 | 2 / 7. | Mu50 | Kuroda et al. 2001 (15) | BA00017* | 869074 | 869557 | SEQ ID No. : 555 TTGGCACTTACTATTTCCGT | SEQ ID No. : 556 AACGTCCAATAATTCAATCG |
| 118. | 3 | *mecR1-1* | BAA82219 MecR1 | 3 / 7. | N315 | Kuroda et al. 2001 (15) | BA00018* | 48407 | 48828 | SEQ ID No. : 557 AAAGAATGGAACCAAGATCA | SEQ ID No. : 558 TCTTCGCCTTTTAAATGTGT |
| 119. | 6 | *aadD* | Aminoside adenyltransferase: AAD(3") | 3 / 7. | RN450(pUB110) | | AF181950# | 3409 | 3847 | SEQ ID No. : 559 GGAAGTGAATTTTGATAGCG | SEQ ID No. : 560 CTCAGAGTCGGAAAGTTGAC |
| 120. | 10 | *ble* | Bleomycin resistance protein. | 3 / 7. | RN450(pUB110) | | A31894# | 10 | 337 | SEQ ID No. : 561 GTTACAGTCTATCCGGGCAT | SEQ ID No. : 562 ATCCCACCACTGATCTTTTA |
| 121. | 11 | *ermA* | ARNr 23S methylase: MLS resistance | 3 / 7. | N315 | Kuroda et al. 2001 (15) | BA00018* | 4641 | 5023 | SEQ ID No. : 563 AATTTTCCTTCCCAAAACAT | SEQ ID No. : 564 CTGTCGGAATTGGTTTTTAG |
| 122. | 58 | *sec* | Enterotoxin C | 3 / 7. | Mu50 | Kuroda et al. 2001 (15) | BA00017* | 2134734 | 2135202 | SEQ ID No. : 565 GATAAATTTTTGGCACATGA | SEQ ID No. : 566 ACCAAAAAGTATTGCCGTTA |
| 123. | 60 | *seg** | Enterotoxin G | 3 / 7. | N315 | Kuroda et al. | BA00018* | 187650 | 18769 | SEQ ID No. : 567 | SEQ ID No. : 568 |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| | | | | | | 2001 (15) | | 5 | 84 | CCGATCCTAAATTAGACGAA | GCCAGTGTCTTGCTTTGTA |
| 124. | 64 | *entK* | Enterotoxin K | 3 / 7. | MW2 | Baba et al. 2002 (5) | BA00033* | 2086888 | 2087341 | SEQ ID No. : 569 GGAGAAAAGGCAATGAAAA | SEQ ID No. : 570 TCTCTTGAGCGGTAACAAAT |
| 125. | 65 | *entQ* | Enterotoxin Q | 3 / 7. | MW2 | Baba et al. 2002 (5) | BA00033* | 2086221 | 2086715 | SEQ ID No. : 571 ATGCTGATGTAGGGGTAATC | SEQ ID No. : 572 TTTTTCCTTTGTTAAACCCA |
| 126. | 66 | *sel** | Enterotoxin L | 3 / 7. | N315 | Kuroda et al. 2001 (15) | BA00018* | 2057196 | 2057678 | SEQ ID No. : 573 ACAGTCTTATCTAACGGCGA | SEQ ID No. : 574 TTTTGAAGAAGTGCCGTATT |
| 127. | 67 | *seo** | Enterotoxin O | 3 / 7. | N315 | Kuroda et al. 2001 (15) | BA00018* | 1880948 | 1881356 | SEQ ID No. : 575 TTGGTTAGATGGAATATCTGC | SEQ ID No. : 576 TGATTCTTTATGCTCCGAAT |
| 128. | 68 | *sem** | Enterotoxin M | 3 / 7. | N315 | Kuroda et al. 2001 (15) | BA00018* | 1879865 | 1880353 | SEQ ID No. : 577 AGCTGAATTTAAGAACGTTGA | SEQ ID No. : 578 TGGAATTTTTCAGTTTCGAC |
| 129. | 69 | *sen** | Enterotoxin N | 3 / 7. | N315 | Kuroda et al. 2001 (15) | BA00018* | 1877491 | 1877950 | SEQ ID No. : 579 ATATAACGTGGCAATTAGACG | SEQ ID No. : 580 GAATGAAAGAAAATGCATCC |
| 130. | 75 | *set21* | Exotoxin Set 21 | 3 / 7. | MW2 | Baba et al. 2002 (5) | BA00033* | 435148 | 435635 | SEQ ID No. : 581 AAGTCAAACAGTAAAAGCGG | SEQ ID No. : 582 TTCTATGGTTAATTGCATCG |
| 131. | 105 | *splE** | Serine protease SplE | 3 / 7. | NCTC8325 | | ftp://ftp.genome.ou.edu/pub/staph/ | 978047 | 978524 | SEQ ID No. : 583 ATCTGGAACAGGTTTCATTG | SEQ ID No. : 584 TGTACTTTTATTTCCAGGGC |
| 132. | 124 | *cna** | Collagen-binding protein | 3 / 7. | Cowan | | M81736# | 450 | 874 | SEQ ID No. : 585 GGTGGGTCAAGCAGTTATTA | SEQ ID No. : 586 CAGTAATTGCACTTTGTCCA |
| 133. | 125 | MW2612 | Collagen-binding protein Q8NUH0 | 3 / 7. | MW2 | Baba et al. 2002 (5) | BA00033* | 2801357 | 2801780 | SEQ ID No. : 587 GATGGTGGTAAAACGACAGT | SEQ ID No. : 588 ACCATCGTACATGTGTCGTA |
| 134. | 151 | *cap8I** | Capsular polysaccharide synthesis Cap8I | 3 / 7. | Becker | Luong *et al.* 2002 (16) | U73374# | 8806 | 9209 | SEQ ID No. : 589 GCCATTTTAATGTTTCCTTG | SEQ ID No. : 590 AGCTACAAAAATCCCACAAA |
| 135. | 152 | *cap8I* | Capsular polysaccharide synthesis Cap8I | 3 / 7. | MW2 | Baba et al. 2002 (5) | BA00033* | 153975 | 154382 | SEQ ID No. : 591 TTGCCATTTTAATGTTTCCT | SEQ ID No. : 592 AAGCTACAAAAATCCCACAA |
| 136. | 153 | *cap8k like* | 74% homology with Cap8K | 3 / 7. | MW2 | Baba et al. 2002 (5) | BA00033* | 155909 | 156348 | SEQ ID No. : 593 TAAATTTATTGGCGATTCGT | SEQ ID No. : 594 CCATTGATCCAGCTCTAAAC |
| 137. | 154 | *cap8J* | Capsular polysaccharide synthesis Cap8J Q8NYP4 | 3 / 7. | MW2 | Baba et al. 2002 (5) | BA00033* | 155430 | 155884 | SEQ ID No. : 595 TGAAAATAAAGTGGCTGGAT | SEQ ID No. : 596 TTTTCTTGATGATTTTCGCT |
| 138. | 155 | *cap8H* | Capsular polysaccharide synthesis Cap8H Q8NYP6 | 3 / 7. | MW2 | Baba et al. 2002 (5) | BA00033* | 152846 | 153303 | SEQ ID No. : 597 CGAAATACTTGGAGGAAATG | SEQ ID No. : 598 AGATAATGTTGTCACCCTGC |
| 139. | 163 | *agrB-MW2* | AgrB (MW2 variable domain) | 3 / 7. | MW2 | Baba et al. 2002 (5) | BA00033* | 2108138 | 2108556 | SEQ ID No. : 599 ATTAGGGATGCAGGTCTTAG | SEQ ID No. : 600 CCAGTTGAATAAATTGGGTA |
| 140. | 166 | *agrC-MW2** | AgrC (MW2 variable domain) | 3 / 7. | MW2 | Baba et al. 2002 (5) | BA00033* | 2108928 | 2109343 | SEQ ID No. : 601 TTCTTTGACAAAATCGGAAG | SEQ ID No. : 602 CCCAAGAATAAAAGCGAATA |
| 141. | 191 | tnpA (Tn*554*) | Tn *554A* transposase | 3 / 7. | Mu50 (Tn554) | Kuroda et al. 2001 (15) | BA00017* | 1767096 | 1767503 | SEQ ID No. : 603 TAGAAGTGGAGAATAAGCCG | SEQ ID No. : 604 ATCCTTTGAAATTTCTTCCC |
| 142. | 192 | tnpB (Tn*554*) | Tn *554B* transposase | 3 / 7. | Mu50 " | Kuroda et al. 2001 (15) | BA00017* | 59475 | 59972 | SEQ ID No. : 605 AATGAATGAAGAAATGCAGG | SEQ ID No. : 606 CAAACGTCTTTATCCCACTC |
| 143. | 202 | ORF2 | Putative transposase Q9AL26 | 3 / 7. | NCTC8325 | | ftp://ftp.genome.ou.edu/pub/staph/ | 10019 | 10458 | SEQ ID No. : 607 AGCCTTATGTGCTAAACTCAA | SEQ ID No. : 608 TTTTTGAGCAACACTGATGA |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 144. | 206 | SA0062 | Transposase IS*150* Q99XD6 | 3 / 7. | N315 | Kuroda et al. 2001 (15) | BA00018* | 70224 | 70670 | SEQ ID No. : 609 AAAGCGAATTGTCCCATAG | SEQ ID No. : 610 GCCTAGTTTTTGTTGAATTTG |
| 145. | 209 | SA1824 | *Tn557* putative protein | 3 / 7. | N315 | Kuroda et al. 2001 (15) | BA00018* | 2063656 | 2064149 | SEQ ID No. : 611 AGAATTGGAACAAGAGCGTA | SEQ ID No. : 612 TGTTATTGTGTTCGTATGGC |
| *146.* | *219* | *phi int12* | Integrase phage phi12 | 3 / 7. | NCTC8325 | | ftp://ftp.genome.ou.edu/pub/staph/ | 1314196 | 1314629 | SEQ ID No. : 613 TCAGGAGGTATAAACATGTGG | SEQ ID No. : 614 CGGGTTACTTGATTTTGAGA |
| 147. | 237 | ORF18* | Anti repressor from phage phi 11 Q8SDX0 | 3 / 7. | NCTC8325 | | ftp://ftp.genome.ou.edu/pub/staph/ | 859524 | 859983 | SEQ ID No. : 615 TAGTGCATCAGGTCAAAACA | SEQ ID No. : 616 TTCTGAGTAAAGCACCCACT |
| 148. | 238 | ORF19* | Tail lengh tape protein from phage phi 13 Q8SDK4 | 3 / 7. | NCTC8325 | | ftp://ftp.genome.ou.edu/pub/staph/ | 773297 | 773705 | SEQ ID No. : 617 TAGGCAAATGAAAATGGTTT | SEQ ID No. : 618 CATGTTACGGCCTACATTTT |
| 149. | 239 | ORF20* | Head protein from phage phi 13 Q8SDK8 | 3 / 7. | NCTC8325 | | ftp://ftp.genome.ou.edu/pub/staph/ | 768796 | 769204 | SEQ ID No. : 619 ACGTTCTCAAATCCAAGAAA | SEQ ID No. : 620 TTCTTCAACTTTTTCAAGGG |
| 150. | 240 | ORF21* | Portal protein from phage phi 13 Q8SDK9 | 3 / 7. | NCTC8325 | | ftp://ftp.genome.ou.edu/pub/staph/ | 766820 | 767304 | SEQ ID No. : 621 CAGGTTTTCAAGGAACAAAA | SEQ ID No. : 622 CGTGCGACTTAATGTGTCTA |
| 151. | 245 | ORF26 | Orf153 from phage phiSLT Q9B0H5 | 3 / 7. | MW2 | Baba et al. 2002 (5) | BA00033* | 1571500 | 1571901 | SEQ ID No. : 623 AATAAGAGAGGCTGATGTGC | SEQ ID No. : 624 TTAAAAACTCTCAACGGCTC |
| 152. | 246 | ORF27 | Phi N315 phage protein Q8LTH2 | 3 / 7. | N315 | Kuroda et al. 2001 (15) | BA00018* | 2029115 | 2029514 | SEQ ID No. : 625 GTTGTATTGAATTTGTTGCG | SEQ ID No. : 626 ATTCTTCCGAATATTTTCCC |
| *153.* | *249* | *repB** | pUB110 RepB replication protein | 3 / 7. | NCTC8325 (pUB110) | | M17852# | 571 | 989 | SEQ ID No. : 627 ACAGTTCGTTGGTTGTTTCT | SEQ ID No. : 628 TCGGTCATAAAATCCGTATC |
| *154.* | *275* | *kdpC* | Potassium-transporting ATPase C chain | 3 / 7. | Mu50(SCCmecII) | Kuroda et al. 2001 (15) | BA00017* | 80929 | 81329 | SEQ ID No. : 629 ACGGCAGTTTAGTGAAACAA | SEQ ID No. : 630 ATAATCATCTGCCATTGGTG |
| *155.* | *276* | *kdpB* | Potassium-transporting ATPase B chain | 3 / 7. | N315(SCCmecII) | Kuroda et al. 2001 (15) | BA00018* | 80355 | 80779 | SEQ ID No. : 631 ATGTAGGTTTGGCAATGAAC | SEQ ID No. : 632 GAGCTTAATGCCGATAAATG |
| *156.* | *277* | *orf N021* | Hypothetical protein kdpD | 3 / 7. | N315(SCCmecII) | Kuroda et al. 2001 (15) | BA00018* | 74302 | 74800 | SEQ ID No. : 633 GCTGAAATACCATAACGAGC | SEQ ID No. : 634 ATAGAGCCCTAATCCCAAAC |
| *157.* | *278* | *orf N022* | Hypothetical protein kdpE | 3 / 7. | N315(SCCmecII) | Kuroda et al. 2001 (15) | BA00018* | 73510 | 73935 | SEQ ID No. : 635 AACAGAGGAACAAACCATTG | SEQ ID No. : 636 CCGATTCTAGGATGTGTGAT |
| 158. | 283 | xylR | Xylose repressor Q93IC6 | 3 / 7. | N315 | Kuroda et al. 2001 (15) | BA00018* | 49793 | 50203 | SEQ ID No. : 637 ACCATCATAATATTTCGCGT | SEQ ID No. : 638 ATGTCACATGCTGCTCATTA |
| 159. | 288 | SA0077 | ORF CN005 Q9XBB4 | 3 / 7. | N315 | Kuroda et al. 2001 (15) | BA00018* | 85565 | 85983 | SEQ ID No. : 639 AATGAAGAAATCTTACGCGA | SEQ ID No. : 640 ATATTCGCATACCCTCCATA |
| 160. | 289 | SA0076 | ORF N007 Q9KX89 | 3 / 7. | N315 | Kuroda et al. 2001 (15) | BA00018* | 84870 | 85335 | SEQ ID No. : 641 AGGCTATAGGCTTCACTCAA | SEQ ID No. : 642 TTGTATTTTTGGGGTTGTTC |
| 161. | 290 | SA0061 | ORF N029 Q9KX84 | 3 / 7. | N315 | Kuroda et al. 2001 (15) | BA00018* | 69372 | 69841 | SEQ ID No. : 643 GATGATTCATGTAGGGCTGT | SEQ ID No. : 644 GCATGTTCATAATCTCTTTGA |
| 162. | 291 | SA0059 | ORF N031 Q9KX82 | 3 / 7. | N315 | Kuroda et | BA00018* | 67820 | 68303 | SEQ ID No. : 645 | SEQ ID No. : 646 |

TABLE IV

|  | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
|  |  |  |  |  |  | al. 2001 (15) |  |  |  | CCTACTGAAGTTGAAATCGC | AACTCGTCACTAAGGCAGAA |
| 163. | 304 | ORF33* | Q9RL83 | 3 / 7. | NCTC8325 |  | ftp://ftp.genome.ou.edu/pub/staph/ | 2643951 | 2644381 | SEQ ID No. : 647 ACTTTCTGAGATGCCCATAG | SEQ ID No. : 648 GCAGTTTCGTCATTGGTATT |
| 164. | 308 | ORF37 | Q9L3N7 | 3 / 7. | NCTC8325 |  | ftp://ftp.genome.ou.edu/pub/staph/ | 2547306 | 2547744 | SEQ ID No. : 649 GATGTTGTTATAATGGGGACA | SEQ ID No. : 650 GTGGCACTATCGTCTTGAAT |
| 165. | 327 | MW1898* | Q8NVQ4 | 3 / 7. | NCTC8325 |  | ftp://ftp.genome.ou.edu/pub/staph/ | 771702 | 772178 | SEQ ID No. : 651 TAGACCGTCCACAAGATACC | SEQ ID No. : 652 TCCATATTTTGTTCTCCTCG |
| 166. | 328 | MW1908* | Q8NVP4 | 3 / 7. | NCTC8325 |  | ftp://ftp.genome.ou.edu/pub/staph/ | 764874 | 765309 | SEQ ID No. : 653 AATTTTGGCAAGTTTGAAGA | SEQ ID No. : 654 TTCACGGATAAACTTGCTTT |
| 167. | 335 | ORF41 | Putative protein | 3 / 7. | NCTC8325 |  | ftp://ftp.genome.ou.edu/pub/staph/ | 9412 | 9839 | SEQ ID No. : 655 CCTATTTTCAAGCACCTGAG | SEQ ID No. : 656 ACAGTGATCATTGAATTCTGA |
| 168. | 340 | SA2259* | Q99RG2 | 3 / 7. | N315 | Kuroda et al. 2001 (15) | BA00018* | 2536822 | 2537278 | SEQ ID No. : 657 AGAGCGTTATGGGTTTACAA | SEQ ID No. : 658 TAATAATTCCCCGTCATTTG |
| 169. | 348 | SA1828 | Q99SM5 | 3 / 7. | N315 | Kuroda et al. 2001 (15) | BA00018* | 2067528 | 2067943 | SEQ ID No. : 659 GGTGTTGCCTTATACCAACT | SEQ ID No. : 660 TGAAACCCTTTATCATTGCT |
| 170. | 352 | SA1806 | Q99SP3 | 3 / 7. | N315 | Kuroda et al. 2001 (15) | BA00018* | 2045769 | 2046206 | SEQ ID No. : 661 TCCACGCAATCTCTAGTTTT | SEQ ID No. : 662 CATCTTGAACACCTTCGACT |
| 171. | 356 | SA1778 | Q99SS4 | 3 / 7. | N315 | Kuroda et al. 2001 (15) | BA00018* | 2031323 | 2031763 | SEQ ID No. : 663 AAAAATCAAAAGCGATCAAA | SEQ ID No. : 664 GCGTTTCTGTGTCTTCTTTC |
| 172. | 357 | SA1777 | Q99SS5 | 3 / 7. | N315 | Kuroda et al. 2001 (15) | BA00018* | 2030720 | 2031174 | SEQ ID No. : 665 GATGAACAGAAAATCGAGGA | SEQ ID No. : 666 TCAAAAATAACACACCCCTC |
| 173. | 358 | SA1775 | Q99SS7 | 3 / 7. | N315 | Kuroda et al. 2001 (15) | BA00018* | 2027927 | 2028423 | SEQ ID No. : 667 CGAAGATAAATGGGTTTACG | SEQ ID No. : 668 TTTGCATATTGTCGTTTTCA |
| 174. | 359 | SA1774 | Q99SS8 | 3 / 7. | N315 | Kuroda et al. 2001 (15) | BA00018* | 2027286 | 2027750 | SEQ ID No. : 669 GCAAATGGATTTTCAAGATT | SEQ ID No. : 670 CACCATAGATTTTACCCCAA |
| 175. | 360 | SA1768 | Q99ST4 | 3 / 7. | N315 | Kuroda et al. 2001 (15) | BA00018* | 2024193 | 2024643 | SEQ ID No. : 671 TCCAATTGAATCAGGGAATA | SEQ ID No. : 672 GTTACCCTCTGTCACGTTTC |
| 176. | 361 | SA1766* | Q99ST6 | 3 / 7. | N315 | Kuroda et al. 2001 (15) | BA00018* | 2022571 | 2023040 | SEQ ID No. : 673 CAAGCAATATGGCAAGGTAT | SEQ ID No. : 674 CTAAAACACCGCCTACAGTC |
| 177. | 362 | SA1755 | Q99SU8 | 3 / 7. | N315 | Kuroda et al. 2001 (15) | BA00018* | 2007579 | 2007974 | SEQ ID No. : 675 TTTAACGGCAGGAATCAGTA | SEQ ID No. : 676 TCATTAGTTTTACCAGGACCA |
| 178. | 363 | SA1641 | Q99T50 | 3 / 7. | N315 | Kuroda et al. 2001 (15) | BA00018* | 1875245 | 1875653 | SEQ ID No. : 677 AAGAAATTACAGCGGATGTT | SEQ ID No. : 678 GCTCTTTCATTTATCGGATG |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 179. | 367 | SA1341 | Q99TY3 | 3 / 7. | N315 | Kuroda et al. 2001 (15) | BA00018* | 1549769 | 1550168 | SEQ ID No. : 679 TAGCTTTTTCTAACATGGGC | SEQ ID No. : 680 TCCGAAACATTTTGAGTAGC |
| 180. | 382 | SA0142 | Q99X68 | 3 / 7. | N315 | Kuroda et al. 2001 (15) | BA00018* | 163051 | 163549 | SEQ ID No. : 681 TACTTTATTAGCGAGCAGCC | SEQ ID No. : 682 GATGTAGAACGAGATGTCCC |
| 181. | 383 | SA0141 | Q99X69 | 3 / 7. | N315 | Kuroda et al. 2001 (15) | BA00018* | 162207 | 162681 | SEQ ID No. : 683 TTAGTGATGCCTATGCCTTT | SEQ ID No. : 684 GAATTTGTTTCTGGATTTGG |
| 182. | 386 | SA0074 | Q99XD0 | 3/ 7. | N315 | Kuroda et al. 2001 (15) | BA00018* | 83977 | 84411 | SEQ ID No. : 685 AATGGTCACGATGATTTTTC | SEQ ID No. : 686 TTCTGATAATTGCAAAGGGT |
| 183. | 387 | SA0073 | Q99XD2 | 3 / 7. | N315 | Kuroda et al. 2001 (15) | BA00018* | 83154 | 83601 | SEQ ID No. : 687 AATGTTATCTGGACCGATTG | SEQ ID No. : 688 TACGCCAAACAACACATCTA |
| 184. | 388 | SA0072 | Q99XD3 | 3 / 7. | N315 | Kuroda et al. 2001 (15) | BA00018* | 81942 | 82388 | SEQ ID No. : 689 GCAGATAAAGCGATGGATAC | SEQ ID No. : 690 TGTGGTGTTTTCACTTTTCA |
| 185. | 390 | SA0047 | Q99XD8 | 3 / 7. | N315 | Kuroda et al. 2001 (15) | BA00018* | 872694 | 873137 | SEQ ID No. : 691 AAGGTTGCAGAAGATTACGA | SEQ ID No. : 692 CGGTAAAAACTCTTGGATTG |
| 186. | 391 | ORF43 | Q93ID1 | 3 / 7. | N315 | Kuroda et al. 2001 (15) | BA00018* | 54085 | 54526 | SEQ ID No. : 693 TTAGTGAAATGATGGGCATT | SEQ ID No. : 694 GGTATGTTGATTGGAAGCAT |
| 187. | 411 | MW0355* | Q8NY78 | 3 / 7. | MW2 | Baba et al. 2002 (5) | BA00033* | 401467 | 401944 | SEQ ID No. : 695 CCCAAGAAAATTACAAAACG | SEQ ID No. : 696 TCCTTTGATACCATACTGCC |
| 188. | 414 | MW0053 | Q8NYV8 | 3 / 7. | MW2 | Baba et al. 2002 (5) | BA00033* | 63193 | 63594 | SEQ ID No. : 697 TTGTTGAGTGATAAAAATTTCG | SEQ ID No. : 698 TTCATCTCTAATTTCAATGCC |
| 189. | 451 | SAV0599 | Q99W13 | 3 / 7. | Mu50 | Kuroda et al. 2001 (15) | BA00017* | 673776 | 674258 | SEQ ID No. : 699 ATTGGTTTGCGAGGTAATAG | SEQ ID No. : 700 AATGAAAAACCACCGAAATA |
| *190.* | *7* | *spc* | Spectinomycin adenyltransferase: AAD9 | 4 / 7. | Mu50 | Kuroda et al. 2001 (15) | BA00017* | 57130 | 57590 | SEQ ID No. : 701 CGGAAAAATACCAAATCAAG | SEQ ID No. : 702 TCAAAGGTACGGAGACAAGT |
| 191. | *142* | *sasK*(SAV2595) | Cell surface protein | 4 / 7. | N315 | Kuroda et al. 2001 (15) | BA00018* | 2668837 | 2669246 | SEQ ID No. : 703 TGAAGGTGTGAATGAAAACA | SEQ ID No. : 704 CTGACTTGCTCTTGCTATCC |
| *192.* | *147* | *cap5H* | Capsular polysaccharide synthesis Cap5H | 4 / 7. | N315 | Kuroda et al. 2001 (15) | BA00018* | 174646 | 175125 | SEQ ID No. : 705 GCGATTGAAAAGATAATTGG | SEQ ID No. : 706 ACCAACAACCTCATATGCTC |
| *193.* | *148* | *cap5K* | Capsular polysaccharide synthesis Cap5K Q99X60 | 4 / 7. | N315 | Kuroda et al. 2001 (15) | BA00018* | 177656 | 178077 | SEQ ID No. : 707 TAGGTAATGAAGCGTTTGGT | SEQ ID No. : 708 TGCAAACAATTCACTGGTAA |
| *194.* | *149* | *cap5J* | Capsular polysaccharide synthesis Cap5J Q99X61 | 4 / 7. | N315 | Kuroda et al. 2001 | BA00018* | 176481 | 176885 | SEQ ID No. : 709 GAGCCGGTGTATTACTCAAC | SEQ ID No. : 710 AAATGCTGAAAGGTACGAAG |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| | | | | | | (15) | | | | | |
| 195. | 150 | *cap5I* | Capsular polysaccharide synthesis Cap5I Q99X62 | 4 / 7. | N315 | Kuroda et al. 2001 (15) | BA00018* | 175337 | 175744 | SEQ ID No. : 711 ATTAAAGGCGTTACGAATGA | SEQ ID No. : 712 TTTTGCTGCATGACTTACTG |
| 196. | 187 | SAV1974* | Pyrophosphatase dUTP Q931L2 | 4 / 7. | Mu50 | Kuroda et al. 2001 (15) | BA00017* | 2110654 | 2111092 | SEQ ID No. : 713 AAATCATAAGACGGATGCAG | SEQ ID No. : 714 TGAAACACTCTCGAATTCCT |
| 197. | 198 | tnp IS *1181* | IS *1181* transposase | 4 / 7. | BM3121 ((IS1181) | Derbise et al. 1996 (11) | L14544# | 624 | 1106 | SEQ ID No. : 715 ACATGGTAAGCGTGTTTCTC | SEQ ID No. : 716 ACCGTCTTAACTTGTTGTCG |
| 198. | 203 | ORF3 | Putative transposase Q8KLQ7 | 4 / 7. | NCTC8325 | | ftp://ftp.genome.ou.edu/pub/staph/ | 583245 | 583701 | SEQ ID No. : 717 TGCGCCTATGTGTAATGATA | SEQ ID No. : 718 TGAATTAGCCGCTTTAGTTC |
| 199. | 213 | MW1750 | Putative HsdS Q8NVX9 | 4 / 7. | NCTC8325 | | ftp://ftp.genome.ou.edu/pub/staph/ | 982052 | 982456 | SEQ ID No. : 719 AGAGTGGGAAAACAAATTCA | SEQ ID No. : 720 TGTTCAGTGAGACAAGGTATTTT |
| 200. | 214 | SA0392* | Putative HsdS Q99WH0 | 4 / 7. | N315 | Kuroda et al. 2001 (15) | BA00018* | 452601 | 453044 | SEQ ID No. : 721 TTGGGGGATCTTACAGATAG | SEQ ID No. : 722 TTGCTGTTCTTCTAAACTTGG |
| 201. | 229 | ORF10 | DNA Polymerase A domain from phage phi 12 Q8SDR7 | 4 / 7. | NCTC8325 | | ftp://ftp.genome.ou.edu/pub/staph/ | 1322439 | 1322914 | SEQ ID No. : 723 ATTTGACCCTGCTGTAAAAA | SEQ ID No. : 724 TCTGCTTATCGAGCACATTA |
| 202. | 230 | ORF11 | APSE P51 from phage phi 12 Q8SDR9 | 4 / 7. | NCTC8325 | | ftp://ftp.genome.ou.edu/pub/staph/ | 1320476 | 1320885 | SEQ ID No. : 725 GAGCAAAACAATGGCTAAAC | SEQ ID No. : 726 TTCAATGCCTTTACCGTATT |
| 203. | 248 | MW0120* | Replication protein Q8NYQ5 | 4 / 7. | NCTC8325 | | ftp://ftp.genome.ou.edu/pub/staph/ | 2707436 | 2707842 | SEQ ID No. : 727 GATTGTTGGGAATCTTAGCA | SEQ ID No. : 728 CACTATTACGCACACCAAAA |
| 204. | 286 | ORF31 | ORF CN004 | 4 / 7. | N315 | Kuroda et al. 2001 (15) | BA00018* | 88968 | 89395 | SEQ ID No. : 729 AATGGAATAGAGTTGGCAGA | SEQ ID No. : 730 CTATCTTCAGTTGGCGTTTC |
| 205. | 287 | ORF32 | ORF beta Q51989 | 4 / 7. | N315 | Kuroda et al. 2001 (15) | BA00018* | 39215 | 39615 | SEQ ID No. : 731 GAGCGAAAAAGTAGAACGAA | SEQ ID No. : 732 AGCCATAACAGTTCTTGACG |
| 206. | 299 | MW0062* | Q8NYV1 | 4 / 7. | NCTC8325 | | ftp://ftp.genome.ou.edu/pub/staph/ | 2776199 | 2776678 | SEQ ID No. : 733 CAAAACTAACGACTCCCAAC | SEQ ID No. : 734 TCCTCTGAATGAGTTGGTTT |
| 207. | 300 | MW0063* | Q8NYV0 | 4 / 7. | NCTC8325 | | ftp://ftp.genome.ou.edu/pub/staph/ | 2775574 | 2776054 | SEQ ID No. : 735 AAAAGATGGCAATTCAGCTA | SEQ ID No. : 736 TGCGAGCTCTTCTATAAATTG |
| 208. | 301 | MW0064* | Q8NYU9 | 4 / 7. | NCTC8325 | | ftp://ftp.genome.ou.edu/pub/staph/ | 2774984 | 2775470 | SEQ ID No. : 737 AGGATTCGATAAAAGGGAAC | SEQ ID No. : 738 TTTTCGTCTGTGATAAGGCT |
| 209. | 302 | MW0065* | Q8NYU8 | 4 / 7. | NCTC8325 | | ftp://ftp.genome.ou.edu/pub/staph/ | 2773690 | 2774096 | SEQ ID No. : 739 GCAATGCAATATCATGTTAAG | SEQ ID No. : 740 GCAACTGCATTATCGAAGA |
| 210. | 303 | MW0066* | Q8NYU7 | 4 / 7. | NCTC8325 | | ftp://ftp.genome.ou.edu/pub/staph/ | 2773010 | 2773428 | SEQ ID No. : 741 AAAGGTGCTAAGGCAACTAA | SEQ ID No. : 742 ATTACCCGTTGTGAATCAAG |
| 211. | 309 | MW0552 | Q8NXV6 | 4 / 7. | NCTC8325 | | ftp://ftp.genome.ou.edu/pub/staph/ | 2234475 | 2234924 | SEQ ID No. : 743 TGCAAAACTTAGTGAGAAGGA | SEQ ID No. : 744 TTACTAATTGAGCCCATGCT |
| 212. | 310 | MW0554 | Q8NXV4 | 4 / 7. | NCTC8325 | | ftp://ftp.genome.ou.edu/pub/staph/ | 2233021 | 2233448 | SEQ ID No. : 745 AGGTGCGCCAAAATTAGT | SEQ ID No. : 746 AAGAAAAACAACATTAACCC |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| | | | | | | | | | | | C |
| 213. | 311 | MW0559 | Q8NXU9 | 4 / 7. | NCTC8325 | | ftp://ftp.genome.ou.edu/pub/staph/ | 2229068 | 2229539 | SEQ ID No. : 747 TGCGATGTCAGAGTCATTTA | SEQ ID No. : 748 AAAAACTTCCAGCGAAAATA |
| 214. | 312 | MW0560 | Q8NXU8 | 4 / 7. | NCTC8325 | | ftp://ftp.genome.ou.edu/pub/staph/ | 2228278 | 2228698 | SEQ ID No. : 749 TAGCACTCTTTTTCCCAATG | SEQ ID No. : 750 TGAACTTCAATCGTCAATAACA |
| 215. | 314 | MW1405 | Q8NWJ3 | 4 / 7. | NCTC8325 | | ftp://ftp.genome.ou.edu/pub/staph/ | 1331598 | 1332032 | SEQ ID No. : 751 GGCTAGGGAAAACAGTATCA | SEQ ID No. : 752 TCAAATAAACTTGAGCCCAT |
| 216. | 315 | MW1742 | Q8NVY7 | 4 / 7. | NCTC8325 | | ftp://ftp.genome.ou.edu/pub/staph/ | 991041 | 991538 | SEQ ID No. : 753 GTTCGATTATTTCAGGCATT | SEQ ID No. : 754 ATACAGCCTTAATCCAGCAG |
| 217. | 316 | MW1744 | Q8NVY5 | 4 / 7. | NCTC8325 | | ftp://ftp.genome.ou.edu/pub/staph/ | 989991 | 990416 | SEQ ID No. : 755 CAGATGTAGCTCATGCTGAA | SEQ ID No. : 756 ATTTTTCTCCTGTCAGAACG |
| 218. | 317 | MW1749* | Q8NVY0 | 4 / 7. | NCTC8325 | | ftp://ftp.genome.ou.edu/pub/staph/ | 983423 | 983869 | SEQ ID No. : 757 GAAACGATCAACGAATCAAG | SEQ ID No. : 758 AAATTCAAAAACTCTTCGCA |
| 219. | 318 | MW1757 | Q8NVX3 | 4 / 7. | NCTC8325 | | ftp://ftp.genome.ou.edu/pub/staph/ | 973057 | 973471 | SEQ ID No. : 759 TAAAACGGCTAAAGCAGAAG | SEQ ID No. : 760 ATTTTCGCCACCACTAGTTA |
| 220. | 319 | bsaG | Q8NVX2 | 4 / 7. | NCTC8325 | | ftp://ftp.genome.ou.edu/pub/staph/ | 971827 | 972321 | SEQ ID No. : 761 AAAGTTGGCGATTAAAATGA | SEQ ID No. : 762 AAAACTCCGACAACAATAGC |
| 221. | 320 | bsaE | Q8NVX1 | 4 / 7. | NCTC8325 | | ftp://ftp.genome.ou.edu/pub/staph/ | 971197 | 971661 | SEQ ID No. : 763 CCAAAAGTAATACGGGAACA | SEQ ID No. : 764 GCAACACCCAAAATAAACTC |
| 222. | 321 | bsaF | Q8NVX0 | 4 / 7. | NCTC8325 | | ftp://ftp.genome.ou.edu/pub/staph/ | 970612 | 971012 | SEQ ID No. : 765 CACAAAGTTGGTTCGCTTAT | SEQ ID No. : 766 TTTAGCTCACCCTCATGAAT |
| 223. | 322 | bsaP | Q8NVW9 | 4 / 7. | NCTC8325 | | ftp://ftp.genome.ou.edu/pub/staph/ | 969185 | 969617 | SEQ ID No. : 767 TGTGGCACAGATTAAAATGA | SEQ ID No. : 768 CGGTGCAACACCTATTAACT |
| 224. | 323 | bsaD | Q8NVW8 | 4 / 7. | NCTC8325 | | ftp://ftp.genome.ou.edu/pub/staph/ | 968492 | 968958 | SEQ ID No. : 769 TTGTTTGTGTGGCTCAGTTA | SEQ ID No. : 770 GTGCGACAACATTCTTTTTA |
| 225. | 324 | bsaC | Q8NVW7 | 4 / 7. | NCTC8325 | | ftp://ftp.genome.ou.edu/pub/staph/ | 967280 | 967775 | SEQ ID No. : 771 AAAAGCATCTACTGAAACCG | SEQ ID No. : 772 AAATTGACTTTCTTGTGGGA |
| 226. | 325 | bsaB | Q8NVW6 | 4 / 7. | NCTC8325 | | ftp://ftp.genome.ou.edu/pub/staph/ | 966612 | 967030 | SEQ ID No. : 773 ACAGCATCTTGAAGAATTGG | SEQ ID No. : 774 AATTTGGTTGCCATCTTTTA |
| 227. | 334 | MW2534 | Q9L4Q0 | 4 / 7. | NCTC8325 | | ftp://ftp.genome.ou.edu/pub/staph/ | 118552 | 118961 | SEQ ID No. : 775 AAGTTGAAGCCCCTTACTTT | SEQ ID No. : 776 GCCCTCACGTACTCTATCAA |
| 228. | 336 | SA2483* | Q99QV0 | 4 / 7. | N315 | Kuroda et al. 2001 (15) | BA00018* | 2793601 | 2794053 | SEQ ID No. : 777 TTATTGGAAGAGCTCGAAGA | SEQ ID No. : 778 TAACCCAACGATGAAACTCT |
| 229. | 339 | SA2272* | Q99RE9 | 4 / 7. | N315 | Kuroda et al. 2001 (15) | BA00018* | 2547487 | 2547969 | SEQ ID No. : 779 TGAAAAGTACGATGGTCACA | SEQ ID No. : 780 TTTTCACCAATTTCATTTCC |
| 230. | 347 | SA1829 | Q99SM4 | 4 / 7. | N315 | Kuroda et al. 2001 (15) | BA00018* | 2068198 | 2068647 | SEQ ID No. : 781 GCAATTACATGAAGCAATCA | SEQ ID No. : 782 CATCTCAATAAATGCCCAAT |
| 231. | 349 | SA1826* | Q99SM7 | 4 / 7. | N315 | Kuroda et al. 2001 (15) | BA00018* | 2065089 | 2065580 | SEQ ID No. : 783 CATAGTAAAGTGCAACCGTG | SEQ ID No. : 784 TGTCGCATTCTCATCTTGTA |
| 232. | 350 | SA1822 | Q99SN1 | 4 / 7. | N315 | Kuroda et | BA00018* | 206284 | 20632 | SEQ ID No. : 785 | SEQ ID No. : 786 |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| | | | | | | al. 2001 (15) | | 7 | 78 | ATTAACGCAATTTAACCCAG | TGGTGATATGCATTTACTCG |
| 233. | 355 | SA1791 | Q99SR0 | 4 / 7. | N315 | Kuroda et al. 2001 (15) | BA00018* | 2036110 | 2036600 | SEQ ID No. : 787 GACCTTTTTAACTGGTCACG | SEQ ID No. : 788 AAGTTTGCATTAAGCCATGT |
| 234. | 369 | SA1208 | Q99UA6 | 4 / 7. | N315 | Kuroda et al. 2001 (15) | BA00018* | 1382172 | 1382649 | SEQ ID No. : 789 GGATATTACGGTTTGGATCA | SEQ ID No. : 790 TTTGTGCAGATTGAGTTTGA |
| 235. | 371 | SA0745 | Q99VJ1 | 4 / 7. | N315 | Kuroda et al. 2001 (15) | BA00018* | 854887 | 855299 | SEQ ID No. : 791 ACAATGGGTGTAATTTGTGC | SEQ ID No. : 792 CTTACTTTTGCAGTTGCCTT |
| 236. | 375 | SA0197* | Q99X18 | 4 / 7. | N315 | Kuroda et al. 2001 (15) | BA00018* | 231800 | 232255 | SEQ ID No. : 793 TTAGAAGTGGGAGCATCAAT | SEQ ID No. : 794 ATCCCTTTAAAATTCGTGTG |
| 237. | 376 | SA0196* | Q99X19 | 4 / 7. | N315 | Kuroda et al. 2001 (15) | BA00018* | 231182 | 231599 | SEQ ID No. : 795 AGTCTTGGAGAAAGAGGGAT | SEQ ID No. : 796 CCATTCTTTGATAAAGGTATGATAA |
| 238. | 377 | SA0195* | Q99X20 | 4 / 7. | N315 | Kuroda et al. 2001 (15) | BA00018* | 229495 | 229931 | SEQ ID No. : 797 TTGGGAAAGCCTCTTTTTA | SEQ ID No. : 798 GCCATACCAACTATCAACAAA |
| 239. | 378 | SA0193* | Q99X22 | 4 / 7. | N315 | Kuroda et al. 2001 (15) | BA00018* | 227994 | 228479 | SEQ ID No. : 799 AACTGCTGCTGATCAATTTT | SEQ ID No. : 800 CCTTGATGTAAGTGTCCCAT |
| 240. | 379 | SA0192* | Q99X23 | 4 / 7. | N315 | Kuroda et al. 2001 (15) | BA00018* | 227398 | 227802 | SEQ ID No. : 801 TCAATTGGCAAACATAAGAA | SEQ ID No. : 802 CGATCAGCTTTTTCTGCTAC |
| 241. | 380 | SA0191 | Q99X24 | 4 / 7. | N315 | Kuroda et al. 2001 (15) | BA00018* | 226056 | 226507 | SEQ ID No. : 803 GGACATAAATTGAGGCGTAA | SEQ ID No. : 804 TGATACAATTGCTGTTGAGC |
| 242. | 381 | SA0190 | Q99X25 | 4 / 7. | N315 | Kuroda et al. 2001 (15) | BA00018* | 225483 | 225912 | SEQ ID No. : 805 AATCACTTTGCAAATATCCG | SEQ ID No. : 806 AGTCGAAAGTACCCAGAATG |
| 243. | 384 | SA0104 | Q99XA5 | 4 / 7. | N315 | Kuroda et al. 2001 (15) | BA00018* | 118553 | 118962 | SEQ ID No. : 807 TACTAAAGTGGGGAGTGGAA | SEQ ID No. : 808 CATCTTGACCTAAAAACCCA |
| 244. | 385 | SA0103 | Q99XA6 | 4 / 7. | N315 | Kuroda et al. 2001 (15) | BA00018* | 117458 | 117887 | SEQ ID No. : 809 ACTCGTCGCATTTATAGGAA | SEQ ID No. : 810 AATAGCAAGGGTTGTTGGTA |
| 245. | 393 | MW1748 | Q8NVY1 | 4 / 7. | MW2 | Baba et al. 2002 (5) | BA00033* | 1894694 | 1895176 | SEQ ID No. : 811 AAAACCAACAATCATATGGG | SEQ ID No. : 812 ATTGGACGTATTTTGCTCTT |
| 246. | 394 | MW1408 | Q8NWJ0 | 4 / 7. | MW2 | Baba et al. 2002 (5) | BA00033* | 1560195 | 1560627 | SEQ ID No. : 813 TATGCAGAAGCTAGCACAAA | SEQ ID No. : 814 TGCATCATTGCTAGTTGAAG |
| 247. | 403 | MW0558 | Q8NXV0 | 4 / 7. | MW2 | Baba et al. 2002 (5) | BA00033* | 643148 | 643579 | SEQ ID No. : 815 CGAAAATGAATTTGAAAGGT | SEQ ID No. : 816 ATCGAAGCCATATTCACTAA |
| 248. | 404 | MW0553* | Q8NXV5 | 4 / 7. | MW2 | Baba et al. 2002 (5) | BA00033* | 639458 | 639869 | SEQ ID No. : 817 TATGTCCAAATCAGCAGAGA | SEQ ID No. : 818 TTGTCATAATAACCCAAGCA |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 249. | 412 | MW0171 | Q8NYL7 | 4 / 7. | MW2 | Baba et al. 2002 (5) | BA00033* | 204473 | 204923 | SEQ ID No. : 819 TAAATTGAGGCGTAAAAGGA | SEQ ID No. : 820 CTTAAATATTGGAGATGCCG |
| 250. | 438 | SAV0866 | Q932A6 | 4 / 7. | Mu50 | Kuroda et al. 2001 (15) | BA00017* | 926479 | 926942 | SEQ ID No. : 821 CAGGTCAACCAATGGACTAT | SEQ ID No. : 822 CTTTTTCTCTCCTTTCAGCA |
| 251. | 442 | SAV0850 | Q932C0 | 4 / 7. | Mu50 | Kuroda et al. 2001 (15) | BA00017* | 919848 | 920224 | SEQ ID No. : 823 ATTTCCAACCTGGATGCTAT | SEQ ID No. : 824 TTTAAAAACTCTCAACGGCT |
| 252. | 2 | *mecA* | Penicillin-binding protein PLP2a | 5 / 7. | N315 | Kuroda et al. 2001 (15) | BA00018* | 46419 | 46853 | SEQ ID No. : 825 GACTGAACGTCCGATAAAAA | SEQ ID No. : 826 TGTACCCAATTTTGATCCAT |
| 253. | 4 | *mecR1-2* | Q53709 MecR1 | 5 / 7. | MW2 | Baba et al. 2002 (5) | BA00033* | 41739 | 42230 | SEQ ID No. : 827 TCAGTTCATTGCTCACGATA | SEQ ID No. : 828 CCATCGGATTATCAATGTTT |
| 254. | 39 | *fosB** | Protein related to Fos | 5 / 7. | N315 | Kuroda et al. 2001 (15) | BA00018* | 2389241 | 2389674 | SEQ ID No. : 829 CGTCATTTCAAAAATCGATAC | SEQ ID No. : 830 TGTTCTCAAGTGTGCCAGTA |
| 255. | 56 | *entA = entP* | Enterotoxin A | 5 / 7. | Mu50 | Kuroda et al. 2001 (15) | BA00017* | 2088709 | 2089186 | SEQ ID No. : 831 CGAGAAAAGCGAAGAAATAA | SEQ ID No. : 832 TCTTGCTTGAAGATCCAACT |
| 256. | 107 | *pro 8325* | Putative protease | 5 / 7. | NCTC8325 | | ftp://ftp.genome.ou.edu/pub/staph/ | 1337293 | 1337721 | SEQ ID No. : 833 TAAATTCATCTGGAGGCAGT | SEQ ID No. : 834 CGAAACGCTTATATTGCTCT |
| 257. | 161 | *agrB-8325* | AgrB (NCTC8325 variable domain) | 5 / 7. | NCTC8325 | | ftp://ftp.genome.ou.edu/pub/staph/ | 727526 | 727954 | SEQ ID No. : 835 TTGCAAGTACGATTAGGGAT | SEQ ID No. : 836 TAATTGAATGAATTGGGCA |
| 258. | 186 | SAV0039 | Phosphodiesterase Q9S3K5 | 5 / 7. | Mu50 | Kuroda et al. 2001 (15) | BA00017* | 43940 | 44416 | SEQ ID No. : 837 AGATGAACATTTGCCAACTT | SEQ ID No. : 838 AAATGCACCATTTTATCTGC |
| 259. | 197 | tnp IS *431* | IS *431* transposase | 5 / 7. | MW2 (IS431) | Baba et al. 2002 (5) | BA00033* | 36588 | 37071 | SEQ ID No. : 839 TGTTATCACTGTAGCCGTTG | SEQ ID No. : 840 AATATGACGGTGATCTTGCT |
| 260. | 204 | CAC3531 | IS*605*/IS*200*-like transposase Q97DE6 | 5 / 7. | NCTC8325 | | ftp://ftp.genome.ou.edu/pub/staph/ | 1000485 | 1000970 | SEQ ID No. : 841 AAAATGCATCTCTACGTGCT | SEQ ID No. : 842 CTGCAACGATATCCTCTTGT |
| 261. | 205 | SA1622 | Truncated tansposase Q99T66 | 5 / 7. | N315 | Kuroda et al. 2001 (15) | BA00018* | 1855391 | 1855859 | SEQ ID No. : 843 TCGCGCTTTTAATAGAGAAC | SEQ ID No. : 844 TGGCGTCAAATACTGTAATG |
| 262. | 221 | ORF5* | Peptidoglycan hydrolase from phage phi 12 Q8SDN4 | 5 / 7. | NCTC8325 | | ftp://ftp.genome.ou.edu/pub/staph/ | 1356121 | 1356592 | SEQ ID No. : 845 CAGTGTTACGATTACGCAAA | SEQ ID No. : 846 CGGCTACAAGCATAATTTTT |
| 263. | 222 | ORF6 | SLT Orf 488 from phage phi 12 Q8SDN8 | 5 / 7. | NCTC8325 | | ftp://ftp.genome.ou.edu/pub/staph/ | 1353262 | 1353737 | SEQ ID No. : 847 ATTTAGAAAACACAGCGCAT | SEQ ID No. : 848 ACTCGCATAATCTTCACCAT |
| 264. | 223 | ORF7 | SLT Orf 527 from phage phi 12 Q8SDP1 | 5 / 7. | NCTC8325 | | ftp://ftp.genome.ou.edu/pub/staph/ | 1349640 | 1350138 | SEQ ID No. : 849 ACACAATTAAGCAGACAGCG | SEQ ID No. : 850 TAATTTTTCTTTTCCTCGGC |
| 265. | 224 | ORF8 | Tail fiber protein from phage phi 12 Q8SDP3 | 5 / 7. | NCTC8325 | | ftp://ftp.genome.ou.edu/pub/staph/ | 1342441 | 1342868 | SEQ ID No. : 851 GACCATTTAGGTGTCCAAGA | SEQ ID No. : 852 TGTTCTGCATCTCTCAACTG |
| 266. | 225 | MW1393 | Major tail protein Q8NWK5 | 5 / 7. | NCTC8325 | | ftp://ftp.genome.ou.edu/pub/staph/ | 1340638 | 1341112 | SEQ ID No. : 853 TTGGAGTTTTTAACCCAGAA | SEQ ID No. : 854 ATACCTTGTGACGTTCCATC |
| 267. | 226 | ORF9 | SLT Orf 37 from phage phi 12 Q8SDP8 | 5 / 7. | NCTC8325 | | ftp://ftp.genome.ou.edu/pub/staph/ | 1337974 | 1338451 | SEQ ID No. : 855 TGAATTGAGTCAGAAAGCAA | SEQ ID No. : 856 TTTTGCTGTTTCTACGTCTG |

TABLE IV

| | N° | Gene (1) | Protein | P (2) | Strains used In PCR (3) | Référence (4) | Acc. Nb (5) | Pos1 (6) | Pos2 (6) | Forward primer | Reverse primer |
|---|---|---|---|---|---|---|---|---|---|---|---|
| 268. | 227 | MW1400 | Portal protein Q8NWJ8 | 5 / 7. | NCTC8325 | | ftp://ftp.genome.ou.edu/pub/staph/ | 1335956 | 1336450 | SEQ ID No. : 857 AATTGGATTGATCAGTCAGC | SEQ ID No. : 858 CGGACTAATACCTTGAACCA |
| 269. | 228 | MW1401* | Terminase large subunit Q8NWJ7 | 5 / 7. | NCTC8325 | | ftp://ftp.genome.ou.edu/pub/staph/ | 1334193 | 1334635 | SEQ ID No. : 859 GGTGGATTCGGTGACTATTA | SEQ ID No. : 860 CGTTTGCTAAAAGATGGATT |
| 270. | 313 | MW1386 | Q8NWL2 | 5 / 7. | NCTC8325 | | ftp://ftp.genome.ou.edu/pub/staph/ | 1351341 | 1351822 | SEQ ID No. : 861 AAAGGATTTGGGAGCTTTAG | SEQ ID No. : 862 TATCAAACCAGAAGGATTGC |
| 271. | 329 | MW2125 | Q8NVC6 | 5 / 7. | NCTC8325 | | ftp://ftp.genome.ou.edu/pub/staph/ | 538626 | 539051 | SEQ ID No. : 863 GTTTTCGCCTTCTGATTCTA | SEQ ID No. : 864 TCTTCATTATCAAATTCCGA |
| 272. | 344 | SA2005 | Q99S65 | 5 / 7. | N315 | Kuroda et al. 2001 (15) | BA00018* | 2277416 | 2277852 | SEQ ID No. : 865 ATGGAAAAATTGTTGGAAAA | SEQ ID No. : 866 AATGGATTGCTTCGACTAAA |
| 273. | 345 | SA2004 | Q99S66 | 5 / 7. | N315 | Kuroda et al. 2001 (15) | BA00018* | 2276128 | 2276610 | SEQ ID No. : 867 ACTTTAAAATACGCAGTGCC | SEQ ID No. : 868 CAAAGACGTACATGCTTCAA |
| 274. | 353 | SA1795 | Q99SQ6 | 5 / 7. | N315 | Kuroda et al. 2001 (15) | BA00018* | 2040276 | 2040707 | SEQ ID No. : 869 TTTAGACGGTTCAACCAAAT | SEQ ID No. : 870 ATTTCGTGATCTCCAAGAAT |
| 275. | 373 | SA0378* | Q99WI6 | 5 / 7. | N315 | Kuroda et al. 2001 (15) | BA00018* | 437096 | 437542 | SEQ ID No. : 871 TTGGAAGTGAGTTTTCTGGT | SEQ ID No. : 872 TTTCGATTTCATTCATGTCA |
| 276. | 374 | SA0377* | Q99WI7 | 5 / 7. | N315 | Kuroda et al. 2001 (15) | BA00018* | 436138 | 436537 | SEQ ID No. : 873 ACAAAAGGGACGATACACAC | SEQ ID No. : 874 AATAAGGCAATTTTCCGTG |
| 277. | 396 | MW0919 | Q8NXA7 | 5 / 7. | MW2 | Baba et al. 2002 (5) | BA00033* | 1011228 | 1011707 | SEQ ID No. : 875 TGGTATGACCTCTTCGATGT | SEQ ID No. : 876 TATGGGTATCCCTTTTCATC |
| 278. | 444 | SAV0804 | Q932C8 | 5 / 7. | Mu50 | Kuroda et al. 2001 (15) | BA00017* | 882088 | 882575 | SEQ ID No. : 877 TGGCTTAGATACAAGGTTTCA | SEQ ID No. : 878 CCCACCTTTTCTTATGGTAGT |
| 279. | 445 | SAV0803 | Q932C9 | 5 / 7. | Mu50 | Kuroda et al. 2001 (15) | BA00017* | 880896 | 881338 | SEQ ID No. : 879 TTATTTTGACTGCGTGTTCA | SEQ ID No. : 880 CCACTCTTCAATCCATTTTT |
| 280. | 62 | *sei** | Enterotoxin I | 6 / 7. | N315 | Kuroda et al. 2001 (15) | BA00018* | 1879150 | 1879635 | SEQ ID No. : 881 ACCTATTGCAAATCAACTCG | SEQ ID No. : 882 AAACTTACAGGCAGTCCATC |
| 281. | 72 | *set8** | Exotoxin 8 | 6 / 7. | N315 | Kuroda et al. 2001 (15) | BA00018* | 443159 | 443604 | SEQ ID No. : 883 GCTTAAAGCAGAGCGATTAG | SEQ ID No. : 884 GTTTGAGCAAAAATCCAAAC |
| 282. | 73 | *set9* | Exotoxin 9 | 6 / 7. | N315 | Kuroda et al. 2001 (15) | BA00018* | 444520 | 445009 | SEQ ID No. : 885 GTGTAATCACAACGACAACG | SEQ ID No. : 886 ACCGAGTATCTTTCCACTCC |
| 283. | 74 | *set10* | Exotoxin 10 | 6 / 7. | N315 | Kuroda et al. 2001 (15) | BA00018* | 445755 | 446157 | SEQ ID No. : 887 GCAACAGGAACAATAACGTC | SEQ ID No. : 888 TACCGTCACCTTCATCTCTC |
| 284. | 76 | *set11* | Exotoxin 11 | 6 / 7. | N315 | Kuroda et al. 2001 (15) | BA00018* | 447084 | 447487 | SEQ ID No. : 889 AGAAAAACAAAATCACC | SEQ ID No. : 890 GCTCGAATTGCAGTTTATCA |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| 285. | 77 | *set12* | Exotoxin 12 | 6 / 7. | N315 | Kuroda et al. 2001 (15) | BA00018* | 448103 | 448536 | SEQ ID No. : 891 CGTTTTGCTTTTTAACCAAC | SEQ ID No. : 892 CCATACGCTCGAAATCTAAT |
| 286. | 80 | *set15** | Exotoxin 15 | 6 / 7. | N315 | Kuroda et al. 2001 (15) | BA00018* | 454263 | 454718 | SEQ ID No. : 893 AGGGAGAGGATTTGAATTAAC | SEQ ID No. : 894 AATGTATAATAGCCACCGTT |
| 287. | 91 | *lukD* | Leucocidin D | 6 / 7. | N315 | Kuroda et al. 2001 (15) | BA00018* | 1869485 | 1869980 | SEQ ID No. : 895 ATTAGTACTTAAGGCAGCCG | SEQ ID No. : 896 TAAAGGCATTTGATGTGTTG |
| 288. | 97 | *sak* | Staphylokinase | 6 / 7. | N315 | Kuroda et al. 2001 (15) | BA00018* | 2009094 | 2009557 | SEQ ID No. : 897 GCCATGCTCAAAAGAAGTT | SEQ ID No. : 898 AAGTTGAATCCAGGGTTTTT |
| 289. | 101 | *splA** | Serine protease SplA | 6 / 7. | NCTC8325 | | ftp://ftp.genome.ou.edu/pub/staph/ | 974668 | 975086 | SEQ ID No. : 899 ATTACCGATGCAACTAAGGA | SEQ ID No. : 900 TCAAATTCCATAAACGTTCC |
| 290. | 103 | *splB* | Serine protease SplB | 6 / 7. | N315 | Kuroda et al. 2001 (15) | BA00018* | 1863774 | 1864194 | SEQ ID No. : 901 AACAACATTGGTTGAGGAAG | SEQ ID No. : 902 ATTTTTGTATGGGTGTGGAT |
| 291. | 129 | *sdrD* | Serine-Aspartate protein | 6 / 7. | COL | | http://www.tigr.org/tigr-scripts/CMR2/GenomePage3.spl?database=gsa : SA0609 | 79 | 551 | SEQ ID No. : 903 AAGTACACAGTGGGAACAGC | SEQ ID No. : 904 TCAGTTTTGGCATCTACCTT |
| 292. | 134 | *bbp=sdrE* | Bone sialoprotein-binding Serine-Aspartate protein | 6 / 7. | O24 | Tung *et al.* 2000 (21) | Y18653# | 323 | 802 | SEQ ID No. : 905 TTGATTTTTGGTCTAGGGAA | SEQ ID No. : 906 GTCTACTTTTGAAGGCGTTG |
| 293. | 179 | *sarH2** | Staphylococcal accessory regulator A | 6 / 7. | NCTC8325 | | ftp://ftp.genome.ou.edu/pub/staph/ | 245078 | 245484 | SEQ ID No. : 907 GGTGGACTATCAAACTTTCG | SEQ ID No. : 908 CTTTGCTATATTTTGGAGCC |
| 294. | 220 | *phi int13** | Integrase phage phi13 | 6 / 7. | NCTC8325 | | ftp://ftp.genome.ou.edu/pub/staph/ | 747969 | 748368 | SEQ ID No. : 909 TGAAAACACGTGTTACGAT | SEQ ID No. : 910 TCATCTAAAGCTGACCGAAT |
| 295. | 274 | vraF (SA0616) | ABC protein | 6 / 7. | N315 | Kuroda et al. 2001 (15) | BA00018* | 710121 | 710534 | SEQ ID No. : 911 AAAATGGCACAAGAAGTGTT | SEQ ID No. : 912 AGCAGATGTTCGTTGTCTTT |
| 296. | 282 | SA1801 | Anti repressor protein Q99SP8 | 6 / 7. | N315 | Kuroda et al. 2001 (15) | BA00018* | 2042963 | 2043436 | SEQ ID No. : 913 CACGCACTCACACTAGACAC | SEQ ID No. : 914 TTGTCCTTTGCCTGTTACTT |
| 297. | 292 | SA0024 | ORF CN050 Q9XB68 | 6 / 7. | N315 | Kuroda et al. 2001 (15) | BA00018* | 34654 | 35138 | SEQ ID No. : 915 AGTTGTAGGGAAGGTTGCTA | SEQ ID No. : 916 TGCTATTGACCAAGCTATCA |
| 298. | 326 | MW1769 | Q8NVW3 | 6 / 7. | NCTC8325 | | ftp://ftp.genome.ou.edu/pub/staph/ | 959692 | 960179 | SEQ ID No. : 917 TTTTGGTTGTGTATGCATCT | SEQ ID No. : 918 AAGTTATAGATGTCGGGGA |
| 299. | 337 | SA2314* | Q99RA9 | 6 / 7. | N315 | Kuroda et al. 2001 (15) | BA00018* | 2598248 | 2598725 | SEQ ID No. : 919 TCTTGGTCAGATCAATCACA | SEQ ID No. : 920 CAAAGCACTTAAATCGGATAA |
| 300. | 338 | ORF42* | Putative protein | 6 / 7. | N315 | Kuroda et al. 2001 (15) | BA00018* | 2558335 | 2558802 | SEQ ID No. : 921 GATTGTCCAAGTCCCACTAA | SEQ ID No. : 922 ACTAGTTGGTAATGTTGGCG |
| 301. | 368 | SA1320 | Q99U01 | 6 / 7. | N315 | Kuroda et al. 2001 | BA00018* | 1529059 | 1529461 | SEQ ID No. : 923 AAACCAACTGGTGAACAAAC | SEQ ID No. : 924 GTCTAGCAAATTTCTGCCAA |

TABLE IV

| | N° | Gene (1) | Protein | P (2) | Strains used in PCR (3) | Référence (4) | Acc. Nb (5) | Pos1 (6) | Pos2 (6) | Forward primer | Reverse primer |
|---|---|---|---|---|---|---|---|---|---|---|---|
| | | | | | | (15) | | | | | |
| 302. | 370 | SA1010 | Q99UU2 | 6 / 7. | N315 | Kuroda et al. 2001 (15) | BA00018* | 1143883 | 1144334 | SEQ ID No. : 925 GCAAAAAGATAACGGTACGA | SEQ ID No. : 926 TTGATGGCAACCTTTTACTT |
| 303. | 372 | SA0704 | Q99VM6 | 6 / 7. | N315 | Kuroda et al. 2001 (15) | BA00018* | 802448 | 802919 | SEQ ID No. : 927 TGTGACTTTTGATGATGGAA | SEQ ID No. : 928 TTTCAATAATGTTCCAACCC |
| 304. | 389 | SA0054 | Q99XD7 | 6 / 7. | N315 | Kuroda et al. 2001 (15) | BA00018* | 61587 | 62021 | SEQ ID No. : 929 ACTCAAGAAAACATGGGATG | SEQ ID No. : 930 GCTTTCAAAGTCCATTTTTG |

TABLE IV

# Table V : Relevant characteristics of the S.*aureus* clinical isolates

| Isolate Designation | Year | Source City/Country/Hospital | SmaI genotype [a] | Reference | Particular characteristics / Antibiotic resistance markers : common marker(s) + additionnal marker(s) | Antibiotic resistance genes (DNA macroarrays): *common gene(s )*+ additional gene(s) |
|---|---|---|---|---|---|---|
| | | | | This study | **Outbreak-related isolates from the same ward** | |
| | | | | | **PEN +** | ***blaZ* +** |
| **IPF735** | | | **101** | | MLSi | *ermC* |
| **IPF736** | | | " | | MLSi | *ermC* |
| **IPF738** | **2000** | Calais/France/A | " | | No additional marker | No additional gene |
| **IPF741** | | | " | | MLSi | *ermC* |
| **IPF743** | | | " | | MLSi | *ermC* |
| | | | | This study | **Outbreak-related isolates producing exfoliative toxinA and responsible for scalded skin syndrome in neonates** | |
| | | | | | **PEN +** | ***blaZ*** |
| **IPF308** | | | **100** | | No additional marker | No additional gene |
| **IPF310** | **2001** | **Villeneuve St Georges / France/B** | " | | No additional marker | No additional gene |
| **IPF311** | | | " | | No additional marker | No additional gene |
| **IPF313** | | | " | | No additional marker | No additional gene |
| | | **Paris / France/ C** | | Guerin *et al.*, 2000 | **Outbreak-related h-VISA[b] isolates, indistinguishable from isolates previously detected at low frequencies** | |
| | | | | | **PEN, OXA, STR, TET, MIN, SPT, KAN, NEO, TOB, GEN, MLSc, PEF, RIF, FUC, h-VISA +** | ***blaZ, mecA, tetM, spc, aadD, aacA-aphD, ermA*** |
| **IPF555** | | | **39a[A]** | | No additional marker | No additional gene |
| **IPF557** | **1999** | - | " | | FOF | No additional gene |
| **IPF562** | | | " | | FOF | No additional gene |

| Isolate Designation | Year | Source City/Country/Hospital | SmaI genotype [a] | Reference | Particular characteristics / Antibiotic resistance markers : common marker(s) + additionnal marker(s) | Antibiotic resistance genes (DNA macroarrays): *common gene(s)* + additional gene(s) |
|---|---|---|---|---|---|---|
| | | | | | **MSRA isolates with decreased susceptibility to glycopeptides, endemically spread in several European cities** | |
| | | | | | **PEN, OXA, STR, TET, MIN, SPT, KAN, NEO, TOB,GEN,MLSc, PEF, RIF** | ***blaZ, mecA, tetM, spc, aadD, aacA-aphD, ermA*** |
| **BM12612(CIP106757)** | **1998** | **Villiers St Denis / France/ D** | 39a[A] | Chesneau et *al.*, 2000 | GISA, FOF | No additional gene |
| **BM10828(CIP106759)** | **1993** | **Bordeaux / France/ E** | 39a[A] | Morvan et *al.*, 1997 | h-VISA[b], SUL, FUC, FOF | No additional gene |
| **SPAIN E1** | **1989** | **Seville / Spain/-** | 39a[A] | Murchan et *al.*, 2003 | h h-VISA[b] ,SUL, FUC | No additional gene |
| **FINLAND E7** | **1990** | **Turku / Finland/-** | 39b[A] | Murchan et *al.*, 2003 | No additional marker | No additional gene |
| **97130(CIP106761)** | **1997** | **Toulouse/ France/ F** | 39c[A] | Chesneau et *al.*, 2000 | h-VISA[b] ,FUC, FOF | No additional gene |
| **96145(CIP106762)** | **1996** | **Blois/France/ G** | 39d[A] | Chesneau et *al.*, 2000 | h-VISA[b] ,FOF | No additional gene |
| **BM10829** | **1993** | **Bordeaux / France/ E** | 39e[A] | Morvan et *al.*, 1997 | h h-VISA[b], SUL, FUC | No additional gene |

| Isolate Designation | Year | Source City/Country/Hospital | SmaI genotype a | Reference | Particular characteristics / Antibiotic resistance markers : common marker(s) + additionnal marker(s) | *Antibiotic resistance genes (DNA macroarrays): common gene(s )+ additional gene(s)* |
|---|---|---|---|---|---|---|
| | | | | Morvan et *al.*, 1997 | **Phage-type 77 MSRA isolates endemically spread in European cities** | |
| | | | | | **OXA, STR, TET, MIN, SPT, KAN, TOB ,GEN, MLSc, PEF +** | ***mecA, tetM, spc, aacA-aphD, ermA* +** |
| **BM9290** | **1987** | **Paris / France/ H** | **$45a^{A}$** | | PEN, RIF | *blaZ* |
| **BM9586** | **1987** | **Paris / France / C** | **$45a^{A}$** | | PEN, RIF, FOF | *blaZ, fos* |
| **BM12184** | **1987** | **Paris / France / C** | **$45a^{A}$** | | PEN, RIF, FOF | *blaZ, fos* |
| **BM12188** | **1987** | **Paris / France / C** | **$45a^{A}$** | | RIF | No additional gene |
| **BM10761** | **1993** | **Toulouse / France/ F** | **$45b^{A}$** | | PEN, NEO, RIF, FUC, FOF, PRI, SGA,SXT | *blaZ, aadD* |
| **BM10759** | **1993** | **Toulouse / France/ F** | **$45c^{A}$** | | PEN, NEO, RIF, FUC, FOF, PRI, SGA,SXT | *blaZ, aadD* |
| **BM9343** | **1987** | **Toulouse / France / F** | **$45d^{A}$** | | PEN, RIF, FUC, FOF | *blaZ, fos* |
| **BM10872** | **1992** | **Aalst / Belgium / I** | **$45e^{A}$** | | PEN | *blaZ* |
| **BM10888** | **1993** | **Aalst / Belgium / I** | **$45e^{A}$** | | PEN | *blaZ* |
| **BM10896** | **1994** | **Ghent / Belgium / J** | **$45f^{A}$** | | PEN, NEO | *blaZ, aadD* |
| **BM10914** | **1991** | **Paris / France / C** | **$45f^{A}$** | | PEN, NEO, RIF, FOF | *blaZ, aadD* |
| **BM10130** | **1989** | **Barcelona / Spain / K** | **$45g^{A}$** | | PEN, NEO, RIF | *blaZ, aadD* |
| **BM10138** | **1989** | **Barcelona / Spain / K** | **$45g^{A}$** | | PEN, NEO, RIF | *blaZ, aadD* |
| **BM12152** | **1989** | **Barcelona / Spain / K** | **$45i^{A}$** | | PEN, NEO, RIF | *blaZ, aadD* |

| Isolate Designation | Year | Source City/Country/Hospital | SmaI genotype a | Reference | Particular characteristics / Antibiotic resistance markers : common marker(s) + additionnal marker(s) | *Antibiotic resistance genes (DNA macroarrays): common gene(s )+ additional gene(s)* |
|---|---|---|---|---|---|---|
| | | | | Haroche et *al.*, 2003 | **SGAr isolates whose streptogramin resistant genes were previously investigated by PCR** | |
| | | | | | **PEN, KAN, NEO, SGA +** | |
| **BM3364** | **1981** | **Paris / France / C** | 13 | | OXA,TET, MIN, SPT, TOB, GEN, MLSc | *blaZ, mecA, tetM, spc, aadE, aacA-aphD, ermA, aphA-3, vgaAv,vgaB, vatB* |
| **BM12828** | **1999** | **Paris / France / C** | **16a[B]** | | OXA, SPT, TOB, MLSc, PRI, SXT, PEF | *mecA,spc, aadD, ermA, vatA,vgbA* |
| **BM12830** | **1999** | **Paris / France / C** | **16a[B]** | | OXA, SPT, TOB, GEN, MLSc, PRI, PEF,RIF | *mecA, spc, aadD, aacA-aphD, ermA, vatA, vgbA* |
| **BM12714** | **1996** | **Grenoble /France / L** | **17b[B]** | | OXA, SPT, TOB, GEN, MLSc, PRI, SXT, PEF, FOF | *blaZ, mecA, spc, aadD, aacA-aphD, ermA, vgaAv, vatB,vgaB, dfrA* |
| **BM12942** | **1999** | **Paris / France / C** | **19** | | STR, MLSc, PRI, SUL, SXT, FUC | *blaZ,aadE,aphA-3 ermC, vatA,vgbA* |
| **BM12286** | **1996** | **Paris / France / M** | **36a** | | STR, PRI | *blaZ,aadE,aphA-3, vatA,vgbA* |
| **BM12827** | **1996** | **Paris / France / M** | **36a** | | STR,PRI | *blaZ,aadE,aphA-3, vatA, vgbA* |
| **97233** | **1997** | **Paris / France / C** | **24h[F]** | | OXA, TOB, MLSc, PRI, SXT, PEF | *blaZ,mecA,aadD, vgaAv* |
| **IPF083** | **1998** | **Toulouse / France / F** | **24a[F]** | | OXA, TOB, LIN, PEF | *blaZ,mecA,aadD, vgaAv* |
| **93184** | **1993** | **Paris / France/ N** | **26 F** | | OXA, TOB, PRI, PEF, RIF | *blaZ,mecA,aadD, vatA, vgbA* |

| Isolate Designation | Year | Source City/Country/Hospital | SmaI genotype a | Reference | Particular characteristics / Antibiotic resistance markers : common marker(s) + additionnal marker(s) | *Antibiotic resistance genes (DNA macroarrays): common gene(s ) + additional gene(s)* |
|---|---|---|---|---|---|---|
| | | **Tunis/ Tunisia / O** | | This study | **Isolates from uninfected nasal carriers (NC)** | |
| **IPF139** | **2000** | | 133 | | PEN, FOF | *blaZ, fos* |
| **IPF140** | **2001** | | 139 | | PEN, | *blaZ* |
| **IPF143** | **2001** | | **149a[C]** | | PEN | *blaZ* |
| **IPF145** | **2001** | | **155a** | | PEN, MLSi, FUC, RIF | *blaZ,ermC* |
| **IPF147** | **2001** | | **121b** | | PEN, MLSi | *blaZ, ermC* |
| **IPF150** | **2001** | | **137a** | | PEN | *blaZ* |
| **IPF153** | **2001** | | 128 | | PEN, | *blaZ* |
| **IPF157** | **2001** | | 127 | | PEN,TET, MIN, STR, KAN, NEO | *blaZ, tetK, tetM, aadE, aphA-3* |
| **IPF159** | **2001** | | **108[D]** | | PEN | *blaZ* |
| **IPF511** | **2001** | | 103 | | PEN | *blaZ* |
| **IPF520** | **2002** | | 161 | | No resistance marker | No resistance gene |
| **IPF524** | **2002** | | 104 | | PEN, TET | *blaZ. tetK* |

| .Isolate Designation | Year | Source City/Country/Hospital | SmaI genotype a | Reference | Particular characteristics / Antibiotic resistance markers : common marker(s) + additionnal marker(s) | *Antibiotic resistance genes (DNA macroarrays): common gene(s )+ additional gene(s)* |
|---|---|---|---|---|---|---|
| | | | | | **Isolates responsable for bone infections ( BI)** | |
| **BM12623** | **1998** | **Tunis / Tunisia / O** | **163** | This study | PEN, TET, STR, KAN NEO, ERY | *blaZ, tetK, aadE,aphA-3, msrA* |
| **BM12633** | **1998** | **Tunis / Tunisia / O** | **136a** | " | PEN, STR, KAN NEO, ERY | *blaZ, aadE,aphA-3, msrA* |
| **BM12681** | **1997** | **Tunis / Tunisia / O** | **125a** | " | PEN, OXA,TET, MIN, STR, SPT, KAN, GEN, TOB, RIF | *blaZ,mecA, tetM, spc, aacA-aphD, ermA* |
| **BM12685** | **1997** | **Tunis / Tunisia / O** | **151a** | " | PEN | *blaZ* |
| **BM12718** | **1998** | **Tunis / Tunisia / O** | **120a** | " | PEN, TET, MIN | *blaZ, tetM* |
| **BM12881** | **1999** | **Tunis / Tunisia / O** | **140i** | " | PEN, MLSi | *blaZ, ermC* |
| **BM12889** | **1999** | **Tunis / Tunisia / O** | **117a** | " | PEN, TET, MIN | *blaZ, tetM* |
| **BM12987 (O24)** | 1989 | **Sweden** | **144e**[C] | Tung et *al.*, *2000* | PEN | *blaZ* |
| **IPF161** | **2000** | **Tunis / Tunisia / O** | **141b** | This study | STR, KAN, NEO, FUC | *mecA, aadE,aphA-3* |
| **IPF166** | **2000** | **Tunis / Tunisia / O** | **116a** | " | PEN, TET, MIN , LIN | *tetM, lnuA* |
| **IPF488** | **2001** | **Tunis / Tunisia / O** | **102** | " | PEN | *blaZ* |
| **IPF490** | **2001** | **Tunis / Tunisia / O** | **111**[D] | " | PEN | *blaZ* |
| **IPF493** | **2001** | **Tunis / Tunisia / O** | **143** | " | PEN | *blaZ* |
| **IPF494** | **2001** | **Tunis / Tunisia / O** | **132** | " | PEN | *blaZ* |
| **IPF497** | **2001** | **Tunis / Tunisia / O** | **119** | " | TET, MIN | *tetM* |
| **IPF498** | **2001** | **Tunis / Tunisia / O** | **162a** | " | PEN | *blaZ* |

| Isolate Designation | Year | Source City/Country/Hospital | SmaI genotype a | Reference | Particular characteristics / Antibiotic resistance markers : common marker(s) + additionnal marker(s) | *Antibiotic resistance genes (DNA macroarrays): common gene(s )+ additional gene(s)* |
|---|---|---|---|---|---|---|
| | | Tunis / Tunisia / O | | This study | **Isolates responsable for cutaneous infections (CI)** | |
| **BM12666** | 1998 | | 105 | | PEN | *blaZ* |
| **BM12755** | 1998 | | 123 | | PEN, OXA,TET, MIN, SPT, KAN, GEN, TOB, MLSc,RIF | *blaZ, mecA, tetK, tetM, spc, aacA-aphD, ermA, ermC* |
| **BM12764** | 1998 | | 131 | | TET, MIN | *tetM* |
| **BM12766** | 1998 | | 117a | | TET, MIN | *tetM* |
| **BM12771** | 1997 | | 120a | | PEN, TET, MIN | *blaZ, tetM* |
| **BM12816** | 1998 | | 118 | | PEN, OXA,TET, MIN, KAN, TOB, MLSi, RIF | *mecA, tetM, aadD, ermC* |
| **BM12863** | 1998 | | 130 | | PEN | *blaZ* |
| **BM12947** | 1999 | | 144a[C] | | PEN, MLSi | *blaZ, msrA* |
| **IPF505** | 2001 | | 126 | | PEN, OXA, TET, MIN, SPT, STR, KAN, GEN, TOB, MLSc, SXT, RIF | *blaZ, mecA, tetK, tetM, spc, aacA-aphD, ermA, ermC* |

Abbreviations : ERY, erythromycin ; FUC, fucidic acid; FOF, fosfomycin; h-VISA, heterogeneous vancomycin intermediate S.*aureus*; GEN, gentamicin; GISA, glycopeptide intermediate S.*aureus* ; KAN, kanamycin; LIN, lincomycin; MLSci, macrolides-lincosamides-streptograminB inducible resistance; MLSc, macrolides-lincosamides-streptograminB constitutive resistance; MIN, minocycline; NEO, neomycin ; OXA, oxacillin; PEF, pefloxacin; PEN, penicillinase; PRI, pristinamycin; RIF, rifampicin; SGA, streptograminA; SPT, spectinomycin; STR, streptomycin; SUL, sulfonamides; SXT, trimethoprim-sulfamethoxazole; TET, tetracycline; TOB, tobramycin.

[a] Strains were clustered according to the following criteria proposed by Tenover et *al., 1995*; (i) strains were grouped in the same major genotype if their patterns differed by no more than three bands (these strains were considered to be closely related and monoclonal) ; (ii) if patterns differed by betwen four and six bands, the strains were scored as being possibly related but were, nevertheless, classified into distinct genotypes to discriminate them from the closely related strains ; and (iii) if patterns differed by seven or more bands, strains were considered to be different. Major genotypes are designated by arabic numerals. Strains with indistinguable patterns were classified with in the same subtype. Subtypes are designated by arabic numerals with letter suffixes. Genotypes which include strains that are possibly relateted (less than seven bands differences) are marked with the superscript letter.

[b] All h-VISA strains were either intermediate or resistant to teicoplanin.

**Table VI : Comparative analysis of the mecA+ and mecA- isolates included in this study.**

| | Categories of isolates | | |
|---|---|---|---|
| | **mecA +** | **mecA -** | |
| **• Nb of isolates** | **36** | **44** | |
| **• Nb of *Sma*I genotypes (Table 1)** | **12** | **33** | |
| **• Nb of clusters based on gene content (Fig 1)** | **20** | **32** | |
| **• Nb of isolates harboring :** | | | **$p_g$ (1)** |
| → *seg*, *sei*, *sem*, *sen* and *seo* | **1** | **30** | $1,0x10^{-7}$ |
| → *ent*A | **32** | **9** | $1,4x10^{-8}$ |
| → *cna* | **4** | **20** | $2,9x10^{-6}$ |
| → *bbp* | **10** | **38** | 0,023 |
| → *sas*k (SAV2595) | **3** | **21** | 0,003 |

**(1)** $p_g$= normalized probability, g=388 genes used for typing.

## Claims

**1.** A method for identification at species level of *Staphylococcus aureus* bacteria from an extract of bacterial genomic DNA, **characterized in that** it comprises:

- labeling the genomic DNA after its extraction from the bacterial strain, and
- putting said labeled genomic DNA into contact with a solid support coated with a set of amplicons in suitable conditions for hybridization, each amplicon being able to specifically hybridize with a relevant gene or with a representative fragment thereof, susceptible to be present in said genomic DNA to be tested, wherein said relevant genes whose presence or absence is to be determined using the set of amplicons comprise the following genes:

  a) at least one gene considered as a positive control whose presence in a genome is characteristic for the *Staphylococcus aureus* species, and

  b) at least one gene considered as a negative control, said gene being absent in the genome of the *Staphylococcus aureus* species, and

  c) genes selected from the group A, said group A consisting in the following categories of genes :

    (i) genes encoding staphylococcal and enterococcal proteins mediating drug resistances,

    (ii) genes encoding factors known to be involved in *Staphylococcus aureus* pathogenicity and genes encoding structurally related proteins,

    (iii)genes encoding proteins produced by mobile elements,

    (iv)genes encoding factors involved in the regulation of pathological proteins,

(v) genes encoding proteins involved in the expression of antibiotic resistance or in the transfer of antibiotics. so as to obtain an hybridization profile containing detectable signals characteristic for the presence or the absence of the relevant genes in said genomic DNA to be tested, and

- identifying the *Staphylococcus aureus* bacterial strain relative to the characterization of the presence or absence of the relevant genes in its genomic DNA.

**2.** A method for typing a bacterial strain from an extract of its genomic DNA, **characterized in that** it comprises:

- identifying a least one *Staphylococcus aureus* bacterial strain according to claim 1, so as to obtain at least one hybridization profile corresponding to said *Staphylococcus aureus* bacterial strain,
- labeling the genomic DNA of the bacterial strain to be tested after its extraction and obtaining its hybridization profile using the solid support of claim 1,
- comparing the obtained hybridization profile of the bacterial strain to be tested with the hybridization profile of the *Staphylococcus aureus* bacterial strain previously identified, and
- typing the bacterial strain to be tested as a *Staphylococcus aureus* bacterial strain when the hybridization profile of the bacterial strain to be tested is close up to the hybridization profile of of said *Staphylococcus aureus* bacterial strain previously identified.

**3.** The method for identification or typing according to claim 1 or 2, **characterized in that** at least one of the two genes *nuc SA* and *sodM* as defined in Table I, preferably the two genes, is selected as positive control for the *Staphylococcus aureus* species.

**4.** The method for identification or typing according to claim 3, **characterized in that** the gene *nuc SI* as defined in Table I is selected as a negative control for the *Staphylococcus aureus* species.

**5.** The method for identification or typing according to claim 4, **characterized in that** the genes selected from the group A are all the genes of the Table T30.

**6.** The method for identification or typing according to claim 4 or 5, **characterized in that** the genes selected from the group A are all the genes of the Table T60.

**7.** The method for identification or typing according to claim 4, **characterized in that** the genes selected from the group A comprise all the genes of the Table II.

**8.** The method for identification or typing according to claim 7, **characterized in that** the genes selected from the group A comprise all the genes of the Table III.

**9.** The method for identification or typing according to claim 8, **characterized in that** the genes selected from the group A comprise putative genes selected from at least n *Staphylococcus aureus* strains whose genomes have been sequenced or may be sequenced, in the following manner, with $n \geq 3$ :

a) the sequences of the putative genes of the *Staphylococcus aureus* strains genomes are identified, and the sequences identities of the putative genes compared with each other are determined, and

b) the putative genes which have the following characteristics are selected:

- putative genes which are considered as distinct from other putative genes previously selected, and
- putative genes which are not present in all of the n *Staphylococcus aureus* strains whose genomes have been sequenced or may be sequenced, and
- putative genes which encode putative proteins having at least 150 amino-acids.

**10.** The method for identification or typing according to claim 9, **characterized in that** $n \geq 7$, the *Staphylococcus aureus* strains used for the selection of the putative genes being the strains N315, Mu50, COL, MW2, NCTC8325, MSSA-strain and EMRSA 16-strain 252.

**11.** The method for identification or typing according to claim 10, **characterized in that** the putative genes are all the genes of the Table IV.

**12.** The method for identification or typing according to claim 1 or 2, **characterized in that** the relevant genes whose presence or absence is to be determined using the set of amplicons comprise the following genes:

- all the control genes of the Table I ;
- all the genes of the Table II ;
- all the genes of the Table III ;
- all the putative genes of the Table IV.

**13.** The method for identification or typing according to anyone of claims 1 to 12, **characterized in that** each amplicon of the set of amplicons is an amplification product of a relevant gene, said amplification product being obtained from the genomic DNA of a bacterial strain containing said gene with a couple of primers specific for the gene.

**14.** The method for identification or typing according to claim 13, **characterized in that** the couple of primers used for obtention of each amplicon of the set of amplicons is selected in a manner such that the amplicons have a size of 400 to 500 base pairs.

**15.** The method for identification or typing according to claim 13 or 14, **characterized in that** when at least one of the genes *nuc SA* and *sodM* as defined in the Table I, preferably the two genes, is selected as positive control, the couple of primers specific for the gene *nuc SA* is SEQ ID No. 1 and SEQ ID No. 2, and the couple of primers specific for the gene *sodM* is SEQ ID No. 3 and SEQ ID No. 4.

**16.** The method for identification or typing according to anyone of claim 13 to 15, **characterized in that** when the gene *nuc SI* as defined in the Table I is selected as negative control, the couple of primers specific for this gene is SEQ ID No. 5 and SEQ ID No. 6.

**17.** The method for identification or typing according to anyone of claims 13 to 16, **characterized in that** the couples of primers specific for the genes of the Tables II, III and IV are the couples of primers corresponding to these genes as defined in column "couple of primers" of these Tables.

**18.** The method for identification or typing according to anyone of claims 13 to 17, **characterized in that** the bacterial strains whose genomic DNA containing said gene is used for obtaining amplification products are the following:

- the strain N315 for the gene *nuc SA,* and/or
- the strain NCTC8325 for the gene *sodM,* and/or
- the strain LRA076 of *Staphylococcus intermedius* for the gene *nuc SI,* and/or
- the corresponding strains to the genes of Table II as defined in column "Strains" of Table II, and/or
- the corresponding strains to the genes of Table III as defined in column "Strains" of Table III. and/or
- the corresponding strains to the genes of Table IVas defined in column "Strains" of Table IV.

**19.** The method for identification or typing according to anyone of claims 1 to 18, **characterized in that** the absence or the presence of a relevant gene in the genomic DNA to be tested is determined in the following manner:

after the specific hybridization step and after reading of the signal obtained for one amplicon, the value 0 is attributed when this signal is significant of the absence of the gene, or the value 1 is attributed when this signal is significant of the presence of the gene,
said values being attributed in the following manner, after determination of the following ratio R:

$$R \geq \frac{\text{Normalized Hybridization Intensity Value (NHIV)}}{\text{Normalized Hybridization Intensity Value of Reference (NHIVR)}},$$

- 0, when the ratio R is strictly inferior to 0.3,

- 1, when the ratio R is equal or superior to 0.3;

where NHIV is the ratio of the hybridization intensity value of the signal obtained for one amplicon to the average of all hybridization intensity values of the significant signals obtained on the solid support, and
where NHIVR is obtained by combining the average of NHIV values obtained for each of the amplicons after hybridization in suitable conditions of the amplified genomic DNA of the previously identified *S. aureus* bacterial strains with the set of amplicons coated on the solid support.

**20.** The method for identification at species level of *Staphylococcus aureus* bacteria according to anyone of claims 1, 3 to 19, **characterized in that** a phylogenetic tree is designed on the basis of a plurality of the hybridization profiles obtained for each of the identified *Staphylococcus aureus* bacterial strains, said hybridization profiles being clustered relative to the presence or the absence of the relevant genes.

**21.** The method for typing a bacterial strain according to anyone of claims 2 to 19, **characterized in that** a phylogenetic tree is designed on the basis of a plurality of the hybridization profiles obtained for each of the identified *Staphylococcus aureus* bacterial strains, said hybridization profiles being clustered relative to the presence or the absence of the relevant genes, and the bacterial strain to be tested is typed as a known *Staphylococcus aureus* bacterial strain when its hybridization profile is close up to the clustered hybridization profiles of the identified *Stapliylococcus aureus* bacterial strains.

**22.** A solid support coated with a set of amplicons for identification or typing of a *Staphylococcus aureus* bacterial strain from an extract of its genomic DNA, each amplicon being able to specifically hybridize with a relevant gene or with a representative fragment thereof susceptible to be present in said genomic DNA to be tested,
**characterized in that** said relevant genes whose presence or absence is to be determined using the set of amplicons comprise the following genes :

a) at least one gene considered as a positive control whose presence in a genome is characteristic for the *Staphylococcus aureus* species, and

b) at least one gene considered as a negative control, said gene being absent in the genome of said *Staphylococcus aureus* species, and

c) genes selected from the group A, said group A consisting in the following categories of genes :

(i) genes encoding staphylococcal and enterococcal proteins mediating drug resistances,

(ii) genes encoding factors known to be involved in *Staphylococcus aureus* pathogenicity and genes encoding structurally related proteins,

(iii)genes encoding proteins produced by mobile elements,

(iv)genes encoding factors involved in the regulation of pathological proteins,

(v)genes encoding proteins involved in the expression of antibiotic resistance or in the transfer of antibiotics.

**23.** The solid support according to claim 22, **characterized in that** the set of amplicons comprises the amplicons necessary for specifically hybridizing with at least 50 % of the relevant genes of the group A.

**24.** The solid support according to claim 22 or 23, **characterized in that** at least one of the two genes *nuc SA* and *sodM* as defined in Table I, preferably the two genes, is selected as positive control for the *Staphylococcus aureus* species.

**25.** The solid support according to anyone of claims 22 to 24, **characterized in that** the gene *nuc SI* as defined in Table I is selected as a negative control for the *Staphylococcus aureus* species.

**26.** The solid support according to claim 25, **characterized in that** the genes selected from the group A are all the genes of the Table T30.

**27.** The solid support according to claim 25 or 26, **characterized in that** the genes selected from the group A are all the genes of the Table T60.

**28.** The solid support according to claim 25, **characterized in that** the genes selected from the group A comprise all the genes of the Table II.

**29.** The solid support according to claim 28, **characterized in that** the genes selected from the group A comprise all the genes of the Table III.

**30.** The solid support according to claim 29, **characterized in that** the genes selected from the group A comprise all the putative genes of the Table IV.

**31.** The solid support according to claim 23, **characterized in that** the relevant genes whose presence or absence is to be determined using the set of amplicons comprise the following genes:

- all the control genes of the Table I ;
- all the genes of the Table II ;
- all the genes of the Table III ;
- all the putative genes of the Table IV.

**32.** The solid support according to anyone of claims 23 to 31, **characterized in that** each amplicon of the set of amplicons is an amplification product of a relevant gene, said amplification product being obtained from the genomic DNA of a bacterial strain containing said gene with a couple of primers specific for the gene.

**33.** The solid support according to claim 32, **characterized in that** the couple of primers used for the obtention of each amplicon of the set of amplicons is selected in a manner such that the amplicons have a size of 400 to 500 base pairs.

**34.** The solid support according to claim 32 or 33, **characterized in that** when at least one of the genes *nuc SA* and *sodM* as defined in the Table I, preferably the two genes, is selected as positive control, the couple of primers specific for the gene *nuc SA* is SEQ ID No. 1 and SEQ ID No. 2, and the couple of primers specific for the gene *sodM* is SEQ ID No. 3 and SEQ ID No. 4.

**35.** The solid support according to anyone of claims 32 to 34, **characterized in that** when the gene *nuc SI* as defined in the Table I is selected as negative control, the couple of primers specific for this gene is SEQ ID No. 5 and SEQ ID No. 6.

**36.** The solid support according to anyone of claims 32 to 35, **characterized in that** the couples of primers specific for the genes of the Tables II, III and IV are the couples of primers corresponding to these genes as defined in column "couple of primers" of these Tables.

**37.** The solid support according to anyone of claims 32 to 36, **characterized in that** the bacterial strains whose genomic DNA containing said gene is used for obtaining amplification products are the following:

- the strain N315 for the gene *nuc SA,* and/or

- the strain NCTC8325 for the gene *sodM,* and/or
- the strain LRA076 of *Staphylococcus intermedius* for the gene *nuc SI,* and/or
- the corresponding strains to the genes of Table II as defined in column "Strains" of Table II, and/or
- the corresponding strains to the genes of Table III as defined in column "Strains" of Table III, and/or
- the corresponding strains to the genes of Table IV as defined in column "Strains" of Table IV.

**38.** A DNA macro-array for identification and typing of a *Staphylococcus aureus* bacterial strain comprising the solid support of anyone of claims 23 to 37.

**39.** A kit for typing a bacterial strain comprising :

- the solid support of anyone of claims 23 to 37 or the DNA macro-array of claim 38.

**40.** The kit for typing a bacterial strain according to claim 39, **characterized in that** it also comprises :

- a plurality of hybridization profiles obtained from previously identified *Staphylococcus aureus* bacterial strains and/or possibly a phylogenetic tree designed on the basis of this plurality of hybridization profiles.

**41.** A set of couples of primers capable of specifically amplifying, in suitable conditions for hybridization, genes or representative fragments which are relevant for the identification at the species level of a *Staphylococcus aureus* strain or for typing it, said relevant genes comprising the following genes:

a) at least one gene considered as a positive control whose presence in a genome is characteristic for the *Staphylococcus aureus* species, and

b) at least one gene considered as a negative control, said gene being absent in the genome of the *Staphylococcus aureus* species, and

c) genes selected from the group A, said group A consisting in the following categories of genes :

(i) genes encoding staphylococcal and enterococcal proteins mediating drug resistances,

(ii) genes encoding factors known to be involved in *Staphylococcus aureus* pathogenicity and genes encoding structurally related proteins,

(iii)genes encoding proteins produced by mobile elements,

(iv)genes encoding factors involved in the regulation of pathological proteins,

(v) genes encoding proteins involved in the expression of antibiotic resistance or in the transfer of antibiotics.

**42.** The set of couples of primers according to claim 41, **characterized in that** at least one of the two genes *nuc SA* and *sodM* as defined in Table I, preferably the two genes, is selected as positive control for the *Staphylococcus aureus* species, the couple of primers specific for the gene *nuc SA* having the sequences SEQ ID No. 1 and SEQ ID No. 2, and the couple of primers specific for the gene *sodM* having the sequences SEQ ID No. 3 and SEQ ID No. 4.

**43.** The set of couples of primers according to claim 42, **characterized in that** the gene *nuc SI* as defined in Table I is selected as a negative control for the *Staphylococcus aureus* species, the couple of primers specific for this gene having the sequences SEQ ID No. 5 and SEQ ID No. 6.

**44.** The set of couples of primers according to claim 43, **characterized in that** the genes selected from the group A comprise all the genes of the Table II, the couples of primers corresponding to these genes being defined in column "couple of primers" of this Table II.

**45.** The set of couples of primers according to claim 44, **characterized in that** the genes selected from the group A comprise all the genes of the Table III, the couples of primers corresponding to these genes being defined in column "couple of primers" of this Table III.

**46.** The set of couples of primers according to claim 45, **characterized in that** the genes selected from the group A comprise all the putative genes of the Table IV, the couples of primers corresponding to these genes being defined in column "couple of primers" of this Table IV.

**47.** The solid support according to claim 41, **characterized in that** the genes relevant for the *Staphylococcus aureus* species comprise the following genes:

- all the control genes of the Table I;
- all the genes of the Table II ;
- all the genes of the Table III ;
- all the putative genes of the Table IV;

the couples of primers corresponding to these genes being defined in column "couple of primers" of these Tables.

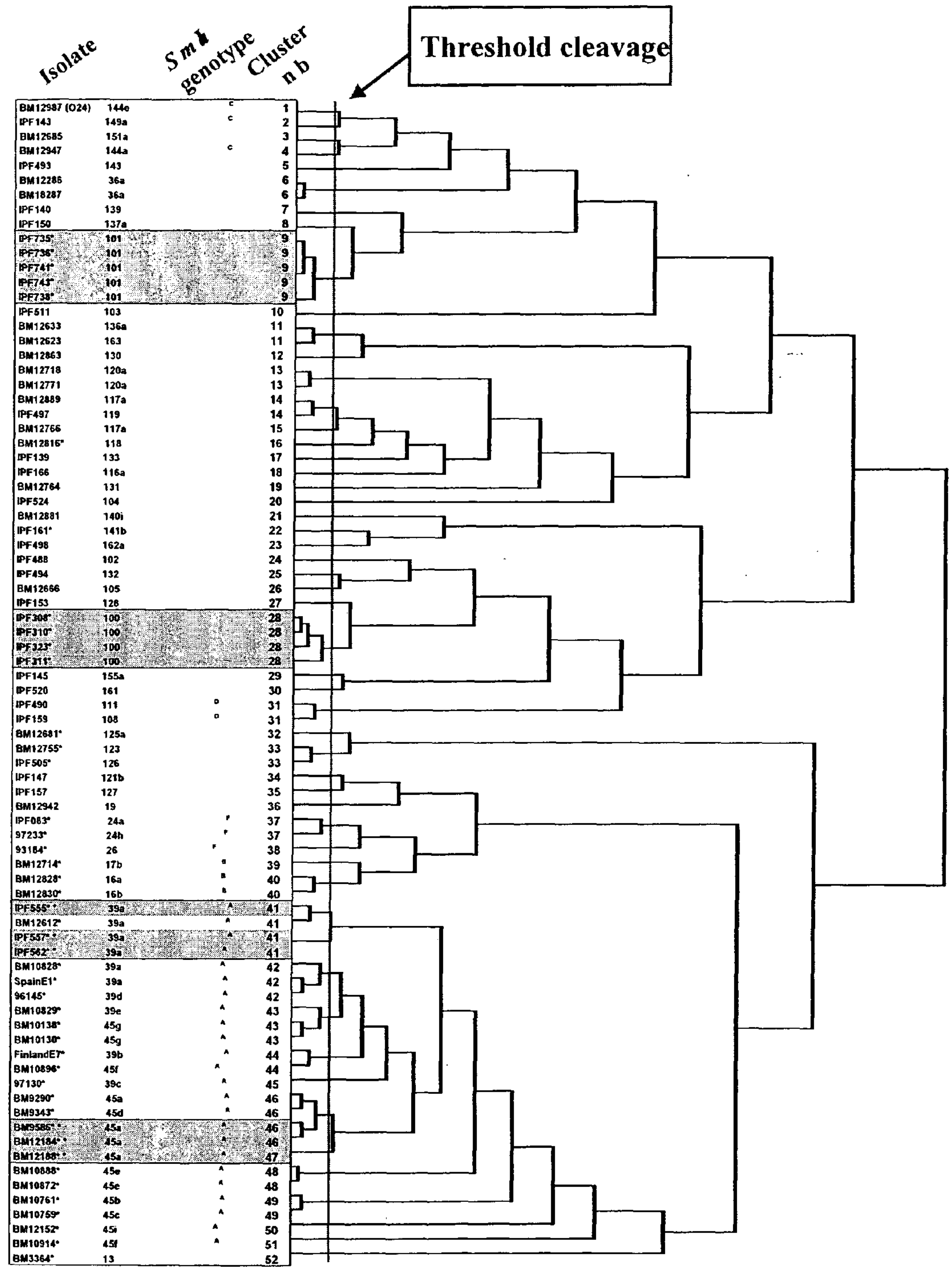
Threshold cleavage
Isolate
Smal genotype
Cluster nb
BM12987 (O24) 144e c 1
IPF143 149a c 2
BM12685 151a 3
BM12947 144a c 4
IPF493 143 5
BM12286 36a 6
BM18287 36a 6
IPF140 139 7
IPF150 137a 8
IPF735* 101 9
IPF736* 101 9
IPF741* 101 9
IPF743* 101 9
IPF738* 101 9
IPF511 103 10
BM12633 136a 11
BM12623 163 11
BM12863 130 12
BM12718 120a 13
BM12771 120a 13
BM12889 117a 14
IPF497 119 14
BM12766 117a 15
BM12816* 118 16
IPF139 133 17
IPF166 116a 18
BM12764 131 19
IPF524 104 20
BM12881 140i 21
IPF161* 141b 22
IPF498 162a 23
IPF488 102 24
IPF494 132 25
BM12666 105 26
IPF153 128 27
IPF308* 100 28
IPF310* 100 28
IPF323* 100 28
IPF311* 100 28
IPF145 155a 29
IPF520 161 30
IPF490 111 D 31
IPF159 108 D 31
BM12681* 125a 32
BM12755* 123 33
IPF505* 126 33
IPF147 121b 34
IPF157 127 35
BM12942 19 36
IPF083* 24a F 37
97233* 24h F 37
93184* 26 F 38
BM12714* 17b 39
BM12828* 16a 40
BM12830* 16b 40
IPF555* * 39a A 41
BM12612* 39a A 41
IPF557* * 39a A 41
IPF562* * 39a A 41
BM10828* 39a A 42
SpainE1* 39a A 42
96145* 39d A 42
BM10829* 39e A 43
BM10138* 45g A 43
BM10130* 45g A 43
FinlandE7* 39b A 44
BM10896* 45f A 44
97130* 39c A 45
BM9290* 45a A 46
BM9343* 45d A 46
BM9586* * 45a A 46
BM12184* * 45a A 46
BM12188* * 45a A 47
BM10888* 45e A 48
BM10872* 45e A 48
BM10761* 45b A 49
BM10759* 45c A 49
BM12152* 45i A 50
BM10914* 45f A 51
BM3364* 13 52

Figure 1

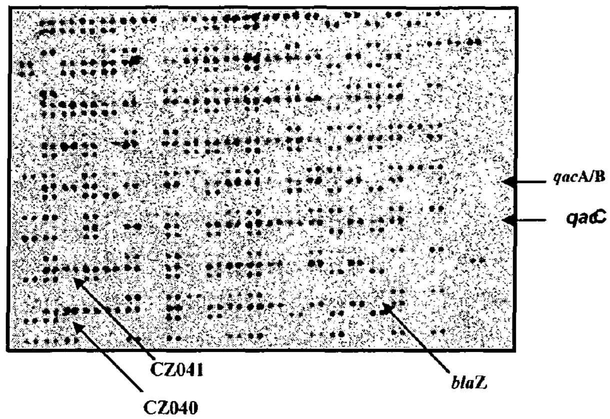


Figure 2 A

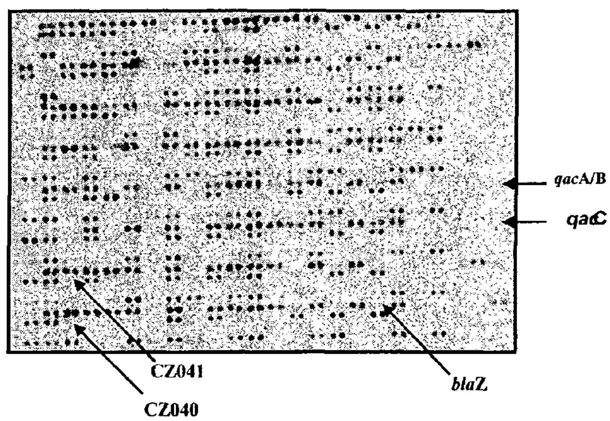


Figure 2B

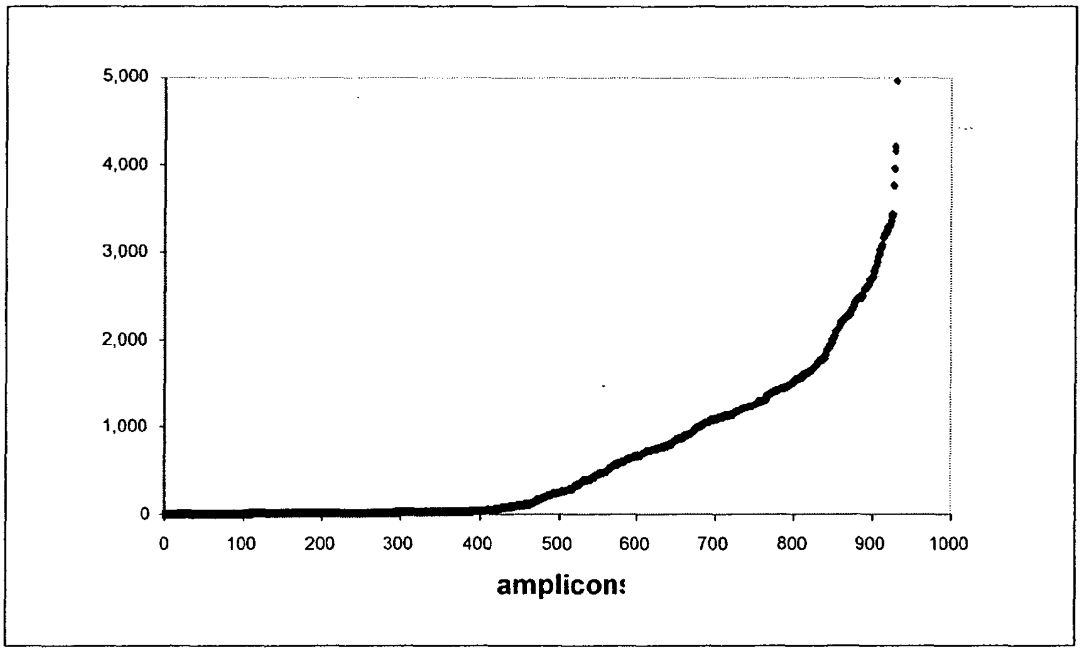
5,000
4,000
3,000
2,000
1,000
0
0
100
200
300
400
500
600
700
800
900
1000
amplicon!

Figure 3

European Patent Office

# PARTIAL EUROPEAN SEARCH REPORT

which under Rule 45 of the European Patent Convention shall be considered, for the purposes of subsequent proceedings, as the European search report

Application Number
EP 03 29 3079

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | VAN LEEUWEN ET AL.: "Multilocus sequence typing of Staphylococcus aureus with DNA array technology" JOURNAL OF CLINICAL MICROBIOLOGY, vol. 41, no. 7, July 2003 (2003-07), pages 3323-3326, XP002280375 | 1,22,23,32 | C12Q1/68 |
| Y | Multilocus sequence typing of S. aureus using an array of several internal fragments of housekeeping genes * the whole document * ----- | 3,4,34,35 | |
| X | WO 02/099034 A (INFECTIO DIAGNOSTIC INC ; HULETSKY ANN (CA); ROSSBACH VALERY (CA)) 12 December 2002 (2002-12-12) | 1,22,23,32,33 | |
| Y | Sequences, and methods using them, for the detection of methicillin-resistant Staphylococcus aureus ----- -/-- | 3,4,34,35 | |

TECHNICAL FIELDS SEARCHED (Int.Cl.7)

C12Q

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 2 June 2004 | Bort, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C07)

European Patent Office

# PARTIAL EUROPEAN SEARCH REPORT

Application Number
EP 03 29 3079

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| D,X | DATABASE GENEBANK Staphylococcus aureus subsp. aureus N315 10 December 2002 (2002-12-10), AOKI ET AL.: "Whole genome sequence of meticillin-resistant Staphylococcus aureus" XP002280617 Database accession no. NC_002745.1 | 24 | |
| Y | Complete sequence of S. aureus subsp. aureus N315; primers corresponding to SEQ ID Nos. 1 and 2 are placed at nt. 1321407-1321426 and 1321811-1321831; only relevant sequence data supplied * abstract * ----- | 3,4,34 | |
| D,X | DATABASE GENEBANK 9 June 1994 (1994-06-09), EL SOLH N: "S. intermedius nuc gene for thermonuclease" XP002280616 Database accession no. X67678 | 25 | TECHNICAL FIELDS SEARCHED (Int.Cl.7) |
| Y | Complete sequence of nuc SI gene; primers corresponding to SEQ ID No 5 and 6 are placed at nt 693-711 and 978-998, respectively * abstract * ----- | 4,35 | |

4

EPO FORM 1503 03.82 (P04C10)

European Patent Office

**INCOMPLETE SEARCH SHEET C**

Application Number
EP 03 29 3079

Claim(s) searched completely:
1, 3, 4, 22-25, 32 (all partially), 34, 35 (all completely)

Claim(s) not searched:
5, 26

Reason for the limitation of the search:

Claims 5 and 26 do not meet the requirements of Art. 84 EPC, in the sense that the matter for which protection is sought is not clearly defined. Said claims refer to genes listed in Table T30; however, table T30 does not disclose specific genes but clomplete Staphylococcus aureus clones, which comprise more than one gene. Consequently, no search could be carried out for said claims

European Patent Office

Application Number

EP 03 29 3079

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1, 3, 4, 22-25, 32 (partially); 5, 26, 34, 35 (completely)

European Patent Office

LACK OF UNITY OF INVENTION
SHEET B

Application Number
EP 03 29 3079

The Search Division considers that the present European patent application does not comply with the requirements of unity of invention and relates to several inventions or groups of inventions, namely:

1. claims: 1, 3, 4, 22-25, 32 (all partially), 5, 26, 34, 35 (all completely)

Invention 1
A solid support comprising: 1) at least one of the amplicons obtained using the primers corresponding to SEQ ID 1 and 2, for determining the presence of the nuc SA gene, or the primers corresponding to SEQ ID 3 and 4, for determining the presence of the sod M gene; 2) the amplicon obtained using the primers corresponding to SEQ ID 5 and 6, for determining the presence of nuc SI gene; and 3) a set of amplicons for determining the genes of Table T30; and, methods using said solid support for identification at species level of Staphyloccoccus aeureus bacteria from an extract of bacterial genomic DNA

---

2. claims: 1, 3, 4, 22-25 (all partially), 6, 27 (all completely)

Invention 2
A solid support comprising: 1) at least one of the amplicons obtained using the primers corresponding to SEQ ID 1 and 2, for determining the presence of the nuc SA gene, or the primers corresponding to SEQ ID 3 and 4, for determining the presence of the sod M gene; 2) the amplicon obtained using the primers corresponding to SEQ ID 5 and 6, for determining the presence of nuc SI gene; and 3) a set of amplicons for determining the genes of Table T60; and, methods using said solid support for identification at species level of Staphyloccoccus aeureus bacteria from an extract of bacterial genomic DNA

---

3. claims: 1, 3, 4, 13, 22, 24, 25, 32 (all partially) 2, 7-12, 14, 15, 17, 21, 28-31, 36, 38-47 (all completely)

European Patent Office

**LACK OF UNITY OF INVENTION SHEET B**

Application Number
EP 03 29 3079

The Search Division considers that the present European patent application does not comply with the requirements of unity of invention and relates to several inventions or groups of inventions, namely:

Invention 3
A solid support/DNA macro-array/kit comprising: 1) at least one of the amplicons obtained using the primers corresponding to SEQ ID 1 and 2, for determining the presence of the nuc SA gene, or the amplicon obtained using the primers corresponding to SEQ ID 3 and 4, for determining the presence of the sod M gene; 2) the amplicon obtained using the primers corresponding to SEQ ID 5 and 6, for determining the presence of nuc SI gene; and 3) a set of amplicons for determining at least all the genes of Table II; a set of couples of primers, comprising the primers corresponding to SEQ ID 1-6 and all the primers listed in column "couple of primers" of Table II; and, methods using said solid support for identification at species level of Staphyloccoccus aeureus bacteria from an extract of bacterial genomic DNA

---

4. claims: 1, 13, 22, 32 (all partially), 14, 33 (all completely)

Invention 4
A solid support for the identification at species level of Staphyloccoccus aeureus bacteria from an extract of bacterial genomic DNA, wherein the method comprises: labelling the genomic DNA; puting said labelled genomic DNA into contact with a solid support coated with a set of amplicons, wherein said amplicons have a saize of 400 to 500 base pairs, and wherein said amplicons can hybridise to: at least one gene whose presence in a genome is characteristic for the S. aureus; at least one gene being absent in the genome of S. aureus; and at least one gene coding for a staphyloccoccal protein; and methods using it

---

5. claims: 1, 13, 32 (partially), 18, 37 (all completely)

Invention 5
A solid support coated with amplicons, where said amplicons have been obtained from the following strains: N315, and/or NCT8325, and/or LRA076, and/or the strains listed in Table II, and/or those listed in Table III, and/or those listed in Table IV; as well as methods using it

---

6. claims: 1 (partially), 19 (completely)

European Patent Office

# LACK OF UNITY OF INVENTION SHEET B

Application Number
EP 03 29 3079

The Search Division considers that the present European patent application does not comply with the requirements of unity of invention and relates to several inventions or groups of inventions, namely:

Invention 6
A method for identification at species level of Staphyloccoccus aeureus bacteria from an extract of bacterial genomic DNA, wherein the method comprises: labelling the genomic DNA; puting said labelled genomic DNA into contact with a solid support coated with a set of amplicons, including relevant genes, and wherein the presence or absence of a relevant gene in the genomic DNA is determined by determining a ratio (R)

---

7. claims: 1 (partially), 20 (completely)

Invention 7
A method for identification at species level of Staphyloccoccus aeureus bacteria from an extract of bacterial genomic DNA, wherein the method comprises: labelling the genomic DNA; puting said labelled genomic DNA into contact with a solid support coated with a set of amplicons, and design of a phylogenetic tree

---

## ANNEX TO THE EUROPEAN SEARCH REPORT ON EUROPEAN PATENT APPLICATION NO. EP 03 29 3079

This annex lists the patent family members relating to the patent documents cited in the above-mentioned European search report. The members are as contained in the European Patent Office EDP file on
The European Patent Office is in no way liable for these particulars which are merely given for the purpose of information.

02-06-2004

| Patent document cited in search report | Publication date | Patent family member(s) | Publication date |
|---|---|---|---|
| WO 02099034 A | 12-12-2002 | CA 2348042 A1 | 04-12-2002 |
| | | WO 02099034 A2 | 12-12-2002 |
| | | CA 2448975 A1 | 12-12-2002 |
| | | EP 1397510 A2 | 17-03-2004 |

EPO FORM P0459

For more details about this annex : see Official Journal of the European Patent Office, No. 12/82

SEQUENCE LISTING

<110> INSTITUT PASTEUR

<120> DNA macro-array for Staphylococcus aureus identification and typing

<130> D21773

<140>
<141>

<160> 930

<170> PatentIn version 3.2

<210> 1
<211> 20
<212> DNA
<213> artificial

<220>
<221> source
<222> (1)..(20)
<223> /note=«Description of artificial sequence: Forward primer, nuc SA* gene encoding Thermonuclease»

<400> 1
aagtcaaata aatcgcttgc 20

<210> 2
<211> 20
<212> DNA
<213> Artificial

<220>
<221> source
<222> (1)..(20)
<223> /note=«Description of artificial sequence: Reverse primer, nuc SA* gene encoding Thermonuclease»

<400> 2
cccttttcca ctaattcctt 20

<210> 3
<211> 20
<212> DNA
<213> artificial

<220>
<221> source
<222> (1)..(20)
<223> /note=«Description of artificial sequence: Forward primer, sodM* gene encoding Superoxide dismutase»

<400> 3
ttcatcacga caaacatcac 20

<210> 4
<211> 20
<212> DNA
<213> artificial

<220>
<221> source
<222> (1)..(20)
<223> /note=«Description of artificial sequence: Reverse primer, sodM*gene encoding Superoxide dismutase»

<400> 4
atgttccaaa atgcagtcat 20

<210> 5
<211> 19
<212> DNA
<213> artificial

<220>
<221> source
<222> (1)..(19)
<223> /note=«Description of artificial sequence: Forward primer, nuc SIgene encoding S. intermedius thermonuclease»

<400> 5
ggtgtggata caccagaac 19

<210> 6
<211> 21
<212> DNA
<213> artificial

<220>
<221> source
<222> (1)..(21)
<223> /note=«Description of artificial sequence: Reverse primer, nuc SI* gene encoding S. intermedius thermonuclease»

<400> 6
gcgttcccaa atgttcagct g 21

<210> 7
<211> 20
<212> DNA
<213> artificial

<220>
<221> source
<222> (1)..(20)
<223> /note=«Description of artificial sequence: Forward primer, aadE gene encoding Streptomycin adenyltransferase: AAD6'»

<400> 7
tgaacgtatt cgaattgtga 20

<210> 8

```
<211> 20
<212> DNA
<213> artificial

<220>
<221> source
<222> (1)..(20)
<223> /note=«Description of artificial sequence: Reverse primer,
      aadE gene encoding Streptomycin adenyltransferase: AAD6'»

<400> 8
cggcacaatc ctttaataac                                                   20


<210> 9
<211> 20
<212> DNA
<213> artificial

<220>
<221> source
<222> (1)..(20)
<223> /note=«Description of artificial sequence: Forward primer,
      aphA-3 gene encoding Aminoside phosphotransferase:
      APH3'-III»

<400> 9
taaagggacc acctatgatg                                                   20


<210> 10
<211> 20
<212> DNA
<213> artificial

<220>
<221> source
<222> (1)..(20)
<223> /note=«Description of artificial sequence: Reverse primer,
      aphA-3 gene encoding Aminoside phosphotransferase:
      APH3'-III»

<400> 10
acaaagatgt tgctgtctcc                                                   20


<210> 11
<211> 20
<212> DNA
<213> artificial

<220>
<221> source
<222> (1)..(20)
<223> /note=«Description of artificial sequence: Forward primer,
      aphA-3 gene encoding Aminoside phosphotransferase:
      APH3'-III»

<400> 11
gtttacgaaa ttggaacagg                                                   20
```

<210> 12
<211> 20
<212> DNA
<213> artificial

<220>
<221> source
<222> (1)..(20)
<223> /note=«Description of artificial sequence: Reverse primer, ermB gene encoding ARNr 23S methylase: MLS resistance

<400> 12
tgaatcgaga cttgagtgtg 20

<210> 13
<211> 21
<212> DNA
<213> artificial

<220>
<221> source
<222> (1)..(21)
<223> /note=«Description of artificial sequence: Forward primer, ermC gene encoding ARNr 23S methylase: MLS resistance»

<400> 13
tgatcacgat aatttccaag t 21

<210> 14
<211> 20
<212> DNA
<213> artificial

<220>
<221> source
<222> (1)..(20)
<223> /note=«Description of artificial sequence: Reverse primer, ermC gene encoding ARNr 23S methylase: MLS resistance»

<400> 14
attcctgcat gttttaagga 20

<210> 15
<211> 20
<212> DNA
<213> artificial

<220>
<221> source
<222> (1)..(20)
<223> /note=«Description of artificial sequence: Forward primer, ermY gene encoding ARNr 23S methylase: MLS resistance»

<400> 15
gggcatttca cactagaact 20

<210> 16
<211> 20
<212> DNA
<213> artificial

<220>
<221> source
<222> (1)..(20)
<223> /note=«Description of artificial sequence: Reverse primer, ermY gene econding ARNr 23S methylase: MLS resistance»

<400> 16
ttaattttag gctttggatg 20

<210> 17
<211> 20
<212> DNA
<213> artificial

<220>
<221> source
<222> (1)..(20)
<223> /note=«Description of artificial sequence: Forward primer, mphC gene encoding Erythromycine phosphotransferase»

<400> 17
atttgaaata ccgaagtgga 20

<210> 18
<211> 20
<212> DNA
<213> artificial

<220>
<221> source
<222> (1)..(20)
<223> /note=«Description of artificial sequence: Reverse primer, mphC gene encoding Erythromycine phosphotransferase»

<400> 18
cgtttgcttg gttatctacc 20

<210> 19
<211> 20
<212> DNA
<213> artificial

<220>
<221> source
<222> (1)..(20)
<223> /note=«Description of artificial sequence: Forward primer, msrA gene encoding ABC protein : MS resistance»

<400> 19
aaatagcact tattgggggt 20

<210> 20
<211> 20
<212> DNA
<213> artificial

<220>
<221> source
<222> (1)..(20)
<223> /note=«Description of artificial sequence: primer»

<400> 20
tgtgtcagca atttggtcta 20

<210> 21
<211> 20
<212> DNA
<213> artificial

<220>
<221> source
<222> (1)..(20)
<223> /note=«Description of artificial sequence: Forward primer, msrC gene encoding ABC protein : MS resistance»

<400> 21
tccaaacaga gatcaccttc 20

<210> 22
<211> 20
<212> DNA
<213> artificial

<220>
<221> source
<222> (1)..(20)
<223> /note=«Description of artificial sequence: Reverse primer, msrC gene encoding ABC protein : MS resistance»

<400> 22
ataattcccc gaaaactctc 20

<210> 23
<211> 20
<212> DNA
<213> artificial

<220>
<221> source
<222> (1)..(20)
<223> /note=«Description of artificial sequence: Forward primer, lnuA gene encoding Lincosamide nucleotidyltransferase»

<400> 23
actcattggt tagatggagg 20

<210> 24

<211> 20
<212> DNA
<213> artificial

<220>
<221> source
<222> (1)..(20)
<223> /note=«Description of artificial sequence: Reverse primer, lnuA gene encoding Lincosamide nucleotidyltransferase»

<400> 24
aaccggaatg aaaaagaagt 20

<210> 25
<211> 20
<212> DNA
<213> artificial

<220>
<221> source
<222> (1)..(20)
<223> /note=«Description of artificial sequence: Forward primer, lnuBgene encoding Lincosamide nucleotidyltransferase»

<400> 25
cttatacgtg gggaatttca 20

<210> 26
<211> 20
<212> DNA
<213> artificial

<220>
<221> source
<222> (1)..(20)
<223> /note=«Description of artificial sequence: Reverse primer, lnuBgene encoding Lincosamide nucleotidyltransferase»

<400> 26
tctaatcgag cagtggtctt 20

<210> 27
<211> 20
<212> DNA
<213> artificial

<220>
<221> source
<222> (1)..(20)
<223> /note=«Description of artificial sequence: Forward primer, lsa gene encoding ABC protein: streptogramin A and lincosamide resistance»

<400> 27
cgtctaggct acgagaaaaa 20

<210> 28

```
<211> 20
<212> DNA
<213> artificial

<220>
<221> source
<222> (1)..(20)
<223> /note=«Description of artificial sequence: Reverse primer,
       lsa gene encoding ABC protein: streptogramin A and
       lincosamide resistance»

<400> 28
aaagctctgc tgattgagac                                                20


<210> 29
<211> 20
<212> DNA
<213> artificial

<220>
<221> source
<222> (1)..(20)
<223> /note=«Description of artificial sequence: Forward primer,
       vga gene encoding ABC protein: Streptogramin A
       resistance»

<400> 29
tgttgaacat aaatcgccta                                                20


<210> 30
<211> 20
<212> DNA
<213> artificial

<220>
<221> source
<222> (1)..(20)
<223> /note=«Description of artificial sequence: Reverse primer,
       vga gene encoding ABC protein: Streptogramin A
       resistance»

<400> 30
cagttattct tccctcgtca                                                20


<210> 31
<211> 22
<212> DNA
<213> artificial

<220>
<221> source
<222> (1)..(22)
<223> /note=«Description of artificial sequence: Forward primer,
       vgaB* gene encoding ABC protein: Streptogramin A
       resistance»

<400> 31
tctctcaatt agaagaacca ca                                             22
```

<210> 32
<211> 20
<212> DNA
<213> artificial

<220>
<221> source
<222> (1)..(20)
<223> /note=«Description of artificial sequence: Reverse primer, vgaB* gene encoding ABC protein: Streptogramin A resistance»

<400> 32
ttgctttcgt agaagcattt 20

<210> 33
<211> 20
<212> DNA
<213> artificial

<220>
<221> source
<222> (1)..(20)
<223> /note=«Description of artificial sequence: Forward primer, vgav gene encoding ABC protein: Streptogramin A resistance»

<400> 33
atgaccgaat tgggttagtt 20

<210> 34
<211> 20
<212> DNA
<213> artificial

<220>
<221> source
<222> (1)..(20)
<223> /note=«Description of artificial sequence: Reverse primer, vgav gene encoding ABC protein: Streptogramin A resistance»

<400> 34
agttcccttt gtattcggtt 20

<210> 35
<211> 20
<212> DNA
<213> artificial

<220>
<221> source
<222> (1)..(20)
<223> /note=«Description of artificial sequence: Forward primer, vatA gene encoding Streptogramin A acetyl transferase»

<400> 35
agtaagcgag gagaatcctt 20

<210> 36
<211> 20
<212> DNA
<213> artificial

<220>
<221> source
<222> (1)..(20)
<223> /note=«Description of artificial sequence: Forward primer, vatA gene encoding Streptogramin A acetyl transferase»

<400> 36
tccaccgaca atagaatagg 20

<210> 37
<211> 20
<212> DNA
<213> artificial

<220>
<221> source
<222> (1)..(20)
<223> /note=«Description of artificial sequence: Forward primer, vatB gene encoding Streptogramin A acetyl transferase»

<400> 37
atatggccct gatccaaata 20

<210> 38
<211> 20
<212> DNA
<213> artificial

<220>
<221> source
<222> (1)..(20)
<223> /note=«Description of artificial sequence: Reverse primer, vatB gene encoding Streptogramin A acetyl transferase»

<400> 38
aacaattgct ccatctccta 20

<210> 39
<211> 20
<212> DNA
<213> artificial

<220>
<221> source
<222> (1)..(20)
<223> /note=«Description of artificial sequence: Forward primer, vatC gene encoding Streptogramin A acetyl transferase»

<400> 39

accatctata acaaagccca 20

<210> 40
<211> 20
<212> DNA
<213> artificial

<220>
<221> source
<222> (1)..(20)
<223> /note=«Description of artificial sequence: Reverse primer, vatC gene encoding Streptogramin A acetyl transferase»

<400> 40
ataatagccc cgtttcctat 20

<210> 41
<211> 22
<212> DNA
<213> artificial

<220>
<221> source
<222> (1)..(22)
<223> /note=«Description of artificial sequence: Forward primer, vatD gene encoding Streptogramin A acetyl transferase»

<400> 41
cctatagaag gaaacaaatc ag 22

<210> 42
<211> 23
<212> DNA
<213> artificial

<220>
<221> source
<222> (1)..(23)
<223> /note=«Description of artificial sequence: Reverse primer, vatD gene encoding Streptogramin A acetyl transferase»

<400> 42
ccatatgact tctctaatga tgc 23

<210> 43
<211> 20
<212> DNA
<213> artificial

<220>
<221> source
<222> (1)..(20)
<223> /note=«Description of artificial sequence: Forward primer, vatE gene encoding Streptogramin A acetyl transferase»

<400> 43
gaaacacgtt acccatcact 20

<210> 44
<211> 20
<212> DNA
<213> artificial

<220>
<221> source
<222> (1)..(20)
<223> /note=«Description of artificial sequence: Reverse primer, vatE gene encoding Streptogramin A acetyl transferase»

<400> 44
tatctttatt ccaccatgcc 20

<210> 45
<211> 20
<212> DNA
<213> artificial

<220>
<221> source
<222> (1)..(20)
<223> /note=«Description of artificial sequence: Forward primer, vgb gene encoding Streptogramine B lyase»

<400> 45
gcaccctacg gtattacaga 20

<210> 46
<211> 20
<212> DNA
<213> artificial

<220>
<221> source
<222> (1)..(20)
<223> /note=«Description of artificial sequence: Reverse primer, vgb gene encoding Streptogramine B lyase»

<400> 46
agtaattgca tgaggtcgag 20

<210> 47
<211> 20
<212> DNA
<213> artificial

<220>
<221> source
<222> (1)..(20)
<223> /note=«Description of artificial sequence: Forward primer, vgbB gene encoding Streptogramine B lyase»

<400> 47
gtgcaaataa aatcggaaag 20

<210> 48
<211> 20
<212> DNA
<213> artificial

<220>
<221> source
<222> (1)..(20)
<223> /note=«Description of artificial sequence: Reverse primer, vgbB gene encoding Streptogramine B lyase»

<400> 48
acctattttg ttgcccataa 20

<210> 49
<211> 20
<212> DNA
<213> artificial

<220>
<221> source
<222> (1)..(20)
<223> /note=«Description of artificial sequence: Forward primer, tetL gene encoding Tetracycline resistance»

<400> 49
gaacagtagc agggtttgtc 20

<210> 50
<211> 20
<212> DNA
<213> artificial

<220>
<221> source
<222> (1)..(20)
<223> /note=«Description of artificial sequence: Reverse primer, tetL gene encoding Tetracycline resistance»

<400> 50
tggatcaagt aagggtatgg 20

<210> 51
<211> 20
<212> DNA
<213> artificial

<220>
<221> source
<222> (1)..(20)
<223> /note=«Description of artificial sequence: Forward primer, cat gene encoding Chloramphenicol resistance»

<400> 51
ttacaatagc gacggagagt 20

<210> 52
<211> 20
<212> DNA
<213> artificial

<220>
<221> source
<222> (1)..(20)
<223> /note=«Description of artificial sequence: Reverse primer, cat gene encoding Chloramphenicol resistance»

<400> 52
aacaatcctg catgataacc 20

<210> 53
<211> 21
<212> DNA
<213> artificial

<220>
<221> source
<222> (1)..(21)
<223> /note=«Description of artificial sequence: Forward primer, far1 gene encoding Fucidic acid resistance»

<400> 53
tcaaagttat tcaatcggaa a 21

<210> 54
<211> 20
<212> DNA
<213> artificial

<220>
<221> source
<222> (1)..(20)
<223> /note=«Description of artificial sequence: Reverse primer, far1 gene encoding Fucidic acid resistance»

<400> 54
cgaattggtt tttgactttt 20

<210> 55
<211> 20
<212> DNA
<213> artificial

<220>
<221> source
<222> (1)..(20)

<223> /note=«Description of artificial sequence: Forward primer, mupA gene encoding Mupirocin resistance»

<400> 55
cttctgtggg accaaaacta 20

<210> 56
<211> 20
<212> DNA
<213> artificial

<220>
<221> source
<222> (1)..(20)
<223> /note=«Description of artificial sequence: Reverse primer, mupA gene encoding Mupirocin resistance»

<400> 56
aataattccc cagttacacc 20

<210> 57
<211> 20
<212> DNA
<213> artificial

<220>
<221> source
<222> (1)..(20)
<223> /note=«Description of artificial sequence: Forward primer, dfrA gene encoding Trimethoprime resistance»

<400> 57
aattgtcgct cacgataaac 20

<210> 58
<211> 20
<212> DNA
<213> artificial

<220>
<221> source
<222> (1)..(20)
<223> /note=«Description of artificial sequence: Reverse primer, dfrA gene encoding Trimethoprime resistance»

<400> 58
gaagattcga cttcccagtt 20

<210> 59
<211> 24
<212> DNA
<213> artificial

<220>
<221> source
<222> (1)..(24)
<223> /note=«Description of artificial sequence: Forward primer, fos gene encoding Fosfomycin resistance»

<400> 59
aaatcatatt acttattcgg tttc 24

<210> 60
<211> 23
<212> DNA
<213> artificial

<220>
<221> source
<222> (1)..(23)
<223> /note=«Description of artificial sequence: Reverse primer, fos gene encoding Fosfomycin resistance»

<400> 60
aaacttcctg tatgcaattc taa 23

<210> 61
<211> 20
<212> DNA
<213> artificial

<220>
<221> source
<222> (1)..(20)
<223> /note=«Description of artificial sequence: Forward primer, vanA gene responsible for Resistance to vancomycin»

<400> 61
aatcggcaag acaatatgac 20

<210> 62
<211> 20
<212> DNA
<213> artificial

<220>
<221> source
<222> (1)..(20)
<223> /note=«Description of artificial sequence: Reverse primer, vanA gene responsible for Resistance to vancomycin»

<400> 62
tacggggata acgactgtat 20

<210> 63
<211> 20
<212> DNA
<213> artificial

<220>
<221> source
<222> (1)..(20)
<223> /note=«Description of artificial sequence: Forward primer, vanB gene responsible of Resistance to vancomycin»

<400> 63
aaaagtcgca attatcttcg 20

<210> 64
<211> 20
<212> DNA
<213> artificial

<220>
<221> source
<222> (1)..(20)
<223> /note=«Description of artificial sequence: Reverse primer, vanB gene responsible of Resistance to vancomycin»

<400> 64
aatgtaggcc agtgatttgt 20

<210> 65
<211> 20
<212> DNA
<213> artificial

<220>
<221> source
<222> (1)..(20)
<223> /note=«Description of artificial sequence: Forward primer, vanC gene responsible of Resistance to vancomycin»

<400> 65
acccgctgaa atatgaagta 20

<210> 66
<211> 20
<212> DNA
<213> artificial

<220>
<221> source
<222> (1)..(20)
<223> /note=«Description of artificial sequence: Reverse primer, vanC gene responsible of Resistance to vancomycin»

<400> 66
ggaatccatg gtcttgaata 20

<210> 67
<211> 20
<212> DNA
<213> artificial

<220>
<221> source
<222> (1)..(20)
<223> /note=«Description of artificial sequence: Forward primer, sat4 gene encoding Streptothricine-acetyl-transferase»

<400> 67
gaaccatttg aggtgatagg 20

<210> 68

<211> 20
<212> DNA
<213> artificial

<220>
<221> source
<222> (1)..(20)
<223> /note=«Description of artificial sequence: Reverse primer, sat4 gene encoding Streptothricine-acetyl-transferase»

<400> 68
agcattagtc catgcaagtt 20

<210> 69
<211> 20
<212> DNA
<213> artificial

<220>
<221> source
<222> (1)..(20)
<223> /note=«Description of artificial sequence: Forward primer, qacA gene reponsible of Antiseptic resistance»

<400> 69
ttatggcttt accggaatta 20

<210> 70
<211> 20
<212> DNA
<213> artificial

<220>
<221> source
<222> (1)..(20)
<223> /note=«Description of artificial sequence: Reverse primer, qacA gene reponsible of Antiseptic resistance»

<400> 70
caagtaaagc tcctccgata 20

<210> 71
<211> 21
<212> DNA
<213> artificial

<220>
<221> source
<222> (1)..(21)
<223> /note=«Description of artificial sequence: Forward primer, smr(qacC)* gene responsible of Antiseptic resistance»

<400> 71
ctgaagttat tggaagtgca t 21

<210> 72
<211> 20

<212> DNA
<213> artificial

<220>
<221> source
<222> (1)..(20)
<223> /note=«Description of artificial sequence: Reverse primer, smr(qacC)* gene responsible of Antiseptic resistance»

<400> 72
gttccgaaaa tgtttaacga 20

<210> 73
<211> 20
<212> DNA
<213> artificial

<220>
<221> source
<222> (1)..(20)
<223> /note=«Description of artificial sequence: Forward primer, CZ040 gene encoding SSCmecIII Organomercurial lyase»

<400> 73
tatttcagaa ttctcagccc 20

<210> 74
<211> 20
<212> DNA
<213> artificial

<220>
<221> source
<222> (1)..(20)
<223> /note=«Description of artificial sequence: Reverse primer, CZ040 gene encoding SSCmecIII Organomercurial lyase»

<400> 74
tggtgtaaca atcgagacaa 20

<210> 75
<211> 20
<212> DNA
<213> artificial

<220>
<221> source
<222> (1)..(20)
<223> /note=«Description of artificial sequence: Forward primer, CZ041 gene encoding SSCmecIII Mercuric reductase»

<400> 75
agtgtagatt tcaatcggga 20

<210> 76
<211> 20
<212> DNA

<213> artificial

<220>
<221> source
<222> (1)..(20)
<223> /note=«Description of artificial sequence: Reverse primer, CZ041 gene encoding SSCmecIII Mercuric reductase»

<400> 76
tagacgtgat gttttgtcca 20

<210> 77
<211> 22
<212> DNA
<213> artificial

<220>
<221> source
<222> (1)..(22)
<223> /note=«Description of artificial sequence: Forward primer, entD gene encoding Enterotoxin D»

<400> 77
ttcttatgca gataaaaatc ca 22

<210> 78
<211> 20
<212> DNA
<213> artificial

<220>
<221> source
<222> (1)..(20)
<223> /note=«Description of artificial sequence: Reverse primer, EntD gene encoding Enterotoxin D»

<400> 78
tttttcctcc gagagtatca 20

<210> 79
<211> 20
<212> DNA
<213> artificial

<220>
<221> source
<222> (1)..(20)
<223> /note=«Description of artificial sequence: Forward primer, sej gene encoding Enterotoxin J»

<400> 79
cgaaaagggt atctctgaaa 20

<210> 80
<211> 20
<212> DNA
<213> artificial

<220>
<221> source
<222> (1)..(20)
<223> /note=«Description of artificial sequence: Reverse primer, sej gene encoding Enterotoxin J»

<400> 80
aacggttctt ttgaggtatg 20

<210> 81
<211> 21
<212> DNA
<213> artificial

<220>
<221> source
<222> (1)..(21)
<223> /note=«Description of artificial sequence: Forward primer, eta gene encoding Exfoliative toxin A»

<400> 81
aaaacatgaa gagaaatgga a 21

<210> 82
<211> 20
<212> DNA
<213> artificial

<220>
<221> source
<222> (1)..(20)
<223> /note=«Description of artificial sequence: Reverse primer, Eta gene encoding Exfoliative toxin A»

<400> 82
ttttgctggc gatattttat 20

<210> 83
<211> 20
<212> DNA
<213> artificial

<220>
<221> source
<222> (1)..(20)
<223> /note=«Description of artificial sequence: Forward primer, etd gene encoding Exfoliative toxin D»

<400> 83
atatttctct ccccgttgat 20

<210> 84
<211> 20
<212> DNA
<213> artificial

<220>
<221> source
<222> (1)..(20)
<223> /note=«Description of artificial sequence: Reverse primer, Etd gene encoding Exfoliative toxin D»

<400> 84
tccgtcttta ttgggtttta 20

<210> 85
<211> 20
<212> DNA
<213> artificial

<220>
<221> source
<222> (1)..(20)
<223> /note=«Description of artificial sequence: Forward primer, edinB gene encoding Epidermall cellular diferenciation inhibitor B»

<400> 85
tagctgccga gactaaaaat 20

<210> 86
<211> 20
<212> DNA
<213> artificial

<220>
<221> source
<222> (1)..(20)
<223> /note=«Description of artificial sequence: Reverse primer, edinB gene encoding Epidermall cellular diferenciation inhibitor B»

<400> 86
taattcaatt ggcctacctg 20

<210> 87
<211> 20
<212> DNA
<213> artificial

<220>
<221> source
<222> (1)..(20)
<223> /note=«Description of artificial sequence: Forward primer, sdrG gene encoding fibrinogen-binding Serine-Aspartate protein»

<400> 87
ccaaagctga ggagaataca 20

<210> 88
<211> 20
<212> DNA

<213> artificial

<220>
<221> source
<222> (1)..(20)
<223> /note=«Description of artificial sequence: Reverse primer, sdrG gene encoding fibrinogen-binding Serine-Aspartate protein»

<400> 88
cgggaatttt cttcattatc 20

<210> 89
<211> 20
<212> DNA
<213> artificial

<220>
<221> source
<222> (1)..(20)
<223> /note=«Description of artificial sequence: Forward primer, sdrH* gene encoding Serine-Aspartate protein»

<400> 89
cattcattta tgcttacggg 20

<210> 90
<211> 20
<212> DNA
<213> artificial

<220>
<221> source

<222> (1)..(20)
<223> /note=«Description of artificial sequence: Reverse primer, sdrH* gene encoding Serine-Aspartate protein»

<400> 90
ttcatactaa ttcctgtgcg 20

<210> 91
<211> 20
<212> DNA
<213> artificial

<220>
<221> source
<222> (1)..(20)
<223> /note=«Description of artificial sequence: Forward primer, sdrY gene encoding Serine-Aspartate protein»

<400> 91
aaacaaccat aacgtccaac 20

<210> 92
<211> 20

<212> DNA
<213> artificial

<220>
<221> source
<222> (1)..(20)
<223> /note=«Description of artificial sequence: Reverse primer, sdrY gene encoding Serine-Aspartate protein»

<400> 92
gcgatgtttg taactgtgaa 20

<210> 93
<211> 20
<212> DNA
<213> artificial

<220>
<221> source
<222> (1)..(20)
<223> /note=«Description of artificial sequence: Forward primer, sdrZ* gene encoding Serine-Aspartate protein»

<400> 93
ttgtattaac aggctttgcc 20

<210> 94
<211> 20
<212> DNA
<213> artificial

<220>
<221> source
<222> (1)..(20)
<223> /note=«Description of artificial sequence: Reverse primer, sdrZ* gene encoding Serine-Aspartate protein»

<400> 94
acctcactcg ttgtatttgc 20

<210> 95
<211> 21
<212> DNA
<213> artificial

<220>
<221> source
<222> (1)..(21)
<223> /note=«Description of artificial sequence: Forward primer, bap gene encoding biofilm associated surface protein»

<400> 95
ggcaatactt ctgaaagtac c 21

<210> 96
<211> 24
<212> DNA

<213> artificial

<220>
<221> source
<222> (1)..(24)
<223> /note=«Description of artificial sequence: Reverse primer, bap gene encoding biofilm associated surface protein»

<400> 96
ttctacgacg tttggtaaat aata 24

<210> 97
<211> 22
<212> DNA
<213> artificial

<220>
<221> source
<222> (1)..(22)
<223> /note=«Description of artificial sequence: Forward primer, cap1G gene responsible of Capsular polysaccharide synthesis Cap1G»

<400> 97
gataagataa ggaaattggg tg 22

<210> 98
<211> 20
<212> DNA
<213> artificial

<220>
<221> source
<222> (1)..(20)
<223> /note=«Description of artificial sequence: Reverse primer, cap1G gene responsible of Capsular polysaccharide synthesis Cap1G»

<400> 98
tgtgtaatcc gcattttcta 20

<210> 99
<211> 20
<212> DNA
<213> artificial

<220>
<221> source
<222> (1)..(20)
<223> /note=«Description of artificial sequence: Forward primer, tnpA (Tn5406) * gene encoding Tn 5406A transposase»

<400> 99
aaatcgtaga agcctgtcac 20

<210> 100
<211> 20

<212> DNA
<213> artificial

<220>
<221> source
<222> (1)..(20)
<223> /note=«Description of artificial sequence: Reverse primer, tnpA (Tn5406) * gene encoding Tn 5406A transposase»

<400> 100
cctgttcttt tattcaaccg 20

<210> 101
<211> 20
<212> DNA
<213> artificial

<220>
<221> source
<222> (1)..(20)
<223> /note=«Description of artificial sequence: Forward primer, tnpB (Tn5406) * gene encoding Tn 5406B transposase»

<400> 101
ttagaaggat attgggctga 20

<210> 102
<211> 20
<212> DNA
<213> artificial

<220>
<221> source
<222> (1)..(20)
<223> /note-«Description of artificial sequence: Reverse primer, tnpB (Tn5406) * gene encoding Tn 5406B transposase»

<400> 102
ttctttcttg tttggcattt 20

<210> 103
<211> 20
<212> DNA
<213> artificial

<220>
<221> source
<222> (1)..(20)
<223> /note=«Description of artificial sequence: Forward primer, tnp IS 256 gene ecoding IS 256 transposase»

<400> 103
gaaaagcgaa gagattcaaa 20

<210> 104
<211> 20
<212> DNA

<213> artificial

<220>
<221> source
<222> (1)..(20)
<223> /note=«Description of artificial sequence: Reverse primer, tnp IS 256 gene ecoding IS 256 transposase»

<400> 104
agagacggat ttaccacaaa 20

<210> 105
<211> 20
<212> DNA
<213> artificial

<220>
<221> source
<222> (1)..(20)
<223> /note=«Description of artificial sequence: Forward primer, tnp IS 1182 gene encoding IS 1182 transposase»

<400> 105
tgtcccaatc agaaaagagt 20

<210> 106
<211> 20
<212> DNA
<213> artificial

<220>
<221> source
<222> (1)..(20)
<223> /note=«Description of artificial sequence: Reverse primer, tnp IS 1182 gene encoding IS 1182 transposase»

<400> 106
aaattgtgcg caagaaatac 20

<210> 107
<211> 20
<212> DNA
<213> artificial

<220>
<221> source
<222> (1)..(20)
<223> /note=«Description of artificial sequence: Forward primer, orfX gene. Encoded protein Tn5405»

<400> 107
ccatacagag gattcggata 20

<210> 108
<211> 20
<212> DNA
<213> artificial

<220>
<221> source
<222> (1)..(20)
<223> /note=«Description of artificial sequence: Reverse primer, orfX gene. Encoded protein Tn5405»

<400> 108
aaccctgctt caaacataaa 20

<210> 109
<211> 20
<212> DNA
<213> artificial

<220>
<221> source
<222> (1)..(20)
<223> /note=«Description of artificial sequence: Forward primer, orfZ gene. Encoded protein Tn5405»

<400> 109
gctttgataa tccggaagtt 20

<210> 110
<211> 20
<212> DNA
<213> artificial

<220>
<221> source
<222> (1)..(20)
<223> /note=«Description of artificial sequence: Reverse primer, orfZ gene. Encoded protein Tn5405»

<400> 110
tgtctgtccg catgtattta 20

<210> 111
<211> 20
<212> DNA
<213> artificial

<220>
<221> source
<222> (1)..(20)
<223> /note=«Description of artificial sequence: Forward primer, hsdS- TY114 gen encoding Restriction modification enzyme subunit HsdS»

<400> 111
aacaaggttt gattcgaaaa 20

<210> 112
<211> 20
<212> DNA
<213> artificial

<220>
<221> source
<222> (1)..(20)
<223> /note=«Description of artificial sequence: Reverse primer,
hsdS- TY114 gen encoding Restriction modification enzyme
subunit HsdS»

<400> 112
acatcgattg aagtaagcct 20

<210> 113
<211> 21
<212> DNA
<213> artificial

<220>
<221> source1
<222> (1)..(20)
<223> /note=«Description of artificial sequence: Forward primer,
Frep pC194* gene encoding pC194 Rep protein»

<400> 113
ttgattctct ccaatatgac g 21

<210> 114
<211> 20
<212> DNA
<213> artificial

<220>
<221> source
<222> (1)..(20)
<223> /note=«Description of artificial sequence: Reverse primer,
Frep pC194* gene encoding pC194 Rep protein»

<400> 114
ttaaaggatt tgagcgtagc 20

<210> 115
<211> 23
<212> DNA
<213> artificial

<220>
<221> source
<222> (1)..(23)
<223> /note=«Description of artificial sequence: Forward primer,
rep1pIP1629* gene encoding pIP1629 replication protein»

<400> 115
aacttagaga aatgacacac ctt 23

<210> 116
<211> 19
<212> DNA
<213> artificial

<220>
<221> source
<222> (1)..(19)
<223> /note=«Description of artificial sequence: Reverse primer, replpIP1629* gene encoding pIP1629 replication protein»

<400> 116
ccaaccattt tggtgtttt 19

<210> 117
<211> 20
<212> DNA
<213> artificial

<220>
<221> source
<222> (1)..(20)
<223> /note=«Description of artificial sequence: Forward primer, traA ( pSK1) gene ecoding Transfert protein from pSK1 plasmid»

<400> 117
aataagttag tcatggccga 20

<210> 118
<211> 20
<212> DNA
<213> artificial

<220>
<221> source
<222> (1)..(20)
<223> /note=«Description of artificial sequence: Reverse primer, traA ( pSK1) gene ecoding Transfert protein from pSK1 plasmid»

<400> 118
atgctgttgc tgtaagtcct 20

<210> 119
<211> 20
<212> DNA
<213> artificial

<220>
<221> source
<222> (1)..(20)
<223> /note=«Description of artificial sequence: Forward primer, traC ( pSK1) gene encodingTransfert protein from pSK1 plasmid»

<400> 119
tgtctgttga gcaagaagtt 20

<210> 120
<211> 20

<212> DNA
<213> artificial

<220>
<221> source
<222> (1)..(20)
<223> /note=«Description of artificial sequence: Reverse primer, traC ( pSK1) gene encodingTransfert protein from pSK1 plasmid»

<400> 120
aaacatcatc atcacccaat 20

<210> 121
<211> 20
<212> DNA
<213> artificial

<220>
<221> pimer
<222> (1)..(20)
<223> /note=«Description of artificial sequence: Forward primer, traD ( pSK1) gene encoding Transfert protein from pSK1 plasmid»

<400> 121
gatatataaa aggggacgga 20

<210> 122
<211> 20
<212> DNA
<213> artificial

<220>
<221> source
<222> (1)..(20)
<223> /note=«Description of artificial sequence: Reverse primer, traD ( pSK1) gene encoding Transfert protein from pSK1 plasmid»

<400> 122
ccttttgcta gcttttcttg 20

<210> 123
<211> 20
<212> DNA
<213> artificial

<220>
<221> source
<222> (1)..(20)
<223> /note=«Description of artificial sequence: Forward primer, traE ( pSK1) encoding Transfert protein from pSK1 plasmid»

<400> 123
cagtttctta tgggacgaaa 20

<210> 124
<211> 20
<212> DNA
<213> artificial

<220>
<221> source
<222> (1)..(20)
<223> /note=«Description of artificial sequence: Reverse primer, traE ( pSK1) encoding Transfert protein from pSK1 plasmid»

<400> 124
atgctctttg tgttccgtat 20

<210> 125
<211> 20
<212> DNA
<213> artificial

<220>
<221> source
<222> (1)..(20)
<223> /note=«Description of artificial sequence: Forward primer, traF ( pSK1) gene encoding Transfert protein from pSK1 plasmid»

<400> 125
ggaacattgg taggaaaaca 20

<210> 126
<211> 20
<212> DNA
<213> artificial

<220>
<221> source
<222> (1)..(20)
<223> /note=«Description of artificial sequence: Reverse primer, traF ( pSK1) gene encoding Transfert protein from pSK1 plasmid»

<400> 126
ggtaaatatt catggccttt 20

<210> 127
<211> 20
<212> DNA
<213> artificial

<220>
<221> source
<222> (1)..(20)
<223> /note=«Description of artificial sequence: Forward primer, traG ( pSK1) gene encodingTransfert protein from pSK1

plasmid
«

<400> 127
aatgaaagag gctgaatcaa 20

<210> 128
<211> 20
<212> DNA
<213> artificial

<220>
<221> source
<222> (1)..(20)
<223> /note=«Description of artificial sequence: Reverse primer, traG ( pSK1) gene encodingTransfert protein from pSK1 plasmid»

<400> 128
cattactagc gcctactggt 20

<210> 129
<211> 20
<212> DNA
<213> artificial

<220>
<221> source
<222> (1)..(20)
<223> /note=«Description of artificial sequence: Forward primer, traH ( pSK1) gene encoding Transfert protein from pSK1 plasmid»

<400> 129
tattcacttt ggttctggct 20

<210> 130
<211> 20
<212> DNA
<213> artificial

<220>
<221> source
<222> (1)..(20)
<223> /note=«Description of artificial sequence: Reverse primer, traH ( pSK1) gene encoding Transfert protein from pSK1 plasmid»

<400> 130
cgttaattca attttcccat 20

<210> 131
<211> 20
<212> DNA
<213> artificial

<220>

<221> source
<222> (1)..(20)
<223> /note=«Description of artificial sequence: Forward primer, traI ( pSK1) gene encoding Transfert protein from pSK1 plasmid»

<400> 131
cttttaact tgggcagttg 20

<210> 132
<211> 19
<212> DNA
<213> artificial

<220>
<221> source
<222> (1)..(19)
<223> /note=«Description of artificial sequence: Reverse primer, traI ( pSK1) gene encoding Transfert protein from pSK1 plasmid»

<400> 132
tgccctcttt gtaaaatcc 19

<210> 133
<211> 24
<212> DNA
<213> artificial

<220>
<221> source
<222> (1)..(24)
<223> /note=«Description of artificial sequence: Forward primer, traJ ( pSK1) gene encoding Transfert protein from pSK1 plasmid»

<400> 133
tttaactggt attatttggc tagt 24

<210> 134
<211> 20
<212> DNA
<213> artificial

<220>
<221> source
<222> (1)..(20)
<223> /note=«Description of artificial sequence: Reverse primer, traJ ( pSK1) gene encoding Transfert protein from pSK1 plasmid»

<400> 134
cttctgaaaa caattcgcta 20

<210> 135
<211> 20
<212> DNA

<213> artificial

<220>
<221> source
<222> (1)..(20)
<223> /note=«Description of artificial sequence: Forward primer, traK ( pSK1) gene encoding Transfert protein from pSK1 plasmid»

<400> 135
agtggcgaag tttatgaaaa 20

<210> 136
<211> 20
<212> DNA
<213> artificial

<220>
<221> source
<222> (1)..(20)
<223> /note=«Description of artificial sequence: Reverse primer, traK ( pSK1) gene encoding Transfert protein from pSK1 plasmid»

<400> 136
ctctagctgg gtggtcttta 20

<210> 137
<211> 20
<212> DNA
<213> artificial

<220>
<221> source
<222> (1)..(20)
<223> /note=«Description of artificial sequence: Forward primer, traL ( pSK1) gene encoding Transfert protein from pSK1 plasmid»

<400> 137
aggttctatg gggactatgg 20

<210> 138
<211> 20
<212> DNA
<213> artificial

<220>
<221> source
<222> (1)..(20)
<223> /note=«Description of artificial sequence: Reverse primer, traL ( pSK1) gene encoding Transfert protein from pSK1 plasmid»

<400> 138
tgatctaata aaggaaggcg 20

<210> 139
<211> 20
<212> DNA
<213> artificial

<220>
<221> source
<222> (1)..(20)
<223> /note=«Description of artificial sequence: Forward primer, traM ( pSK1) gene encoding Transfert protein from pSK1 plasmid»

<400> 139
tatagtggac gtatcgcaac 20

<210> 140
<211> 20
<212> DNA
<213> artificial

<220>
<221> source
<222> (1)..(20)
<223> /note=«Description of artificial sequence: Reverse primer, traM ( pSK1) gene encoding Transfert protein from pSK1 plasmid»

<400> 140
actatcttct gctgggctta 20

<210> 141
<211> 20
<212> DNA
<213> artificial

<220>
<221> source
<222> (1)..(20)
<223> /note=«Description of artificial sequence: Forward primer, fmtB(mrp) fmtB gene encoding : Beta-lactam resistance regulator»

<400> 141
atgccaatac acagcctaac 20

<210> 142
<211> 20
<212> DNA
<213> artificial

<220>
<221> source
<222> (1)..(20)
<223> /note=«Description of artificial sequence: Reverse primer, fmtB(mrp) fmtB gene encoding : Beta-lactam resistance regulator»

<400> 142

atcgcttcgt cttaattctg 20

<210> 143
<211> 20
<212> DNA
<213> artificial

<220>
<221> source
<222> (1)..(20)
<223> /note=«Description of artificial sequence: Forward primer, fmtC(mrpF) FmtC gene encoding : Beta-lactam resistance regulator»

<400> 143
ggcatcgctt gttattctat 20

<210> 144
<211> 20
<212> DNA
<213> artificial

<220>
<221> source
<222> (1)..(20)
<223> /note=«Description of artificial sequence: Reverse primer, fmtC(mrpF) FmtC gene encoding : Beta-lactam resistance regulator»

<400> 144
ccattcaaca cacgacacta 20

<210> 145
<211> 20
<212> DNA
<213> artificial

<220>
<221> source
<222> (1)..(20)
<223> /note=«Description of artificial sequence: Forward primer, dltA gene encoding D-alanine-D-alanyl carrier protein ligase»

<400> 145
gaaaccgatg attttattcg 20

<210> 146
<211> 20
<212> DNA
<213> artificial

<220>
<221> source
<222> (1)..(20)
<223> /note=«Description of artificial sequence: Reverse primer, dltA gene encoding D-alanine-D-alanyl carrier protein

ligase»

<400> 146
cttggttaag ccattgttgt 20

<210> 147
<211> 20
<212> DNA
<213> artificial

<220>
<221> source
<222> (1)..(20)
<223> /note=«Description of artificial sequence: Forward primer, femB gene encoding Beta-lactam resistance regulator»

<400> 147
cagcaagcct tttctctaaa 20

<210> 148
<211> 20
<212> DNA
<213> artificial

<220>
<221> source
<222> (1)..(20)
<223> /note=«Description of artificial sequence: Reverse primer, femB gene encoding Beta-lactam resistance regulator»

<400> 148
cgttcaagga atctgacttt 20

<210> 149
<211> 20
<212> DNA
<213> artificial

<220>
<221> source
<222> (1)..(20)
<223> /note=«Description of artificial sequence: Forward primer, femA gene encoding Beta-lactam resistance regulator»

<400> 149
acagctaaag agtttggtgc 20

<210> 150
<211> 20
<212> DNA
<213> artificial

<220>
<221> source
<222> (1)..(20)
<223> /note=«Description of artificial sequence: Reverse primer, femA gene encoding Beta-lactam resistance regulator»

<400> 150
acgaatttgt agcacaggat 20

<210> 151
<211> 20
<212> DNA
<213> artificial

<220>
<221> source
<222> (1)..(20)
<223> /note=«Description of artificial sequence: Forward primer, set6* gene encoding Exotoxin 6»

<400> 151
ttggggctaa aaattatgaa 20

<210> 152
<211> 20
<212> DNA
<213> artificial

<220>
<221> source
<222> (1)..(20)
<223> /note=«Description of artificial sequence: Reverse primer, set6* gene encoding Exotoxin 6»

<400> 152
ttcaaggatt ggtgtgtgta 20

<210> 153
<211> 20
<212> DNA
<213> artificial

<220>
<221> source
<222> (1)..(20)
<223> /note=«Description of artificial sequence: Forward primer, set7 gene encoding Exotoxin 7»

<400> 153
aattggcaaa gataagcaac 20

<210> 154
<211> 20
<212> DNA
<213> artificial

<220>
<221> source
<222> (1)..(20)
<223> /note=«Description of artificial sequence: Reverse primer, set7 gene encoding Exotoxin 7»

<400> 154
tgtgacattg ataacatcgg 20

<210> 155
<211> 20
<212> DNA
<213> artificial

<220>
<221> source
<222> (1)..(20)
<223> /note=«Description of artificial sequence: Forward primer, set13* gene encoding Exotoxin 13»

<400> 155
tgaatcaaca aacattagcg 20

<210> 156
<211> 20
<212> DNA
<213> artificial

<220>
<221> source
<222> (1)..(20)
<223> /note=«Description of artificial sequence: Reverse primer, set13* gene encoding Exotoxin 13»

<400> 156
tatctgacgc gcctttatac 20

<210> 157
<211> 20
<212> DNA
<213> artificial

<220>
<221> source
<222> (1)..(20)
<223> /note=«Description of artificial sequence: Forward primer, set14 gene encoding Exotoxin 14»

<400> 157
aggtcatgca aaacaaaatc 20

<210> 158
<211> 20
<212> DNA
<213> artificial

<220>
<221> source
<222> (1)..(20)
<223> /note=«Description of artificial sequence: Reverse primer, set14 gene encoding Exotoxin 14»

<400> 158

tttgacccta ccatcttttg 20

<210> 159
<211> 20
<212> DNA
<213> artificial

<220>
<221> source
<222> (1)..(20)
<223> /note=«Description of artificial sequence: Forward primer, hlb gene encoding Beta-hemolysin»

<400> 159
ttatcgaccg ttttgtatcc 20

<210> 160
<211> 20
<212> DNA
<213> artificial

<220>
<221> source
<222> (1)..(20)
<223> /note=«Description of artificial sequence: Reverse primer, hlb gene encoding Beta-hemolysin»

<400> 160
aatttttcga tcatgtccag 20

<210> 161
<211> 20
<212> DNA
<213> artificial

<220>
<221> source
<222> (1)..(20)
<223> /note=«Description of artificial sequence: Forward primer, hla gene encoding Alpha-hemolysin»

<400> 161
aatgaatcct gtcgctaatg 20

<210> 162
<211> 20
<212> DNA
<213> artificial

<220>
<221> source
<222> (1)..(20)
<223> /note=«Description of artificial sequence: Reverse primer, hla gene encoding Alpha-hemolysin»

<400> 162
tttcagtgta tgaccaatcg 20

<210> 163
<211> 20
<212> DNA
<213> artificial

<220>
<221> source
<222> (1)..(20)
<223> /note=«Description of artificial sequence: Forward primer, hlgA gene encoding Gamma-hemolysin component A»

<400> 163
tgccctagtt gttaagatgc 20

<210> 164
<211> 20
<212> DNA
<213> artificial

<220>
<221> source
<222> (1)..(20)
<223> /note=«Description of artificial sequence: Reverse primer, hlgA gene encoding Gamma-hemolysin component A»

<400> 164
cgaaatagtc tcttgctgct 20

<210> 165
<211> 21
<212> DNA
<213> artificial

<220>
<221> source
<222> (1)..(21)
<223> /note=«Description of artificial sequence: Forward primer, hlgB gene encoding Gamma-hemolysin component B»

<400> 165
gctatacatt tggtggtgac a 21

<210> 166
<211> 19
<212> DNA
<213> artificial

<220>
<221> source
<222> (1)..(19)
<223> /note=«Description of artificial sequence: Reverse primer, hlgB gene encoding Gamma-hemolysin component B»

<400> 166
tagaagccat tccaacgaa 19

<210> 167
<211> 21
<212> DNA
<213> artificial

<220>
<221> source
<222> (1)..(21)
<223> /note=«Description of artificial sequence: Forward primer, hlgC gene encoding Gamma-hemolysin component C»

<400> 167

aatcatgtta aagctatgcg a 21

<210> 168
<211> 20
<212> DNA
<213> artificial

<220>
<221> source
<222> (1)..(20)
<223> /note=«Description of artificial sequence: Reverse primer, hlgC gene encoding Gamma-hemolysin component C»

<400> 168
catgagatac tgtggcgata 20

<210> 169
<211> 20
<212> DNA
<213> artificial

<220>
<221> source
<222> (1)..(20)
<223> /note=«Description of artificial sequence: Forward primer, SAV2170* gene encoding Putative hemolysin»

<400> 169
gagaagaaat tggtaatgcg 20

<210> 170
<211> 20
<212> DNA
<213> artificial

<220>
<221> source
<222> (1)..(20)
<223> /note=«Description of artificial sequence: Reverse primer, SAV2170* gene encoding Putative hemolysin»

<400> 170
agataatacc tacccagccc 20

<210> 171
<211> 20
<212> DNA
<213> artificial

<220>
<221> source
<222> (1)..(20)
<223> /note=«Description of artificial sequence: Forward primer, lukM(SA1813) gene encoding Putative leucocidine»

<400> 171
aatattagac caattgccga 20

<210> 172
<211> 20
<212> DNA
<213> artificial

<220>
<221> source
<222> (1)..(20)
<223> /note=«Description of artificial sequence: Reverse primer, lukM(SA1813) gene encoding Putative leucocidine»

<400> 172
ttctaaaatt ggaggtgcat 20

<210> 173
<211> 20
<212> DNA
<213> artificial

<220>
<221> source
<222> (1)..(20)
<223> /note=«Description of artificial sequence: Forward primer, hySA* gene encoding Hyaluronate lyase precursor»

<400> 173
atgttcaaac gccagattat 20

<210> 174
<211> 20
<212> DNA
<213> artificial

<220>
<221> source
<222> (1)..(20)
<223> /note=«Description of artificial sequence: Reverse primer, hySA* gene encoding Hyaluronate lyase precursor»

<400> 174
gttccaattt cataatccca 20

<210> 175
<211> 20
<212> DNA
<213> artificial

<220>
<221> source
<222> (1)..(20)
<223> /note=«Description of artificial sequence: Forward primer, aur(SA2430) gene encodingZinc metalloproteinase aureolysin»

<400> 175
caagtatggc agcagtaaca 20

<210> 176
<211> 20
<212> DNA
<213> artificial

<220>
<221> source
<222> (1)..(20)
<223> /note=«Description of artificial sequence: Reverse primer, aur(SA2430) gene encodingZinc metalloproteinase aureolysin»

<400> 176
tactgtcacc atcgatttca 20

<210> 177
<211> 18
<212> DNA
<213> artificial

<220>
<221> source
<222> (1)..(18)
<223> /note=«Description of artificial sequence: Forward primer, spa gene encoding Immunoglobulin G binding protein A precursor»

<400> 177
cgcaacacga tgaagctc 18

<210> 178
<211> 23
<212> DNA
<213> artificial

<220>
<221> source
<222> (1)..(23)
<223> /note=«Description of artificial sequence: Reverse primer, spa gene encoding Immunoglobulin G binding protein A precursor»

<400> 178

ttagcttctg acaataggtt agc 23

<210> 179
<211> 20
<212> DNA
<213> artificial

<220>
<221> source
<222> (1)..(20)
<223> /note=«Description of artificial sequence: Forward primer, putB ene encoding High affinity proline permease»

<400> 179
caactggtaa cctaagcgag 20

<210> 180
<211> 20
<212> DNA
<213> artificial

<220>
<221> source
<222> (1)..(20)
<223> /note=«Description of artificial sequence: Reverse primer, putB ene encoding High affinity proline permease»

<400> 180
ttaccaccag atacgaaacc 20

<210> 181
<211> 20
<212> DNA
<213> artificial

<220>
<221> source
<222> (1)..(20)
<223> /note=«Description of artificial sequence: Forward primer, sspA* gene encoding Staphylococcal serine protease V8»

<400> 181
aaaaggcggt aaccttaaac 20

<210> 182
<211> 20
<212> DNA
<213> artificial

<220>
<221> source
<222> (1)..(20)
<223> /note=«Description of artificial sequence: Reverse primer, sspA* gene encoding Staphylococcal serine protease V8»

<400> 182
gtttgttttg ctcattaggg 20

<210> 183
<211> 20
<212> DNA
<213> artificial

<220>
<221> source
<222> (1)..(20)
<223> /note=«Description of artificial sequence: Forward primer, splC* gene encoding Serine protease SplC»

<400> 183
cagcattagc cattctaacc 20

<210> 184
<211> 20
<212> DNA
<213> artificial

<220>
<221> source
<222> (1)..(20)
<223> /note=«Description of artificial sequence: Reverse primer, splC* gene encoding Serine protease SplC»

<400> 184
tattttgagc aggtaatggg 20

<210> 185
<211> 21
<212> DNA
<213> artificial

<220>
<221> source
<222> (1)..(21)
<223> /note=«Description of artificial sequence: Forward primer, splD = splF ene encoding Serine protease SplD, splF»

<400> 185
attaattacc aacacgaatg t 21

<210> 186
<211> 21
<212> DNA
<213> artificial

<220>
<221> source
<222> (1)..(21)
<223> /note=«Description of artificial sequence: Reverse primer, splD = splF ene encoding Serine protease SplD, splF»

<400> 186
acataacacc aattgcttct c 21

<210> 187
<211> 20
<212> DNA
<213> artificial

<220>
<221> source
<222> (1)..(20)
<223> /note=«Description of artificial sequence: Forward primer, htrA gene encoding Serine protease HtrA»

<400> 187
gaacagagaa accgtgatgt 20

<210> 188
<211> 20
<212> DNA
<213> artificial

<220>
<221> source
<222> (1)..(20)
<223> /note=«Description of artificial sequence: Reverse primer, htrA gene encoding Serine protease HtrA»

<400> 188
tcatcagatt ccgacaattt 20

<210> 189
<211> 20
<212> DNA
<213> artificial

<220>
<221> source
<222> (1)..(20)
<223> /note=«Description of artificial sequence: Forward primer, clpB gene encoding ClpB chaperone protein»

<400> 189
atattggaca tgatgaaggg 20

<210> 190
<211> 20
<212> DNA
<213> artificial

<220>
<221> source
<222> (1)..(20)
<223> /note=«Description of artificial sequence: Reverse primer, clpB gene encoding ClpB chaperone protein»

<400> 190
ccgtattgag gttcataagc 20

```
<210> 191
<211> 20
<212> DNA
<213> artificial

<220>
<221> source
<222> (1)..(20)
<223> /note=«Description of artificial sequence: Forward primer,
      clpL* gene encoding ATP-dependent Clp proteinase chain
      clpL»

<400> 191
gaagaactag cgcaactcac                                              20


<210> 192
<211> 20
<212> DNA
<213> artificial

<220>
<221> source
<222> (1)..(20)
<223> /note=«Description of artificial sequence: Reverse primer,
      clpL* gene encoding ATP-dependent Clp proteinase chain
      clpL»

<400> 192
ccgataattt gatggatttc                                              20


<210> 193
<211> 20
<212> DNA
<213> artificial

<220>
<221> source
<222> (1)..(20)
<223> /note=«Description of artificial sequence: Forward primer,
      SA1725 gene encoding Cysteine protease : staphylopain»

<400> 193
tgtaacgcaa ctaaagcaaa                                              20


<210> 194
<211> 20
<212> DNA
<213> artificial

<220>
<221> source
<222> (1)..(20)
<223> /note=«Description of artificial sequence: Reverse primer,
      SA1725 gene encoding Cysteine protease : staphylopain»

<400> 194
tctaatttag cgttcccaac                                              20
```

<210> 195
<211> 20
<212> DNA
<213> artificial

<220>
<221> source
<222> (1)..(20)
<223> /note=«Description of artificial sequence: Forward primer, sspB*(SA0900) gene encoding Cysteine protease»

<400> 195
aagccgattc acactctaaa 20

<210> 196
<211> 20
<212> DNA
<213> artificial

<220>
<221> source
<222> (1)..(20)
<223> /note=«Description of artificial sequence: Reverse primer, sspB*(SA0900) gene encoding Cysteine protease»

<400> 196
tttgccatct tcttcaaaat 20

<210> 197
<211> 20
<212> DNA
<213> artificial

<220>
<221> source
<222> (1)..(20)
<223> /note=«Description of artificial sequence: Forward primer, srtA gene encoding Sortase»

<400> 197
aatcgattaa tgacaatcgc 20

<210> 198
<211> 20
<212> DNA
<213> artificial

<220>
<221> source
<222> (1)..(20)

<223> /note=«Description of artificial sequence: Reverse primer, srtA gene encoding Sortase»

<400> 198
ttggctgctt taagatttgt 20

<210> 199
<211> 20
<212> DNA
<213> artificial

<220>
<221> source
<222> (1)..(20)
<223> /note=«Description of artificial sequence: Forward primer, vwb* gene encoding Secreted von Willebrand factor-binding protein»

<400> 199
aaaatcacaa acctgcatct 20

<210> 200
<211> 20
<212> DNA
<213> artificial

<220>
<221> source
<222> (1)..(20)
<223> /note=«Description of artificial sequence: Reverse primer, vwb* gene encoding Secreted von Willebrand factor-binding protein»

<400> 200
aattacacgc ttctcacgtt 20

<210> 201
<211> 19
<212> DNA
<213> artificial

<220>
<221> source
<222> (1)..(19)
<223> /note=«Description of artificial sequence: Forward primer, fnbA gene encoding Fibronectin-binding protein»

<400> 201
gcatcagaac aaagacaac 19

<210> 202
<211> 20
<212> DNA
<213> artificial

<220>
<221> source
<222> (1)..(20)
<223> /note=«Description of artificial sequence: Reverse primer, fnbA gene encoding Fibronectin-binding protein»

<400> 202
ttacatctgt acccgtttcc 20

<210> 203
<211> 19
<212> DNA
<213> artificial

<220>
<221> source
<222> (1)..(19)
<223> /note=«Description of artificial sequence: Forward primer, fnbB gene encoding Fibronectin binding protein B»

<400> 203
gaaagtaaag cagcgaaac 19

<210> 204
<211> 20
<212> DNA
<213> artificial

<220>
<221> source
<222> (1)..(20)
<223> /note=«Description of artificial sequence: Reverse primer, fnbB gene encoding Fibronectin binding protein B»

<400> 204
aattccttct ccaaatttcc 20

<210> 205
<211> 19
<212> DNA
<213> artificial

<220>
<221> source
<222> (1)..(19)
<223> /note=«Description of artificial sequence: Forward primer, efb=fib gene encoding Fibrinogen-binding Serine-Aspartate protein»

<400> 205
ctacaattgc gtcaacagc 19

<210> 206
<211> 20
<212> DNA
<213> artificial

<220>
<221> source
<222> (1)..(20)
<223> /note=«Description of artificial sequence: Reverse primer, Efb=fib gene encoding Fibrinogen-binding Serine-Aspartate protein»

<400> 206

cgaaactaag ttgactgcct 20

<210> 207
<211> 20
<212> DNA
<213> artificial

<220>
<221> source
<222> (1)..(20)
<223> /note=«Description of artificial sequence: Forward primer, coa* gene encoding Staphylocoagulase (conserved domain)»

<400> 207
gaaaccagca agaggttaaa 20

<210> 208
<211> 20
<212> DNA
<213> artificial

<220>
<221> source
<222> (1)..(20)
<223> /note=«Description of artificial sequence: Reverse primer, coa* gene encoding Staphylocoagulase (conserved domain)»

<400> 208
cgttgtattc acggatacct 20

<210> 209
<211> 20
<212> DNA
<213> artificial

<220>
<221> source
<222> (1)..(20)
<223> /note=«Description of artificial sequence: Forward primer, ebpS gene encoding Cell surface elastin binding protein EbpS»

<400> 209
agaccatacg gaagatgttg 20

<210> 210
<211> 20
<212> DNA
<213> artificial

<220>
<221> source
<222> (1)..(20)
<223> /note=«Description of artificial sequence: Reverse primer, ebpS gene encoding Cell surface elastin binding protein

EbpS»

<400> 210
tggtttaggt tgctgagatt 20

<210> 211
<211> 20
<212> DNA
<213> artificial

<220>
<221> source
<222> (1)..(20)
<223> /note=«Description of artificial sequence: Forward primer, ssaA(SA2093) gene encoding Secretory antigen precursor SsaA homolog»

<400> 211
acgacccaac atcatatagc 20

<210> 212
<211> 20
<212> DNA
<213> artificial

<220>
<221> source
<222> (1)..(20)
<223> /note=«Description of artificial sequence: Reverse primer, ssaA(SA2093) gene encoding Secretory antigen precursor SsaA homolog»

<400> 212
actgtgtaac cagctcttgc 20

<210> 213
<211> 20
<212> DNA
<213> artificial

<220>
<221> source
<222> (1)..(20)
<223> /note=«Description of artificial sequence: Forward primer, map* gene encoding Cell surface binding protein»

<400> 213
ataataatga agcgtctgcc 20

<210> 214
<211> 20
<212> DNA
<213> artificial

<220>
<221> source
<222> (1)..(20)

<223> /note=«Description of artificial sequence: Reverse primer, map* gene encoding Cell surface binding protein»

<400> 214
tggaacttgg taatttgctt 20

<210> 215
<211> 20
<212> DNA
<213> artificial

<220>
<221> source
<222> (1)..(20)
<223> /note=«Description of artificial sequence: Forward primer, emp gene encoding Plasma binding protein»

<400> 215
cattactacg caaacccact 20

<210> 216
<211> 21
<212> DNA
<213> artificial

<220>
<221> source
<222> (1)..(21)
<223> /note=«Description of artificial sequence: Reverse primer, emp gene encoding Plasma binding protein»

<400> 216
aaccatttga ttgtgaaaat g 21

<210> 217
<211> 19
<212> DNA
<213> artificial

<220>
<221> source
<222> (1)..(19)
<223> /note=«Description of artificial sequence: Forward primer, clfB* gene encoding Serine- Aspartate Clumping factor B»

<400> 217
ataatgcaag tgcgattcc 19

<210> 218
<211> 20
<212> DNA
<213> artificial

<220>
<221> source
<222> (1)..(20)
<223> /note=«Description of artificial sequence: Reverse primer,

clfB* gerne encoding Serine- Aspartate Clumping factor B»

<400> 218
gctctcgttc taacacttgg 20

<210> 219
<211> 20
<212> DNA
<213> artificial

<220>
<221> source
<222> (1)..(20)
<223> /note=«Description of artificial sequence: Forward primer, SA 0423 gene encoding Putative autolysin»

<400> 219
ttgatttttg ggttaagtgg 20

<210> 220
<211> 20
<212> DNA
<213> artificial

<220>
<221> source
<222> (1)..(20)
<223> /note=«Description of artificial sequence: Reverse primer, sdrC gene encoding Serine-Aspartate protein»

<400> 220
atttgttcct tgttgtggag 20

<210> 221
<211> 20
<212> DNA
<213> artificial

<220>
<221> source
<222> (1)..(20)
<223> /note=«Description of artificial sequence: Forward primer, fus(SA0505) gene encoding Elongation factor G and vitronectin-binding protein»

<400> 221
ctccaatggt ttcatatcgt 20

<210> 222
<211> 20
<212> DNA
<213> artificial

<220>
<221> source
<222> (1)..(20)
<223> /note=«Description of artificial sequence: Reverse primer,

fus(SA0505) gene encoding Elongation factor G and vitronectin-binding protein»

<400> 222
aggttccata ccatcaacac 20

<210> 223
<211> 20
<212> DNA
<213> artificial

<220>
<221> source
<222> (1)..(20)
<223> /note=«Description of artificial sequence: Forward primer, sbi(SA2206) gene encoding IgG-binding protein Sbi»

<400> 223
cacgcaacaa acttcaacta 20

<210> 224
<211> 20
<212> DNA
<213> artificial

<220>
<221> source
<222> (1)..(20)
<223> /note=«Description of artificial sequence: Reverse primer, sbi(SA2206) gene encoding IgG-binding protein Sbi»

<400> 224
agcttcatat gctgctgatt 20

<210> 225
<211> 20
<212> DNA
<213> artificial

<220>
<221> source
<222> (1)..(20)
<223> /note=«Description of artificial sequence: Forward primer, atl-glucosamin gene encoding Atl : autolysin glucosaminidase domain»

<400> 225
acactggtat tcgtgcttct 20

<210> 226
<211> 20
<212> DNA
<213> artificial

<220>
<221> source
<222> (1)..(20)

<223> /note=«Description of artificial sequence: Reverse primer, atl-glucosamin gene encoding Atl : autolysin glucosaminidase domain»

<400> 226
ggtgcagtta aatcttttgc 20

<210> 227
<211> 20
<212> DNA
<213> artificial

<220>
<221> source
<222> (1)..(20)
<223> /note=«Description of artificial sequence: Forward primer, atl-amidase gene encoding Atl : autolysin amidase domain»

<400> 227
tcgacgataa atggtgaaat 20

<210> 228
<211> 20
<212> DNA
<213> artificial

<220>
<221> source
<222> (1)..(20)
<223> /note=«Description of artificial sequence: Reverse primer, atl-amidase gene encoding Atl : autolysin amidase domain»

<400> 228
ttttgatggt gtagtagggg 20

<210> 229
<211> 20
<212> DNA
<213> artificial

<220>
<221> source
<222> (1)..(20)
<223> /note=«Description of artificial sequence: Forward primer, SA 0423 gene encoding Putative autolysin»

<400> 229
gcagctatta ttgggacaag 20

<210> 230
<211> 20
<212> DNA
<213> artificial

<220>
<221> source

<222> (1)..(20)
<223> /note=«Description of artificial sequence: Reverse primer, SA 0423 gene encoding Putative autolysin»

<400> 230
gcgttacttg agctagcagt 20

<210> 231
<211> 20
<212> DNA
<213> artificial

<220>
<221> source
<222> (1)..(20)
<223> /note=«Description of artificial sequence: Forward primer, SA0977 gene encoding Cell surface protein Q99UX4»

<400> 231
tgtatacata ggcgcagaca 20

<210> 232
<211> 20
<212> DNA
<213> artificial

<220>
<221> source
<222> (1)..(20)
<223> /note=«Description of artificial sequence: Reverse primer, SA0977 gene encoding Cell surface protein Q99UX4»

<400> 232
cggtttaaca ttgtttggtt 20

<210> 233
<211> 20
<212> DNA
<213> artificial

<220>
<221> source
<222> (1)..(20)
<223> /note=«Description of artificial sequence: Forward primer, icaA gene encoding Intercellular adhesion protein A»

<400> 233
tgttcttgca ctcaaatacg 20

<210> 234
<211> 20
<212> DNA
<213> artificial

<220>
<221> source
<222> (1)..(20)

<223> /note=«Description of artificial sequence: Reverse primer, icaA gene encoding Intercellular adhesion protein A»

<400> 234
atagagtgaa gacacccgaa 20

<210> 235
<211> 21
<212> DNA
<213> artificial

<220>
<221> source
<222> (1)..(21)
<223> /note=«Description of artificial sequence: Forward primer, icaC gene encoding Intercellular adhesion protein C»

<400> 235
tttggtacac cttgctttat t 21

<210> 236
<211> 18
<212> DNA
<213> artificial

<220>
<221> source
<222> (1)..(18)
<223> /note=«Description of artificial sequence: Reverse primer, icaC gene encoding Intercellular adhesion protein C»

<400> 236
ggtatcgtga aacgctgt 18

<210> 237
<211> 20
<212> DNA
<213> artificial

<220>
<221> source
<222> (1)..(20)
<223> /note=«Description of artificial sequence: Forward primer, putB gene encoding High affinity proline permease»

<400> 237
caactggtaa cctaagcgag 20

<210> 238
<211> 20
<212> DNA
<213> artificial

<220>
<221> source

<222> (1)..(20)
<223> /note=«Description of artificial sequence: Reverse primer, putB gene encoding High affinity proline permease»

<400> 238
ttaccaccag atacgaaacc 20

<210> 239
<211> 20
<212> DNA
<213> artificial

<220>
<221> source
<222> (1)..(20)
<223> /note=«Description of artificial sequence: Forward primer, recA gene encoding Recombinase RecA»

<400> 239
ggctatccta aaggacgaat 20

<210> 240
<211> 20
<212> DNA
<213> artificial

<220>
<221> source
<222> (1)..(20)
<223> /note=«Description of artificial sequence: Reverse primer, recA gene encoding Recombinase RecA»

<400> 240
ccgaacataa caccaacttt 20

<210> 241
<211> 20
<212> DNA
<213> artificial

<220>
<221> source
<222> (1)..(20)
<223> /note=«Description of artificial sequence: Forward primer, lexA gene encoding SOS regulatory LexA protein»

<400> 241
aaaggttatc cgcctagtgt 20

<210> 242
<211> 20
<212> DNA
<213> artificial

<220>
<221> source

<222> (1)..(20)
<223> /note=«Description of artificial sequence: Reverse primer, lexA gene encoding SOS regulatory LexA protein»

<400> 242
tctccatttt ctgctatggt 20

<210> 243
<211> 20
<212> DNA
<213> artificial

<220>
<221> source
<222> (1)..(20)
<223> /note=«Description of artificial sequence: Forward primer, agrA gene encoding Accessory gene regulator (Agr) protein A»

<400> 243
gaagacgatc caaaacaaag 20

<210> 244
<211> 20
<212> DNA
<213> artificial

<220>
<221> source
<222> (1)..(20)
<223> /note=«Description of artificial sequence: Reverse primer, agrA gene encoding Accessory gene regulator (Agr) protein A»

<400> 244
actgaattac tgccacgttt 20

<210> 245
<211> 20
<212> DNA
<213> artificial

<220>
<221> source
<222> (1)..(20)
<223> /note=«Description of artificial sequence: Forward primer, agrC* gene encoding AgrC (N terminal conserved domain)»

<400> 245
acggtgatag cttaattcca 20

<210> 246
<211> 20
<212> DNA
<213> artificial

<220>

<221> source
<222> (1)..(20)
<223> /note=«Description of artificial sequence: Reverse primer, agrC* gene encoding AgrC (N terminal conserved domain)»

<400> 246
ttacttcatc gggtatttcg 20

<210> 247
<211> 20
<212> DNA
<213> artificial

<220>
<221> source
<222> (1)..(20)
<223> /note=«Description of artificial sequence: Forward primer, agrJ gene encoding AgrJ»

<400> 247
tttgggtgga tttactcatc 20

<210> 248
<211> 20

<212> DNA
<213> artificial

<220>
<221> source
<222> (1)..(20)
<223> /note=«Description of artificial sequence: Reverse primer, agrJ gene encoding AgrJ»

<400> 248
atttgggtga atcatacctg 20

<210> 249
<211> 20

<212> DNA
<213> artificial

<220>
<221> source
<222> (1)..(20)
<223> /note=«Description of artificial sequence: Forward primer, norA* gene encoding Quinolones transporter NorA»

<400> 249
caaatgatta tatcgccgtt 20

<210> 250
<211> 20
<212> DNA
<213> artificial

<220>
<221> source
<222> (1)..(20)
<223> /note=«Description of artificial sequence: Reverse primer, norA* gene encoding Quinolones transporter NorA»

<400> 250
tgtcgtagac tttttcggat 20

<210> 251
<211> 20
<212> DNA
<213> artificial

<220>
<221> source
<222> (1)..(20)
<223> /note=«Description of artificial sequence: Forward primer, radA gene encoding DNA repair protein»

<400> 251
ggaaattgtt gaaaaagcag 20

<210> 252
<211> 20
<212> DNA
<213> artificial

<220>
<221> source
<222> (1)..(20)
<223> /note=«Description of artificial sequence: Reverse primer, radA gene encoding DNA repair protein»

<400> 252
acttgtgaaa cagaacctgg 20

<210> 253
<211> 20
<212> DNA
<213> artificial

<220>
<221> source
<222> (1)..(20)
<223> /note=«Description of artificial sequence: Forward primer, rnc(SA1076) gene encoding Ribonuclease III»

<400> 253
ggaatttaac aaaaatgcgt 20

<210> 254
<211> 20
<212> DNA
<213> artificial

<220>

<221> source
<222> (1)..(20)
<223> /note=«Description of artificial sequence: Reverse primer, rnc(SA1076) gene encoding Ribonuclease III»

<400> 254
agcacgttgt tctgattctt 20

<210> 255
<211> 20
<212> DNA
<213> artificial

<220>
<221> source
<222> (1)..(20)
<223> /note=«Description of artificial sequence: Forward primer, rpoD* gene encoding RNA polymerase sigma factor rpoD»

<400> 255
acaaacaatt gatccgacat 20

<210> 256
<211> 21
<212> DNA
<213> artificial

<220>
<221> source
<222> (1)..(21)
<223> /note=«Description of artificial sequence: Reverse primer, rpoD* gene encoding RNA polymerase sigma factor rpoD»

<400> 256
cgacctacgt atcttttagc a 21

<210> 257
<211> 20
<212> DNA
<213> artificial

<220>
<221> source
<222> (1)..(20)
<223> /note=«Description of artificial sequence: Forward primer, sigB* gene encoding Sigma factor B»

<400> 257
taaaggacaa tcacatcacg 20

<210> 258
<211> 20
<212> DNA
<213> artificial

<220>
<221> source

<222> (1)..(20)
<223> /note=«Description of artificial sequence: Reverse primer, sigB* gene encoding Sigma factor B»

<400> 258
tcttgttgcc ccataatatc 20

<210> 259
<211> 20
<212> DNA
<213> artificial

<220>
<221> source
<222> (1)..(20)
<223> /note=«Description of artificial sequence: Forward primer, rsbU* gene encoding SigmaB regulation protein RsbU»

<400> 259
atcgtggaag aatttaagca 20

<210> 260
<211> 20
<212> DNA
<213> artificial

<220>
<221> source
<222> (1)..(20)
<223> /note=«Description of artificial sequence: Reverse primer, rsbU* gene encoding SigmaB regulation protein RsbU»

<400> 260
tggtatacct ttcccaatga 20

<210> 261
<211> 20
<212> DNA
<213> artificial

<220>
<221> source
<222> (1)..(20)
<223> /note=«Description of artificial sequence: Forward primer, mdr* gene encoding Putative multidrug resistance protein»

<400> 261
aaatggcgat caaattaaaa 20

<210> 262
<211> 20
<212> DNA
<213> artificial

<220>
<221> source

<222> (1)..(20)
<223> /note=«Description of artificial sequence: Reverse primer, mdr* gene encoding Putative multidrug resistance protein»

<400> 262
catcactatt tgatgatggc 20

<210> 263
<211> 20
<212> DNA
<213> artificial

<220>
<221> source
<222> (1)..(20)
<223> /note=«Description of artificial sequence: Forward primer, vraR (SA1700) gene encoding Two-component response regulator»

<400> 263
gtggatgatc atgaaatggt 20

<210> 264
<211> 20
<212> DNA
<213> artificial

<220>
<221> source
<222> (1)..(20)
<223> /note=«Description of artificial sequence: Reverse primer, vraR (SA1700) gene encoding Two-component response regulator»

<400> 264
ccatttctcg ttctgtaagc 20

<210> 265
<211> 20
<212> DNA
<213> artificial

<220>
<221> source
<222> (1)..(20)
<223> /note=«Description of artificial sequence: Forward primer, dnaK gene encoding Heat shock chaperone protein dnaK»

<400> 265
tcacgtacaa caccatctgt 20

<210> 266
<211> 20
<212> DNA
<213> artificial

<220>
<221> source
<222> (1)..(20)
<223> /note=«Description of artificial sequence: Reverse primer, dnaK gene encoding Heat shock chaperone protein dnaK»

<400> 266
cgaatacacc gtcacctaat 20

<210> 267
<211> 20
<212> DNA
<213> artificial

<220>
<221> source
<222> (1)..(20)
<223> /note=«Description of artificial sequence: Forward primer, lytS+lytR gene encodingTWO-component response regulator»

<400> 267
aaatgaaatt ggtggttttg 20

<210> 268
<211> 20
<212> DNA
<213> artificial

<220>
<221> source
<222> (1)..(20)
<223> /note=«Description of artificial sequence: Reverse primer, lytS+lytR gene encodingTWO-component response regulator»

<400> 268
gattcaatgg ctctgttgtt 20

<210> 269
<211> 20
<212> DNA
<213> artificial

<220>
<221> source
<222> (1)..(20)
<223> /note=«Description of artificial sequence: Forward primer, geh gene encoding Glycerol ester hydrolase»

<400> 269
gtattcaata ggcgtggtgt 20

<210> 270
<211> 20
<212> DNA
<213> artificial

<220>

<221> source
<222> (1)..(20)
<223> /note=«Description of artificial sequence: Reverse primer, geh gene encoding Glycerol ester hydrolase»

<400> 270
gttcagcatg atgggtattt 20

<210> 271
<211> 20
<212> DNA
<213> artificial

<220>
<221> source
<222> (1)..(20)
<223> /note=«Description of artificial sequence: Forward primer, sacP (SA0295) gene encoding Acidic phosphatase»

<400> 271
ggcttggtat caaaattcag 20

<210> 272
<211> 20
<212> DNA
<213> artificial

<220>
<221> source
<222> (1)..(20)
<223> /note=«Description of artificial sequence: Reverse primer, sacP (SA0295) gene encoding Acidic phosphatase»

<400> 272
atttgtctgc gtgattcttt 20

<210> 273
<211> 20
<212> DNA
<213> artificial

<220>
<221> source
<222> (1)..(20)
<223> /note=«Description of artificial sequence: Forward primer, lip(SA2463) gene encoding Triacylglycerol lipase»

<400> 273
gtgtaggtgc atcttccatt 20

<210> 274
<211> 20
<212> DNA
<213> artificial

<220>
<221> source

<222> (1)..(20)
<223> /note=«Description of artificial sequence: Reverse primer, lip(SA2463) gene encoding Triacylglycerol lipase»

<400> 274
gtttgccatc attaatagcc 20

<210> 275
<211> 20
<212> DNA
<213> artificial

<220>
<221> source
<222> (1)..(20)
<223> /note=«Description of artificial sequence: Forward primer, SA0587 gene encoding Putative lipoprotein»

<400> 275
ctgttggtca agatcctcat 20

<210> 276
<211> 20
<212> DNA
<213> artificial

<220>
<221> source
<222> (1)..(20)
<223> /note=«Description of artificial sequence: Reverse primer, SA0587 gene encoding Putative lipoprotein»

<400> 276
acgttgttct tttggaatgt 20

<210> 277
<211> 20
<212> DNA
<213> artificial

<220>
<221> source
<222> (1)..(20)
<223> /note=«Description of artificial sequence: Forward primer, SA0691 gene encoding Protein similar to ferrichrome ABC transporter»

<400> 277
tgcggtaaca attctgataa 20

<210> 278
<211> 20
<212> DNA
<213> artificial

<220>
<221> source

<222> (1)..(20)
<223> /note=«Description of artificial sequence: Reverse primer, SA0691 gene encoding Protein similar to ferrichrome ABC transporter»

<400> 278
ttttcatctg caccaacata 20

<210> 279
<211> 20
<212> DNA
<213> artificial

<220>
<221> source
<222> (1)..(20)
<223> /note=«Description of artificial sequence: Forward primer, plc gene encoding Phosphatidylinositol phosphodiesterase SA091»

<400> 279
tgagattaat atgccaggct 20

<210> 280
<211> 20
<212> DNA
<213> artificial

<220>
<221> source
<222> (1)..(20)
<223> /note=«Description of artificial sequence: Reverse primer, plc gene encoding Phosphatidylinositol phosphodiesterase SA091»

<400> 280
gtcagcacca taaccactct 20

<210> 281
<211> 20
<212> DNA
<213> artificial

<220>
<221> source
<222> (1)..(20)
<223> /note=«Description of artificial sequence: Forward primer, SA0746* gene encoding Staphylococcal nuclease»

<400> 281
agaggttttt ctttttcgct 20

<210> 282
<211> 20
<212> DNA
<213> artificial

<220>
<221> source
<222> (1)..(20)
<223> /note=«Description of artificial sequence: Reverse primer, SA0746* gene encoding Staphylococcal nuclease»

<400> 282
tttaccattt ttccatcagc 20

<210> 283
<211> 20
<212> DNA
<213> artificial

<220>
<221> source
<222> (1)..(20)
<223> /note=«Description of artificial sequence: Forward primer, hsdM gene encoding Restriction modification enzyme subunit HsdM»

<400> 283
aataacaatt gcgccactaa 20

<210> 284
<211> 18
<212> DNA
<213> artificial

<220>
<221> source
<222> (1)..(18)
<223> /note=«Description of artificial sequence: Reverse primer, hsdM gene encoding Restriction modification enzyme subunit HsdM»

<400> 284
gccgtcgcta gatgttct 18

<210> 285
<211> 20
<212> DNA
<213> artificial

<220>
<221> source
<222> (1)..(20)
<223> /note=«Description of artificial sequence: Forward primer, fur* gene encoding Iron uptake regulatory protein»

<400> 285
attaaatcgc gttaagcaac 20

<210> 286

<211> 20
<212> DNA
<213> artificial

<220>
<221> source
<222> (1)..(20)
<223> /note=«Description of artificial sequence: Reverse primer, fur* gene encoding Iron uptake regulatory protein»

<400> 286
ttacctttag cttggcatgt 20

<210> 287
<211> 20
<212> DNA
<213> artificial

<220>
<221> source
<222> (1)..(20)
<223> /note=«Description of artificial sequence: Forward primer, fhuD2 (SA2079) gene encoding Ferric hydroxamatereceptor 2»

<400> 287
gttgctaaag aaaagccaga 20

<210> 288
<211> 20
<212> DNA
<213> artificial

<220>
<221> source
<222> (1)..(20)
<223> /note=«Description of artificial sequence: Reverse primer, fhuD2 (SA2079) gene encoding Ferric hydroxamatereceptor 2»

<400> 288
agctttcaaa ttcttccaca 20

<210> 289
<211> 20
<212> DNA
<213> artificial

<220>
<221> source
<222> (1)..(20)
<223> /note=«Description of artificial sequence: Forward primer, fhuD=fhuG*(SA0604) gene encoding Ferrichrome transport permease FhuG»

<400> 289
tgattactat tttggctggc 20

<210> 290
<211> 20
<212> DNA
<213> artificial

<220>
<221> source
<222> (1)..(20)
<223> /note=«Description of artificial sequence: Reverse primer, fhuD=fhuG*(SA0604) gene encoding Ferrichrome transport permease FhuG»

<400> 290
ataataatca acacccacgg 20

<210> 291
<211> 20
<212> DNA
<213> artificial

<220>
<221> source
<222> (1)..(20)
<223> /note=«Description of artificial sequence: Forward primer, fhuB gene encoding Ferrichrome transport permease FhuB»

<400> 291
ttaataggtg acgccaaaat 20

<210> 292
<211> 20
<212> DNA
<213> artificial

<220>
<221> source
<222> (1)..(20)
<223> /note=«Description of artificial sequence: Reverse primer, fhuB gene encoding Ferrichrome transport permease FhuB»

<400> 292
catagcactt actgctgcac 20

<210> 293
<211> 20
<212> DNA
<213> artificial

<220>
<221> source
<222> (1)..(20)
<223> /note=«Description of artificial sequence: Forward primer, fhuA=fhuC (SA0602) gene encoding Ferrichrome transport ATP-binding protein FhuA (FhuC)»

<400> 293
gttttccaca tcaaaaagga 20

<210> 294
<211> 20
<212> DNA
<213> artificial

<220>
<221> source
<222> (1)..(20)
<223> /note=«Description of artificial sequence: Reverse primer, fhuA-fhuC (SA0602) gene encoding Ferrichrome transport ATP-binding protein FhuA (FhuC)»

<400> 294
agctctgcga cataagtcat 20

<210> 295
<211> 20
<212> DNA
<213> artificial

<220>
<221> source
<222> (1)..(20)
<223> /note=«Description of artificial sequence: Forward primer, SA0675 gene encoding ABC protein»

<400> 295
gtacaccagc caaatgttct 20

<210> 296
<211> 20
<212> DNA
<213> artificial

<220>
<221> source
<222> (1)..(20)
<223> /note=«Description of artificial sequence: Reverse primer, SA0675 gene encoding ABC protein»

<400> 296
ttttcccata atctgcactt 20

<210> 297
<211> 20
<212> DNA
<213> artificial

<220>
<221> source
<222> (1)..(20)
<223> /note=«Description of artificial sequence: Forward primer, SA1852 gene encoding ABC protein»

<400> 297

ctccaacatt ttgaaggaac 20

<210> 298
<211> 20
<212> DNA
<213> artificial

<220>
<221> source
<222> (1)..(20)
<223> /note=«Description of artificial sequence: Reverse primer, SA1852 gene encoding ABC protein»

<400> 298
tcgctatctt gtttttggat 20

<210> 299
<211> 20
<212> DNA
<213> artificial

<220>
<221> source
<222> (1)..(20)
<223> /note=«Description of artificial sequence: Forward primer, SAV1392* gene encoding ABC protein»

<400> 299
tcagagttct ttgagggtgt 20

<210> 300
<211> 20
<212> DNA
<213> artificial

<220>
<221> source
<222> (1)..(20)
<223> /note=«Description of artificial sequence: Reverse primer, SAV1392* gene encoding ABC protein»

<400> 300
attttaaaac gccgatttct 20

<210> 301
<211> 20
<212> DNA
<213> artificial

<220>
<221> source
<222> (1)..(20)
<223> /note=«Description of artificial sequence: Forward primer, SA1580* Gene encoding ABC protein»

<400> 301
actgttgctg gtttagtgct 20

<210> 302
<211> 20
<212> DNA
<213> artificial

<220>
<221> source
<222> (1)..(20)
<223> /note=«Description of artificial sequence: Reverse primer, SA1580* Gene encoding ABC protein»

<400> 302
aaagctaaat tctgcgacaa 20

<210> 303
<211> 20
<212> DNA
<213> artificial

<220>
<221> source
<222> (1)..(20)
<223> /note=«Description of artificial sequence: Forward primer, fabH gene encoding FabH protein»

<400> 303
tatgcgccag aaaagattat 20

<210> 304
<211> 20
<212> DNA
<213> artificial

<220>
<221> source
<222> (1)..(20)
<223> /note=«Description of artificial sequence: Reverse primer, fabH gene encoding FabH protein
«

<400> 304
tacctctgcc atctgaaact 20

<210> 305
<211> 20
<212> DNA
<213> artificial

<220>
<221> source
<222> (1)..(20)
<223> /note=«Description of artificial sequence: Forward primer, ORF30 gene encoding Putative Cyclase hisF Q8NUI3»

<400> 305
aggattaagg gatattggga 20

<210> 306
<211> 20
<212> DNA
<213> artificial

<220>
<221> source
<222> (1)..(20)
<223> /note=«Description of artificial sequence: Reverse primer, ORF30 gene encoding Putative Cyclase hisF Q8NUI3»

<400> 306
ccatacttgt aacgaggagc 20

<210> 307
<211> 20
<212> DNA
<213> artificial

<220>
<221> source
<222> (1)..(20)
<223> /note=«Description of artificial sequence: Forward primer, SA0102 gene encoding Putative protein Q99XA7»

<400> 307
aaagcaggtg gtagtcttga 20

<210> 308
<211> 20
<212> DNA
<213> artificial

<220>

<221> source
<222> (1)..(20)
<223> /note=«Description of artificial sequence: Reverse primer, SA0102 gene encoding Putative protein Q99XA7»

<400> 308
ataacgacgc atttccatag 20

<210> 309
<211> 20
<212> DNA
<213> artificial

<220>
<221> source
<222> (1)..(20)
<223> /note=«Description of artificial sequence: Forward primer, SA0610 gene encoding Putative protein Q99VW6»

<400> 309
gcatttcatc tgaaacaaca 20

<210> 310
<211> 20
<212> DNA
<213> artificial

<220>
<221> source
<222> (1)..(20)
<223> /note=«Description of artificial sequence: Reverse primer, SA0610 gene encoding Putative protein Q99VW6»

<400> 310
aagcgatcat tcgtctgtat 20

<210> 311
<211> 21
<212> DNA
<213> artificial

<220>
<221> source
<222> (1)..(21)
<223> /note=«Description of artificial sequence: Forward primer, SA 1970 Gene encoding Putative protein Q99S99»

<400> 311
gaacatttgc attatgtacg a 21

<210> 312
<211> 20
<212> DNA
<213> artificial

<220>
<221> source
<222> (1)..(20)
<223> /note=«Description of artificial sequence: Reverse primer, SA 1970 Gene encoding Putative protein Q99S99»

<400> 312
ttttgcacca aaactcatta 20

<210> 313
<211> 20
<212> DNA
<213> artificial

<220>
<221> source
<222> (1)..(20)
<223> /note=«Description of artificial sequence: Forward primer, SA0022 gene encoding Putative protein Q99XE9»

<400> 313
tgacagttaa tggagggaac 20

<210> 314
<211> 20
<212> DNA
<213> artificial

<220>
<221> source
<222> (1)..(20)
<223> /note=«Description of artificial sequence: Reverse primer, SA0022 gene encoding Putative protein Q99XE9»

<400> 314
ggtgaaagtg atgctgtttt 20

<210> 315
<211> 20
<212> DNA
<213> artificial

<220>
<221> source
<222> (1)..(20)
<223> /note=«Description of artificial sequence: Forward primer, SA0276 gene encoding Putative diarrheal toxin-like protein Q99WT9»

<400> 315
cgtgcagata ttacgttgaa 20

<210> 316
<211> 20
<212> DNA
<213> artificial

<220>
<221> source
<222> (1)..(20)
<223> /note=«Description of artificial sequence: Reverse primer, SA0276 gene encoding Putative diarrheal toxin-like protein Q99WT9»

<400> 316
ttaatgacga tgccactgta 20

<210> 317
<211> 20
<212> DNA
<213> artificial

<220>
<221> source
<222> (1)..(20)
<223> /note=«Description of artificial sequence: Forward primer, SA1552 gene encoding Putative protein Q99TD3»

<400> 317
tgcaactaca actcagcaac 20

<210> 318
<211> 20
<212> DNA
<213> artificial

<220>
<221> source
<222> (1)..(20)
<223> /note=«Description of artificial sequence: Reverse primer, SA1552 gene encoding Putative protein Q99TD3»

<400> 318
atttcaatt cttgtgtgcc 20

<210> 319
<211> 20
<212> DNA
<213> artificial

<220>
<221> source
<222> (1)..(20)
<223> /note=«Description of artificial sequence: Forward primer, MW2409 gene encoding Q8NUV5»

<400> 319
ccagcttaaa ggctcaacta 20

<210> 320
<211> 20
<212> DNA
<213> artificial

<220>
<221> source
<222> (1)..(20)
<223> /note=«Description of artificial sequence: Reverse primer, MW2409 gene encoding Q8NUV5»

<400> 320
tatcagctag cagcatttga 20

<210> 321
<211> 19
<212> DNA
<213> artificial

<220>
<221> source
<222> (1)..(19)
<223> /note=«Description of artificial sequence: Forward primer, clfA* gene encoding Serine- Aspartate Clumping factor A»

<400> 321
taaaacagac acacaaacg 19

<210> 322
<211> 20
<212> DNA
<213> artificial

<220>
<221> source
<222> (1)..(20)
<223> /note=«Description of artificial sequence: Reverse primer, clfA* gene encoding Serine- Aspartate Clumping factor A»

<400> 322
agctactgcc gctaaactaa 20

<210> 323
<211> 20
<212> DNA
<213> artificial

<220>
<221> source
<222> (1)..(20)
<223> /note=«Description of artificial sequence: Forward primer, aacA-aphD gene encoding Acetyl transferase-phosphotransferase: AAC6'-APH2»«

<400> 323
tatacagagc cttgggaaga 20

<210> 324
<211> 20
<212> DNA
<213> artificial

<220>
<221> source
<222> (1)..(20)
<223> /note=«Description of artificial sequence: Reverse primer, aacA-aphD gene encoding Acetyl transferase-phosphotransferase: AAC6'-APH2»«

<400> 324
tcgtgtaatt catgttctgg 20

<210> 325
<211> 20
<212> DNA
<213> artificial

<220>
<221> source
<222> (1)..(20)
<223> /note=«Description of artificial sequence: Forward primer, tetK gene encoding Tetracycline resistance»

<400> 325
gaaaaacatt ccgtttatgc 20

<210> 326
<211> 20
<212> DNA
<213> artificial

<220>
<221> source
<222> (1)..(20)
<223> /note=«Description of artificial sequence: Reverse primer, tetK gene encoding Tetracycline resistance»

<400> 326
gctatacctg ttccctctga 20

<210> 327
<211> 20
<212> DNA
<213> artificial

<220>
<221> source
<222> (1)..(20)
<223> /note=«Description of artificial sequence: Forward primer, tetM gene encoding Tetracycline resistance»

<400> 327
acgcttttag aacgtcagag 20

<210> 328
<211> 20
<212> DNA
<213> artificial

<220>
<221> source
<222> (1)..(20)
<223> /note=«Description of artificial sequence: Reverse primer, tetM gene encoding Tetracycline resistance»

<400> 328
tcagattcgg taaagttcgt 20

<210> 329
<211> 20
<212> DNA
<213> artificial

<220>
<221> source
<222> (1)..(20)
<223> /note=«Description of artificial sequence: Forward primer, entB gene encoding Enterotoxin B»

<400> 329
tgcacaaatc gagtaaattc 20

<210> 330
<211> 21
<212> DNA
<213> artificial

<220>
<221> source
<222> (1)..(21)
<223> /note=«Description of artificial sequence: Reverse primer, EntB gene encoding Enterotoxin B»

<400> 330
tttcaccaaa tagtgacgag t 21

<210> 331
<211> 20
<212> DNA
<213> artificial

<220>
<221> source
<222> (1)..(20)
<223> /note=«Description of artificial sequence: Forward primer, lukS gene encoding Leucocidin S»

<400> 331
tataaaagca atgaggtggc 20

<210> 332
<211> 20
<212> DNA
<213> artificial

<220>
<221> source
<222> (1)..(20)
<223> /note=«Description of artificial sequence: Reverse primer, lukS gene encoding Leucocidin S»

<400> 332
cactgtgtac taatgggggt 20

<210> 333
<211> 31
<212> DNA
<213> artificial

<220>
<221> source
<222> (1)..(31)
<223> /note=«Description of artificial sequence: Forward primer, lukS+F*gene encoding Leucocidins F + S»

<400> 333
atcattaggt aaaatgtctg gacatgatcc a 31

<210> 334
<211> 27
<212> DNA
<213> artificial

<220>
<221> source
<222> (1)..(27)
<223> /note=«Description of artificial sequence: Reverse primer, lukS+F'gene encoding Leucocidins F + S»

<400> 334
gcatcaastg tattggatag caaaagc 27

<210> 335
<211> 20
<212> DNA
<213> artificial

<220>
<221> source
<222> (1)..(20)
<223> /note=«Description of artificial sequence: Forward primer, SAV 0415 gene encoding Putative transposase»

<400> 335
aattattccg tgtccatttg 20

<210> 336
<211> 20
<212> DNA
<213> artificial

<220>
<221> source
<222> (1)..(20)
<223> /note=«Description of artificial sequence: Reverse primer, SAV 0415 gene encoding Putative transposase»

<400> 336
ctgcgatatg aatagcttcc 20

<210> 337
<211> 20
<212> DNA
<213> artificial

<220>
<221> source
<222> (1)..(20)
<223> /note=«Description of artificial sequence: Forward primer, SAV0847 Gene encoding Putative integrase»

<400> 337
tgaagctaag agagcagagg 20

<210> 338
<211> 20
<212> DNA
<213> artificial

<220>
<221> source
<222> (1)..(20)
<223> /note=«Description of artificial sequence: Reverse primer, SAV0847 Gene encoding Putative integrase»

<400> 338
cttcaaattc ttcacgcatc 20

<210> 339
<211> 20
<212> DNA
<213> artificial

<220>
<221> source
<222> (1)..(20)

<223> /note=«Description of artificial sequence: Forward primer, SAV2028 gene encoding Putative integrase»

<400> 339
agctttttcc aaataaaggc 20

<210> 340
<211> 20
<212> DNA
<213> artificial

<220>
<221> source

<222> (1)..(20)
<223> /note=«Description of artificial sequence: Reverse primer, SAV2028 gene encoding Putative integrase»

<400> 340
attggtttgt tcttaaagcg 20

<210> 341
<211> 20
<212> DNA
<213> artificial

<220>
<221> source
<222> (1)..(20)
<223> /note=«Description of artificial sequence: Forward primer, Int459 (SAV0392) gene encoding Putative integrase Int459 Q9AGN0»

<400> 341
aaaagcaaag aatggaacct 20

<210> 342
<211> 20
<212> DNA
<213> artificial

<220>
<221> source
<222> (1)..(20)
<223> /note=«Description of artificial sequence: Reverse primer, Int459 (SAV0392) gene encoding Putative integrase Int459 Q9AGN0»

<400> 342
tagatggatt tgtggtggtt 20

<210> 343
<211> 20
<212> DNA
<213> artificial

<220>
<221> source
<222> (1)..(20)
<223> /note=«Description of artificial sequence: Forward primer, phi int11 Integrase phage phi11»

<400> 343
gagaagcaaa atgccagtat 20

<210> 344
<211> 20
<212> DNA
<213> artificial

<220>
<221> source
<222> (1)..(20)
<223> /note=«Description of artificial sequence: Reverse primer, phi int11 Integrase phage phi11»

<400> 344
tggttttgtt ctgggaatag 20

<210> 345
<211> 20
<212> DNA
<213> artificial

<220>
<221> source
<222> (1)..(20)
<223> /note=«Description of artificial sequence: Forward primer, ORF15 gene encoding DUT Pase from phage phiPVL O80091»

<400> 345
gaacgaaatc ataagacgga 20

<210> 346
<211> 20
<212> DNA
<213> artificial

<220>
<221> source
<222> (1)..(20)
<223> /note=«Description of artificial sequence: Reverse primer, ORF15 gene encoding DUT Pase from phage phiPVL O80091
«

<400> 346
tgaaacactt tcgaattcct 20

<210> 347
<211> 20
<212> DNA
<213> artificial

<220>
<221> source
<222> (1)..(20)
<223> /note=«Description of artificial sequence: Forward primer, ORF17 gene encoding Ssb from phage phi 11 Q8SDW4»

<400> 347
aaagttgcaa atgtcgagtt 20

<210> 348
<211> 20
<212> DNA
<213> artificial

<220>
<221> source
<222> (1)..(20)
<223> /note=«Description of artificial sequence: Reverse primer, ORF17 gene encoding Ssb from phage phi 11 Q8SDW4»

<400> 348
gaatgggttt gattgttgac 20

<210> 349
<211> 20
<212> DNA
<213> artificial

<220>
<221> source
<222> (1)..(20)
<223> /note=«Description of artificial sequence: Forward primer, ORF22* Gene encoding Orf178 from phage phiSLT Q9B0G4»

<400> 349
tgggttttta agaaactgga 20

<210> 350
<211> 20
<212> DNA
<213> artificial

<220>
<221> source
<222> (1)..(20)
<223> /note=«Description of artificial sequence: Reverse primer, ORF22* Gene encoding Orf178 from phage phiSLT Q9B0G4»

<400> 350
tagcaccctc taaaacttcg 20

<210> 351
<211> 20
<212> DNA
<213> artificial

<220>
<221> source
<222> (1)..(20)
<223> /note=«Description of artificial sequence: Forward primer, ORF23* gene encoding PV83 Orf 3 from phage phi 11Q8SDX4»

<400> 351
tcaaatgttt aacttgagcg 20

<210> 352
<211> 20
<212> DNA
<213> artificial

<220>
<221> source
<222> (1)..(20)
<223> /note=«Description of artificial sequence: Reverse primer, ORF23* gene encoding PV83 Orf 3 from phage phi 11Q8SDX4»

<400> 352
agcagctttg actgaatgtt 20

<210> 353
<211> 20
<212> DNA
<213> artificial

<220>
<221> source
<222> (1)..(20)
<223> /note=«Description of artificial sequence: Forward primer, ORF24* Gene encoding DnaC from phage phi 13 Q9B0F8»

<400> 353
tcaaacgtta atccgtcttt 20

<210> 354
<211> 20
<212> DNA
<213> artificial

<220>
<221> source
<222> (1)..(20)
<223> /note=«Description of artificial sequence: Reverse primer, ORF24* Gene encoding DnaC from phage phi 13 Q9B0F8»

<400> 354
aattgatacg ttgccagttc 20

<210> 355
<211> 20
<212> DNA
<213> artificial

<220>
<221> source
<222> (1)..(20)
<223> /note=«Description of artificial sequence: Forward primer, repC* gene encoding pT181 RepC protein (polypeptide A)»

<400> 355
tgtgcatatc tgatccaaaa 20

<210> 356
<211> 20
<212> DNA
<213> artificial

<220>
<221> source
<222> (1)..(20)
<223> /note=«Description of artificial sequence: Reverse primer, repC* gene encoding pT181 RepC protein (polypeptide A)»

<400> 356
tttgtttctg gcttaccatt 20

<210> 357
<211> 21
<212> DNA
<213> artificial

<220>
<221> source
<222> (1)..(21)

<223> /note=«Description of artificial sequence: Forward primer, SAV1998* gene encoding Repressor homolog Q931J0»

<400> 357
tctgaacgga agatatctca a 21

<210> 358
<211> 20
<212> DNA
<213> artificial

<220>
<221> source
<222> (1)..(20)
<223> /note=«Description of artificial sequence: Reverse primer, SAV1998* gene encoding Repressor homolog Q931J0»

<400> 358
aaccatttcg gtaaacaatg 20

<210> 359
<211> 20
<212> DNA
<213> artificial

<220>
<221> source
<222> (1)..(20)
<223> /note=«Description of artificial sequence: Forward primer, MW0751 gene encoding Putative primase Q8NXK4»

<400> 359
taattgggta ttatggcgag 20

<210> 360
<211> 20
<212> DNA
<213> artificial

<220>
<221> source
<222> (1)..(20)
<223> /note=«Description of artificial sequence: Reverse primer, MW0751 gene encoding Putative primase Q8NXK4»

<400> 360
ttgtgcatcc tgtaacagtc 20

<210> 361
<211> 20
<212> DNA
<213> artificial

<220>
<221> source
<222> (1)..(20)
<223> /note=«Description of artificial sequence: Forward primer, SA1808 gene encoding Q99SP1»

<400> 361
tgacaatggt gagactttga 20

<210> 362
<211> 21
<212> DNA
<213> artificial

<220>
<221> source
<222> (1)..(21)
<223> /note=«Description of artificial sequence: Reverse primer, SA1808 gene encoding Q99SP1»

<400> 362
tggtcttcag cagttttaga g 21

<210> 363
<211> 20
<212> DNA
<213> artificial

<220>
<221> source
<222> (1)..(20)
<223> /note=«Description of artificial sequence: Forward primer, SA1794* Gene encoding Q99SQ7»

<400> 363
cctgctaaga atcaaggcta 20

<210> 364

<211> 20
<212> DNA
<213> artificial

<220>
<221> source
<222> (1)..(20)
<223> /note=«Description of artificial sequence: Reverse primer, SA1794* Gene encoding Q99SQ7»

<400> 364
aatcttttta catgcacggt 20

<210> 365
<211> 23
<212> DNA
<213> artificial

<220>
<221> source
<222> (1)..(23)
<223> /note=«Description of artificial sequence: Forward primer, MW0757 gene encoding Q8NXJ8»

<400> 365
aaagagaaat taggaactgg ttt 23

<210> 366
<211> 24
<212> DNA
<213> artificial

<220>
<221> source
<222> (1)..(24)
<223> /note=«Description of artificial sequence: Reverse primer, MW0757 gene encoding Q8NXJ8»

<400> 366
aagataatgt gtaccttttt attg 24

<210> 367
<211> 19
<212> DNA
<213> artificial

<220>
<221> source
<222> (1)..(19)
<223> /note=«Description of artificial sequence: Forward primer, MW0756 gene encoding Q8NXJ9»

<400> 367
cgcttgatga tgttgaaaa 19

<210> 368
<211> 21
<212> DNA
<213> artificial

<220>
<221> source
<222> (1)..(21)
<223> /note=«Description of artificial sequence: Reverse primer, MW0756 gene encoding Q8NXJ9»

<400> 368
ttccctaatt taagtccatc c 21

<210> 369
<211> 21
<212> DNA
<213> artificial

<220>
<221> source
<222> (1)..(21)
<223> /note=«Description of artificial sequence: Forward primer, MW0755 Gene encoding Q8NXK0»

<400> 369
tctgactttg aaagcaatga t 21

<210> 370
<211> 20
<212> DNA
<213> artificial

<220>
<221> source
<222> (1)..(20)
<223> /note=«Description of artificial sequence: Reverse primer, MW0755 Gene encoding Q8NXK0»

<400> 370
tcgatgtaat tcttctcgct 20

<210> 371
<211> 20
<212> DNA
<213> artificial

<220>
<221> source
<222> (1)..(20)
<223> /note=«Description of artificial sequence: Forward primer, MW0754 gene encoding Q8NXK1»

<400> 371
tgattgaaca ggggttaaag 20

<210> 372
<211> 20
<212> DNA
<213> artificial

<220>
<221> source
<222> (1)..(20)
<223> /note=«Description of artificial sequence: Reverse primer, MW0754 gene encoding Q8NXK1»

<400> 372
tcatatgcga cacactcatt 20

<210> 373
<211> 21
<212> DNA
<213> artificial

<220>
<221> source
<222> (1)..(21)
<223> /note=«Description of artificial sequence: Forward primer, MW0746 gene encoding Q8NXK9»

<400> 373
aaatgttgat gttgcagatt t 21

<210> 374

<211> 20
<212> DNA
<213> artificial

<220>
<221> source
<222> (1)..(20)
<223> /note=«Description of artificial sequence: Reverse primer, MW0746 gene encoding Q8NXK9»

<400> 374
ctgggaatga ggaaagaata 20

<210> 375
<211> 20
<212> DNA
<213> artificial

<220>
<221> source
<222> (1)..(20)
<223> /note=«Description of artificial sequence: Forward primer, MW0047 gene encoding Q8VUW0»

<400> 375
cgaacatctt gtaaaggcat 20

<210> 376
<211> 20
<212> DNA
<213> artificial

<220>
<221> source
<222> (1)..(20)
<223> /note=«Description of artificial sequence: Reverse primer, MW0047 gene encoding Q8VUW0»

<400> 376
atgatttctt catcaaaccg 20

<210> 377
<211> 20
<212> DNA
<213> artificial

<220>
<221> source
<222> (1)..(20)
<223> /note=«Description of artificial sequence: Forward primer, MW0043 gene encoding Q8VUV8»

<400> 377
ggtgttgaag atccagagaa 20

<210> 378
<211> 20

<212> DNA
<213> artificial

<220>
<221> source
<222> (1)..(20)
<223> /note=«Description of artificial sequence: Reverse primer, MW0043 gene encoding Q8VUV8»

<400> 378
aatccagttg gctcatattc 20

<210> 379
<211> 20
<212> DNA
<213> artificial

<220>
<221> source
<222> (1)..(20)
<223> /note=«Description of artificial sequence: Forward primer, MW0042 Gene encoding Q8VUV7»

<400> 379

attatgatta gacgcggaac 20

<210> 380
<211> 20
<212> DNA
<213> artificial

<220>
<221> source
<222> (1)..(20)
<223> /note=«Description of artificial sequence: Reverse primer, MW0042 Gene encoding Q8VUV7»

<400> 380
ttgcgttttg tttatttctg 20

<210> 381
<211> 20
<212> DNA
<213> artificial

<220>
<221> source
<222> (1)..(20)
<223> /note=«Description of artificial sequence: Forward primer, SAV1999 gene encoding Q931I9

<400> 381
accaataatc agcgacagtt 20

<210> 382
<211> 20

<212> DNA
<213> artificial

<220>
<221> source
<222> (1)..(20)
<223> /note=«Description of artificial sequence: Reverse primer, SAV1999 gene encoding Q931I9»

<400> 382
gttcattccc tttcaaatca 20

<210> 383
<211> 21
<212> DNA
<213> artificial

<220>
<221> source
<222> (1)..(21)
<223> /note=«Description of artificial sequence: Forward primer, SAV0916* gene encoding Q931V6»

<400> 383
ttttaaaaga ggagggagaa a 21

<210> 384
<211> 20
<212> DNA
<213> artificial

<220>
<221> source
<222> (1)..(20)
<223> /note=«Description of artificial sequence: Reverse primer, SAV0916* gene encoding Q931V6»

<400> 384
aaaactaagt tgcggattga 20

<210> 385
<211> 20
<212> DNA
<213> artificial

<220>
<221> source
<222> (1)..(20)
<223> /note=«Description of artificial sequence: Forward primer, SAV0869 gene encoding Q932A3»

<400> 385
aagcgtttga tttgaaatgt 20

<210> 386
<211> 20
<212> DNA

<213> artificial

<220>
<221> source
<222> (1)..(20)
<223> /note=«Description of artificial sequence: Reverse primer, SAV0869 gene encoding Q932A3»

<400> 386
ggtcatagca gtttggttgt 20

<210> 387
<211> 20
<212> DNA
<213> artificial

<220>
<221> source
<222> (1)..(20)
<223> /note=«Description of artificial sequence: Forward primer, SAV0868 gene encoding Q932A4»

<400> 387
tactgcacag caagtaatgg 20

<210> 388
<211> 20
<212> DNA
<213> artificial

<220>
<221> source
<222> (1)..(20)
<223> /note=«Description of artificial sequence: Reverse primer, SAV0868 gene encoding Q932A4»

<400> 388
ctcgagtacc ttgcatgatt 20

<210> 389
<211> 20
<212> DNA
<213> artificial

<220>
<221> source
<222> (1)..(20)
<223> /note=«Description of artificial sequence: Forward primer, SAV0865 Gene encoding Q932A7»

<400> 389
gttacgaggg catatacgaa 20

<210> 390
<211> 20
<212> DNA
<213> artificial

<220>
<221> source
<222> (1)..(20)
<223> /note=«Description of artificial sequence: Reverse primer, SAV0865 Gene encoding Q932A7»

<400> 390
tctgcctaag aattttcctg 20

<210> 391
<211> 20
<212> DNA
<213> artificial

<220>
<221> source
<222> (1)..(20)
<223> /note=«Description of artificial sequence: Forward primer, SAV0858 Gene encoding Q932B4»

<400> 391
cgtatgttat gaacgctctg 20

<210> 392
<211> 20
<212> DNA
<213> artificial

<220>
<221> source
<222> (1)..(20)
<223> /note=«Description of artificial sequence: Reverse primer, SAV0858 Gene encoding Q932B4»

<400> 392
cggagatgtt actccgatag 20

<210> 393
<211> 20
<212> DNA
<213> artificial

<220>
<221> source
<222> (1)..(20)
<223> /note=«Description of artificial sequence: Forward primer, SAV0849 gene encoding Q932C1»

<400> 393
tcccattcat gagaaaagac 20

<210> 394
<211> 20
<212> DNA
<213> artificial

<220>
<221> source
<222> (1)..(20)
<223> /note=«Description of artificial sequence: Reverse primer, SAV0849 gene encoding Q932C1»

<400> 394
aaatctaagt ggttgaacgg 20

<210> 395
<211> 20
<212> DNA
<213> artificial

<220>
<221> source
<222> (1)..(20)
<223> /note=«Description of artificial sequence: Forward primer, SAV0784 Gene encoding Q932E4»

<400> 395
gatggaagag tttggtgaaa 20

<210> 396
<211> 20
<212> DNA
<213> artificial

<220>
<221> source
<222> (1)..(20)
<223> /note=«Description of artificial sequence: Reverse primer, SAV0784 gene encoding Q932E4»

<400> 396
attcttgcgc atttattgtt 20

<210> 397
<211> 20
<212> DNA
<213> artificial

<220>
<221> source
<222> (1)..(20)
<223> /note=«Description of artificial sequence: Forward primer, SAV0414 Gene encoding Q932H2»

<400> 397
gtgagaatga ttcagggaaa 20

<210> 398
<211> 22
<212> DNA
<213> artificial

<220>

<221> source
<222> (1)..(22)
<223> /note=«Description of artificial sequence: Reverse primer, SAV0414 gene encoding Q932H2»

<400> 398
cattgagtct tcctaccaat tt 22

<210> 399
<211> 20
<212> DNA
<213> artificial

<220>
<221> source
<222> (1)..(20)
<223> /note=«Description of artificial sequence: Forward primer, SAV0413 gene encoding Q932H3»

<400> 399
gaagaaatta aggaacgcct 20

<210> 400
<211> 20
<212> DNA
<213> artificial

<220>
<221> source
<222> (1)..(20)
<223> /note=«Description of artificial sequence: Reverse primer, SAV0413 gene encoding Q932H3»

<400> 400
aattcatcca cataaatcgc 20

<210> 401
<211> 20
<212> DNA
<213> artificial

<220>
<221> source
<222> (1)..(20)
<223> /note=«Description of artificial sequence: Forward primer, SAV0410 Gene encoding Q932H6»

<400> 401
acaaaccata cgcaaactct 20

<210> 402
<211> 20
<212> DNA
<213> artificial

<220>
<221> source

<222> (1)..(20)
<223> /note=«Description of artificial sequence: Reverse primer, SAV0410 gene encoding Q932H6»

<400> 402
tccattgatt ttctcaatcc 20

<210> 403
<211> 20
<212> DNA
<213> artificial

<220>
<221> source
<222> (1)..(20)
<223> /note=«Description of artificial sequence: Forward primer, SAV0409 gene encoding Q932H7»

<400> 403
gaatcaaagc caacgataag 20

<210> 404
<211> 20
<212> DNA
<213> artificial

<220>
<221> source
<222> (1)..(20)
<223> /note=«Description of artificial sequence: Reverse primer, SAV0409 gene encoding Q932H7»

<400> 404
ctaggtgcaa aagctgatct 20

<210> 405
<211> 20
<212> DNA
<213> artificial

<220>
<221> source
<222> (1)..(20)
<223> /note=«Description of artificial sequence: Forward primer, SAV0408 gene encoding Q932H8»

<400> 405
atggcgtttc tcaaaataaa 20

<210> 406
<211> 20
<212> DNA
<213> artificial

<220>
<221> source
<222> (1)..(20)

<223> /note=«Description of artificial sequence: Reverse primer, SAV0408 gene encoding Q932H8»

<400> 406
tatgttccat gcaatcttga 20

<210> 407
<211> 20
<212> DNA
<213> artificial

<220>
<221> source
<222> (1)..(20)
<223> /note=«Description of artificial sequence: Forward primer, SAV0406 Gene encoding Q932I0»

<400> 407
gatttgatta cccagacagc 20

<210> 408
<211> 20
<212> DNA
<213> artificial

<220>
<221> source
<222> (1)..(20)
<223> /note=«Description of artificial sequence: Reverse primer, SAV0406 gene encoding Q932I0»

<400> 408
tgcctatttc ttgcttcatt 20

<210> 409
<211> 20
<212> DNA
<213> artificial

<220>
<221> source
<222> (1)..(20)
<223> /note=«Description of artificial sequence: Forward primer, SAV0405 gene encoding Q932I1»

<400> 409
atgaaggtga attagtcgga 20

<210> 410
<211> 19
<212> DNA
<213> artificial

<220>
<221> source
<222> (1)..(19)
<223> /note=«Description of artificial sequence: Reverse primer,

SAV0405 gene encoding Q932I1»

<400> 410
tatcacgtcc caaagattg 19

<210> 411
<211> 20
<212> DNA
<213> artificial

<220>
<221> source
<222> (1)..(20)
<223> /note=«Description of artificial sequence: Forward primer, SAV0404 gene encoding Q932I2»

<400> 411
aaagataaag gctgggtgat 20

<210> 412
<211> 20
<212> DNA
<213> artificial

<220>
<221> source
<222> (1)..(20)
<223> /note=«Description of artificial sequence: Reverse primer, SAV0404 gene encoding Q932I2»

<400> 412
gctgggtcta tacaatctcg 20

<210> 413
<211> 20
<212> DNA
<213> artificial

<220>
<221> source
<222> (1)..(20)
<223> /note=«Description of artificial sequence: Forward primer, SAV0403 gene encoding Q932I3»

<400> 413
cattgaaaga gcaatcacct 20

<210> 414
<211> 20
<212> DNA
<213> artificial

<220>
<221> source
<222> (1)..(20)
<223> /note=«Description of artificial sequence: Reverse primer, SAV0403 gene encoding Q932I3»

<400> 414
gccatctagt tgttcactcc 20

<210> 415
<211> 20
<212> DNA
<213> artificial

<220>
<221> source
<222> (1)..(20)
<223> /note=«Description of artificial sequence: Forward primer, SAV0402 gene encoding Q932I4»

<400> 415
aaaaagaaat cactggacga 20

<210> 416
<211> 20
<212> DNA
<213> artificial

<220>
<221> source
<222> (1)..(20)
<223> /note=«Description of artificial sequence: Reverse primer, SAV0402 gene encoding Q932I4»

<400> 416
ttcgtatcgt ttcggtaaat 20

<210> 417
<211> 20
<212> DNA
<213> artificial
«

<400> 417
tgtgggcgtt attttacttt 20

<210> 418
<211> 20
<212> DNA
<213> artificial

<220>
<221> source
<222> (1)..(20)
<223> /note=«Description of artificial sequence: Reverse primer, SAV0402 gene encoding Q932I4»

<400> 418
acagctcgta tcactttcgt 20

<210> 419

<211> 20
<212> DNA
<213> artificial

<220>
<221> source
<222> (1)..(20)
<223> /note=«Description of artificial sequence: Forward primer, GSAV0400 gene encoding Q932I6»

<400> 419
cagaacaaca aggagtggat 20

<210> 420
<211> 20
<212> DNA
<213> artificial

<220>
<221> source
<222> (1)..(20)
<223> /note=«Description of artificial sequence: Reverse primer, GSAV0400 gene encoding Q932I6»

<400> 420
tccatagaga tttcttgcgt 20

<210> 421
<211> 20
<212> DNA
<213> artificial

<220>
<221> source
<222> (1)..(20)
<223> /note=«Description of artificial sequence: Forward primer, SAV0399 gene encoding Q932I7»

<400> 421
gtccgtgaaa cagaaatcat 20

<210> 422
<211> 20
<212> DNA
<213> artificial

<220>
<221> source
<222> (1)..(20)
<223> /note=«Description of artificial sequence: Reverse primer, SAV0399 gene encoding Q932I7»

<400> 422
ctagctgttt gggttggtag 20

<210> 423
<211> 20

<212> DNA
<213> artificial

<220>
<221> source
<222> (1)..(20)
<223> /note=«Description of artificial sequence: Forward primer, SAV0395 gene encoding Q932J0»

<400> 423
gggtttagt atgaagggag 20

<210> 424
<211> 20
<212> DNA
<213> artificial

<220>
<221> source
<222> (1)..(20)
<223> /note=«Description of artificial sequence: Reverse primer, SAV0395 gene encoding Q932J0»

<400> 424
aaacagtcga acgattcaaa 20

<210> 425
<211> 20
<212> DNA
<213> artificial

<220>
<221> source
<222> (1)..(20)
<223> /note=«Description of artificial sequence: Forward primer, blaZ gene encoding Beta-lactamase»

<400> 425
taaaagaaat cggtggaatc 20

<210> 426
<211> 20
<212> DNA
<213> artificial

<220>
<221> source
<222> (1)..(20)
<223> /note=«Description of artificial sequence: Reverse primer, blaZ gene encoding Beta-lactamase»

<400> 426
tccttcatta cactcttggc 20

<210> 427
<211> 20
<212> DNA

<213> artificial

<220>
<221> source
<222> (1)..(20)
<223> /note=«Description of artificial sequence: Forward primer, ccrB1gene encoding SSCmecI Recombinase B1»

<400> 427
ttgtaggagg ctacattcgt 20

<210> 428
<211> 20
<212> DNA
<213> artificial

<220>
<221> source
<222> (1)..(20)
<223> /note=«Description of artificial sequence: Reverse primer, ccrB1gene encoding SSCmecI Recombinase B1»

<400> 428
atggaagatt gccttgataa 20

<210> 429
<211> 20
<212> DNA
<213> artificial

<220>
<221> source
<222> (1)..(20)
<223> /note=«Description of artificial sequence: Forward primer, ccrA1 gene encoding SSCmecI Recombinase A1»

<400> 429
ctcaagttac ccgaactcag 20

<210> 430
<211> 20
<212> DNA
<213> artificial

<220>
<221> source
<222> (1)..(20)
<223> /note=«Description of artificial sequence: Reverse primer,ccrA1 gene encoding SSCmecI Recombinase A1»

<400> 430
ttgttgaatg atgttttgga 20

<210> 431
<211> 20
<212> DNA
<213> artificial

<220>
<221> source
<222> (1)..(20)
<223> /note=«Description of artificial sequence: Forward primer, SA1633gene encoding Beta-lactamase Q99T58»

<400> 431
aaaacagcaa aagcagaaga 20

<210> 432
<211> 21
<212> DNA
<213> artificial

<220>
<221> source
<222> (1)..(21)
<223> /note=«Description of artificial sequence: Reverse primer, SA1633gene encoding Beta-lactamase Q99T58»

<400> 432
ctgctcctac tttacctgaa a 21

<210> 433
<211> 20
<212> DNA
<213> artificial

<220>
<221> source
<222> (1)..(20)
<223> /note=«Description of artificial sequence: Forward primer, seh* gene encoding Enterotoxin H»

<400> 433
ggtgatagtg gcaatgattt 20

<210> 434
<211> 22
<212> DNA
<213> artificial

<220>
<221> source
<222> (1)..(22)
<223> /note=«Description of artificial sequence: Reverse primer, seh* gene encoding Enterotoxin H»

<400> 434
tccttttaaa tcataaatgt cg 22

<210> 435
<211> 20
<212> DNA
<213> artificial

<220>
<221> source
<222> (1)..(20)
<223> /note=«Description of artificial sequence: Forward primer, tst gene encoding Toxic shock syndrome toxin-1»

<400> 435
aatcaaaact gcaaaagcat 20

<210> 436
<211> 20
<212> DNA
<213> artificial

<220>
<221> source
<222> (1)..(20)
<223> /note=«Description of artificial sequence: Reverse primer, tst gene encoding Toxic shock syndrome toxin-1»

<400> 436
ttgagttagc tgatgacgaa 20

<210> 437
<211> 20
<212> DNA
<213> artificial

<220>
<221> source
<222> (1)..(20)
<223> /note=«Description of artificial sequence: Forward primer, acoa-N315* gene encoding Staphylocoagulase (N315 variable domain)»

<400> 437
catgggataa caaagcagat 20

<210> 438
<211> 20
<212> DNA
<213> artificial

<220>
<221> source
<222> (1)..(20)
<223> /note=«Description of artificial sequence: Reverse primer, acoa-N315* gene encoding Staphylocoagulase (N315 variable domain)»

<400> 438
caaactgctt caagtcaaca 20

<210> 439
<211> 23
<212> DNA
<213> artificial

<220>
<221> source
<222> (1)..(23)
<223> /note=«Description of artificial sequence: Forward primer, coa-8325 Gene encoding Staphylocoagulase (NCTC8325 variable domain)»

<400> 439
aaatgggaca ttaatagata gca 23

<210> 440
<211> 21
<212> DNA
<213> artificial

<220>
<221> source
<222> (1)..(21)
<223> /note=«Description of artificial sequence: Reverse primer, coa-8325 Gene encoding Staphylocoagulase (NCTC8325 variable domain)»

<400> 440
tcttctgctg cattaaaagt t 21

<210> 441
<211> 20
<212> DNA
<213> artificial

<220>
<221> source
<222> (1)..(20)
<223> /note=«Description of artificial sequence: Forward primer, ami 8325 gene encoding Putative amidase from bacteriophage phi 11»

<400> 441
ttgaaaactt ctgagggaaa 20

<210> 442
<211> 20
<212> DNA
<213> artificial

<220>
<221> source
<222> (1)..(20)
<223> /note=«Description of artificial sequence: Reverse primer, ami 8325 gene encoding Putative amidase from bacteriophage phi 11»

<400> 442
tttaaaattc ggacggataa 20

<210> 443

<211> 20
<212> DNA
<213> artificial

<220>
<221> source
<222> (1)..(20)
<223> /note=«Description of artificial sequence: Forward primer, agrB-N315 gene encoding AgrB (N315 variable domain)»

<400> 443
acgtttaggg atgcaaatta 20

<210> 444
<211> 20
<212> DNA
<213> artificial

<220>
<221> source
<222> (1)..(20)
<223> /note=«Description of artificial sequence: Reverse primer, agrB-N315 gene encoding AgrB (N315 variable domain)»

<400> 444

taatcctcct tagggaaaaa 20

<210> 445
<211> 20
<212> DNA
<213> artificial

<220>
<221> source
<222> (1)..(20)
<223> /note=«Description of artificial sequence: Forward primer, agrC-8325 gene encoding AgrC (NCTC8325 variable domain)»

<400> 445
atagcgcgtc cttaatcata 20

<210> 446
<211> 23
<212> DNA
<213> artificial

<220>
<221> source
<222> (1)..(23)
<223> /note=«Description of artificial sequence: Reverse primer, agrC-8325 gene encoding AgrC (NCTC8325 variable domain)»

<400> 446
aaggagaaat tgagaaataa caa 23

<210> 447

<211> 20
<212> DNA
<213> artificial

<220>
<221> source
<222> (1)..(20)
<223> /note=«Description of artificial sequence: Forward primer, agrC-N315* gene encoding AgrC (N315 variable domain)»

<400> 447
atggaagtag agccgtattg 20

<210> 448
<211> 20
<212> DNA
<213> artificial

<220>
<221> source
<222> (1)..(20)
<223> /note=«Description of artificial sequence: Reverse primer, agrC-N315* gene encoding AgrC (N315 variable domain)»

<400> 448
tctctttaag ggtgaaaagc 20

<210> 449
<211> 20
<212> DNA
<213> artificial

<220>
<221> source
<222> (1)..(20)
<223> /note=«Description of artificial sequence: Forward primer, tnp 480 (Tn552) gene encoding Tn552 Tnp 480 transposase»

<400> 449
tgaagccaaa cgtaaagaag 20

<210> 450
<211> 20
<212> DNA
<213> artificial

<220>
<221> source
<222> (1)..(20)
<223> /note=«Description of artificial sequence: Reverse primer, tnp 480 (Tn552) gene encoding Tn552 Tnp 480 transposase»

<400> 450
tatttcattg ggtcttgagg 20

<210> 451
<211> 20

<212> DNA
<213> artificial

<220>
<221> source
<222> (1)..(20)
<223> /note=«Description of artificial sequence: Forward primer, tnp IS 1272 gene encoding IS 1272 transposase»

<400> 451
ggattctaaa gccctctacc 20

<210> 452
<211> 20
<212> DNA
<213> artificial

<220>
<221> source
<222> (1)..(20)
<223> /note=«Description of artificial sequence: Reverse primer, tnp IS 1272 gene encoding IS 1272 transposase»

<400> 452
ttgggaattt gttgctattt 20

<210> 453
<211> 21
<212> DNA
<213> artificial

<220>
<221> source
<222> (1)..(21)
<223> /note=«Description of artificial sequence: Forward primer, SA1818 gene encoding Tn557 putative protein»

<400> 453
ggttcaggtt ttcattcttc t 21

<210> 454
<211> 22
<212> DNA
<213> artificial

<220>
<221> source
<222> (1)..(22)
<223> /note=«Description of artificial sequence: Reverse primer, SA1818 gene encoding Tn557 putative protein»

<400> 454
cagtctcatc tttagggtct gt 22

<210> 455
<211> 21
<212> DNA

<213> artificial

<220>
<221> source
<222> (1)..(21)
<223> /note=«Description of artificial sequence: Forward primer, ORF12 gene encoding Na/K ATPase from phage phi 12, 13 Q8SBD6»

<400> 455
agatgactca atttctaggg g 21

<210> 456
<211> 20
<212> DNA
<213> artificial

<220>
<221> source
<222> (1)..(20)
<223> /note=«Description of artificial sequence: Reverse primer, ORF12 gene encoding Na/K ATPase from phage phi 12, 13 Q8SBD6»

<400> 456
ttcaccctct tcaatgattc 20

<210> 457
<211> 20
<212> DNA
<213> artificial

<220>

<223> /note=«Description of artificial sequence: PRIMER

<220>
<221> source
<222> (1)..(20)
<223> /note=«Description of artificial sequence: Forward primer, ORF13 gene encoding Small terminase from phage phi 11 Q8SDV0»

<400> 457
tcgcaattag gaataaagga 20

<210> 458
<211> 20
<212> DNA
<213> artificial

<220>
<221> source
<222> (1)..(20)
<223> /note=«Description of artificial sequence: Reverse primer, ORF13 gene encoding Small terminase from phage phi 11 Q8SDV0»

<400> 458
caaaagttgg tgtgattgtg 20

<210> 459
<211> 20
<212> DNA
<213> artificial

<220>
<221> source
<222> (1)..(20)
<223> /note=«Description of artificial sequence: Forward primer, ORF14 gene encoding Terminase from phage phi 11 Q8SDU9»

<400> 459
ggtgagtacg atgacgaaag 20

<210> 460
<211> 20
<212> DNA
<213> artificial

<220>
<221> source
<222> (1)..(20)
<223> /note=«Description of artificial sequence: Reverse primer, ORF14 gene encoding Terminase from phage phi 11 Q8SDU9»

<400> 460
cgcttcttcc atgactatgt 20

<210> 461
<211> 20
<212> DNA
<213> artificial

<220>
<221> source
<222> (1)..(20)
<223> /note=«Description of artificial sequence: Forward primer, ORF16 gene encoding Helicase DnaB from phage phi 11 Q8SDW1»

<400> 461
aattatgtaa tggacgtcgg 20

<210> 462
<211> 20
<212> DNA
<213> artificial

<220>
<221> source
<222> (1)..(20)
<223> /note=«Description of artificial sequence: Reverse primer, ORF16 gene encoding Helicase DnaB from phage phi 11 Q8SDW1»

<400> 462
tccattttgt gctatgttca 20

<210> 463
<211> 20
<212> DNA
<213> artificial

<220>
<221> source
<222> (1)..(20)
<223> /note=«Description of artificial sequence: Forward primer, ORF25 gene encoding Hypothetical protein from phage phi 11 Q8SDT8»

<400> 463
cggatatttt aacggagttg 20

<210> 464
<211> 20

<212> DNA
<213> artificial

<220>
<221> source
<222> (1)..(20)
<223> /note=«Description of artificial sequence: Reverse primer, ORF25 gene encoding Hypothetical protein from phage phi 11 Q8SDT8»

<400> 464
tcatcacgtt cgctactaaa 20

<210> 465
<211> 20
<212> DNA
<213> artificial

<220>
<221> source
<222> (1)..(20)
<223> /note=«Description of artificial sequence: Forward primer, ORF28 gene encoding Hypothetical protein from plasmid pSV41 O87359»

<400> 465
aagctagtga gcgtaaatgc 20

<210> 466
<211> 20
<212> DNA
<213> artificial

<220>
<221> source

<222> (1)..(20)
<223> /note=«Description of artificial sequence: Reverse primer, ORF28 gene encoding Hypothetical protein from plasmid pSV41 O87359»

<400> 466
taagcacgct caagagaaat 20

<210> 467
<211> 20
<212> DNA
<213> artificial

<220>
<221> source
<222> (1)..(20)
<223> /note=«Description of artificial sequence: Forward primer, ORF29 gene encoding Putative ATP/GTP-binding protein Q935V0»

<400> 467
caatatcata gtgcgagcaa 20

<210> 468
<211> 20
<212> DNA
<213> artificial

<220>
<221> source
<222> (1)..(20)
<223> /note=«Description of artificial sequence: Reverse primer, ORF29 gene encoding Putative ATP/GTP-binding protein Q935V0»

<400> 468
ggctaattct gtttttccaa 20

<210> 469
<211> 20
<212> DNA
<213> artificial

<220>
<221> source
<222> (1)..(20)
<223> /note=«Description of artificial sequence: Forward primer, ORF34* gene encoding Q9RL82»

<400> 469
ttgcaatact gtggtgaaaa 20

<210> 470
<211> 20
<212> DNA
<213> artificial

```
<220>
<221> source
<222> (1)..(20)
<223> /note=«Description of artificial sequence: Reverse primer,
      ORF34* gene encoding Q9RL82»

<400> 470
tgctacgtat ccatcttcct                                                20


<210> 471
<211> 20
<212> DNA
<213> artificial

<220>
<221> source
<222> (1)..(20)
<223> /note=«Description of artificial sequence: Forward primer,
      ORF35 gene encoding Putative protein»

<400> 471
ggattttgtg agagcttgaa                                                20


<210> 472
<211> 21
<212> DNA
<213> artificial

<220>
<221> source
<222> (1)..(21)
<223> /note=«Description of artificial sequence: Reverse primer,
      ORF35 gene encoding Putative protein»

<400> 472
gcttggattt gttattcctt t                                              21


<210> 473
<211> 20
<212> DNA
<213> artificial

<220>
<221> source
<222> (1)..(20)
<223> /note=«Description of artificial sequence: Forward primer,
      ORF36 gene encoding Putative protein»

<400> 473
tggaattgaa agtcgatgat                                                20


<210> 474
<211> 20
<212> DNA
<213> artificial

<220>
```

<221> source
<222> (1)..(20)
<223> /note=«Description of artificial sequence: Reverse primer, ORF36 gene encoding Putative protein»

<400> 474
ggcattaact gcaggattag 20

<210> 475
<211> 20
<212> DNA
<213> artificial

<220>
<221> source
<222> (1)..(20)
<223> /note=«Description of artificial sequence: Forward primer, ORF38 gene encoding Q935U9»

<400> 475
aaagtgaaca tggaatttgg 20

<210> 476
<211> 20
<212> DNA
<213> artificial

<220>
<221> source
<222> (1)..(20)
<223> /note=«Description of artificial sequence: Reverse primer, ORF38 gene encoding Q935U9»

<400> 476
tctgttttca ttccctcttg 20

<210> 477
<211> 20
<212> DNA
<213> artificial

<220>
<221> source
<222> (1)..(20)
<223> /note=«Description of artificial sequence: Forward primer, ORF39 gene encoding Putative protein»

<400> 477
atacacgcct attattccga 20

<210> 478
<211> 20
<212> DNA
<213> artificial

<220>
<221> source

<222> (1)..(20)
<223> /note=«Description of artificial sequence: Reverse primer, ORF39 gene encoding Putative protein»

<400> 478
cccatcttgc acctaaagta 20

<210> 479
<211> 24
<212> DNA
<213> artificial

<220>
<221> source
<222> (1)..(24)
<223> /note=«Description of artificial sequence: Forward primer, ORF40 gene encoding Putative protein»

<400> 479
agaggaggaa taatagtgga tatg 24

<210> 480
<211> 21
<212> DNA
<213> artificial

<220>
<221> source
<222> (1)..(21)
<223> /note=«Description of artificial sequence: Reverse primer, ORF40 gene encoding Putative protein»

<400> 480
catttcatca tcatcaaaga c 21

<210> 481
<211> 21
<212> DNA
<213> artificial

<220>
<221> source
<222> (1)..(21)
<223> /note=«Description of artificial sequence: Forward primer, SA2012 gene encoding Q99S58»

<400> 481
tcggaaaatt tctaagtcaa a 21

<210> 482
<211> 20
<212> DNA
<213> artificial

<220>
<221> source
<222> (1)..(20)

<223> /note=«Description of artificial sequence: Reverse primer, SA2012 gene encoding Q99S58»

<400> 482
attgcatcaa taaagtcgct 20

<210> 483
<211> 21
<212> DNA
<213> artificial

<220>
<221> source
<222> (1)..(21)
<223> /note=«Description of artificial sequence: Forward primer, SA2011 gene encoding Q99S59»

<400> 483
ctccaaataa tacactgagc c 21

<210> 484
<211> 20
<212> DNA
<213> artificial

<220>
<221> source
<222> (1)..(20)
<223> /note=«Description of artificial sequence: Reverse primer, SA2011 gene encoding Q99S59»

<400> 484
ttctgctgat gtaatccgtt 20

<210> 485
<211> 20
<212> DNA
<213> artificial
«

<400> 485
tggagaaaat tgagaatgaa 20

<210> 486
<211> 20
<212> DNA
<213> artificial

<220>
<221> source
<222> (1)..(20)
<223> /note=«Description of artificial sequence: Reverse primer, SA2011 gene encoding Q99S59»

<400> 486
atatatggac tggttggtgc 20

<210> 487
<211> 20
<212> DNA
<213> artificial

<220>
<221> source
<222> (1)..(20)
<223> /note=«Description of artificial sequence: Forward primer, SA1834 gene encoding Q99SL9»

<400> 487
cgtgttgcaa aagatgtaaa 20

<210> 488
<211> 20
<212> DNA
<213> artificial

<220>
<221> source
<222> (1)..(20)
<223> /note=«Description of artificial sequence: Reverse primer, SA1834 gene encoding Q99SL9»

<400> 488
gtttttaagg cctgcagata 20

<210> 489
<211> 20
<212> DNA
<213> artificial

<220>
<221> source
<222> (1)..(20)
<223> /note=«Description of artificial sequence: Forward primer, SA1640 gene encoding Q99T51»

<400> 489
ggagataaca gcatgaggag 20

<210> 490
<211> 20
<212> DNA
<213> artificial

<220>
<221> source
<222> (1)..(20)
<223> /note=«Description of artificial sequence: Reverse primer, SA1640 gene encoding Q99T51»

<400> 490
tttggtcgct attttcttgt 20

<210> 491
<211> 20
<212> DNA
<213> artificial

<220>
<221> source
<222> (1)..(20)
<223> /note=«Description of artificial sequence: Forward primer, SA1636 gene encoding Q99T55»

<400> 491
cctatcgtcg attattttgg 20

<210> 492
<211> 20
<212> DNA
<213> artificial

<220>
<221> source
<222> (1)..(20)
<223> /note=«Description of artificial sequence: Reverse primer, SA1636 gene encoding Q99T55»

<400> 492
ctctgtagct tctcctggaa 20

<210> 493
<211> 20
<212> DNA
<213> artificial

<220>
<221> source
<222> (1)..(20)
<223> /note=«Description of artificial sequence: Forward primer, SA1635 gene encoding Q99T56»

<400> 493
tttgaaactg aacgatgaag 20

<210> 494
<211> 21
<212> DNA
<213> artificial

<220>
<221> source
<222> (1)..(21)
<223> /note=«Description of artificial sequence: Reverse primer, SA1635 gene encoding Q99T56»

<400> 494
acaaaatcag agctatcgtc a 21

<210> 495

<211> 20
<212> DNA
<213> artificial

<220>
<221> source
<222> (1)..(20)
<223> /note=«Description of artificial sequence: Forward primer, MW2292 gene encoding Q8NV38»

<400> 495
agttttacaa tggacgcaaa 20

<210> 496
<211> 20
<212> DNA
<213> artificial

<220>
<221> source
<222> (1)..(20)
<223> /note=«Description of artificial sequence: Reverse primer, MW2292 gene encoding Q8NV38»

<400> 496
aggattattt cttgcagggt 20

<210> 497
<211> 20
<212> DNA
<213> artificial

<220>
<221> source
<222> (1)..(20)
<223> /note=«Description of artificial sequence: Forward primer, MW1197 gene encoding Q8NWX1»

<400> 497
agatgacaca accaaagatg 20

<210> 498
<211> 22
<212> DNA
<213> artificial

<220>
<221> source
<222> (1)..(22)
<223> /note=«Description of artificial sequence: Reverse primer, MW1197 gene encoding Q8NWX1»

<400> 498
tgatttgcaa tattcttttg tc 22

<210> 499
<211> 23

<212> DNA
<213> artificial

<220>
<221> source
<222> (1)..(23)
<223> /note=«Description of artificial sequence: Forward primer, MW0758 gene encoding Q8NXJ7»

<400> 499
ggttttcatt cttcttcaaa tta 23

<210> 500
<211> 18
<212> DNA
<213> artificial

<220>
<221> source
<222> (1)..(18)
<223> /note=«Description of artificial sequence: Reverse primer, MW0758 gene encoding Q8NXJ7»

<400> 500
tagctgaaat tgggggat 18

<210> 501
<211> 20
<212> DNA
<213> artificial

<220>
<221> source
<222> (1)..(20)
<223> /note=«Description of artificial sequence: Forward primer, MW0379* gene encoding Q8NY57»

<400> 501
ggaaaagtta cagcgaaaga 20

<210> 502
<211> 20
<212> DNA
<213> artificial

<220>
<221> source
<222> (1)..(20)
<223> /note=«Description of artificial sequence: Reverse primer, MW0379* gene encoding Q8NY57»

<400> 502
ggtacaaaat cgctatcacc 20

<210> 503
<211> 20
<212> DNA

<213> artificial

<220>
<221> source
<222> (1)..(20)
<223> /note=«Description of artificial sequence: Forward primer, MW0378* gene encoding Q8NY58»

<400> 503
cgtgtttgat aggcgataat 20

<210> 504
<211> 20
<212> DNA
<213> artificial

<220>
<221> source
<222> (1)..(20)
<223> /note=«Description of artificial sequence: Reverse primer, MW0378* gene encoding Q8NY58»

<400> 504
cgtttcattc atgttttggt 20

<210> 505
<211> 20
<212> DNA
<213> artificial

<220>
<221> source
<222> (1)..(20)
<223> /note=«Description of artificial sequence: Forward primer, ORF44* Gene encoding Putative protein»

<400> 505
aatttaataa aggacaacgc 20

<210> 506
<211> 20
<212> DNA
<213> artificial

<220>
<221> source
<222> (1)..(20)
<223> /note=«Description of artificial sequence: Reverse primer, ORF44* Gene encoding Putative protein»

<400> 506
ttatttcgcc agtttctttg 20

<210> 507
<211> 20
<212> DNA
<213> artificial

<220>
<221> source
<222> (1)..(20)
<223> /note=«Description of artificial sequence: Forward primer, MW0374* gene encoding Q8NY62»

<400> 507
caagaatttt tctcaacgct 20

<210> 508
<211> 20
<212> DNA
<213> artificial

<220>
<221> source
<222> (1)..(20)
<223> /note=«Description of artificial sequence: Reverse primer, MW0374* gene encoding Q8NY62»

<400> 508
ccttcaaaga agatacacgg 20

<210> 509
<211> 20
<212> DNA
<213> artificial

<220>
<221> source
<222> (1)..(20)
<223> /note=«Description of artificial sequence: Forward primer, MW0372* Q8NY65»

<400> 509
gtcttgactg ccgttttatt 20

<210> 510
<211> 20
<212> DNA
<213> artificial

<220>
<221> source
<222> (1)..(20)
<223> /note=«Description of artificial sequence: Reverse primer, MW0372* gene encoding Q8NY65»

<400> 510
gcaatggctt ctttgactac 20

<210> 511
<211> 20
<212> DNA
<213> artificial

<220>
<221> source
<222> (1)..(20)
<223> /note=«Description of artificial sequence: Forward primer, MW0368* Gene encoding Q8NY68»

<400> 511
ttctgccaaa aagctaaaag 20

<210> 512
<211> 23
<212> DNA
<213> artificial

<220>
<221> source
<222> (1)..(23)
<223> /note=«Description of artificial sequence: Reverse primer, MW0368* Gene encoding Q8NY68»

<400> 512
aaaaatgtaa ctaacctttg ctg 23

<210> 513
<211> 20
<212> DNA
<213> artificial

<220>
<221> source
<222> (1)..(20)
<223> /note=«Description of artificial sequence: Forward primer, MW0074 gene encoding Q8NYT9»

<400> 513
gtgggaattt tcctaaaaac 20

<210> 514
<211> 20
<212> DNA
<213> artificial

<220>
<221> source
<222> (1)..(20)
<223> /note=«Description of artificial sequence: Reverse primer, MW0074 gene encoding Q8NYT9»

<400> 514
gatgttataa cgaagcccaa 20

<210> 515
<211> 20
<212> DNA

<213> artificial

<220>
<221> source
<222> (1)..(20)
<223> /note=«Description of artificial sequence: Forward primer,
MW0045 Gene encoding Q8VUV9»

<400> 515
tactcatgca gctgaaacac 20

<210> 516
<211> 20
<212> DNA
<213> artificial

<220>
<221> source
<222> (1)..(20)
<223> /note=«Description of artificial sequence: Reverse primer,
MW0045 gene encoding gene encoding Q8VUV9»

<400> 516
atagccaagc gtagatgttg 20

<210> 517
<211> 20
<212> DNA
<213> artificial

<220>
<221> source
<222> (1)..(20)
<223> /note=«Description of artificial sequence: Forward primer,
SAV2596 gene encoding Q931E7»

<400> 517
gatttccagg agacaagtca 20

<210> 518
<211> 22
<212> DNA
<213> artificial

<220>
<221> source
<222> (1)..(22)
<223> /note=«Description of artificial sequence: Reverse primer,
SAV2596 gene encoding Q931E7»

<400> 518
tcattgctat taaagtaacc ca 22

<210> 519
<211> 20
<212> DNA
<213> artificial

<220>
<221> source
<222> (1)..(20)
<223> /note=«Description of artificial sequence: Forward primer, SAV2025 Gene encoding Q931I0»

<400> 519
gaggcataaa ccaatgttca 20

<210> 520
<211> 20
<212> DNA
<213> artificial

<220>
<221> source
<222> (1)..(20)
<223> /note=«Description of artificial sequence: Reverse primer, SAV2025 gene encoding Q931I0»

<400> 520
atgaacgcta aaagcaacat 20

<210> 521
<211> 20
<212> DNA
<213> artificial

<220>
<221> source
<222> (1)..(20)
<223> /note=«Description of artificial sequence: Forward primer, SAV2001 gene encoding Q931I8»

<400> 521
aagcaaagga gaaatgagaa 20

<210> 522

<211> 20
<212> DNA
<213> artificial

<220>
<221> source
<222> (1)..(20)
<223> /note=«Description of artificial sequence: Reverse primer, SAV2001 gene encoding Q931I8»

<400> 522
cttcaatttg tttttgagcc 20

<210> 523
<211> 20
<212> DNA
<213> artificial

<220>
<221> source
<222> (1)..(20)
<223> /note=«Description of artificial sequence: Forward primer, SAV0912* gene encoding Q931W0»

<400> 523
aaggagcaaa caaatggata 20

<210> 524
<211> 20
<212> DNA
<213> artificial

<220>
<221> source
<222> (1)..(20)
<223> /note=«Description of artificial sequence: Reverse primer, SAV0912* gene encoding Q931W0»

<400> 524
attcacatca aagccaacat 20

<210> 525
<211> 20
<212> DNA
<213> artificial

<220>
<221> source
<222> (1)..(20)
<223> /note=«Description of artificial sequence: Forward primer, SAV0910* gene encoding Q931W2»

<400> 525
tttgtttatg gaagatggct 20

<210> 526
<211> 20
<212> DNA
<213> artificial

<220>
<221> Reverse
<222> (1)..(20)
<223> /note=«Description of artificial sequence: Reverse primer, SAV0910* gene encoding Q931W2»

<400> 526
atcaattcgc cacattttag 20

<210> 527
<211> 20
<212> DNA
<213> artificial

<220>
<221> source
<222> (1)..(20)
<223> /note=«Description of artificial sequence: Forward primer, SAV0909* gene encoding Q931W3»

<400> 527
gatgctatgg ctcaaaaatc 20

<210> 528
<211> 20
<212> DNA
<213> artificial

<220>
<221> source
<222> (1)..(20)
<223> /note=«Description of artificial sequence: Reverse primer, SAV0909* gene encoding Q931W3»

<400> 528
atcgtttgtg cgtttctaat 20

<210> 529
<211> 20
<212> DNA
<213> artificial

<220>
<221> source
<222> (1)..(20)
<223> /note=«Description of artificial sequence: Forward primer, SAV0905* gene encoding Q931W7»

<400> 529
tgcgatgact agtattgtgc 20

<210> 530
<211> 20
<212> DNA
<213> artificial

<220>
<221> source
<222> (1)..(20)
<223> /note=«Description of artificial sequence: Reverse primer, SAV0905* gene encoding Q931W7»

<400> 530
aatcatctgt accgcttcat 20

<210> 531
<211> 20
<212> DNA
<213> artificial

<220>
<221> source
<222> (1)..(20)
<223> /note=«Description of artificial sequence: Forward primer, SAV0904* gene encoding Q931W8»

<400> 531
acggatagaa aaggaggaag 20

<210> 532
<211> 20
<212> DNA
<213> artificial

<220>
<221> source
<222> (1)..(20)
<223> /note=«Description of artificial sequence: Reverse primer, SAV0904* gene encoding Q931W8»

<400> 532
tcggtattct gtagctcgat 20

<210> 533
<211> 20
<212> DNA
<213> artificial

<220>
<221> source
<222> (1)..(20)
<223> /note=«Description of artificial sequence: Forward primer, SAV0903 gene encoding Q931W9»

<400> 533
aaggtgaaaa cgacaagaaa 20

<210> 534
<211> 20
<212> DNA
<213> artificial

<220>
<221> source
<222> (1)..(20)
<223> /note=«Description of artificial sequence: Reverse primer, SAV0903 gene encoding Q931W9»

<400> 534
tatcctctaa tgaaggtggc 20

<210> 535
<211> 20
<212> DNA
<213> artificial

<220>

<221> source
<222> (1)..(20)
<223> /note=«Description of artificial sequence: Forward primer, SAV0902 gene encoding Q931X0»

<400> 535
gttccttgtt tttgtgtggt 20

<210> 536
<211> 20
<212> DNA
<213> artificial

<220>
<221> source
<222> (1)..(20)
<223> /note=«Description of artificial sequence: Reverse primer, SAV0902 gene encoding Q931X0»

<400> 536
acctttgctc aaattttcaa 20

<210> 537
<211> 20
<212> DNA
<213> artificial

<220>
<221> source
<222> (1)..(20)
<223> /note=«Description of artificial sequence: Forward primer, SAV0901 gene encoding Q931X1»

<400> 537
gaggtgttaa tgtgacggaa 20

<210> 538
<211> 21
<212> DNA
<213> artificial

<220>
<221> source
<222> (1)..(21)
<223> /note=«Description of artificial sequence: Reverse primer, SAV0901 gene encoding Q931X1»

<400> 538
ttactgttat cagcattgac g 21

<210> 539
<211> 20
<212> DNA
<213> artificial

<220>
<221> source

<222> (1)..(20)
<223> /note=«Description of artificial sequence: Forward primer, SAV0898 gene encoding Q931X4»

<400> 539
ttttgtttag aaaagctggc 20

<210> 540
<211> 20
<212> DNA
<213> artificial

<220>
<221> source
<222> (1)..(20)
<223> /note=«Description of artificial sequence: Reverse primer, SAV0898 gene encoding Q931X4»

<400> 540
atctgtatcc agtcgcctta 20

<210> 541
<211> 20
<212> DNA
<213> artificial

<220>
<221> source
<222> (1)..(20)
<223> /note=«Description of artificial sequence: Forward primer, SAV0890 gene encoding Q931Y2»

<400> 541
ataacaacga tgatgaaggc 20

<210> 542
<211> 20
<212> DNA
<213> artificial

<220>
<221> source
<222> (1)..(20)
<223> /note=«Description of artificial sequence: Reverse primer, SAV0890 gene encoding Q931Y2»

<400> 542
aaatgaatct ccaccagtca 20

<210> 543
<211> 20
<212> DNA
<213> artificial

<220>
<221> source
<222> (1)..(20)

<223> /note=«Description of artificial sequence: Forward primer, SAV0888 gene encoding Q931Y4»

<400> 543
tatgatgatt aagcgcattg 20

<210> 544
<211> 20
<212> DNA
<213> artificial

<220>
<221> source
<222> (1)..(20)
<223> /note=«Description of artificial sequence: Reverse primer, SAV0888 gene encoding Q931Y4»

<400> 544
cattgcttcg gtattagtcc 20

<210> 545
<211> 20
<212> DNA
<213> artificial

<220>
<221> source
<222> (1)..(20)
<223> /note=«Description of artificial sequence: Forward primer, SAV0887 Gene encoding Q931Y5»

<400> 545
cagatacaga tctacgggga 20

<210> 546
<211> 21
<212> DNA
<213> artificial

<220>
<221> source
<222> (1)..(21)
<223> /note=«Description of artificial sequence: Reverse primer, SAV0887 Gene encoding Q931Y5»

<400> 546
gcatcactct tgtataaacg c 21

<210> 547
<211> 20
<212> DNA
<213> artificial

<220>
<221> source
<222> (1)..(20)
<223> /note=«Description of artificial sequence: Forward primer,

ORF45 gene encoding Putative protein»

<400> 547
atggtcatat gcacatcaag 20

<210> 548
<211> 20
<212> DNA
<213> artificial

<220>
<221> source
<222> (1)..(20)
<223> /note=«Description of artificial sequence: Reverse primer, ORF45 gene encoding Putative protein»

<400> 548
tttttagcca tctcatcgtt 20

<210> 549
<211> 20
<212> DNA
<213> artificial

<220>
<221> source
<222> (1)..(20)
<223> /note=«Description of artificial sequence: Forward primer, SAV0801 gene encoding Q932D1»

<400> 549
tcgggttatg gctactttta 20

<210> 550
<211> 20
<212> DNA
<213> artificial

<220>
<221> source
<222> (1)..(20)
<223> /note=«Description of artificial sequence: Reverse primer, SAV0801 gene encoding Q932D1»

<400> 550
aaccatttc acgatgctat 20

<210> 551
<211> 20
<212> DNA
<213> artificial

<220>
<221> source

<222> (1)..(20)
<223> /note=«Description of artificial sequence: Forward primer, SAV0792 gene encoding Q932D6»

<400> 551
gaaatgggac acctatcaaa 20

<210> 552
<211> 20
<212> DNA
<213> artificial

<220>
<221> source
<222> (1)..(20)
<223> /note=«Description of artificial sequence: Reverse primer, SAV0792 gene encoding Q932D6»

<400> 552
gacacactcc gtttctttct 20

<210> 553
<211> 20
<212> DNA
<213> artificial

<220>
<221> source
<222> (1)..(20)
<223> /note=«Description of artificial sequence: Forward primer, SAV0791 gene encoding Q932D7»

<400> 553
gggctagtac aatatgacgc 20

<210> 554
<211> 20
<212> DNA
<213> artificial

<220>
<221> source
<222> (1)..(20)
<223> /note=«Description of artificial sequence: Reverse primer, SAV0791 gene encoding Q932D7»

<400> 554
tggttatacc aatgacctcc 20

<210> 555
<211> 20
<212> DNA
<213> artificial

<220>
<221> source
<222> (1)..(20)

<223> /note=«Description of artificial sequence: Forward primer, SAV0783 gene encoding Q932E5»

<400> 555
ttggcactta ctatttccgt 20

<210> 556
<211> 20
<212> DNA
<213> artificial

<220>
<221> source
<222> (1)..(20)
<223> /note=«Description of artificial sequence: Reverse primer, SAV0783 gene encoding Q932E5»

<400> 556
aacgtccaat aattcaatcg 20

<210> 557
<211> 20
<212> DNA

<213> artificial

<220>
<221> source
<222> (1)..(20)
<223> /note=«Description of artificial sequence: Forward primer, mecR1-1 Gene encoding BAA82219 MecR1.»

<400> 557
aaagaatgga accaagatca 20

<210> 558
<211> 20
<212> DNA
<213> artificial

<220>
<221> source
<222> (1)..(20)
<223> /note=«Description of artificial sequence: Reverse primer, mecR1-1 Gene encoding BAA82219 MecR1.»

<400> 558
tcttcgcctt ttaaatgtgt 20

<210> 559
<211> 20
<212> DNA
<213> artificial

<220>
<221> source
<222> (1)..(20)

<223> /note=«Description of artificial sequence: Forward primer, aadD gene encoding Aminoside adenyltransferase: AAD(3»)»

<400> 559
ggaagtgaat tttgatagcg 20

<210> 560
<211> 20
<212> DNA
<213> artificial

<220>
<221> source
<222> (1)..(20)
<223> /note=«Description of artificial sequence: Reverse primer, aadD gene encoding Aminoside adenyltransferase: AAD(3»)»

<400> 560
ctcagagtcg gaaagttgac 20

<210> 561
<211> 20
<212> DNA
<213> artificial

<220>
<221> source
<222> (1)..(20)
<223> /note=«Description of artificial sequence: Forward primer, ble gene encoding Bleomycin resistance protein»

<400> 561
gttacagtct atccgggcat 20

<210> 562
<211> 20
<212> DNA
<213> artificial

<220>
<221> source
<222> (1)..(20)
<223> /note=«Description of artificial sequence: Reverse primer, ble gene encoding Bleomycin resistance protein»

<400> 562
atcccaccac tgatctttta 20

<210> 563
<211> 20
<212> DNA
<213> artificial

<220>
<221> source
<222> (1)..(20)
<223> /note=«Description of artificial sequence: Forward primer,

ermA gene encoding ARNr 23S methylase: MLS resistance»

<400> 563
aattttcctt cccaaaacat 20

<210> 564
<211> 20
<212> DNA
<213> artificial

<220>
<221> source
<222> (1)..(20)
<223> /note=«Description of artificial sequence: Reverse primer, ErmA gene encoding ARNr 23S methylase: MLS resistance»

<400> 564
ctgtcggaat tggtttttag 20

<210> 565
<211> 20
<212> DNA
<213> artificial

<220>
<221> source
<222> (1)..(20)
<223> /note=«Description of artificial sequence: Forward primer, sec gen encoding Enterotoxin C

<220>
<221> source
<222> (1)..(20)
<223> /note=«Description of artificial sequence: Forward primer, sec gene encoding Enterotoxin C»

<400> 565
gataaatttt tggcacatga 20

<210> 566
<211> 20
<212> DNA
<213> artificial

<220>
<221> source
<222> (1)..(20)
<223> /note=«Description of artificial sequence: Reverse primer, sec gene encoding Enterotoxin C»

<400> 566
accaaaaagt attgccgtta 20

<210> 567
<211> 20
<212> DNA
<213> artificial

<220>
<221> source
<222> (1)..(20)
<223> /note=«Description of artificial sequence: Forward primer, seg* gene encoding Enterotoxin G»

<400> 567
ccgatcctaa attagacgaa 20

<210> 568
<211> 19
<212> DNA
<213> artificial

<220>
<221> source
<222> (1)..(19)
<223> /note=«Description of artificial sequence: Reverse primer, seg* gene encoding Enterotoxin G»

<400> 568
gccagtgtct tgctttgta 19

<210> 569
<211> 19
<212> DNA
<213> artificial

<220>
<221> source
<222> (1)..(19)
<223> /note=«Description of artificial sequence: Forward primer, entK gene encoding Enterotoxin K»

<400> 569
ggagaaaagg caatgaaaa 19

<210> 570
<211> 20
<212> DNA
<213> artificial

<220>
<221> source
<222> (1)..(20)
<223> /note=«Description of artificial sequence: Reverse primer, EntK gene encoding Enterotoxin K»

<400> 570
tctcttgagc ggtaacaaat 20

<210> 571
<211> 20
<212> DNA
<213> artificial

<220>
<221> source
<222> (1)..(20)
<223> /note=«Description of artificial sequence: Forward primer, entQ gene encoding Enterotoxin Q»

<400> 571
atgctgatgt aggggtaatc 20

<210> 572
<211> 20
<212> DNA
<213> artificial

<220>
<221> source
<222> (1)..(20)
<223> /note=«Description of artificial sequence: Reverse primer, entQ gene encoding Enterotoxin Q»

<400> 572
tttttccttt gttaaaccca 20

<210> 573
<211> 20
<212> DNA
<213> artificial

<220>
<221> source
<222> (1)..(20)
<223> /note=«Description of artificial sequence: Forward primer, sel* gene encoding Enterotoxin L»

<400> 573
acagtcttat ctaacggcga 20

<210> 574
<211> 20
<212> DNA
<213> artificial

<220>
<221> source
<222> (1)..(20)
<223> /note=«Description of artificial sequence: Reverse primer, sel* gene encoding Enterotoxin L»

<400> 574
ttttgaagaa gtgccgtatt 20

<210> 575
<211> 21
<212> DNA
<213> artificial

<220>

<221> source
<222> (1)..(21)
<223> /note=«Description of artificial sequence: Forward primer, seo* ene encoding Enterotoxin O»

<400> 575
ttggttagat ggaatatctg c 21

<210> 576
<211> 20
<212> DNA
<213> artificial

<220>
<221> source
<222> (1)..(20)
<223> /note=«Description of artificial sequence: Reverse primer, seo* ene encoding Enterotoxin O»

<400> 576
tgattcttta tgctccgaat 20

<210> 577
<211> 21
<212> DNA
<213> artificial

<220>
<221> source
<222> (1)..(21)
<223> /note=«Description of artificial sequence: Forward primer, sem* gene encoding Enterotoxin M»

<400> 577
agctgaattt aagaacgttg a 21

<210> 578
<211> 20
<212> DNA
<213> artificial

<220>
<221> source
<222> (1)..(20)
<223> /note=«Description of artificial sequence: Reverse primer, sem* gene encoding Enterotoxin M»

<400> 578
tggaattttt cagtttcgac 20

<210> 579
<211> 21
<212> DNA
<213> artificial

<220>
<221> source

<222> (1)..(21)
<223> /note=«Description of artificial sequence: Forward primer, sen* gene encoding Enterotoxin N»

<400> 579
atataacgtg gcaattagac g 21

<210> 580
<211> 20
<212> DNA
<213> artificial

<220>
<221> source

<222> (1)..(20)
<223> /note=«Description of artificial sequence: Reverse primer, sen* gene encoding Enterotoxin N»

<400> 580
gaatgaaaga aaatgcatcc 20

<210> 581
<211> 20
<212> DNA
<213> artificial

<220>
<221> source
<222> (1)..(20)
<223> /note=«Description of artificial sequence: Forward primer, set21 gene encoding Exotoxin Set 21»

<400> 581
aagtcaaaca gtaaaagcgg 20

<210> 582
<211> 20
<212> DNA
<213> artificial

<220>
<221> source
<222> (1)..(20)
<223> /note=«Description of artificial sequence: Reverse primer, set21 gene encoding Exotoxin Set 21»

<400> 582
ttctatggtt aattgcatcg 20

<210> 583
<211> 20
<212> DNA
<213> artificial

<220>
<221> source

<222> (1)..(20)
<223> /note=«Description of artificial sequence: Forward primer, splE* gene encoding Serine protease SplE»

<400> 583
atctggaaca ggtttcattg 20

<210> 584
<211> 20
<212> DNA
<213> artificial

<220>
<221> source
<222> (1)..(20)
<223> /note=«Description of artificial sequence: Reverse primer, splE* gene encoding Serine protease SplE»

<400> 584
tgtactttta tttccagggc 20

<210> 585
<211> 20
<212> DNA
<213> artificial

<220>
<221> source
<222> (1)..(20)
<223> /note=«Description of artificial sequence: Forward primer, cna* gene encoding Collagen-binding protein»

<400> 585
ggtgggtcaa gcagttatta 20

<210> 586
<211> 20
<212> DNA
<213> artificial

<220>
<221> source
<222> (1)..(20)
<223> /note=«Description of artificial sequence: Reverse primer, cna* gene encoding Collagen-binding protein»

<400> 586
cagtaattgc actttgtcca 20

<210> 587
<211> 20
<212> DNA
<213> artificial

<220>
<221> source
<222> (1)..(20)

<223> /note=«Description of artificial sequence: Forward primer, MW2612 gene encoding Collagen-binding protein Q8NUH0»

<400> 587
gatggtggta aaacgacagt 20

<210> 588
<211> 20
<212> DNA
<213> artificial

<220>
<221> source
<222> (1)..(20)
<223> /note=«Description of artificial sequence: Reverse primer, MW2612 gene encoding Collagen-binding protein Q8NUH0»

<400> 588
accatcgtac atgtgtcgta 20

<210> 589
<211> 20
<212> DNA
<213> artificial

<220>
<221> source
<222> (1)..(20)
<223> /note=«Description of artificial sequence: Forward primer, cap8I* gene encoding Capsular polysaccharide synthesis Cap8I»

<400> 589
gccattttaa tgtttccttg 20

<210> 590
<211> 20
<212> DNA
<213> artificial

<220>
<221> source
<222> (1)..(20)
<223> /note=«Description of artificial sequence: Reverse primer, cap8I* gene encoding Capsular polysaccharide synthesis Cap8I»

<400> 590
agctacaaaa atcccacaaa 20

<210> 591
<211> 20
<212> DNA
<213> artificial .

<220>
<221> source

<222> (1)..(20)
<223> /note=«Description of artificial sequence: Forward primer, cap8I gene encoding Capsular polysaccharide synthesis Cap8I»

<400> 591
ttgccatttt aatgtttcct 20

<210> 592
<211> 20
<212> DNA
<213> artificial

<220>
<221> source
<222> (1)..(20)
<223> /note=«Description of artificial sequence: Reverse primer, cap8I gene encoding Capsular polysaccharide synthesis Cap8I»

<400> 592
aagctacaaa aatcccacaa 20

<210> 593
<211> 20
<212> DNA
<213> artificial

<220>
<221> source
<222> (1)..(20)
<223> /note=«Description of artificial sequence: Forward primer, cap8k like gene encoding protein 74% homology with Cap8K»

<400> 593
taaatttatt ggcgattcgt 20

<210> 594
<211> 20
<212> DNA
<213> artificial

<220>
<221> source
<222> (1)..(20)
<223> /note=«Description of artificial sequence: Reverse primer, cap8k like gene encoding protein 74% homology with Cap8K»

<400> 594
ccattgatcc agctctaaac 20

<210> 595
<211> 20
<212> DNA

<213> artificial

<220>
<221> source
<222> (1)..(20)
<223> /note=«Description of artificial sequence: Forward primer, cap8J gene encoding Capsular polysaccharide synthesis Cap8J Q8NYP4»

<400> 595
tgaaaataaa gtggctggat 20

<210> 596
<211> 20
<212> DNA
<213> artificial

<220>
<221> source
<222> (1)..(20)
<223> /note=«Description of artificial sequence: Reverse primer, cap8J gene encoding Capsular polysaccharide synthesis Cap8J Q8NYP4»

<400> 596
ttttcttgat gattttcgct 20

<210> 597
<211> 20
<212> DNA
<213> artificial

<220>
<221> source
<222> (1)..(20)
<223> /note=«Description of artificial sequence: Forward primer, cap8H gene encoding Capsular polysaccharide synthesis Cap8H Q8NYP6»

<400> 597
cgaaatactt ggaggaaatg 20

<210> 598
<211> 20
<212> DNA
<213> artificial

<220>
<221> source
<222> (1)..(20)
<223> /note=«Description of artificial sequence: Reverse primer, cap8H gene encoding Capsular polysaccharide synthesis Cap8H Q8NYP6»

<400> 598
agataatgtt gtcaccctgc 20

<210> 599
<211> 20
<212> DNA
<213> artificial

<220>
<221> source
<222> (1)..(20)
<223> /note=«Description of artificial sequence: Forward primer, agrB-MW2 gene encoding AgrB (MW2 variable domain)»

<400> 599
attagggatg caggtcttag 20

<210> 600
<211> 20
<212> DNA
<213> artificial

<220>
<221> source
<222> (1)..(20)
<223> /note=«Description of artificial sequence: Reverse primer, agrB-MW2 gene encoding AgrB (MW2 variable domain)»

<400> 600
ccagttgaat aaattgggta 20

<210> 601
<211> 20
<212> DNA
<213> artificial

<220>
<221> source
<222> (1)..(20)
<223> /note=«Description of artificial sequence: Forward primer, agrC-MW2* gene encoding AgrC (MW2 variable domain)»

<400> 601
ttctttgaca aaatcggaag 20

<210> 602
<211> 20
<212> DNA
<213> artificial

<220>
<221> source
<222> (1)..(20)
<223> /note=«Description of artificial sequence: Reverse primer, agrC-MW2* gene encoding AgrC (MW2 variable domain)»

<400> 602
cccaagaata aaagcgaata 20

<210> 603

<211> 20
<212> DNA
<213> artificial

<220>
<221> source
<222> (1)..(20)
<223> /note=«Description of artificial sequence: Forward primer, tnpA (Tn554) gene encoding Tn 554A transposase»

<400> 603
tagaagtgga gaataagccg 20

<210> 604
<211> 20
<212> DNA
<213> artificial

<220>
<221> source
<222> (1)..(20)
<223> /note=«Description of artificial sequence: Reverse primer, tnpA (Tn554) gene encoding Tn 554A transposase»

<400> 604
atcctttgaa atttcttccc 20

<210> 605
<211> 20
<212> DNA
<213> artificial

<220>
<221> source
<222> (1)..(20)
<223> /note=«Description of artificial sequence: Forward primer, tnpB (Tn554) gene encoding Tn 554B transposase»

<400> 605
aatgaatgaa gaaatgcagg 20

<210> 606
<211> 20
<212> DNA
<213> artificial

<220>
<221> source
<222> (1)..(20)
<223> /note=«Description of artificial sequence: Reverse primer, tnpB (Tn554) gene encoding Tn 554B transposase»

<400> 606
caaacgtctt tatcccactc 20

<210> 607

<211> 21
<212> DNA
<213> artificial

<220>
<221> source
<222> (1)..(21)
<223> /note=«Description of artificial sequence: Forward primer, ORF2 gene encoding Putative transposase Q9AL26»

<400> 607
agccttatgt gctaaactca a 21

<210> 608
<211> 20
<212> DNA
<213> artificial

<220>
<221> source
<222> (1)..(20)
<223> /note=«Description of artificial sequence: Reverse primer, ORF2 gene encoding Putative transposase Q9AL26»

<400> 608
tttttgagca acactgatga 20

<210> 609
<211> 19
<212> DNA
<213> artificial

<220>
<221> source
<222> (1)..(19)
<223> /note=«Description of artificial sequence: Forward primer, SA0062 Gene encoding Transposase IS150 Q99XD6»

<400> 609
aaagcgaatt gtcccatag 19

<210> 610
<211> 21
<212> DNA
<213> artificial

<220>
<221> source
<222> (1)..(21)
<223> /note=«Description of artificial sequence: Reverse primer, SA0062 Gene encoding Transposase IS150 Q99XD6

<400> 610
gcctagtttt tgttgaattt g 21

<210> 611
<211> 20

<212> DNA
<213> artificial

<220>
<221> source
<222> (1)..(20)
<223> /note=«Description of artificial sequence: Forward primer, SA1824 gene encoding Tn557 putative protein»

<400> 611
agaattggaa caagagcgta 20

<210> 612
<211> 20
<212> DNA
<213> artificial

<220>
<221> source
<222> (1)..(20)
<223> /note=«Description of artificial sequence: Reverse primer, SA1824 gene encoding Tn557 putative protein»

<400> 612
tgttattgtg ttcgtatggc 20

<210> 613
<211> 21
<212> DNA
<213> artificial

<220>
<221> source
<222> (1)..(21)
<223> /note=«Description of artificial sequence: Forward primer, phi int12 gene encoding Integrase phage phi12»

<400> 613
tcaggaggta taaacatgtg g 21

<210> 614
<211> 20
<212> DNA
<213> artificial

<220>
<221> source
<222> (1)..(20)
<223> /note=«Description of artificial sequence: Reverse primer, phi int12 gene encoding Integrase phage phi12»

<400> 614
cgggttactt gattttgaga 20

<210> 615
<211> 20
<212> DNA

<213> artificial

<220>
<221> source
<222> (1)..(20)
<223> /note=«Description of artificial sequence: Forward primer, ORF18* gene encoding Anti repressor from phage phi 11 Q8SDX0»

<400> 615
tagtgcatca ggtcaaaaca 20

<210> 616
<211> 20
<212> DNA
<213> artificial

<220>
<221> source
<222> (1)..(20)
<223> /note=«Description of artificial sequence: Reverse primer, ORF18* gene encoding Anti repressor from phage phi 11 Q8SDX0»

<400> 616
ttctgagtaa agcacccact 20

<210> 617
<211> 20
<212> DNA
<213> artificial

<220>
<221> source
<222> (1)..(20)
<223> /note=«Description of artificial sequence: Forward primer, ORF19* gene encoding Tail lengh tape protein from phage phi 13 Q8SDK4»

<400> 617
taggcaaatg aaaatggttt 20

<210> 618
<211> 20
<212> DNA
<213> artificial

<220>
<221> source
<222> (1)..(20)
<223> /note=«Description of artificial sequence: Reverse primer, ORF19* gene encoding Tail lengh tape protein from phage phi 13 Q8SDK4»

<400> 618
catgttacgg cctacatttt 20

<210> 619
<211> 20
<212> DNA
<213> artificial

<220>
<221> source
<222> (1)..(20)
<223> /note=«Description of artificial sequence: Forward primer, ORF20* gene encoding Head protein from phage phi 13 Q8SDK8»

<400> 619
acgttctcaa atccaagaaa 20

<210> 620
<211> 20
<212> DNA
<213> artificial

<220>
<221> source
<222> (1)..(20)
<223> /note=«Description of artificial sequence: Reverse primer, ORF20* gene encoding Head protein from phage phi 13 Q8SDK8»

<400> 620
ttcttcaact ttttcaaggg 20

<210> 621
<211> 20
<212> DNA
<213> artificial

<220>
<221> source
<222> (1)..(20)
<223> /note=«Description of artificial sequence: Forward primer, ORF21* gene encoding Portal protein from phage phi 13 Q8SDK9»

<400> 621
caggttttca aggaacaaaa 20

<210> 622
<211> 20
<212> DNA
<213> artificial

<220>
<221> source
<222> (1)..(20)
<223> /note=«Description of artificial sequence: Reverse primer, ORF21* gene encoding Portal protein from phage phi 13 Q8SDK9»

<400> 622

cgtgcgactt aatgtgtcta 20

<210> 623
<211> 20
<212> DNA
<213> artificial

<220>
<221> source
<222> (1)..(20)
<223> /note=«Description of artificial sequence: Forward primer, ORF26 gene encoding Orf153 from phage phiSLT Q9B0H5»

<400> 623
aataagagag gctgatgtgc 20

<210> 624
<211> 20
<212> DNA
<213> artificial

<220>
<221> source
<222> (1)..(20)
<223> /note=«Description of artificial sequence: Reverse primer, ORF26 gene encoding Orf153 from phage phiSLT Q9B0H5»

<400> 624
ttaaaaactc tcaacggctc 20

<210> 625
<211> 20
<212> DNA
<213> artificial

<220>
<221> source
<222> (1)..(20)
<223> /note=«Description of artificial sequence: Forward primer, ORF27 gene encoding Phi N315 phage protein Q8LTH2»

<400> 625
gttgtattga atttgttgcg 20

<210> 626
<211> 20
<212> DNA
<213> artificial

<220>
<221> source
<222> (1)..(20)
<223> /note=«Description of artificial sequence: Reverse primer, ORF27 gene encoding Phi N315 phage protein Q8LTH2»

<400> 626
attcttccga atattttccc 20

<210> 627
<211> 20
<212> DNA
<213> artificial

<220>

<221> source
<222> (1)..(20)
<223> /note=«Description of artificial sequence: Forward primer, repB* gene encoding pUB110 RepB replication protein»

<400> 627
acagttcgtt ggttgtttct 20

<210> 628
<211> 20
<212> DNA
<213> artificial

<220>
<221> source
<222> (1)..(20)
<223> /note=«Description of artificial sequence: Reverse primer, repB* gene encoding pUB110 RepB replication protein»

<400> 628
tcggtcataa aatccgtatc 20

<210> 629
<211> 20
<212> DNA
<213> artificial

<220>
<221> source
<222> (1)..(20)
<223> /note=«Description of artificial sequence: Forward primer, kdpC gene encoding Potassium-transporting ATPase C chain»

<400> 629
acggcagttt agtgaaacaa 20

<210> 630
<211> 20
<212> DNA
<213> artificial

<220>
<221> source
<222> (1)..(20)
<223> /note=«Description of artificial sequence: Reverse primer, kdpC gene encoding Potassium-transporting ATPase C chain»

<400> 630

ataatcatct gccattggtg 20

<210> 631
<211> 20
<212> DNA
<213> artificial

<220>
<221> source
<222> (1)..(20)
<223> /note=«Description of artificial sequence: Forward primer, kdpB gene encoding Potassium-transporting ATPase B chain»

<400> 631
atgtaggttt ggcaatgaac 20

<210> 632
<211> 20
<212> DNA
<213> artificial

<220>
<221> source
<222> (1)..(20)
<223> /note=«Description of artificial sequence: Reverse primer, kdpB gene encoding Potassium-transporting ATPase B chain»

<400> 632
gagcttaatg ccgataaatg 20

<210> 633
<211> 20
<212> DNA
<213> artificial

<220>
<221> source
<222> (1)..(20)
<223> /note=«Description of artificial sequence: Forward primer, orf N021 gene encoding Hypothetical protein kdpD»

<400> 633
gctgaaatac cataacgagc 20

<210> 634
<211> 20
<212> DNA
<213> artificial

<220>
<221> source
<222> (1)..(20)
<223> /note=«Description of artificial sequence: Reverse primer, orf N021 gene encoding Hypothetical protein kdpD»

<400> 634
atagagccct aatcccaaac 20

<210> 635
<211> 20
<212> DNA
<213> artificial

<220>
<221> source
<222> (1)..(20)
<223> /note=«Description of artificial sequence: Forward primer, orf N022 gene encoding Hypothetical protein kdpE»

<400> 635
aacagaggaa caaaccattg 20

<210> 636
<211> 20
<212> DNA
<213> artificial

<220>
<221> source
<222> (1)..(20)
<223> /note=«Description of artificial sequence: Reverse primer, orf N022 gene encoding Hypothetical protein kdpE»

<400> 636
ccgattctag gatgtgtgat 20

<210> 637
<211> 20
<212> DNA
<213> artificial

<220>
<221> source
<222> (1)..(20)
<223> /note=«Description of artificial sequence: Forward primer, xylR gene encoding Xylose repressor Q93IC6»

<400> 637
accatcataa tatttcgcgt 20

<210> 638
<211> 20
<212> DNA
<213> artificial

<220>
<221> source
<222> (1)..(20)
<223> /note=«Description of artificial sequence: Reverse primer, xylR gene encoding Xylose repressor Q93IC6»

<400> 638

atgtcacatg ctgctcatta 20

<210> 639
<211> 20
<212> DNA
<213> artificial

<220>
<221> source
<222> (1)..(20)
<223> /note=«Description of artificial sequence: Forward primer, SA0077 gene encoding ORF CN005 Q9XBB4»

<400> 639
aatgaagaaa tcttacgcga 20

<210> 640
<211> 20
<212> DNA
<213> artificial

<220>
<221> source
<222> (1)..(20)
<223> /note=«Description of artificial sequence: Reverse primer, SA0077 gene encoding ORF CN005 Q9XBB4»

<400> 640
atattcgcat accctccata 20

<210> 641
<211> 20
<212> DNA
<213> artificial

<220>
<221> source
<222> (1)..(20)
<223> /note=«Description of artificial sequence: Forward primer, SA0076 gene encoding ORF N007 Q9KX89»

<400> 641
aggctatagg cttcactcaa 20

<210> 642
<211> 20
<212> DNA
<213> artificial

<220>
<221> source
<222> (1)..(20)
<223> /note=«Description of artificial sequence: Reverse primer, SA0076 gene encoding ORF N007 Q9KX89»

<400> 642
ttgtattttt ggggttgttc 20

<210> 643
<211> 20
<212> DNA
<213> artificial

<220>
<221> source
<222> (1)..(20)
<223> /note=«Description of artificial sequence: Forward primer, SA0061 gene encoding ORF N029 Q9KX84»

<400> 643
gatgattcat gtagggctgt 20

<210> 644
<211> 21
<212> DNA
<213> artificial

<220>
<221> source
<222> (1)..(21)
<223> /note=«Description of artificial sequence: Reverse primer, SA0061 gene encoding ORF N029 Q9KX84»

<400> 644
gcatgttcat aatctctttg a 21

<210> 645
<211> 20
<212> DNA
<213> artificial

<220>
<221> source
<222> (1)..(20)
<223> /note=«Description of artificial sequence: Forward primer, SA0059 gene encoding ORF N031 Q9KX82»

<400> 645
cctactgaag ttgaaatcgc 20

<210> 646
<211> 20
<212> DNA
<213> artificial

<220>
<221> source
<222> (1)..(20)
<223> /note=«Description of artificial sequence: Reverse primer, SA0059 gene encoding ORF N031 Q9KX82»

<400> 646
aactcgtcac taaggcagaa 20

<210> 647
<211> 20
<212> DNA
<213> artificial

<220>
<221> source
<222> (1)..(20)
<223> /note=«Description of artificial sequence: Forward primer, ORF33* gene encoding Q9RL83»

<400> 647
actttctgag atgcccatag 20

<210> 648
<211> 20
<212> DNA
<213> artificial

<220>
<221> source
<222> (1)..(20)

<223> /note=«Description of artificial sequence: Reverse primer, ORF33* gene encoding Q9RL83»

<400> 648
gcagtttcgt cattggtatt 20

<210> 649
<211> 21
<212> DNA
<213> artificial

<220>
<221> source
<222> (1)..(21)
<223> /note=«Description of artificial sequence: Forward primer, ORF37 gene encoding Q9L3N7»

<400> 649
gatgttgtta taatggggac a 21

<210> 650
<211> 20
<212> DNA
<213> artificial

<220>
<221> source
<222> (1)..(20)
<223> /note=«Description of artificial sequence: Reverse primer, ORF37 gene encoding Q9L3N7»

<400> 650
gtggcactat cgtcttgaat 20

<210> 651
<211> 20
<212> DNA
<213> artificial

<220>
<221> source
<222> (1)..(20)
<223> /note=«Description of artificial sequence: Forward primer, MW1898* gene encoding Q8NVQ4»

<400> 651
tagaccgtcc acaagatacc 20

<210> 652
<211> 20
<212> DNA
<213> artificial

<220>
<221> source
<222> (1)..(20)
<223> /note=«Description of artificial sequence: Reverse primer, MW1898* gene encoding Q8NVQ4»

<400> 652
tccatatttt gttctcctcg 20

<210> 653
<211> 20
<212> DNA
<213> artificial

<220>
<221> source
<222> (1)..(20)
<223> /note=«Description of artificial sequence: Forward primer, MW1908* Gene encoding Q8NVP4»

<400> 653
aattttggca agtttgaaga 20

<210> 654
<211> 20
<212> DNA
<213> artificial

<220>
<221> source
<222> (1)..(20)
<223> /note=«Description of artificial sequence: Reverse primer, MW1908* gene encoding Q8NVP4»

<400> 654
ttcacggata aacttgcttt 20

<210> 655
<211> 20
<212> DNA
<213> artificial

<220>
<221> source
<222> (1)..(20)
<223> /note=«Description of artificial sequence: Forward primer, ORF41 gene encoding putative protein»

<400> 655
cctattttca agcacctgag 20

<210> 656
<211> 21
<212> DNA
<213> artificial

<220>
<221> source
<222> (1)..(21)
<223> /note=«Description of artificial sequence: Reverse primer, ORF41 gene encoding putative protein»

<400> 656
acagtgatca ttgaattctg a 21

<210> 657
<211> 20
<212> DNA
<213> artificial

<220>
<221> source
<222> (1)..(20)
<223> /note=«Description of artificial sequence: Forward primer, SA2259* gene encoding Q99RG2»

<400> 657
agagcgttat gggtttacaa 20

<210> 658
<211> 20
<212> DNA
<213> artificial

<220>
<221> source
<222> (1)..(20)
<223> /note=«Description of artificial sequence: Reverse primer, SA2259* gene encoding Q99RG2»

<400> 658
taataattcc ccgtcatttg 20

<210> 659
<211> 20
<212> DNA
<213> artificial

<220>
<221> source
<222> (1)..(20)
<223> /note=«Description of artificial sequence: Forward primer, SA1828 gene encoding Q99SM5»

<400> 659
ggtgttgcct tataccaact 20

<210> 660
<211> 20
<212> DNA
<213> artificial

<220>
<221> source
<222> (1)..(20)
<223> /note=«Description of artificial sequence: Reverse primer, SA1828 gene encoding Q99SM5»

<400> 660
tgaaaccctt tatcattgct 20

<210> 661
<211> 20
<212> DNA
<213> artificial

<220>
<221> source
<222> (1)..(20)
<223> /note=«Description of artificial sequence: Forward primer, SA1806 gene encoding Q99SP3»

<400> 661
tccacgcaat ctctagtttt 20

<210> 662
<211> 20
<212> DNA
<213> artificial

<220>
<221> source
<222> (1)..(20)
<223> /note=«Description of artificial sequence: Reverse primer, SA1806 gene encoding Q99SP3»

<400> 662
catcttgaac accttcgact 20

<210> 663
<211> 20
<212> DNA
<213> artificial

<220>
<221> source
<222> (1)..(20)
<223> /note=«Description of artificial sequence: Forward primer, SA1778 gene encoding Q99SS4»

<400> 663
aaaaatcaaa agcgatcaaa 20

<210> 664
<211> 20
<212> DNA
<213> artificial

<220>
<221> source
<222> (1)..(20)
<223> /note=«Description of artificial sequence: Reverse primer, SA1778 gene encoding Q99SS4»

<400> 664
gcgtttctgt gtcttctttc 20

<210> 665
<211> 20
<212> DNA
<213> artificial

<220>
<221> source
<222> (1)..(20)
<223> /note=«Description of artificial sequence: Forward primer, SA1777 gene encoding Q99SS5»

<400> 665
gatgaacaga aaatcgagga 20

<210> 666
<211> 20
<212> DNA
<213> artificial

<220>
<221> source
<222> (1)..(20)
<223> /note=«Description of artificial sequence: Reverse primer, SA1777 gene encoding Q99SS5»

<400> 666
tcaaaaataa cacacccctc 20

<210> 667

<211> 20
<212> DNA
<213> artificial

<220>
<221> source
<222> (1)..(20)
<223> /note=«Description of artificial sequence: Forward primer, SA1775 gene encoding Q99SS7»

<400> 667
cgaagataaa tgggtttacg 20

<210> 668
<211> 20
<212> DNA
<213> artificial

<220>
<221> source
<222> (1)..(20)
<223> /note=«Description of artificial sequence: Reverse primer, SA1775 gene encoding Q99SS7»

<400> 668
tttgcatatt gtcgttttca 20

<210> 669
<211> 20
<212> DNA
<213> artificial

<220>
<221> source
<222> (1)..(20)
<223> /note=«Description of artificial sequence: Forward primer, SA1774 gene encoding Q99SS8»

<400> 669
gcaaatggat tttcaagatt 20

<210> 670
<211> 20
<212> DNA
<213> artificial

<220>
<221> source
<222> (1)..(20)
<223> /note=«Description of artificial sequence: Reverse primer, SA1774 gene encoding Q99SS8»

<400> 670
caccatagat tttaccccaa 20

<210> 671
<211> 20

<212> DNA
<213> artificial

<220>
<221> source
<222> (1)..(20)
<223> /note=«Description of artificial sequence: Forward primer, SA1768 gene encoding Q99ST4»

<400> 671
tccaattgaa tcagggaata 20

<210> 672
<211> 20
<212> DNA
<213> artificial

<220>
<221> source
<222> (1)..(20)
<223> /note=«Description of artificial sequence: Reverse primer, SA1768 gene encoding Q99ST4»

<400> 672
gttaccctct gtcacgtttc 20

<210> 673
<211> 20
<212> DNA
<213> artificial

<220>
<221> source
<222> (1)..(20)
<223> /note=«Description of artificial sequence: Forward primer, SA1766* gene encoding Q99ST6»

<400> 673
caagcaatat ggcaaggtat 20

<210> 674
<211> 20
<212> DNA
<213> artificial

<220>
<221> source
<222> (1)..(20)
<223> /note=«Description of artificial sequence: Reverse primer, SA1766* gene encoding Q99ST6»

<400> 674
ctaaaacacc gcctacagtc 20

<210> 675
<211> 20
<212> DNA

<213> artificial

<220>
<221> source
<222> (1)..(20)
<223> /note=«Description of artificial sequence: Forward primer, SA1755 gene encoding Q99SU8»

<400> 675
tttaacggca ggaatcagta 20

<210> 676
<211> 21
<212> DNA
<213> artificial

<220>
<221> source1
<222> (1)..(21)
<223> /note=«Description of artificial sequence: Reverse primer, SA1755 gene encoding Q99SU8»

<400> 676
tcattagttt taccaggacc a 21

<210> 677
<211> 20
<212> DNA
<213> artificial

<220>
<221> source
<222> (1)..(20)
<223> /note=«Description of artificial sequence: Forward primer, SA1641 gene encoding Q99T50»

<400> 677
aagaaattac agcggatgtt 20

<210> 678
<211> 20
<212> DNA
<213> artificial

<220>
<221> source
<222> (1)..(20)
<223> /note=«Description of artificial sequence: Reverse primer, SA1641 gene encoding Q99T50»

<400> 678
gctctttcat ttatcggatg 20

<210> 679
<211> 20
<212> DNA
<213> artificial

<220>
<221> source
<222> (1)..(20)
<223> /note=«Description of artificial sequence: Forward primer, SA1341 gene encoding Q99TY3»

<400> 679
tagctttttc taacatgggc 20

<210> 680
<211> 20
<212> DNA
<213> artificial

<220>
<221> source
<222> (1)..(20)
<223> /note=«Description of artificial sequence: Reverse primer, SA1341 gene encoding Q99TY3»

<400> 680
tccgaaacat tttgagtagc 20

<210> 681
<211> 20
<212> DNA
<213> artificial

<220>
<221> source
<222> (1)..(20)
<223> /note=«Description of artificial sequence: Forward primer, SA0142 gene encoding Q99X68»

<400> 681
tactttatta gcgagcagcc 20

<210> 682
<211> 20
<212> DNA
<213> artificial

<220>
<221> source
<222> (1)..(20)
<223> /note=«Description of artificial sequence: Reverse primer, SA0142 gene encoding Q99X68»

<400> 682
gatgtagaac gagatgtccc 20

<210> 683
<211> 20
<212> DNA
<213> artificial

<220>
<221> source
<222> (1)..(20)
<223> /note=«Description of artificial sequence: Forward primer, SA0141 gene encoding Q99X69»

<400> 683
ttagtgatgc ctatgccttt 20

<210> 684
<211> 20
<212> DNA
<213> artificial

<220>
<221> source
<222> (1)..(20)
<223> /note=«Description of artificial sequence: Reverse primer, SA0141 gene encoding Q99X69»

<400> 684
gaatttgttt ctggatttgg 20

<210> 685
<211> 20
<212> DNA
<213> artificial

<220>
<221> source
<222> (1)..(20)
<223> /note=«Description of artificial sequence: Forward primer, SA0074 gene encoding Q99XD0
«

<400> 685
aatggtcacg atgatttttc 20

<210> 686
<211> 20
<212> DNA
<213> artificial

<220>
<221> source
<222> (1)..(20)
<223> /note=«Description of artificial sequence: Reverse primer, SA0074 gene encoding Q99XD0»

<400> 686
ttctgataat tgcaaagggt 20

<210> 687
<211> 20
<212> DNA
<213> artificial

<220>
<221> source
<222> (1)..(20)
<223> /note=«Description of artificial sequence: Forward primer, SA0073 gene encoding Q99XD2»

<400> 687
aatgttatct ggaccgattg 20

<210> 688
<211> 20
<212> DNA
<213> artificial

<220>
<221> source
<222> (1)..(20)
<223> /note=«Description of artificial sequence: Reverse primer, SA0073 gene encoding Q99XD2»

<400> 688
tacgccaaac aacacatcta 20

<210> 689
<211> 20
<212> DNA
<213> artificial

<220>
<221> source
<222> (1)..(20)
<223> /note=«Description of artificial sequence: Forward primer, SA0072 gene encoding Q99XD3»

<400> 689
gcagataaag cgatggatac 20

<210> 690
<211> 20
<212> DNA
<213> artificial

<220>
<221> source
<222> (1)..(20)
<223> /note=«Description of artificial sequence: Reverse primer, SA0072 gene encoding Q99XD3»

<400> 690
tgtggtgttt tcacttttca 20

<210> 691
<211> 20
<212> DNA
<213> artificial

<220>

<221> source
<222> (1)..(20)
<223> /note=«Description of artificial sequence: Forward primer, SA0047 gene encoding Q99XD8»

<400> 691
aaggttgcag aagattacga 20

<210> 692
<211> 20
<212> DNA
<213> artificial

<220>
<221> source
<222> (1)..(20)
<223> /note=«Description of artificial sequence: Reverse primer, SA0047 gene encoding Q99XD8»

<400> 692
cggtaaaaac tcttggattg 20

<210> 693
<211> 20
<212> DNA
<213> artificial

<220>
<221> source
<222> (1)..(20)
<223> /note=«Description of artificial sequence: Forward primer, ORF43 gene encoding Q93ID1»

<400> 693
ttagtgaaat gatgggcatt 20

<210> 694
<211> 20
<212> DNA
<213> artificial

<220>
<221> source
<222> (1)..(20)
<223> /note=«Description of artificial sequence: Reverse primer, ORF43 gene encoding Q93ID1»

<400> 694
ggtatgttga ttggaagcat 20

<210> 695
<211> 20
<212> DNA
<213> artificial

<220>
<221> source

<222> (1)..(20)
<223> /note=«Description of artificial sequence: Forward primer, MW0355* Gene encoding Q8NY78»

<400> 695
cccaagaaaa ttacaaaacg 20

<210> 696
<211> 20
<212> DNA
<213> artificial

<220>
<221> source
<222> (1)..(20)
<223> /note=«Description of artificial sequence: Reverse primer, MW0355* gene encoding Q8NY78»

<400> 696
tcctttgata ccatactgcc 20

<210> 697
<211> 22
<212> DNA
<213> artificial

<220>
<221> source
<222> (1)..(22)
<223> /note=«Description of artificial sequence: Forward primer, MW0053 gene encoding Q8NYV8»

<400> 697
ttgttgagtg ataaaaattt cg 22

<210> 698
<211> 21
<212> DNA
<213> artificial

<220>
<221> source
<222> (1)..(21)
<223> /note=«Description of artificial sequence: Reverse primer, MW0053 gene encoding Q8NYV8»

<400> 698
ttcatctcta atttcaatgc c 21

<210> 699
<211> 20
<212> DNA
<213> artificial

<220>
<221> source
<222> (1)..(20)

```
<223> /note=«Description of artificial sequence: Forward primer,
      SAV0599 gene encoding Q99W13»

<400> 699
attggtttgc gaggtaatag                                          20


<210> 700
<211> 20
<212> DNA
<213> artificial

<220>
<221> source
<222> (1)..(20)
<223> /note=«Description of artificial sequence: Reverse primer,
      SAV0599 gene encoding Q99W13»

<400> 700
aatgaaaaac caccgaaata                                          20


<210> 701
<211> 20
<212> DNA
<213> artificial

<220>
<221> source
<222> (1)..(20)
<223> /note=«Description of artificial sequence: Forward primer,
      spc gene encoding Spectinomycin adenyltransferase:
      AAD9'«

<400> 701
cggaaaaata ccaaatcaag                                          20


<210> 702
<211> 20
<212> DNA
<213> artificial

<220>
<221> source
<222> (1)..(20)
<223> /note=«Description of artificial sequence: Reverse primer,
      spc gene encoding Spectinomycin adenyltransferase:
      AAD9'«

<400> 702
tcaaaggtac ggagacaagt                                          20


<210> 703
<211> 20
<212> DNA
<213> artificial

<220>
<221> source
```

<222> (1)..(20)
<223> /note=«Description of artificial sequence: Forward primer, sasK(SAV2595) gene encoding Cell surface protein»

<400> 703
tgaaggtgtg aatgaaaaca 20

<210> 704
<211> 20
<212> DNA
<213> artificial

<220>
<221> source
<222> (1)..(20)
<223> /note=«Description of artificial sequence: Reverse primer, sasK(SAV2595) gene encoding Cell surface protein»

<400> 704
ctgacttgct cttgctatcc 20

<210> 705
<211> 20
<212> DNA
<213> artificial

<220>
<221> source
<222> (1)..(20)
<223> /note=«Description of artificial sequence: Forward primer, cap5H gene encoding Capsular polysaccharide synthesis Cap5H»

<400> 705
gcgattgaaa agataattgg 20

<210> 706
<211> 20
<212> DNA
<213> artificial

<220>
<221> source
<222> (1)..(20)
<223> /note=«Description of artificial sequence: Reverse primer, cap5H gene encoding Capsular polysaccharide synthesis Cap5H»

<400> 706
accaacaacc tcatatgctc 20

<210> 707
<211> 20
<212> DNA
<213> artificial

<220>

<221> source
<222> (1)..(20)
<223> /note=«Description of artificial sequence: Forward primer, cap5K gene encoding Capsular polysaccharide synthesis Cap5K Q99X60»

<400> 707
taggtaatga agcgtttggt 20

<210> 708
<211> 20
<212> DNA
<213> artificial

<220>
<221> source
<222> (1)..(20)
<223> /note=«Description of artificial sequence: Reverse primer, cap5K gene encoding Capsular polysaccharide synthesis Cap5K Q99X60»

<400> 708
tgcaaacaat tcactggtaa 20

<210> 709
<211> 20
<212> DNA
<213> artificial

<220>
<221> source
<222> (1)..(20)
<223> /note=«Description of artificial sequence: Forward primer, cap5J gene encoding Capsular polysaccharide synthesis Cap5J Q99X61»

<400> 709
gagccggtgt attactcaac 20

<210> 710
<211> 20
<212> DNA
<213> artificial

<220>
<221> source
<222> (1)..(20)
<223> /note=«Description of artificial sequence: Reverse primer, cap5J gene encoding Capsular polysaccharide synthesis Cap5J Q99X61»

<400> 710
aaatgctgaa aggtacgaag 20

<210> 711
<211> 20
<212> DNA

<213> artificial

<220>
<221> source
<222> (1)..(20)
<223> /note=«Description of artificial sequence: Forward primer, cap5I gene encoding Capsular polysaccharide synthesis Cap5I Q99X62»

<400> 711
attaaaggcg ttacgaatga 20

<210> 712
<211> 20
<212> DNA
<213> artificial

<220>
<221> source
<222> (1)..(20)
<223> /note=«Description of artificial sequence: Reverse primer, cap5I gene encoding Capsular polysaccharide synthesis Cap5I Q99X62»

<400> 712
ttttgctgca tgacttactg 20

<210> 713
<211> 20
<212> DNA
<213> artificial

<220>
<221> source
<222> (1)..(20)
<223> /note=«Description of artificial sequence: Forward primer, SAV1974* gene encoding Pyrophosphatase dUTP Q931L2»

<400> 713
aaatcataag acggatgcag 20

<210> 714
<211> 20
<212> DNA
<213> artificial

<220>
<221> source
<222> (1)..(20)
<223> /note=«Description of artificial sequence: Reverse primer, SAV1974* gene encoding Pyrophosphatase dUTP Q931L2»

<400> 714
tgaaacactc tcgaattcct 20

<210> 715
<211> 20

<212> DNA
<213> artificial

<220>
<221> source
<222> (1)..(20)
<223> /note=«Description of artificial sequence: Forward primer,
tnp IS 1181 gene encoding IS 1181 transposase»

<400> 715
acatggtaag cgtgtttctc 20

<210> 716
<211> 20
<212> DNA
<213> artificial

<220>
<221> source
<222> (1)..(20)
<223> /note=«Description of artificial sequence: Reverse primer,
tnp IS 1181 gene encoding IS 1181 transposase»

<400> 716
accgtcttaa cttgttgtcg 20

<210> 717
<211> 20
<212> DNA
<213> artificial

<220>
<221> source
<222> (1)..(20)
<223> /note=«Description of artificial sequence: Forward primer,
ORF3 gene encoding Putative transposase Q8KLQ7»

<400> 717
tgcgcctatg tgtaatgata 20

<210> 718
<211> 20
<212> DNA
<213> artificial

<220>
<221> source
<222> (1)..(20)
<223> /note=«Description of artificial sequence: Reverse primer,
ORF3 gene encoding Putative transposase Q8KLQ7»

<400> 718
tgaattagcc gctttagttc 20

<210> 719
<211> 20
<212> DNA

<213> artificial

<220>
<221> source
<222> (1)..(20)
<223> /note=«Description of artificial sequence: Forward primer, MW1750 gene encoding Putative HsdS Q8NVX9»

<400> 719
agagtgggaa aacaaattca 20

<210> 720
<211> 23
<212> DNA
<213> artificial

<220>
<221> source
<222> (1)..(23)
<223> /note=«Description of artificial sequence: Reverse primer, MW1750 gene encoding Putative HsdS Q8NVX9»

<400> 720
tgttcagtga gacaaggtat ttt 23

<210> 721
<211> 20
<212> DNA
<213> artificial

<220>
<221> source
<222> (1)..(20)
<223> /note=«Description of artificial sequence: Forward primer, SA0392* Gene encoding putative HsdS Q99WH0»

<400> 721
ttgggggatc ttacagatag 20

<210> 722
<211> 21
<212> DNA
<213> artificial

<220>
<221> source
<222> (1)..(21)
<223> /note=«Description of artificial sequence: Reverse primer, SA0392* Gene encoding putative HsdS Q99WH0»

<400> 722
ttgctgttct tctaaacttg g 21

<210> 723
<211> 20
<212> DNA
<213> artificial

<220>
<221> source
<222> (1)..(20)
<223> /note=«Description of artificial sequence: Forward primer, ORF10 gene encoding DNA Polymerase A domain from phage phi 12 Q8SDR7»

<400> 723
atttgaccct gctgtaaaaa 20

<210> 724
<211> 20
<212> DNA
<213> artificial

<220>
<221> source
<222> (1)..(20)
<223> /note=«Description of artificial sequence: Reverse primer, ORF10 gene encoding DNA Polymerase A domain from phage phi 12 Q8SDR7»

<400> 724
tctgcttatc gagcacatta 20

<210> 725
<211> 20
<212> DNA
<213> artificial

<220>
<221> source
<222> (1)..(20)
<223> /note=«Description of artificial sequence: Forward primer, ORF11 gene encoding APSE P51 from phage phi 12 Q8SDR9»

<400> 725
gagcaaaaca atggctaaac 20

<210> 726
<211> 20
<212> DNA
<213> artificial

<220>
<221> source
<222> (1)..(20)
<223> /note=«Description of artificial sequence: Reverse primer, ORF11 gene encoding APSE P51 from phage phi 12 Q8SDR9»

<400> 726
ttcaatgcct ttaccgtatt 20

<210> 727
<211> 20
<212> DNA

<213> artificial

<220>
<221> source
<222> (1)..(20)
<223> /note=«Description of artificial sequence: Forward primer, MW0120* gene encoding replication protein Q8NYQ5»

<400> 727
gattgttggg aatcttagca 20

<210> 728
<211> 20
<212> DNA
<213> artificial

<220>
<221> source
<222> (1)..(20)
<223> /note=«Description of artificial sequence: Reverse primer, MW0120* gene encoding replication protein Q8NYQ5»

<400> 728
cactattacg cacaccaaaa 20

<210> 729
<211> 20
<212> DNA
<213> artificial

<220>
<221> source

<222> (1)..(20)
<223> /note=«Description of artificial sequence: Forward primer, ORF31gene encoding ORF CN004»

<400> 729
aatggaatag agttggcaga 20

<210> 730
<211> 20
<212> DNA
<213> artificial

<220>
<221> source
<222> (1)..(20)
<223> /note=«Description of artificial sequence: Reverse primer, ORF31gene encoding ORF CN004»

<400> 730
ctatcttcag ttggcgtttc 20

<210> 731
<211> 20
<212> DNA

<213> artificial

<220>
<221> source
<222> (1)..(20)
<223> /note=«Description of artificial sequence: Forward primer, ORF32 gene encoding ORF beta Q51989»

<400> 731
gagcgaaaaa gtagaacgaa 20

<210> 732
<211> 20
<212> DNA
<213> artificial

<220>
<221> source
<222> (1)..(20)
<223> /note=«Description of artificial sequence: Reverse primer, ORF32 gene encoding ORF beta Q51989»

<400> 732
agccataaca gttcttgacg 20

<210> 733
<211> 20
<212> DNA
<213> artificial

<220>
<221> source
<222> (1)..(20)
<223> /note=«Description of artificial sequence: Forward primer, MW0062* gene encoding Q8NYV1»

<400> 733
caaaactaac gactcccaac 20

<210> 734
<211> 20
<212> DNA
<213> artificial

<220>
<221> source
<222> (1)..(20)
<223> /note=«Description of artificial sequence: Reverse primer, MW0062* gene encoding Q8NYV1»

<400> 734
tcctctgaat gagttggttt 20

<210> 735
<211> 20
<212> DNA
<213> artificial

<220>
<221> source
<222> (1)..(20)
<223> /note=«Description of artificial sequence: Forward primer, MW0063* gene encoding Q8NYV0»

<400> 735
aaaagatggc aattcagcta 20

<210> 736
<211> 21
<212> DNA
<213> artificial

<220>
<221> source
<222> (1)..(21)
<223> /note=«Description of artificial sequence: Reverse primer, MW0063* gene encoding Q8NYV0»

<400> 736
tgcgagctct tctataaatt g 21

<210> 737
<211> 20
<212> DNA
<213> artificial

<220>
<221> source
<222> (1)..(20)
<223> /note=«Description of artificial sequence: Forward primer, MW0064* Gene encoding Q8NYU9»

<400> 737
aggattcgat aaaagggaac 20

<210> 738
<211> 20
<212> DNA
<213> artificial

<220>
<221> source
<222> (1)..(20)
<223> /note=«Description of artificial sequence: Reverse primer, MW0064* gene encoding Q8NYU9»

<400> 738
ttttcgtctg tgataaggct 20

<210> 739
<211> 21
<212> DNA
<213> artificial

<220>
<221> source
<222> (1)..(21)
<223> /note=«Description of artificial sequence: Forward primer, MW0065* gene encoding Q8NYU8»

<400> 739
gcaatgcaat atcatgttaa g 21

<210> 740
<211> 19
<212> DNA
<213> artificial

<220>
<221> source
<222> (1)..(19)
<223> /note=«Description of artificial sequence: Reverse primer, MW0065* gene encoding Q8NYU8»

<400> 740
gcaactgcat tatcgaaga 19

<210> 741
<211> 20
<212> DNA
<213> artificial

<220>
<221> source
<222> (1)..(20)
<223> /note=«Description of artificial sequence: Forward primer, MW0066*gene encoding Q8NYU7»

<400> 741
aaaggtgcta aggcaactaa 20

<210> 742
<211> 20
<212> DNA
<213> artificial

<220>
<221> source
<222> (1)..(20)
<223> /note=«Description of artificial sequence: Reverse primer, MW0066*gene encoding Q8NYU7»

<400> 742
attacccgtt gtgaatcaag 20

<210> 743
<211> 21
<212> DNA
<213> artificial

<220>

<221> source
<222> (1)..(21)
<223> /note=«Description of artificial sequence: Forward primer, MW0552 gene encoding Q8NXV6»

<400> 743
tgcaaaactt agtgagaagg a 21

<210> 744
<211> 20
<212> DNA
<213> artificial

<220>
<221> source
<222> (1)..(20)
<223> /note=«Description of artificial sequence: Reverse primer, MW0552 gene encoding Q8NXV6»

<400> 744
ttactaattg agcccatgct 20

<210> 745
<211> 18
<212> DNA
<213> artificial

<220>
<221> source
<222> (1)..(18)
<223> /note=«Description of artificial sequence: Forward primer, MW0554 gene encoding Q8NXV4»

<400> 745
aggtgcgcca aaattagt 18

<210> 746
<211> 21
<212> DNA
<213> artificial

<220>
<221> source
<222> (1)..(21)
<223> /note=«Description of artificial sequence: Reverse primer, MW0554 gene encoding Q8NXV4»

<400> 746
aagaaaaaca acattaaccc c 21

<210> 747
<211> 20
<212> DNA
<213> artificial

<220>
<221> source

<222> (1)..(20)
<223> /note=«Description of artificial sequence: Forward primer, MW0559 gene encoding Q8NXU9»

<400> 747
tgcgatgtca gagtcattta 20

<210> 748
<211> 20
<212> DNA
<213> artificial

<220>
<221> source
<222> (1)..(20)
<223> /note=«Description of artificial sequence: Reverse primer, MW0559 gene encoding Q8NXU9»

<400> 748
aaaaacttcc agcgaaaata 20

<210> 749
<211> 20
<212> DNA
<213> artificial

<220>
<221> source
<222> (1)..(20)
<223> /note=«Description of artificial sequence: Forward primer, MW0560 gene encoding Q8NXU8»

<400> 749
tagcactctt tttcccaatg 20

<210> 750
<211> 22
<212> DNA
<213> artificial

<220>
<221> source
<222> (1)..(22)
<223> /note=«Description of artificial sequence: Reverse primer, MW0560 gene encoding Q8NXU8»

<400> 750
tgaacttcaa tcgtcaataa ca 22

<210> 751
<211> 20
<212> DNA
<213> artificial

<220>
<221> source
<222> (1)..(20)

<223> /note=«Description of artificial sequence: Forward primer, MW1405 gene encoding Q8NWJ3»

<400> 751
ggctagggaa aacagtatca 20

<210> 752
<211> 20
<212> DNA
<213> artificial

<220>
<221> source
<222> (1)..(20)
<223> /note=«Description of artificial sequence: Reverse primer, MW1405 gene encoding Q8NWJ3»

<400> 752
tcaaataaac ttgagcccat 20

<210> 753
<211> 20
<212> DNA
<213> artificial

<220>
<221> source
<222> (1)..(20)
<223> /note=«Description of artificial sequence: Forward primer, MW1742 gene encoding Q8NVY7»

<400> 753
gttcgattat ttcaggcatt 20

<210> 754
<211> 20
<212> DNA
<213> artificial

<220>
<221> source
<222> (1)..(20)
<223> /note=«Description of artificial sequence: Reverse primer, MW1742 gene encoding Q8NVY7»

<400> 754
atacagcctt aatccagcag 20

<210> 755
<211> 20
<212> DNA
<213> artificial

<220>
<221> source
<222> (1)..(20)
<223> /note=«Description of artificial sequence: Forward primer,

MW1744 gene encoding Q8NVY5»

<400> 755
cagatgtagc tcatgctgaa 20

<210> 756
<211> 20
<212> DNA
<213> artificial

<220>
<221> source
<222> (1)..(20)
<223> /note=«Description of artificial sequence: Reverse primer, MW1744 gene encoding Q8NVY5»

<400> 756
attttctcc tgtcagaacg 20

<210> 757
<211> 20
<212> DNA
<213> artificial

<220>
<221> source
<222> (1)..(20)
<223> /note=«Description of artificial sequence: Forward primer, MW1749* gene encoding Q8NVY0»

<400> 757
gaaacgatca acgaatcaag 20

<210> 758
<211> 20
<212> DNA
<213> artificial

<220>
<221> source
<222> (1)..(20)
<223> /note=«Description of artificial sequence: Reverse primer, MW1749* gene encoding Q8NVY0»

<400> 758
aaattcaaaa actcttcgca 20

<210> 759
<211> 20
<212> DNA
<213> artificial

<220>
<221> source
<222> (1)..(20)
<223> /note=«Description of artificial sequence: Forward primer, MW1757 gene encoding Q8NVX3»

<400> 759
taaaacggct aaagcagaag 20

<210> 760
<211> 20
<212> DNA
<213> artificial

<220>
<221> source
<222> (1)..(20)
<223> /note=«Description of artificial sequence: Reverse primer, MW1757 gene encoding Q8NVX3»

<400> 760
attttcgcca ccactagtta 20

<210> 761
<211> 20
<212> DNA
<213> artificial

<220>
<221> source
<222> (1)..(20)
<223> /note=«Description of artificial sequence: Forward primer, bsaG gene encoding Q8NVX2»

<400> 761
aaagttggcg attaaaatga 20

<210> 762
<211> 20
<212> DNA
<213> artificial

<220>
<221> source
<222> (1)..(20)
<223> /note=«Description of artificial sequence: Reverse primer, bsaG gene encoding Q8NVX2»

<400> 762
aaaactccga caacaatagc 20

<210> 763
<211> 20
<212> DNA
<213> artificial

<220>
<221> source
<222> (1)..(20)
<223> /note=«Description of artificial sequence: Forward primer, bsaE gene encoding Q8NVX1»

<400> 763
ccaaaagtaa tacgggaaca 20

<210> 764
<211> 20
<212> DNA
<213> artificial

<220>
<221> source
<222> (1)..(20)
<223> /note=«Description of artificial sequence: Reverse primer, bsaE gene encoding Q8NVX1»

<400> 764
gcaacaccca aaataaactc 20

<210> 765
<211> 20
<212> DNA
<213> artificial

<220>
<221> source
<222> (1)..(20)
<223> /note=«Description of artificial sequence: Forward primer, bsaF gene encoding Q8NVX0»

<400> 765
cacaaagttg gttcgcttat 20

<210> 766
<211> 20
<212> DNA
<213> artificial

<220>
<221> source
<222> (1)..(20)
<223> /note=«Description of artificial sequence: Reverse primer, bsaF gene encoding Q8NVX0»

<400> 766
tttagctcac cctcatgaat 20

<210> 767
<211> 20
<212> DNA
<213> artificial

<220>
<221> source
<222> (1)..(20)
<223> /note=«Description of artificial sequence: Forward primer, bsaP gene encoding Q8NVW9»

<400> 767

tgtggcacag attaaaatga 20

<210> 768
<211> 20
<212> DNA
<213> artificial

<220>
<221> source
<222> (1)..(20)
<223> /note=«Description of artificial sequence: Reverse primer, bsaP gene encoding Q8NVW9»

<400> 768
cggtgcaaca cctattaact 20

<210> 769
<211> 20
<212> DNA
<213> artificial

<220>
<221> source
<222> (1)..(20)
<223> /note=«Description of artificial sequence: Forward primer, bsaD gene encoding Q8NVW8»

<400> 769
ttgtttgtgt ggctcagtta 20

<210> 770
<211> 20
<212> DNA
<213> artificial

<220>
<221> source
<222> (1)..(20)
<223> /note=«Description of artificial sequence: Reverse primer, bsaD gene encoding Q8NVW8»

<400> 770
gtgcgacaac attcttttta 20

<210> 771
<211> 20
<212> DNA
<213> artificial

<220>
<221> source
<222> (1)..(20)
<223> /note=«Description of artificial sequence: Forward primer, bsaC gene encoding Q8NVW7»

<400> 771
aaaagcatct actgaaaccg 20

<210> 772
<211> 20
<212> DNA
<213> artificial

<220>
<221> source
<222> (1)..(20)
<223> /note=«Description of artificial sequence: Reverse primer, bsaC gene encoding Q8NVW7»

<400> 772
aaattgactt tcttgtggga 20

<210> 773
<211> 20
<212> DNA
<213> artificial

<220>
<221> source
<222> (1)..(20)
<223> /note=«Description of artificial sequence: Forward primer, bsaB gene encoding Q8NVW6»

<400> 773
acagcatctt gaagaattgg 20

<210> 774
<211> 20
<212> DNA
<213> artificial

<220>
<221> source
<222> (1)..(20)
<223> /note=«Description of artificial sequence: Reverse primer, bsaB gene encoding Q8NVW6»

<400> 774
aatttggttg ccatctttta 20

<210> 775
<211> 20
<212> DNA
<213> artificial

<220>
<221> source
<222> (1)..(20)
<223> /note=«Description of artificial sequence: Forward primer, MW2534 Gene encoding Q9L4Q0»

<400> 775
aagttgaagc cccttacttt 20

<210> 776
<211> 20
<212> DNA
<213> artificial

<220>
<221> source
<222> (1)..(20)
<223> /note=«Description of artificial sequence: Reverse primer, MW2534 Gene encoding Q9L4Q0»

<400> 776
gccctcacgt actctatcaa 20

<210> 777
<211> 20
<212> DNA
<213> artificial

<220>
<221> source
<222> (1)..(20)
<223> /note=«Description of artificial sequence: Forward primer, SA2483* Gene encoding Q99QV0»

<400> 777
ttattggaag agctcgaaga 20

<210> 778
<211> 20
<212> DNA
<213> artificial

<220>
<221> source
<222> (1)..(20)
<223> /note=«Description of artificial sequence: Reverse primer, SA2483* Gene encoding Q99QV0»

<400> 778
taacccaacg atgaaactct 20

<210> 779
<211> 20
<212> DNA
<213> artificial

<220>
<221> source
<222> (1)..(20)
<223> /note=«Description of artificial sequence: Forward primer, SA2272* gene encoding Q99RE9»

<400> 779
tgaaaagtac gatggtcaca 20

<210> 780
<211> 20
<212> DNA
<213> artificial

<220>
<221> source
<222> (1)..(20)
<223> /note=«Description of artificial sequence: Reverse primer, SA2272* gene encoding Q99RE9»

<400> 780
ttttcaccaa tttcatttcc 20

<210> 781
<211> 20
<212> DNA
<213> artificial

<220>
<221> source
<222> (1)..(20)
<223> /note=«Description of artificial sequence: Forward primer, SA1829 gene encoding Q99SM4»

<400> 781
gcaattacat gaagcaatca 20

<210> 782
<211> 20
<212> DNA
<213> artificial

<220>
<221> source
<222> (1)..(20)
<223> /note=«Description of artificial sequence: Reverse primer, SA1829 gene encoding Q99SM4»

<400> 782
catctcaata aatgcccaat 20

<210> 783
<211> 20
<212> DNA
<213> artificial

<220>
<221> source
<222> (1)..(20)
<223> /note=«Description of artificial sequence: Forward primer, SA1826* gene encoding Q99SM7»

<400> 783
catagtaaag tgcaaccgtg 20

<210> 784

<211> 20
<212> DNA
<213> artificial

<220>
<221> source
<222> (1)..(20)
<223> /note=«Description of artificial sequence: Reverse primer, SA1826* gene encoding Q99SM7»

<400> 784
tgtcgcattc tcatcttgta 20

<210> 785
<211> 20
<212> DNA
<213> artificial

<220>
<221> source
<222> (1)..(20)
<223> /note=«Description of artificial sequence: Forward primer, SA1822 gene encoding Q99SN1»

<400> 785
attaacgcaa tttaacccag 20

<210> 786
<211> 20
<212> DNA
<213> artificial

<220>
<221> source
<222> (1)..(20)
<223> /note=«Description of artificial sequence: Reverse primer, SA1822 gene encoding Q99SN1»

<400> 786
tggtgatatg catttactcg 20

<210> 787
<211> 20
<212> DNA
<213> artificial

<220>
<221> source
<222> (1)..(20)
<223> /note=«Description of artificial sequence: Forward primer, SA1791 gene encoding Q99SR0»

<400> 787
gaccttttta actggtcacg 20

<210> 788
<211> 20

<212> DNA
<213> artificial

<220>
<221> source
<222> (1)..(20)
<223> /note=«Description of artificial sequence: Reverse primer, SA1791 gene encoding Q99SR0»

<400> 788
aagtttgcat taagccatgt 20

<210> 789
<211> 20
<212> DNA
<213> artificial

<220>
<221> source
<222> (1)..(20)
<223> /note=«Description of artificial sequence: Forward primer, SA1208 gene encoding Q99UA6»

<400> 789
ggatattacg gtttggatca 20

<210> 790
<211> 20
<212> DNA
<213> artificial

<220>
<221> source
<222> (1)..(20)
<223> /note=«Description of artificial sequence: Reverse primer, SA1208 gene encoding Q99UA6»

<400> 790
tttgtgcaga ttgagtttga 20

<210> 791
<211> 20
<212> DNA
<213> artificial

<220>
<221> source
<222> (1)..(20)
<223> /note=«Description of artificial sequence: Forward primer, SA0745 gene encoding Q99VJ1»

<400> 791
acaatgggtg taatttgtgc 20

<210> 792

<211> 20
<212> DNA
<213> artificial

<220>
<221> source
<222> (1)..(20)
<223> /note=«Description of artificial sequence: Reverse primer, SA0745 gene encoding Q99VJ1»

<400> 792
cttacttttg cagttgcctt 20

<210> 793
<211> 20
<212> DNA
<213> artificial

<220>
<221> source
<222> (1)..(20)
<223> /note=«Description of artificial sequence: Forward primer, SA0197* gene encoding Q99X18»

<400> 793
ttagaagtgg gagcatcaat 20

<210> 794
<211> 20
<212> DNA
<213> artificial

<220>
<221> source
<222> (1)..(20)
<223> /note=«Description of artificial sequence: Reverse primer, SA0197* gene encoding Q99X18»

<400> 794
atccctttaa aattcgtgtg 20

<210> 795
<211> 20
<212> DNA
<213> artificial

<220>
<221> source
<222> (1)..(20)
<223> /note=«Description of artificial sequence: Forward primer, SA0196* gene encoding Q99X19»

<400> 795
agtcttggag aaagagggat 20

<210> 796
<211> 25

<212> DNA
<213> artificial

<220>
<221> source
<222> (1)..(25)
<223> /note=«Description of artificial sequence: Reverse primer, SA0196* gene encoding Q99X19»

<400> 796
ccattctttg ataaaggtat gataa 25

<210> 797
<211> 19
<212> DNA
<213> artificial

<220>
<221> source
<222> (1)..(19)
<223> /note=«Description of artificial sequence: Forward primer, SA0195* gene encoding Q99X20»

<400> 797
ttgggaaagc ctcttttta 19

<210> 798
<211> 21
<212> DNA
<213> artificial

<220>
<221> source
<222> (1)..(21)
<223> /note=«Description of artificial sequence: Reverse primer, SA0195* gene encoding Q99X20»

<400> 798
gccataccaa ctatcaacaa a 21

<210> 799
<211> 20
<212> DNA
<213> artificial

<220>
<221> source
<222> (1)..(20)
<223> /note=«Description of artificial sequence: Forward primer, SA0193* gene encoding Q99X22»

<400> 799
aactgctgct gatcaatttt 20

<210> 800
<211> 20

<212> DNA
<213> artificial

<220>
<221> source
<222> (1)..(20)
<223> /note=«Description of artificial sequence: Reverse primer, SA0193* gene encoding Q99X22»

<400> 800
ccttgatgta agtgtcccat 20

<210> 801
<211> 20
<212> DNA
<213> artificial

<220>
<221> source
<222> (1)..(20)
<223> /note=«Description of artificial sequence: Forward primer, SA0192* gene encoding Q99X23»

<400> 801
tcaattggca aacataagaa 20

<210> 802
<211> 20
<212> DNA
<213> artificial

<220>
<221> source
<222> (1)..(20)
<223> /note=«Description of artificial sequence: Reverse primer, SA0192* gene encoding Q99X23»

<400> 802
cgatcagctt tttctgctac 20

<210> 803
<211> 20
<212> DNA
<213> artificial

<220>
<221> source
<222> (1)..(20)
<223> /note=«Description of artificial sequence: Forward primer, SA0191 gene encoding Q99X24»

<400> 803
ggacataaat tgaggcgtaa 20

<210> 804
<211> 20
<212> DNA

<213> artificial

<220>
<221> source
<222> (1)..(20)
<223> /note=«Description of artificial sequence: Reverse primer, SA0191 gene encoding Q99X24»

<400> 804
tgatacaatt gctgttgagc 20

<210> 805
<211> 20
<212> DNA
<213> artificial

<220>
<221> source
<222> (1)..(20)
<223> /note=«Description of artificial sequence: Forward primer, SA0190 gene encoding Q99X25»

<400> 805
aatcactttg caaatatccg 20

<210> 806
<211> 20
<212> DNA
<213> artificial

<220>
<221> source
<222> (1)..(20)
<223> /note=«Description of artificial sequence: Reverse primer, SA0190 gene encoding Q99X25»

<400> 806
agtcgaaagt acccagaatg 20

<210> 807
<211> 20
<212> DNA
<213> artificial

<220>
<221> source
<222> (1)..(20)
<223> /note=«Description of artificial sequence: Forward primer, SA0104 gene encoding Q99XA5»

<400> 807
tactaaagtg gggagtggaa 20

<210> 808
<211> 20
<212> DNA
<213> artificial

<220>
<221> source
<222> (1)..(20)
<223> /note=«Description of artificial sequence: Reverse primer, SA0104 gene encoding Q99XA5»

<400> 808
catcttgacc taaaaaccca 20

<210> 809
<211> 20
<212> DNA
<213> artificial

<220>
<221> source
<222> (1)..(20)
<223> /note=«Description of artificial sequence: Forward primer, SA0103 gene encoding Q99XA6»

<400> 809
actcgtcgca tttataggaa 20

<210> 810
<211> 20
<212> DNA
<213> artificial

<220>
<221> source
<222> (1)..(20)
<223> /note=«Description of artificial sequence: Reverse primer, SA0103 gene encoding Q99XA6»

<400> 810
aatagcaagg gttgttggta 20

<210> 811
<211> 20
<212> DNA
<213> artificial

<220>
<221> source
<222> (1)..(20)
<223> /note=«Description of artificial sequence: Forward primer, MW1748 gene encoding Q8NVY1»

<400> 811
aaaaccaaca atcatatggg 20

<210> 812
<211> 20
<212> DNA
<213> artificial

<220>
<221> source
<222> (1)..(20)
<223> /note=«Description of artificial sequence: Reverse primer, MW1748 gene encoding Q8NVY1»

<400> 812
attggacgta ttttgctctt 20

<210> 813
<211> 20
<212> DNA
<213> artificial

<220>
<221> source
<222> (1)..(20)
<223> /note=«Description of artificial sequence: Forward primer, MW1408 gene encoding Q8NWJ0»

<400> 813
tatgcagaag ctagcacaaa 20

<210> 814
<211> 20
<212> DNA
<213> artificial

<220>
<221> source
<222> (1)..(20)
<223> /note=«Description of artificial sequence: Reverse primer, MW1408 gene encoding Q8NWJ0»

<400> 814
tgcatcattg ctagttgaag 20

<210> 815
<211> 20
<212> DNA
<213> artificial

<220>
<221> source
<222> (1)..(20)
<223> /note=«Description of artificial sequence: Forward primer, MW0558 gene encoding Q8NXV0»

<400> 815
cgaaaatgaa tttgaaaggt 20

<210> 816
<211> 20
<212> DNA
<213> artificial

<220>

<221> source
<222> (1)..(20)
<223> /note=«Description of artificial sequence: Reverse primer, MW0558 gene encoding Q8NXV0»

<400> 816
atcgaagcca tattcactaa 20

<210> 817
<211> 20
<212> DNA
<213> artificial

<220>
<221> source
<222> (1)..(20)
<223> /note=«Description of artificial sequence: Forward primer, MW0553* gene encoding Q8NXV5»

<400> 817
tatgtccaaa tcagcagaga 20

<210> 818
<211> 20
<212> DNA
<213> artificial

<220>
<221> source
<222> (1)..(20)
<223> /note=«Description of artificial sequence: Reverse primer, MW0553* gene encoding Q8NXV5»

<400> 818
ttgtcataat aacccaagca 20

<210> 819
<211> 20
<212> DNA
<213> artificial

<220>
<221> source
<222> (1)..(20)
<223> /note=«Description of artificial sequence: Forward primer, MW0171 gene encoding Q8NYL7»

<400> 819
taaattgagg cgtaaaagga 20

<210> 820
<211> 20
<212> DNA
<213> artificial

<220>
<221> source

<222> (1)..(20)
<223> /note=«Description of artificial sequence: Reverse primer, MW0171 gene encoding Q8NYL7»

<400> 820
cttaaatatt ggagatgccg 20

<210> 821
<211> 20
<212> DNA
<213> artificial

<220>
<221> source
<222> (1)..(20)
<223> /note=«Description of artificial sequence: Forward primer, SAV0866 gene encoding Q932A6»

<400> 821
caggtcaacc aatggactat 20

<210> 822
<211> 20
<212> DNA
<213> artificial

<220>
<221> source
<222> (1)..(20)
<223> /note=«Description of artificial sequence: Reverse primer, SAV0866 gene encoding Q932A6»

<400> 822
ctttttctct cctttcagca 20

<210> 823
<211> 20
<212> DNA
<213> artificial

<220>
<221> source
<222> (1)..(20)
<223> /note=«Description of artificial sequence: Forward primer, SAV0850 gene encoding Q932C0»

<400> 823
atttccaacc tggatgctat 20

<210> 824
<211> 20
<212> DNA
<213> artificial

<220>
<221> source
<222> (1)..(20)

<223> /note=«Description of artificial sequence: Reverse primer, SAV0850 gene encoding Q932C0»

<400> 824
tttaaaaact ctcaacggct 20

<210> 825
<211> 20
<212> DNA
<213> artificial

<220>
<221> source
<222> (1)..(20)
<223> /note=«Description of artificial sequence: Forward primer, mecA gene encoding Penicillin-binding protein PLP2a»

<400> 825
gactgaacgt ccgataaaaa 20

<210> 826
<211> 20
<212> DNA
<213> artificial

<220>
<221> source
<222> (1)..(20)
<223> /note=«Description of artificial sequence: Reverse primer, mecA gene encoding Penicillin-binding protein PLP2a»

<400> 826
tgtacccaat tttgatccat 20

<210> 827
<211> 20
<212> DNA
<213> artificial

<220>
<221> source
<222> (1)..(20)
<223> /note=«Description of artificial sequence: Forward primer, mecR1-2 gene encoding Q53709 MecR1»

<400> 827
tcagttcatt gctcacgata 20

<210> 828
<211> 20
<212> DNA
<213> artificial

<220>
<221> source
<222> (1)..(20)
<223> /note=«Description of artificial sequence: Reverse primer,

mecR1-2 gene encoding Q53709 MecR1»

<400> 828
ccatcggatt atcaatgttt 20

<210> 829
<211> 21
<212> DNA
<213> artificial

<220>
<221> source
<222> (1)..(21)
<223> /note=«Description of artificial sequence: Forward primer, fosB* gene encoding Protein related to Fos»

<400> 829
cgtcatttca aaaatcgata c 21

<210> 830
<211> 20
<212> DNA
<213> artificial

<220>
<221> source
<222> (1)..(20)
<223> /note=«Description of artificial sequence: Reverse primer, fosB* gene encoding Protein related to Fos»

<400> 830
tgttctcaag tgtgccagta 20

<210> 831
<211> 20
<212> DNA
<213> artificial

<220>
<221> source
<222> (1)..(20)
<223> /note=«Description of artificial sequence: Forward primer, entA = entP gene encoding Enterotoxin A»

<400> 831
cgagaaaagc gaagaaataa 20

<210> 832
<211> 20
<212> DNA
<213> artificial

<220>
<221> source
<222> (1)..(20)
<223> /note=«Description of artificial sequence: Reverse primer, entA = entP gene encoding Enterotoxin A»

<400> 832
tcttgcttga agatccaact 20

<210> 833
<211> 20
<212> DNA
<213> artificial

<220>
<221> source
<222> (1)..(20)
<223> /note=«Description of artificial sequence: Forward primer, pro 8325 gene encoding Putative protease»

<400> 833
taaattcatc tggaggcagt 20

<210> 834
<211> 20
<212> DNA
<213> artificial

<220>
<221> source
<222> (1)..(20)
<223> /note=«Description of artificial sequence: Reverse primer, pro 8325 gene encoding Putative protease»

<400> 834
cgaaacgctt atattgctct 20

<210> 835
<211> 20
<212> DNA
<213> artificial

<220>
<221> source
<222> (1)..(20)
<223> /note=«Description of artificial sequence: Forward primer, agrB-8325 gene encoding AgrB (NCTC8325 variable domain)»

<400> 835
ttgcaagtac gattagggat 20

<210> 836
<211> 19
<212> DNA
<213> artificial

<220>
<221> source
<222> (1)..(19)
<223> /note=«Description of artificial sequence: Reverse primer, agrB-8325 gene encoding AgrB (NCTC8325 variable domain)»

<400> 836
taattgaatg aattgggca 19

<210> 837
<211> 20
<212> DNA
<213> artificial

<220>
<221> source
<222> (1)..(20)
<223> /note=«Description of artificial sequence: Forward primer, SAV0039 gene encoding Phosphodiesterase Q9S3K5»

<400> 837
agatgaacat ttgccaactt 20

<210> 838
<211> 20
<212> DNA
<213> artificial

<220>
<221> source
<222> (1)..(20)
<223> /note=«Description of artificial sequence: Reverse primer, SAV0039 gene encoding Phosphodiesterase Q9S3K5»

<400> 838
aaatgcacca ttttatctgc 20

<210> 839
<211> 20
<212> DNA
<213> artificial

<220>
<221> source
<222> (1)..(20)
<223> /note=«Description of artificial sequence: Forward primer, tnp IS 431 gene encoding IS 431 transposase»

<400> 839
tgttatcact gtagccgttg 20

<210> 840
<211> 20
<212> DNA
<213> artificial

<220>
<221> source
<222> (1)..(20)
<223> /note=«Description of artificial sequence: Reverse primer, tnp IS 431 gene encoding IS 431 transposase»

<400> 840

aatatgacgg tgatcttgct 20

<210> 841
<211> 20
<212> DNA
<213> artificial

<220>
<221> source
<222> (1)..(20)
<223> /note=«Description of artificial sequence: Forward primer, CAC3531 gene encoding IS605/IS200-like transposase Q97DE6»

<400> 841
aaaatgcatc tctacgtgct 20

<210> 842
<211> 20
<212> DNA
<213> artificial

<220>
<221> source
<222> (1)..(20)
<223> /note=«Description of artificial sequence: Reverse primer, CAC3531 gene encoding IS605/IS200-like transposase Q97DE6»

<400> 842
ctgcaacgat atcctcttgt 20

<210> 843
<211> 20
<212> DNA
<213> artificial

<220>
<221> source
<222> (1)..(20)
<223> /note=«Description of artificial sequence: Forward primer, SA1622 gene encoding Truncated tansposase Q99T66»

<400> 843
tcgcgctttt aatagagaac 20

<210> 844
<211> 20
<212> DNA
<213> artificial

<220>
<221> source
<222> (1)..(20)
<223> /note=«Description of artificial sequence: Reverse primer, SA1622 gene encoding Truncated tansposase Q99T66»

<400> 844
tggcgtcaaa tactgtaatg 20

<210> 845
<211> 20
<212> DNA
<213> artificial

<220>
<221> source
<222> (1)..(20)
<223> /note=«Description of artificial sequence: Forward primer, ORF5* gene encoding Peptidoglycan hydrolase from phage phi 12 Q8SDN4»

<400> 845
cagtgttacg attacgcaaa 20

<210> 846
<211> 20
<212> DNA
<213> artificial

<220>
<221> source
<222> (1)..(20)
<223> /note=«Description of artificial sequence: Reverse primer, ORF5* gene encoding Peptidoglycan hydrolase from phage phi 12 Q8SDN4»

<400> 846
cggctacaag cataatttt 20

<210> 847
<211> 20
<212> DNA
<213> artificial

<220>
<221> source
<222> (1)..(20)
<223> /note=«Description of artificial sequence: Forward primer, ORF6 gene encoding SLT Orf 488 from phage phi 12 Q8SDN8»

<400> 847
atttagaaaa cacagcgcat 20

<210> 848
<211> 20
<212> DNA
<213> artificial

<220>
<221> source
<222> (1)..(20)
<223> /note=«Description of artificial sequence: Reverse primer, ORF6 gene encoding SLT Orf 488 from phage phi 12 Q8SDN8»

<400> 848
actcgcataa tcttcaccat 20

<210> 849
<211> 20
<212> DNA
<213> artificial

<220>
<221> source
<222> (1)..(20)
<223> /note=«Description of artificial sequence: Forward primer, ORF7 gene encoding SLT Orf 527 from phage phi 12 Q8SDP1»

<400> 849
acacaattaa gcagacagcg 20

<210> 850
<211> 20
<212> DNA
<213> artificial

<220>
<221> source
<222> (1)..(20)
<223> /note=«Description of artificial sequence: Reverse primer, ORF7 gene encoding SLT Orf 527 from phage phi 12 Q8SDP1»

<400> 850
taatttttct tttcctcggc 20

<210> 851
<211> 20
<212> DNA
<213> artificial

<220>
<221> source
<222> (1)..(20)
<223> /note=«Description of artificial sequence: Forward primer, ORF8 gene encoding Tail fiber protein from phage phi 12 Q8SDP3»

<400> 851
gaccatttag gtgtccaaga 20

<210> 852
<211> 20
<212> DNA
<213> artificial

<220>
<221> source
<222> (1)..(20)
<223> /note=«Description of artificial sequence: Reverse primer, ORF8 gene encoding Tail fiber protein from phage phi

12 Q8SDP3»

<400> 852
tgttctgcat ctctcaactg 20

<210> 853
<211> 20
<212> DNA
<213> artificial

<220>
<221> source
<222> (1)..(20)
<223> /note=«Description of artificial sequence: Forward primer, MW1393 Gene encoding Major tail protein Q8NWK5»

<400> 853
ttggagtttt taacccagaa 20

<210> 854
<211> 20
<212> DNA
<213> artificial

<220>
<221> source
<222> (1)..(20)
<223> /note=«Description of artificial sequence: Reverse primer, MW1393 Gene encoding Major tail protein Q8NWK5»

<400> 854
ataccttgtg acgttccatc 20

<210> 855
<211> 20
<212> DNA
<213> artificial

<220>
<221> source
<222> (1)..(20)
<223> /note=«Description of artificial sequence: Forward primer, ORF9 gene encoding SLT Orf 37 from phage phi 12 Q8SDP8»

<400> 855
tgaattgagt cagaaagcaa 20

<210> 856
<211> 20
<212> DNA
<213> artificial

<220>
<221> source
<222> (1)..(20)
<223> /note=«Description of artificial sequence: Reverse primer, ORF9 gene encoding SLT Orf 37 from phage phi 12 Q8SDP8»

<400> 856
ttttgctgtt tctacgtctg 20

<210> 857
<211> 20
<212> DNA
<213> artificial

<220>
<221> source
<222> (1)..(20)
<223> /note=«Description of artificial sequence: Forward primer, MW1400 Gene encoding Portal protein Q8NWJ8»

<400> 857
aattggattg atcagtcagc 20

<210> 858
<211> 20
<212> DNA
<213> artificial

<220>
<221> source
<222> (1)..(20)
<223> /note=«Description of artificial sequence: Reverse primer, MW1400 Gene encoding Portal protein Q8NWJ8»

<400> 858
cggactaata ccttgaacca 20

<210> 859
<211> 20
<212> DNA
<213> artificial

<220>
<221> source
<222> (1)..(20)
<223> /note=«Description of artificial sequence: Forward primer, MW1401* gene encoding Terminase large subunit Q8NWJ7»

<400> 859
ggtggattcg gtgactatta 20

<210> 860
<211> 20
<212> DNA
<213> artificial

<220>
<221> source
<222> (1)..(20)

<223> /note=«Description of artificial sequence: Reverse primer, MW1401* gene encoding Terminase large subunit Q8NWJ7»

<400> 860
cgtttgctaa aagatggatt 20

<210> 861
<211> 20
<212> DNA
<213> artificial

<220>
<221> source
<222> (1)..(20)
<223> /note=«Description of artificial sequence: Forward primer, MW1386 gene encoding Q8NWL2»

<400> 861
aaaggatttg ggagctttag 20

<210> 862
<211> 20
<212> DNA
<213> artificial

<220>
<221> source
<222> (1)..(20)
<223> /note=«Description of artificial sequence: Reverse primer, MW1386 gene encoding Q8NWL2»

<400> 862
tatcaaacca gaaggattgc 20

<210> 863
<211> 20
<212> DNA
<213> artificial

<220>
<221> source
<222> (1)..(20)
<223> /note=«Description of artificial sequence: Forward primer, MW2125 gene encoding Q8NVC6»

<400> 863
gttttcgcct tctgattcta 20

<210> 864
<211> 20
<212> DNA
<213> artificial

<220>
<221> source
<222> (1)..(20)
<223> /note=«Description of artificial sequence: Reverse primer, MW2125 gene encoding Q8NVC6»

<400> 864
tcttcattat caaattccga 20

<210> 865
<211> 20
<212> DNA
<213> artificial

<220>
<221> source
<222> (1)..(20)
<223> /note=«Description of artificial sequence: Forward primer, SA2005 gene encoding Q99S65»

<400> 865
atggaaaaat tgttggaaaa 20

<210> 866
<211> 20
<212> DNA
<213> artificial

<220>
<221> source
<222> (1)..(20)
<223> /note=«Description of artificial sequence: Reverse primer, SA2005 gene encoding Q99S65»

<400> 866
aatggattgc ttcgactaaa 20

<210> 867
<211> 20
<212> DNA
<213> artificial

<220>
<221> source
<222> (1)..(20)
<223> /note=«Description of artificial sequence: Forward primer, SA2004 gene encoding Q99S66»

<400> 867
actttaaaat acgcagtgcc 20

<210> 868
<211> 20
<212> DNA
<213> artificial

<220>
<221> source
<222> (1)..(20)
<223> /note=«Description of artificial sequence: Reverse primer, SA2004 gene encoding Q99S66»

<400> 868

caaagacgta catgcttcaa 20

<210> 869
<211> 20
<212> DNA
<213> artificial

<220>
<221> source
<222> (1)..(20)
<223> /note=«Description of artificial sequence: Forward primer, SA1795 gene encoding Q99SQ6»

<400> 869
tttagacggt tcaaccaaat 20

<210> 870
<211> 20
<212> DNA
<213> artificial

<220>
<221> source
<222> (1)..(20)
<223> /note=«Description of artificial sequence: Reverse primer, SA1795 gene encoding Q99SQ6»

<400> 870
atttcgtgat ctccaagaat 20

<210> 871
<211> 20
<212> DNA
<213> artificial

<220>
<221> source
<222> (1)..(20)
<223> /note=«Description of artificial sequence: Forward primer, SA0378* gene encoding Q99WI6»

<400> 871
ttggaagtga gttttctggt 20

<210> 872
<211> 20
<212> DNA
<213> artificial

<220>
<221> source
<222> (1)..(20)
<223> /note=«Description of artificial sequence: Reverse primer, SA0378* gene encoding Q99WI6»

<400> 872

tttcgatttc attcatgtca 20

<210> 873
<211> 20
<212> DNA

<213> artificial

<220>
<221> source
<222> (1)..(20)
<223> /note=«Description of artificial sequence: Forward primer, SA0377* gene encoding Q99WI7»

<400> 873
acaaaaggga cgatacacac 20

<210> 874
<211> 19
<212> DNA
<213> artificial

<220>
<221> source
<222> (1)..(19)
<223> /note=«Description of artificial sequence: Reverse primer, SA0377* gene encoding Q99WI7»

<400> 874
aataaggcaa ttttccgtg 19

<210> 875
<211> 20
<212> DNA
<213> artificial

<220>
<221> source
<222> (1)..(20)
<223> /note=«Description of artificial sequence: Forward primer, MW0919 gene encoding Q8NXA7»

<400> 875
tggtatgacc tcttcgatgt 20

<210> 876
<211> 20
<212> DNA
<213> artificial

<220>
<221> source
<222> (1)..(20)
<223> /note=«Description of artificial sequence: Reverse primer, MW0919 gene encoding Q8NXA7»

<400> 876

tatgggtatc ccttttcatc 20

<210> 877
<211> 21
<212> DNA
<213> artificial

<220>
<221> source
<222> (1)..(21)
<223> /note=«Description of artificial sequence: Forward primer, SAV0804 Gene encoding Q932C8»

<400> 877
tggcttagat acaaggtttc a 21

<210> 878
<211> 21
<212> DNA
<213> artificial

<220>
<221> source
<222> (1)..(21)
<223> /note=«Description of artificial sequence: Reverse primer, SAV0804 gene encoding Q932C8»

<400> 878
cccacctttt cttatggtag t 21

<210> 879
<211> 20
<212> DNA
<213> artificial

<220>
<221> source
<222> (1)..(20)
<223> /note=«Description of artificial sequence: Forward primer, SAV0803 gene encoding Q932C9»

<400> 879
ttattttgac tgcgtgttca 20

<210> 880
<211> 20
<212> DNA
<213> artificial

<220>
<221> source
<222> (1)..(20)
<223> /note=«Description of artificial sequence: Reverse primer, SAV0803 gene encoding Q932C9»

<400> 880
ccactcttca atccattttt 20

<210> 881
<211> 20
<212> DNA
<213> artificial

<220>
<221> source
<222> (1)..(20)
<223> /note=«Description of artificial sequence: Forward primer, sei* gene encoding Enterotoxin I»

<400> 881
acctattgca aatcaactcg 20

<210> 882
<211> 20
<212> DNA
<213> artificial

<220>
<221> source
<222> (1)..(20)
<223> /note=«Description of artificial sequence: Reverse primer, sei* gene encoding Enterotoxin I»

<400> 882
aaacttacag gcagtccatc 20

<210> 883
<211> 20
<212> DNA
<213> artificial

<220>
<221> source
<222> (1)..(20)
<223> /note=«Description of artificial sequence: Forward primer, set8* gene encoding Exotoxin 8»

<400> 883
gcttaaagca gagcgattag 20

<210> 884
<211> 20
<212> DNA
<213> artificial

<220>
<221> source
<222> (1)..(20)
<223> /note=«Description of artificial sequence: Reverse primer, set8* gene encoding Exotoxin 8»

<400> 884
gtttgagcaa aaatccaaac 20

<210> 885
<211> 20
<212> DNA
<213> artificial

<220>
<221> source
<222> (1)..(20)
<223> /note=«Description of artificial sequence: Forward primer, set9 gene encoding Exotoxin 9»

<400> 885
gtgtaatcac aacgacaacg 20

<210> 886
<211> 20
<212> DNA
<213> artificial

<220>
<221> source
<222> (1)..(20)
<223> /note=«Description of artificial sequence: Reverse primer, set9 gene encoding Exotoxin 9»

<400> 886
accgagtatc tttccactcc 20

<210> 887
<211> 20
<212> DNA
<213> artificial

<220>
<221> source
<222> (1)..(20)
<223> /note=«Description of artificial sequence: Forward primer, set10 gene encoding Exotoxin 10»

<400> 887
gcaacaggaa caataacgtc 20

<210> 888
<211> 20
<212> DNA
<213> artificial

<220>
<221> source
<222> (1)..(20)
<223> /note=«Description of artificial sequence: Reverse primer, set10 gene encoding Exotoxin 10»

<400> 888
taccgtcacc ttcatctctc 20

<210> 889
<211> 17
<212> DNA
<213> artificial

<220>
<221> source
<222> (1)..(17)
<223> /note=«Description of artificial sequence: Forward primer, set11 gene encoding Exotoxin 11»

<400> 889
agaaaaacaa aatcacc 17

<210> 890
<211> 20
<212> DNA
<213> artificial

<220>
<221> source
<222> (1)..(20)
<223> /note=«Description of artificial sequence: Reverse primer, set11 gene encoding Exotoxin 11»

<400> 890
gctcgaattg cagtttatca 20

<210> 891
<211> 20
<212> DNA
<213> artificial

<220>
<221> source
<222> (1)..(20)
<223> /note=«Description of artificial sequence: Forward primer, set12 gene encoding Exotoxin 12»

<400> 891
cgttttgctt tttaaccaac 20

<210> 892
<211> 20
<212> DNA
<213> artificial

<220>
<221> source
<222> (1)..(20)
<223> /note=«Description of artificial sequence: Reverse primer, set12 gene encoding Exotoxin 12»

<400> 892
ccatacgctc gaaatctaat 20

<210> 893
<211> 21
<212> DNA
<213> artificial

<220>
<221> source
<222> (1)..(21)
<223> /note=«Description of artificial sequence: Forward primer, set15* gene encoding Exotoxin 15»

<400> 893
agggagagga tttgaattaa c 21

<210> 894
<211> 20
<212> DNA
<213> artificial

<220>
<221> source
<222> (1)..(20)
<223> /note=«Description of artificial sequence: Reverse primer, set15* gene encoding Exotoxin 15»

<400> 894
aatgtataat agccaccgtt 20

<210> 895
<211> 20
<212> DNA
<213> artificial

<220>
<221> source
<222> (1)..(20)

<223> /note=«Description of artificial sequence: Forward primer, lukD gene encoding Leucocidin D»

<400> 895
attagtactt aaggcagccg 20

<210> 896
<211> 20
<212> DNA
<213> artificial

<220>
<221> source
<222> (1)..(20)
<223> /note=«Description of artificial sequence: Reverse primer, lukD gene encoding Leucocidin D»

<400> 896
taaaggcatt tgatgtgttg 20

<210> 897
<211> 19
<212> DNA
<213> artificial

<220>
<221> source

<222> (1)..(19)
<223> /note=«Description of artificial sequence: Forward primer, sak gene encoding Staphylokinase»

<400> 897
gccatgctca aaagaagtt 19

<210> 898
<211> 20
<212> DNA
<213> artificial

<220>
<221> source
<222> (1)..(20)
<223> /note=«Description of artificial sequence: Reverse primer, sak gene encoding Staphylokinase»

<400> 898
aagttgaatc cagggttttt 20

<210> 899
<211> 20
<212> DNA
<213> artificial

<220>
<221> source
<222> (1)..(20)
<223> /note=«Description of artificial sequence: Forward primer, splA* gene encoding Serine protease SplA»

<400> 899
attaccgatg caactaagga 20

<210> 900
<211> 20
<212> DNA
<213> artificial

<220>
<221> source
<222> (1)..(20)
<223> /note=«Description of artificial sequence: Reverse primer, splA* gene encoding Serine protease SplA»

<400> 900
tcaaattcca taaacgttcc 20

<210> 901
<211> 20
<212> DNA
<213> artificial

<220>
<221> source
<222> (1)..(20)
<223> /note=«Description of artificial sequence: Forward primer, splB gene encoding Serine protease SplB»

<400> 901
aacaacattg gttgaggaag 20

<210> 902
<211> 20
<212> DNA
<213> artificial

<220>
<221> source
<222> (1)..(20)
<223> /note=«Description of artificial sequence: Reverse primer, splB gene encoding Serine protease SplB»

<400> 902
atttttgtat gggtgtggat 20

<210> 903
<211> 20
<212> DNA
<213> artificial

<220>
<221> source
<222> (1)..(20)
<223> /note=«Description of artificial sequence: Forward primer, sdrD gene encoding Serine-Aspartate protein»

<400> 903
aagtacacag tgggaacagc 20

<210> 904
<211> 20
<212> DNA
<213> artificial

<220>
<221> source
<222> (1)..(20)
<223> /note=«Description of artificial sequence: Reverse primer, sdrD gene encoding Serine-Aspartate protein»

<400> 904
tcagttttgg catctacctt 20

<210> 905

<211> 20
<212> DNA
<213> artificial

<220>
<221> source
<222> (1)..(20)
<223> /note=«Description of artificial sequence: Forward primer, bbp=sdrE Gene encoding Bone sialoprotein-binding Serine-Aspartate protein»

<400> 905
ttgatttttg gtctagggaa 20

<210> 906
<211> 20
<212> DNA
<213> artificial

<220>
<221> source
<222> (1)..(20)
<223> /note=«Description of artificial sequence: Reverse primer, bbp=sdrE Gene encoding Bone sialoprotein-binding Serine-Aspartate protein»

<400> 906
gtctactttt gaaggcgttg 20

<210> 907
<211> 20
<212> DNA
<213> artificial

<220>
<221> source
<222> (1)..(20)
<223> /note=«Description of artificial sequence: Forward primer, sarH2* gene encoding Staphylococcal accessory regulator A»

<400> 907
ggtggactat caaactttcg 20

<210> 908
<211> 20
<212> DNA
<213> artificial

<220>
<221> source
<222> (1)..(20)
<223> /note=«Description of artificial sequence: Reverse primer, sarH2* gene encoding Staphylococcal accessory regulator A»

<400> 908
ctttgctata ttttggagcc 20

<210> 909
<211> 19
<212> DNA
<213> artificial

<220>
<221> source
<222> (1)..(19)
<223> /note=«Description of artificial sequence: Forward primer, phi int13* gene encoding Integrase phage phi13»

<400> 909
tgaaaacacg tgttacgat 19

<210> 910
<211> 20
<212> DNA
<213> artificial

<220>
<221> source
<222> (1)..(20)
<223> /note=«Description of artificial sequence: Reverse primer, phi int13* gene encoding Integrase phage phi13»

<400> 910
tcatctaaag ctgaccgaat 20

<210> 911
<211> 20
<212> DNA
<213> artificial

<220>
<221> source
<222> (1)..(20)
<223> /note=«Description of artificial sequence: Forward primer, vraF (SA0616) gene encoding ABC protein»

<400> 911
aaaatggcac aagaagtgtt 20

<210> 912
<211> 20
<212> DNA
<213> artificial

<220>
<221> source
<222> (1)..(20)
<223> /note=«Description of artificial sequence: Reverse primer, vraF (SA0616) gene encoding ABC protein»

<400> 912
agcagatgtt cgttgtcttt 20

<210> 913

<211> 20
<212> DNA
<213> artificial

<220>
<221> source
<222> (1)..(20)
<223> /note=«Description of artificial sequence: Forward primer, SA1801 gene encoding Anti repressor protein Q99SP8»

<400> 913
cacgcactca cactagacac 20

<210> 914
<211> 20
<212> DNA
<213> artificial

<220>
<221> source
<222> (1)..(20)
<223> /note=«Description of artificial sequence: Reverse primer, SA1801 gene encoding Anti repressor protein Q99SP8»

<400> 914
ttgtcctttg cctgttactt 20

<210> 915
<211> 20
<212> DNA
<213> artificial

<220>
<221> source
<222> (1)..(20)
<223> /note=«Description of artificial sequence: Forward primer, SA0024 gene encoding ORF CN050 Q9XB68»

<400> 915
agttgtaggg aaggttgcta 20

<210> 916
<211> 20
<212> DNA
<213> artificial

<220>
<221> source
<222> (1)..(20)
<223> /note=«Description of artificial sequence: Reverse primer, SA0024 gene encoding ORF CN050 Q9XB68»

<400> 916
tgctattgac caagctatca 20

<210> 917
<211> 20

<212> DNA
<213> artificial

<220>
<221> source
<222> (1)..(20)
<223> /note=«Description of artificial sequence: Forward primer, MW1769 gene encoding Q8NVW3»

<400> 917
ttttggttgt gtatgcatct 20

<210> 918
<211> 19
<212> DNA
<213> artificial

<220>
<221> source
<222> (1)..(19)
<223> /note=«Description of artificial sequence: Reverse primer, MW1769 gene encoding Q8NVW3»

<400> 918
aagttataga tgtcgggga 19

<210> 919
<211> 20
<212> DNA
<213> artificial

<220>
<221> source
<222> (1)..(20)
<223> /note=«Description of artificial sequence: Forward primer, SA2314* gene encoding Q99RA9»

<400> 919
tcttggtcag atcaatcaca 20

<210> 920
<211> 21
<212> DNA
<213> artificial

<220>
<221> source
<222> (1)..(21)
<223> /note=«Description of artificial sequence: Reverse primer, SA2314* gene encoding Q99RA9»

<400> 920
caaagcactt aaatcggata a 21

<210> 921
<211> 20

```
<212> DNA
<213> artificial

<220>
<221> source
<222> (1)..(20)
<223> /note=«Description of artificial sequence: Forward primer,
      ORF42* gene encoding putative protein»

<400> 921
gattgtccaa gtcccactaa                                                   20


<210> 922
<211> 20
<212> DNA
<213> artificial

<220>
<221> source
<222> (1)..(20)
<223> /note=«Description of artificial sequence: Reverse primer,
      ORF42* gene encoding putative protein»

<400> 922
actagttggt aatgttggcg                                                   20


<210> 923
<211> 20
<212> DNA
<213> artificial

<220>
<221> source
<222> (1)..(20)
<223> /note=«Description of artificial sequence: Forward primer,
      SA1320 gene encoding Q99U01»

<400> 923
aaaccaactg gtgaacaaac                                                   20


<210> 924
<211> 20
<212> DNA
<213> artificial

<220>
<221> source
<222> (1)..(20)
<223> /note=«Description of artificial sequence: Reverse primer,
      SA1320 gene encoding Q99U01»

<400> 924
gtctagcaaa tttctgccaa                                                   20


<210> 925
<211> 20
<212> DNA
```

<213> artificial

<220>
<221> source
<222> (1)..(20)
<223> /note=«Description of artificial sequence: Forward primer, SA1010 gene encoding Q99UU2»

<400> 925
gcaaaaagat aacggtacga 20

<210> 926
<211> 20
<212> DNA
<213> artificial

<220>
<221> source
<222> (1)..(20)
<223> /note=«Description of artificial sequence: Reverse primer, SA1010 gene encoding Q99UU2»

<400> 926
ttgatggcaa ccttttactt 20

<210> 927
<211> 20
<212> DNA
<213> artificial

<220>
<221> source
<222> (1)..(20)
<223> /note=«Description of artificial sequence: Forward primer, SA0704 gene encoding Q99VM6»

<400> 927
tgtgactttt gatgatggaa 20

<210> 928
<211> 20
<212> DNA

<213> artificial

<220>
<221> source
<222> (1)..(20)
<223> /note=«Description of artificial sequence: Reverse primer, SA0704 gene encoding Q99VM6»

<400> 928
tttcaataat gttccaaccc 20

<210> 929
<211> 20
<212> DNA

```
<213> artificial

<220>
<221> source
<222> (1)..(20)
<223> /note=«Description of artificial sequence: Forward primer,
       SA0054 gene encoding Q99XD7»

<400> 929
actcaagaaa acatgggatg


<210> 930
<211> 20
<212> DNA
<213> artificial

<220>
<221> source
<222> (1)..(20)
<223> /note=«Description of artificial sequence: Reverse primer,
      SA0054 gene encoding Q99XD7»

<400> 930
gctttcaaag tccattttg
```